# EUROPEAN PATENT APPLICATION

(11) **EP 4 349 829 A1**
(43) Date of publication of application: **10.04.2024**
(21) Application number: 22810643.1
(22) Date of filing: 27.05.2022
(51) Int. Cl.: C07D 403/02, C07D 403/14, C07D 401/02, C07D 401/14, C07D 405/02, C07D 405/14, C07D 413/02, C07D 413/14, C07D 487/02, A61K 31/435, A61K 31/41, A61P 35/00

(54) **COMPOUND USED AS BCR-ABL INHIBITOR**

(30) Priority: 28.05.2021 CN 202110592542; 17.09.2021 CN 202111094508; 31.12.2021 CN 202111661984
(71) Applicant: CHIA TAI TIANQING PHARMACEUTICAL GROUP CO., LTD., Lianyungang, Jiangsu 222062 (CN)
(72) Inventor: ZHANG, Yinsheng, Lianyungang, Jiangsu 222062 (CN); LIU, Xin, Lianyungang, Jiangsu 222062 (CN); QIN, Hui, Lianyungang, Jiangsu 222062 (CN); YE, Jiawei, Lianyungang, Jiangsu 222062 (CN); WANG, Jinan, Lianyungang, Jiangsu 222062 (CN); WU, Songsong, Lianyungang, Jiangsu 222062 (CN)
(74) Representative: WSL Patentanwälte Partnerschaft mbB
(86) International application number: PCT/CN2022/095436
(87) International publication number: WO 2022/247919

(57) **Abstract**

Provided are a compound used as a BCR-ABL inhibitor, namely a compound of formula (I), or a pharmaceutically acceptable salt thereof, a preparation method therefor, and a pharmaceutical composition comprising the compound. Moreover, the present invention relates to a use of the compound in the preparation of a drug for treating BCR-ABL related diseases.

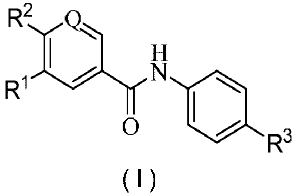

## Description

### CROSS-REFERENCE TO RELATED APPLICATIONS

The present application claims the benefit and priority to the Chinese Patent Application Nos. 202110592542.2, 202111094508.9 and 202111661984.4 filed with National Intellectual Property Administration, PRC on May 28, 2021, September 17, 2021, and December 31, 2021, which are incorporated herein by reference in their entireties.

### TECHNICAL FIELD

The present application belongs to the field of pharmaceutical chemistry, provides a compound used as a BCR-ABL inhibitor, a preparation method therefor, and a pharmaceutical composition comprising the same, and relates to use of the compound in the preparation of a medicament for treating BCR-ABL related diseases.

### BACKGROUND

Chronic myeloid leukemia (CML) is the main disease species of chronic leukemia in China, and accounts for about 70% of chronic leukemia. 90% or more cases of this disease have chromosomal abnormalities, and mainly the long arm of chromosome 9 and chromosome 22 is translocated to form Bcr-Abl fusion gene that expresses protein p-210. The tyrosine kinase activity of p-210 is much stronger than that of p-150 (normal c-Abl gene expression product), which causes abnormal proliferation and differentiation of hematopoietic stem cells, and finally triggers CML.

Imatinib, the first marketed Bcr-Abl-targeting therapeutic agent, is currently used as a first-line therapeutic agent for the treatment of various stages of CML. Nilotinib, dasatinib and bosutinib (second-generation Bcr-Abl inhibitors) are approved for use in the treatment of CML that is resistant or intolerant to imatinib. The second-generation Bcr-Abl inhibitors all have the drug effect superior to imatinib and are effective against almost all imatinib-resistant mutation types, but the problem of T315I (gatekeeper)-resistant mutation remains unsolved. About 20% of patients with drug resistance against the first- and second-generation Bcr-Abl inhibitors have the T315I mutation, and the marketing of ponatinib (third-generation Bcr-Abl inhibitor) relieves the predicament that patients with the T315I-resistant mutation are not medically available, and becomes the only choice for the patients whose treatment fails by using the first- and second-generation Bcr-Abl inhibitors.

ABL001 is a Bcr-ABL allosteric inhibitor developed by Novartis, which is disclosed in WO2013171639, currently in phase III clinical research. ABL001 is a potent and selective BCR-ABL inhibitor, active against a number of mutants, such as T315I (Andrew A. Wylie et al. (2017) Nature 543, 733-737).

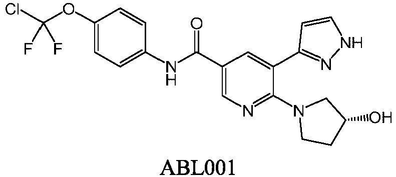

At present, no BCR-ABL allosteric inhibitor is on the market, so a high-potency and low-toxicity Bcr-Abl inhibitor effective against T315I is urgently needed clinically, and a new BCR-ABL allosteric inhibitor needs to be further developed.

### SUMMARY

In one aspect, the present application provides a compound of formula (I) or a pharmaceutically acceptable salt thereof, 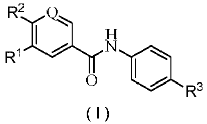 wherein,
Q is selected from the group consisting of N and CH;
R¹ is selected from the group consisting of 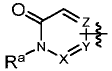 and 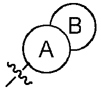 , wherein the 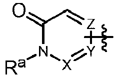 or 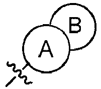 is optionally substituted with one or more R^{a'};
X, Y and Z are each independently selected from the group consisting of CH and N, and at least one of X, Y and Z is selected from CH;
the ring A is selected from 5-membered heteroaryl containing 1-3 heteroatoms selected from the group consisting of N, O and S atoms;
the ring B is selected from the group consisting of 5- to 10-membered heterocyclyl containing 1-3 heteroatoms selected from the group consisting of N, O and S atoms, and 5- to 8-membered heteroaryl containing 1-3 heteroatoms selected from the group consisting of N, O and S atoms;
R² is selected from the group consisting of hydrogen, amino, C₁₋₆ alkyl, C₁₋₆ alkoxy, amino-C₁₋₆ alkyl-, 3- to 10-membered heterocyclyl, and 5- to 6-membered heteroaryl, wherein the amino, C₁₋₆ alkyl, C₁₋₆ alkoxy, amino-C₁₋₆ alkyl-, 3- to 10-membered heterocyclyl, or 5- to 6-membered heteroaryl is optionally substituted with one or more R^{b};
R³ is selected from -OCF₂H, wherein the -OCF₂H is optionally substituted with halogen;
R^{a} and R^{a'} are each independently selected from the group consisting of hydroxy, amino, cyano, halogen, 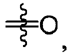 C₁₋₆ alkyl, 3- to 8-membered cycloalkyl, 3- to 8-membered heterocycloalkyl, 3- to 8-membered cycloalkyl-C₁₋₆ alkyl-, 3- to 8-membered heterocycloalkyl-C₁₋₆ alkyl-, and C₁₋₆ alkyl substituted with one or more hydroxy or halogen;
R^{b} is selected from the group consisting of hydroxy, amino, cyano, halogen, 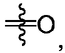 C₁₋₆ alkyl, C₁₋₆ alkoxy, C₁₋₆ alkoxy-C₁₋₆ alkyl-, C₁₋₆ alkyl-C(O)-NH-, and C₁₋₆ alkyl substituted with one or more hydroxy or halogen.

In some embodiments, the compound of formula (I) or a pharmaceutically acceptable salt thereof is selected from the group consisting of a compound of formula (II) and a pharmaceutically acceptable salt thereof, 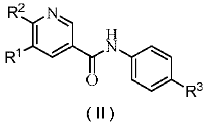 wherein,
R¹ is selected from the group consisting of 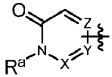 and 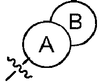 , wherein the 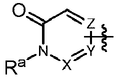 or 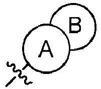 is optionally substituted with one or more R^{a'};
X, Y and Z are each independently selected from the group consisting of CH and N, and at least one of X, Y and Z is selected from CH;
the ring A is selected from 5-membered heteroaryl containing 1-3 heteroatoms selected from the group consisting of N, O and S atoms;
the ring B is selected from the group consisting of 5- to 10-membered heterocyclyl containing 1-3 heteroatoms selected from the group consisting of N, O and S atoms, and 5- to 8-membered heteroaryl containing 1-3 heteroatoms selected from the group consisting of N, O and S atoms;
R² is selected from the group consisting of hydrogen, amino, C₁₋₆ alkyl, C₁₋₆ alkoxy, amino-C₁₋₆ alkyl-, 3- to 10-membered heterocyclyl, and 5- to 6-membered heteroaryl, wherein the amino, C₁₋₆ alkyl, C₁₋₆ alkoxy, amino-C₁₋₆ alkyl-, 3- to 10-membered heterocyclyl, or 5- to 6-membered heteroaryl is optionally substituted with one or more R^{b};
R³ is selected from -OCF₂H, wherein the -OCF₂H is optionally substituted with halogen;
R^{a} and R^{a'} are each independently selected from the group consisting of hydroxy, amino, cyano, halogen, 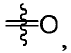 C₁₋₆ alkyl, 3- to 8-membered cycloalkyl, 3- to 8-membered heterocycloalkyl, 3- to 8-membered cycloalkyl-C₁₋₆ alkyl-, 3- to 8-membered heterocycloalkyl-C₁₋₆ alkyl-, and C₁₋₆ alkyl substituted with one or more hydroxy or halogen;
R^{b} is selected from the group consisting of hydroxy, amino, cyano, halogen, 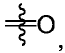 C₁₋₆ alkyl, C₁₋₆ alkoxy, C₁₋₆ alkoxy-C₁₋₆ alkyl-, C₁₋₆ alkyl-C(O)-NH-, and C₁₋₆ alkyl substituted with one or more hydroxy or halogen. In some embodiments,
R¹ is selected from the group consisting of 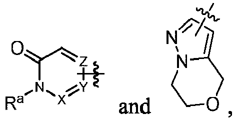 wherein the 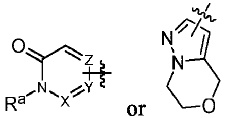 is optionally substituted with one or more R^{a'};
X, Y and Z are each independently selected from the group consisting of CH and N, and at least one of X, Y and Z is selected from CH;
R² is selected from the group consisting of hydrogen and 3- to 10-membered heterocyclyl, wherein the 3- to 10-membered heterocyclyl is optionally substituted with one or more R^{b};
R³ is selected from -OCF₂H, wherein the -OCF₂H is optionally substituted with halogen;
R^{a} and R^{a'} are each independently selected from the group consisting of hydroxy, amino, cyano, halogen, C₁₋₆ alkyl, 3- to 8-membered cycloalkyl, 3- to 8-membered heterocycloalkyl, and C₁₋₆ alkyl substituted with one or more hydroxy or halogen;
R^{b} is selected from the group consisting of hydroxy, amino, cyano, halogen, 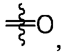 C₁₋₆ alkyl, C₁₋₆ alkoxy, and C₁₋₆ alkyl substituted with one or more hydroxy or halogen.

In some embodiments, the heterocyclyl or heterocycloalkyl contains 1, 2, 3, or 4 heteroatoms or heteroatom groups independently selected from the group consisting of -O-, -NH-, -S-, and N; the other variables are as defined herein. In some embodiments, the heterocyclyl or heterocycloalkyl contains 1, 2, 3, or 4 heteroatoms or heteroatom groups independently selected from the group consisting of -O-, -NH-, and N; the other variables are as defined herein. In some embodiments, the heterocyclyl or heterocycloalkyl contains 1 or 2 heteroatoms or heteroatom groups independently selected from the group consisting of -O-, -NH-, and N; the other variables are as defined herein.

In some embodiments, the heterocyclyl or heterocycloalkyl referred to in the R², R^{a} or R^{a'} contains 1, 2, 3 or 4 heteroatoms or heteroatom groups independently selected from the group consisting of -O-, -NH-, -S-, and N; the other variables are as defined herein. In some embodiments, the heterocyclyl or heterocycloalkyl referred to in the R², R^{a} or R^{a'} contains 1, 2, 3 or 4 heteroatoms or heteroatom groups independently selected from the group consisting of -O-, -NH-, and N; the other variables are as defined herein. In some embodiments, the heterocyclyl or heterocycloalkyl referred to in the R², R^{a} and R^{a'} contains 1 or 2 heteroatoms or heteroatom groups independently selected from the group consisting of -O-, -NH-, and N; the other variables are as defined herein. In some embodiments, Q is selected from N, and the other variables are as defined herein. In some embodiments, Q is selected from CH and the other variables are as defined herein.

In some embodiments, Q is selected from CH;
R¹ is selected from the group consisting of 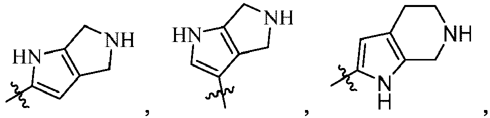 and 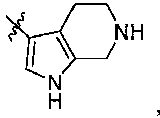 wherein the 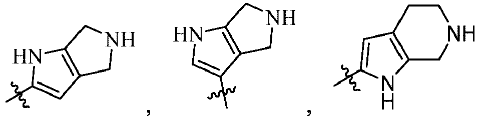 or 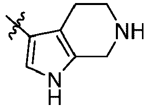 is optionally substituted with one or more R^{a'}; the R^{a'} is selected from the group consisting of halogen, 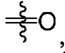 C₁₋₆ alkyl, and 3- to 6-membered cycloalkyl; or, R^{a'} is selected from the group consisting of fluoro, chloro, 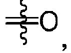 methyl, ethyl, isopropyl, and cyclopropyl;
R² is selected from the group consisting of 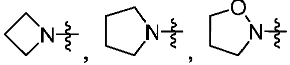 , and 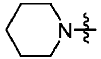 , wherein the 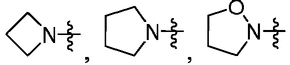 or 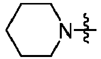 is optionally substituted with 1, 2 or 3 R^{b}; the R^{b} is selected from the group consisting of hydroxy and C₁₋₄ alkyl;
R³ is selected from -OCF₂H, wherein the -OCF₂H is optionally substituted with halogen.

In some embodiments, Q is selected from CH;
R¹ is selected from the group consisting of 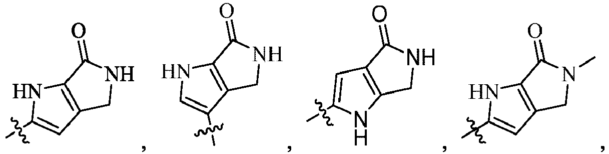 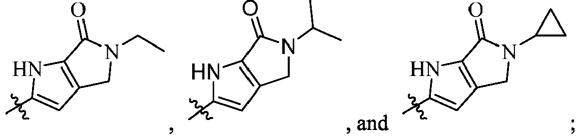
R² is selected from the group consisting of 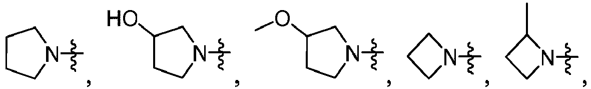 and 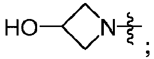
R³ is selected from -OCF₂H, wherein the -OCF₂H is optionally substituted with chlorine.

In some embodiments, X, Y and Z are all selected from CH; the other variables are as defined herein. In some embodiments, one of X, Y and Z is selected from N, and the others are selected from CH; the other variables are as defined herein. In some embodiments, X is selected from N, and Y and Z are selected from CH; the other variables are as defined herein. In some embodiments, Y is selected from N, and X and Z are selected from CH; the other variables are as defined herein. In some embodiments, Z is selected from N, and X and Y are selected from CH; the other variables are as defined herein. In some embodiments, one of X, Y and Z is selected from CH, and the others are selected from N; the other variables are as defined herein.

In some embodiments, the ring A is selected from 5-membered heteroaryl containing 1 or 2 heteroatoms selected from the group consisting of N, O and S atoms; the other variables are as defined herein.

In some embodiments, the ring A is selected from 5-membered heteroaryl containing 1 or 2 heteroatoms selected from the group consisting of N and O atoms; the other variables are as defined herein.

In some embodiments, the ring A is selected from the group consisting of pyrrolyl, pyrazolyl, imidazolyl, furanyl, thienyl, thiazolyl, oxazolyl, and isoxazolyl; the other variables are as defined herein. In some embodiments, the ring A is selected from the group consisting of pyrrolyl, pyrazolyl, imidazolyl, and furanyl; the other variables are as defined herein.

In some embodiments, the ring B is selected from the group consisting of 5- to 8-membered heterocycloalkyl containing 1-3 heteroatoms selected from the group consisting of N and O atoms, and 5- to 8-membered heteroaryl containing 1-3 heteroatoms selected from the group consisting of N and O atoms; the other variables are as defined herein.

In some embodiments, the ring B is selected from the group consisting of 5- or 6-membered heterocycloalkyl containing 1 or 2 heteroatoms selected from the group consisting of N and O atoms, and 5- or 6-membered heteroaryl containing 1 or 2 heteroatoms selected from the group consisting of N and O atoms; the other variables are as defined herein.

In some embodiments, the ring B is selected from the group consisting of 5- or 6-membered heterocycloalkyl containing 1 or 2 heteroatoms selected from the group consisting of N and O atoms, and 5-membered heteroaryl containing 1 or 2 N atoms; the other variables are as defined herein.

In some embodiments, the ring B is selected from the group consisting of tetrahydropyrrolyl, piperidinyl, piperazinyl, morpholinyl, imidazolyl, and pyrazolyl; the other variables are as defined herein. In some embodiments, the ring B is selected from the group consisting of tetrahydropyrrolyl, piperidinyl, morpholinyl, and imidazolyl; the other variables are as defined herein.

In some embodiments, R¹ is selected from 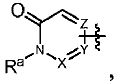 wherein the 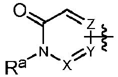 is optionally substituted with one or more R^{a'}; the other variables are as defined herein.

In some embodiments, R¹ is selected from the group consisting of 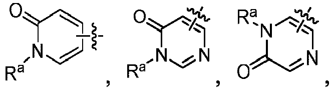 and 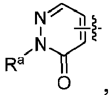 wherein the 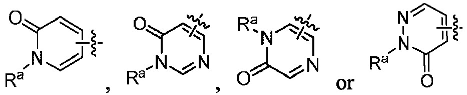 is optionally substituted with one or more R^{a'}; the other variables are as defined herein.

In some embodiments, R¹ is selected from the group consisting of 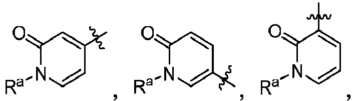 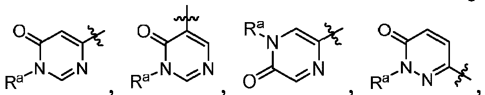 and 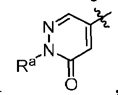 , wherein the 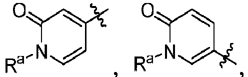 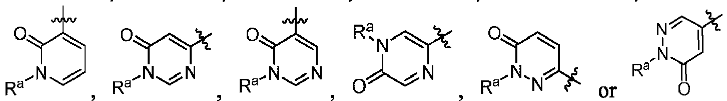 is optionally substituted with one or more R^{a'}; the other variables are as defined herein.

In some embodiments, R¹ is selected from 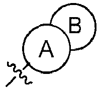 , wherein the 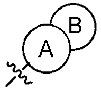 is optionally substituted with one or more R^{a'}; the other variables are as defined herein.

In some embodiments. R¹ is selected from the group consisting of 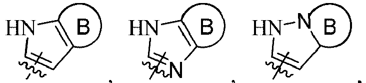 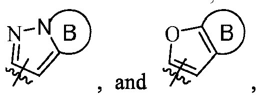 wherein the 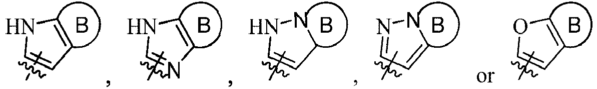 is optionally substituted with one or more R^{a'}; the other variables are as defined herein.

In some embodiments, R¹ is selected from the group consisting of 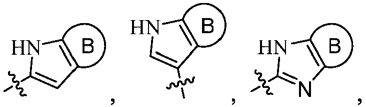 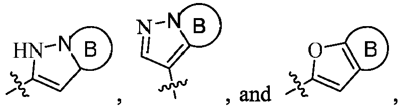 wherein the 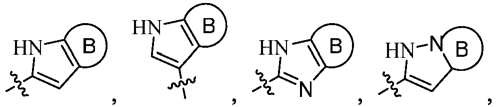 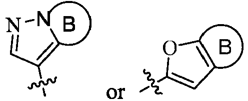 is optionally substituted with one or more R^{a'}; the other variables are as defined herein.

In some embodiments, R¹ is selected from the group consisting of 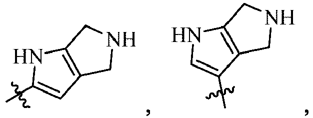 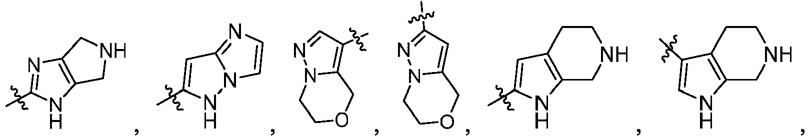 and 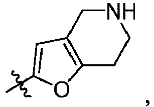 wherein the 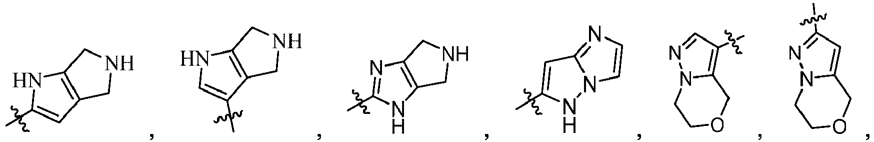 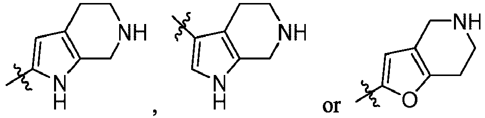 is optionally substituted with one or more R^{a'}; the other variables are as defined herein.

In some embodiments, R¹ is selected from 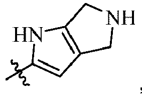 , wherein the 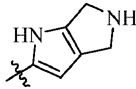 is optionally substituted with one or more R^{a'}; the other variables are as defined herein.

In some embodiments, R¹ is selected from the group consisting of 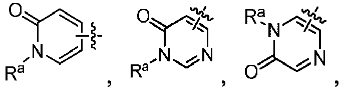 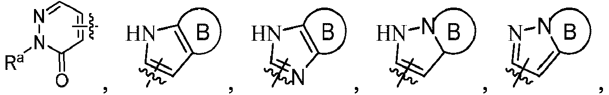 and 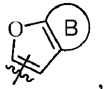 wherein the 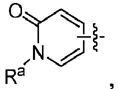 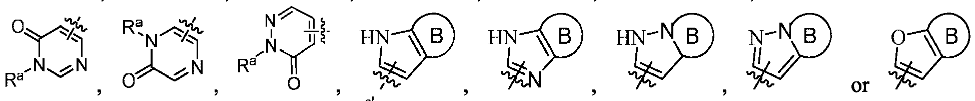 is optionally substituted with one or more R^{a'}; the other variables are as defined herein.

In some embodiments, R¹ is selected from the group consisting of 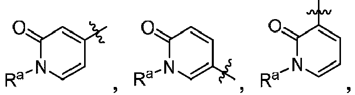 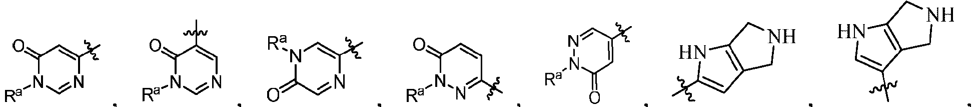 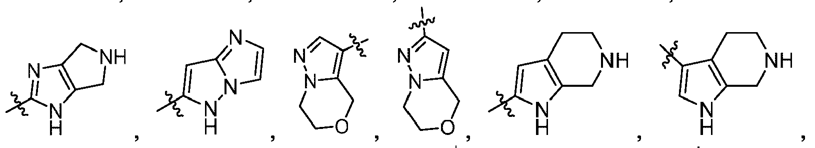 and 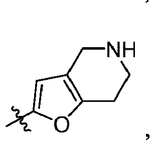 wherein the 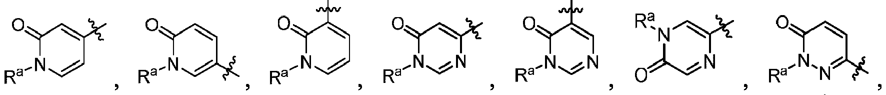 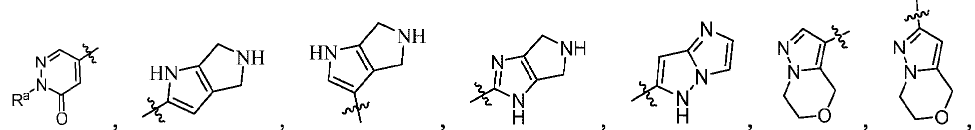 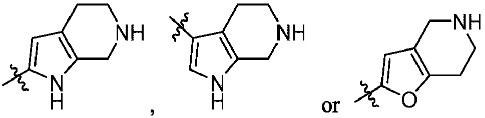 is optionally substituted with one or more R^{a'}; the other variables are as defined herein.

In some embodiments, R¹ is selected from the group consisting of 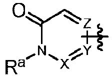 and 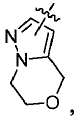 wherein the 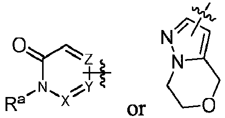 is optionally substituted with one or more R^{a'}; the other variables are as defined herein.

In some embodiments, R¹ is selected from the group consisting of 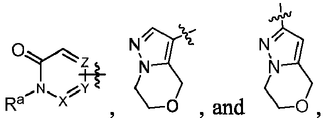 wherein the 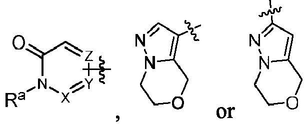 is optionally substituted with one or more R^{a'}; the other variables are as defined herein.

In some embodiments, R¹ is selected from the group consisting of 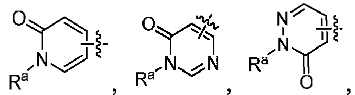 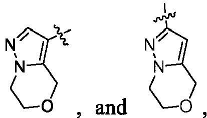 wherein the 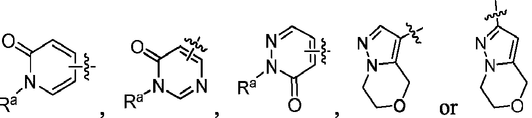 is optionally substituted with one or more R^{a'}; the other variables are as defined herein.

In some embodiments, R¹ is selected from the group consisting of 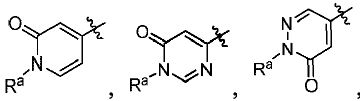 , and 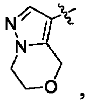 wherein the 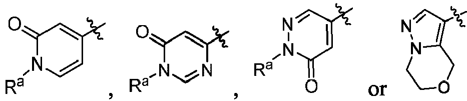 is optionally substituted with one R^{a'}; the other variables are as defined herein.

In some embodiments, R¹ is selected from the group consisting of 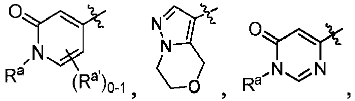 and 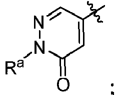 the other variables are as defined herein.

In some embodiments, R^{a} and R^{a'} are each independently selected from the group consisting of halogen, 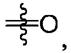 C₁₋₆ alkyl, 3- to 6-membered cycloalkyl, 3- to 8-membered heterocycloalkyl-C₁₋₆ alkyl-, and C₁₋₆ alkyl substituted with 1, 2 or 3 hydroxy; the other variables are as defined herein.

In some embodiments, R^{a} and R^{a'} are each independently selected from the group consisting of halogen, 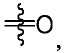 C₁₋₄ alkyl, 3- to 6-membered cycloalkyl, 3- to 6-membered heterocycloalkyl-C₁₋₄ alkyl-, and C₁₋₄ alkyl substituted with 1, 2 or 3 hydroxy; the other variables are as defined herein.

In some embodiments, R^{a} and R^{a'} are each independently selected from the group consisting of hydroxy, amino, cyano, halogen, C₁₋₆ alkyl, 3- to 8-membered cycloalkyl, 3- to 8-membered heterocycloalkyl, and C₁₋₆ alkyl substituted with one or more hydroxy or halogen; the other variables are as defined herein.

In some embodiments, R^{a} and R^{a'} are each independently selected from the group consisting of halogen, C₁₋₆ alkyl, 3- to 6-membered cycloalkyl, and C₁₋₆ alkyl substituted with 1, 2 or 3 hydroxy; the other variables are as defined herein.

In some embodiments, R^{a} and R^{a'} are each independently selected from the group consisting of hydroxy, amino, cyano, halogen, C₁₋₄ alkyl, 3- to 6-membered cycloalkyl, 3- to 6-membered heterocycloalkyl, and C₁₋₄ alkyl substituted with one or more hydroxy or halogen; the other variables are as defined herein.

In some embodiments, R^{a} and R^{a'} are each independently selected from the group consisting of halogen, C₁₋₄ alkyl, 3- to 6-membered cycloalkyl, and C₁₋₄ alkyl substituted with 1, 2 or 3 hydroxy; the other variables are as defined herein.

In some embodiments, R^{a} is selected from the group consisting of C₁₋₄ alkyl, 3- to 6-membered cycloalkyl, 3- to 6-membered heterocycloalkyl-C₁₋₄ alkyl-, and C₁₋₄ alkyl substituted with 1, 2 or 3 hydroxy; the other variables are as defined herein.

In some embodiments, R^{a} is selected from the group consisting of methyl, ethyl, cyclopropyl, 2-hydroxyethyl, and 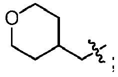 the other variables are as defined herein.

In some embodiments, R^{a} is selected from the group consisting of C₁₋₄ alkyl, 3- to 6-membered cycloalkyl, and C₁₋₄ alkyl substituted with 1, 2 or 3 hydroxy; the other variables are as defined herein.

In some embodiments, R^{a} is selected from the group consisting of methyl, ethyl, cyclopropyl, and 2-hydroxyethyl; the other variables are as defined herein.

In some embodiments, R^{a} is selected from methyl; the other variables are as defined herein.

In some embodiments, R^{a'} is selected from the group consisting of hydroxy, amino, cyano, halogen, 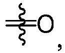 C₁₋₄ alkyl, and 3- to 6-membered cycloalkyl; the other variables are as defined herein.

In some embodiments, R^{a'} is selected from the group consisting of halogen, 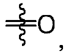 C₁₋₄ alkyl, and 3- to 6-membered cycloalkyl; the other variables are as defined herein.

In some embodiments, R^{a'} is selected from the group consisting of fluoro, chloro, bromo, iodo, 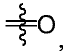 methyl, ethyl, isopropyl, and cyclopropyl; the other variables are as defined herein.

In some embodiments, R^{a'} is selected from the group consisting of fluoro, chloro, 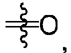 methyl, ethyl, and cyclopropyl; the other variables are as defined herein.

In some embodiments, R^{a'} is selected from the group consisting of hydroxy, amino, cyano, and halogen; the other variables are as defined herein.

In some embodiments, R^{a'} is selected from halogen; the other variables are as defined herein.

In some embodiments, R^{a'} is selected from the group consisting of fluoro and chloro; the other variables are as defined herein.

In some embodiments, R¹ is selected from the group consisting of 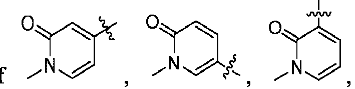 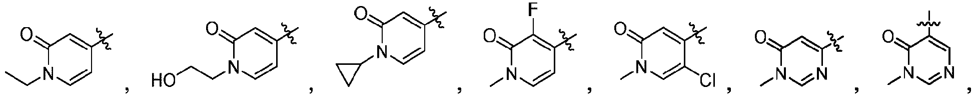 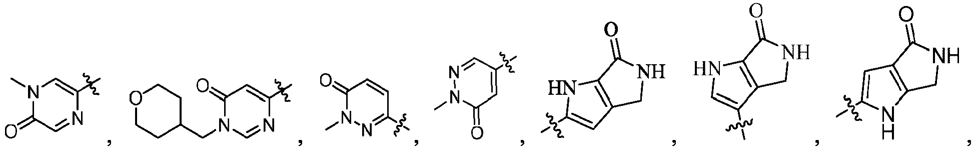 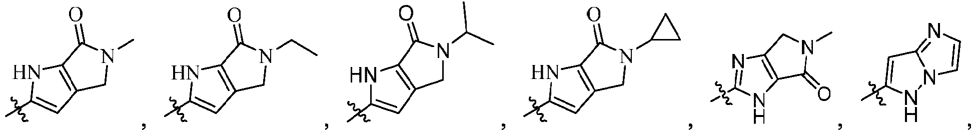 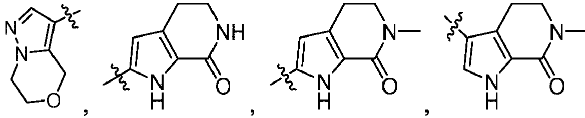 , and 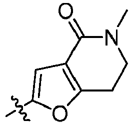 ; the other variables are as defined herein.

In some embodiments, R¹ is selected from the group consisting of 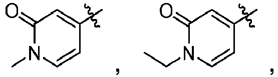 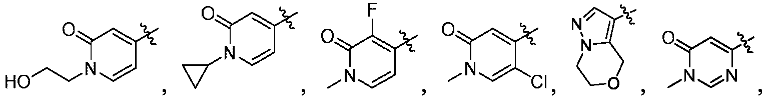 , and 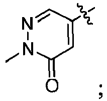 the other variables are as defined herein.

In some embodiments, R² is selected from the group consisting of hydrogen, amino, C₁₋₄ alkoxy, amino-C₁₋₄ alkyl-, 3- to 10-membered heterocycloalkyl, and 5- to 6-membered heteroaryl, wherein the amino, C₁₋₄ alkoxy, amino-C₁₋₄ alkyl-, 3- to 0-membered heterocycloalkyl or 5- to 6-membered heteroaryl is optionally substituted with one or more R^{b}; the other variables are as defined herein.

In some embodiments, R² is selected from the group consisting of hydrogen, amino, C₁₋₄ alkoxy, amino-C₁₋₄ alkyl-, 4- to 6-membered monoheterocycloalkyl, 6- to 9-membered bridged heterocycloalkyl, 7- to 9-membered spiro-heterocycloalkyl, and 5- to 6-membered heteroaryl, wherein the amino, C₁₋₄ alkoxy, amino-C₁₋₄ alkyl-, 4- to 6-membered monoheterocycloalkyl, 6- to 9-membered bridged heterocycloalkyl, 7- to 9-membered spiro-heterocycloalkyl, or 5- to 6-membered heteroaryl is optionally substituted with one or more R^{b}; the other variables are as defined herein.

In some embodiments, R² is selected from the group consisting of amino, C₁₋₃ alkoxy, amino-C₁₋₃ alkyl-, 4-, 5-, or 6-membered monoheterocycloalkyl, 6-, 7-, or 8-membered bridged heterocycloalkyl, 7-, 8-, or 9-membered spiro-heterocycloalkyl, and 5-membered heteroaryl, wherein the amino, C₁₋₃ alkoxy, amino-C₁₋₃ alkyl-, 4-, 5-, or 6-membered monoheterocycloalkyl, 6-, 7-, or 8-membered bridged heterocycloalkyl, 7-, 8-, or 9-membered spiro-heterocycloalkyl, or 5-membered heteroaryl is optionally substituted with one or more R^{b} (e.g., 1, 2, or 3); the other variables are as defined herein.

In some embodiments, R² is selected from the group consisting of hydrogen and 3- to 10-membered heterocyclyl, wherein the 3- to 10-membered heterocyclyl is optionally substituted with one or more R^{b}; the other variables are as defined herein.

In some embodiments, R² is selected from the group consisting of hydrogen and 3- to 10-membered heterocycloalkyl, wherein the 3- to 10-membered heterocycloalkyl is optionally substituted with one or more R^{b}; the other variables are as defined herein.

In some embodiments, R² is selected from the group consisting of amino and 3- to 10-membered heterocycloalkyl, wherein the amino or 3- to 10-membered heterocycloalkyl is optionally substituted with one or more R^{b}; the other variables are as defined herein.

In some embodiments, the heterocycloalkyl contains 1, 2, 3, or 4 heteroatoms or heteroatom groups independently selected from the group consisting of -O-, -NH-, -S-, and N; the other variables are as defined herein. In some embodiments, the heterocycloalkyl contains 1, 2, 3, or 4 heteroatoms or heteroatom groups independently selected from the group consisting of -O-, -NH-, and N; the other variables are as defined herein. In some embodiments, the heterocycloalkyl contains 1 or 2 heteroatoms or heteroatom groups independently selected from the group consisting of -O-, -NH-, and N; the other variables are as defined herein.

In some embodiments, the heterocycloalkyl referred to in the R², R^{a} or R^{a'} contains 1, 2, 3 or 4 heteroatoms or heteroatom groups independently selected from the group consisting of -O-, -NH-, -S-, and N; the other variables are as defined herein. In some embodiments, the heterocycloalkyl referred to in the R², R^{a} or R^{a'} contains 1, 2, 3 or 4 heteroatoms or heteroatom groups independently selected from the group consisting of -O-, -NH-, and N; the other variables are as defined herein. In some embodiments, the heterocycloalkyl referred to in the R², R^{a} and R^{a'} contains 1 or 2 heteroatoms or heteroatom groups independently selected from the group consisting of -O-, -NH-, and N; the other variables are as defined herein. In some embodiments, R² is selected from the group consisting of hydrogen, 4- to 6-membered monoheterocyclyl, 6- to 9-membered bridged heterocyclyl, and 7- to 9-membered spiro-heterocyclyl, wherein the 4- to 6-membered heteromonocyclyl, 6- to 9-membered bridged heterocyclyl, or 7- to 9-membered spiro-heterocyclyl is optionally substituted with one or more R^{b}; the other variables are as defined herein.

In some embodiments, R² is selected from the group consisting of hydrogen, 4- to 6-membered monoheterocycloalkyl, 6- to 9-membered bridged heterocycloalkyl, and 7- to 9-membered spiro-heterocycloalkyl, wherein the 4- to 6-membered monoheterocycloalkyl, 6- to 9-membered bridged heterocycloalkyl, or 7- to 9-membered spiro-heterocycloalkyl is optionally substituted with one or more R^{b}; the other variables are as defined herein.

In some embodiments, R² is not hydrogen; the other variables are as defined herein.

In some embodiments, R² is selected from the group consisting of 4-, 5-, or 6-membered monoheterocycloalkyl, 6-, 7-, or 8-membered bridged heterocycloalkyl, and 7-, 8-, or 9-membered spiro-heterocycloalkyl, wherein the 4-, 5-, or 6-membered monoheterocycloalkyl, 6-, 7-, or 8-membered bridged heterocycloalkyl, or 7-, 8-, or 9-membered spiro-heterocycloalkyl is optionally substituted with one or more R^{b} (e.g., 1, 2, or 3); the other variables are as defined herein.

In some embodiments, R² is selected from the group consisting of amino, methoxy, ethoxy, aminomethyl, pyrrolidinyl, oxazolidinyl, isoxazolidinyl, morpholinyl, thiomorpholinyl, 1,4-dioxanyl, azetidinyl, piperazinyl, piperidinyl, 1,2,3,6-tetrahydropyridinyl, tetrahydropyranyl, 3,4-dihydropyranyl, 3,6-dihydropyranyl, 6-oxa-3-azabicyclo[3.1.1]heptanyl, 3-oxa-6-azabicyclo[3.1.1]heptanyl, 3-oxa-8-azabicyclo[3.2.1]octanyl, 8-oxa-3-azabicyclo[3.2.1]octanyl, 2-oxa-5-azabicyclo[2.2.1]heptanyl, 3-azabicyclo[3.1.0]hexanyl, 2-oxa-6-azaspiro[3.4]octanyl, 2-oxa-7-azaspiro[3.5]nonanyl, 2-oxa-6-azaspiro[3.3]heptanyl, pyrazolyl, and imidazolyl, wherein the amino, methoxy, ethoxy, aminomethyl, pyrrolidinyl, oxazolidinyl, isoxazolidinyl, morpholinyl, thiomorpholinyl, 1,4-dioxanyl, azetidinyl, piperazinyl, piperidinyl, 1,2,3,6-tetrahydropyridinyl, tetrahydropyranyl, 3,4-dihydropyranyl, 3,6-dihydropyranyl, 6-oxa-3-azabicyclo[3.1.1]heptanyl, 3-oxa-6-azabicyclo[3.1.1]heptanyl, 3-oxa-8-azabicyclo[3.2.1]octanyl, 8-oxa-3-azabicyclo[3.2.1]octanyl, 2-oxa-5-azabicyclo[2.2.1]heptanyl, 3-azabicyclo[3.1.0]hexanyl, 2-oxa-6-azaspiro[3.4]octanyl, 2-oxa-7-azaspiro[3.5]nonanyl, 2-oxa-6-azaspiro[3.3]heptanyl, pyrazolyl or imidazolyl is optionally substituted with one or more R^{b}; the other variables are as defined herein.

In some embodiments, R² is selected from the group consisting of amino, methoxy, ethoxy, aminomethyl, pyrrolidinyl, isoxazolidinyl, piperidinyl, morpholinyl, thiomorpholinyl, 1,4-dioxanyl, azetidinyl, 6-oxa-3-azabicyclo[3.1.1]heptanyl, 3-oxa-6-azabicyclo[3.1.1]heptanyl, 3-oxa-8-azabicyclo[3.2.1]octanyl, 8-oxa-3-azabicyclo[3.2.1]octanyl, 2-oxa-5-azabicyclo[2.2.1]heptanyl, 3-azabicyclo[3.1.0]hexanyl, 2-oxa-6-azaspiro[3.4]octanyl, 2-oxa-7-azaspiro[3.5]nonanyl, 2-oxa-6-azaspiro[3.3]heptanyl, pyrazolyl, and imidazolyl, wherein the amino, methoxy, ethoxy, aminomethyl, pyrrolidinyl, isoxazolidinyl, piperidinyl, morpholinyl, thiomorpholinyl, 1,4-dioxanyl, azetidinyl, 6-oxa-3-azabicyclo[3.1.1]heptanyl, 3-oxa-6-azabicyclo[3.1.1]heptanyl, 3-oxa-8-azabicyclo[3.2.1]octanyl, 8-oxa-3-azabicyclo[3.2.1]octanyl, 2-oxa-5-azabicyclo[2.2.1]heptanyl, 3-azabicyclo[3.1.0]hexanyl, 2-oxa-6-azaspiro[3.4]octanyl, 2-oxa-7-azaspiro[3.5]nonanyl, 2-oxa-6-azaspiro[3.3]heptanyl, pyrazolyl or imidazolyl is optionally substituted with one or more R^{b}; the other variables are as defined herein.

In some embodiments, R² is selected from the group consisting of pyrrolidinyl, isoxazolidinyl, morpholinyl, azetidinyl, piperazinyl, piperidinyl, 1,2,3,6-tetrahydropyridinyl, tetrahydropyranyl, 3,4-dihydropyranyl, 3,6-dihydropyranyl, 6-oxa-3-azabicyclo[3.1.1]heptanyl, 3-oxa-6-azabicyclo[3.1.1]heptanyl, 3-oxa-8-azabicyclo[3.2.1]octanyl, 8-oxa-3-azabicyclo[3.2.1]octanyl, 2-oxa-5-azabicyclo[2.2.1]heptanyl, 3-azabicyclo[3.1.0]hexanyl, 2-oxa-6-azaspiro[3.4]octanyl, 2-oxa-7-azaspiro[3.5]nonanyl, and 2-oxa-6-azaspiro[3.3]heptanyl, wherein the pyrrolidinyl, isoxazolidinyl, morpholinyl, azetidinyl, piperazinyl, piperidinyl, 1,2,3,6-tetrahydropyridinyl, tetrahydropyranyl, 3,4-dihydropyranyl, 3,6-dihydropyranyl, 6-oxa-3-azabicyclo[3.1.1]heptanyl, 3-oxa-6-azabicyclo[3.1.1]heptanyl, 3-oxa-8-azabicyclo[3.2.1]octanyl, 8-oxa-3-azabicyclo[3.2.1]octanyl, 2-oxa-5-azabicyclo[2.2.1]heptanyl, 3-azabicyclo[3.1.0]hexanyl, 2-oxa-6-azaspiro[3.4]octanyl, 2-oxa-7-azaspiro[3.5]nonanyl, or 2-oxa-6-azaspiro[3.3]heptanyl is optionally substituted with one or more R^{b}; the other variables are as defined herein.

In some embodiments, R² is selected from the group consisting of pyrrolidinyl, isoxazolidinyl, morpholinyl, azetidinyl, 6-oxa-3-azabicyclo[3.1.1]heptanyl, 3-oxa-6-azabicyclo[3.1.1]heptanyl, 3-oxa-8-azabicyclo[3.2.1]octanyl, 8-oxa-3-azabicyclo[3.2.1]octanyl, 2-oxa-5-azabicyclo[2.2.1]heptanyl, 3-azabicyclo[3.1.0]hexanyl, 2-oxa-6-azaspiro[3.4]octanyl, 2-oxa-7-azaspiro[3.5]nonanyl, and 2-oxa-6-azaspiro[3.3]heptanyl, wherein the pyrrolidinyl, isoxazolidinyl, morpholinyl, azetidinyl, 6-oxa-3-azabicyclo[3.1.1]heptanyl, 3-oxa-6-azabicyclo[3.1.1]heptanyl, 3-oxa-8-azabicyclo[3.2.1]octanyl, 8-oxa-3-azabicyclo[3.2.1]octanyl, 2-oxa-5-azabicyclo[2.2.1]heptanyl, 3-azabicyclo[3.1.0]hexanyl, 2-oxa-6-azaspiro[3.4]octanyl, 2-oxa-7-azaspiro[3.5]nonanyl, or 2-oxa-6-azaspiro[3.3]heptanyl is optionally substituted with one or more R^{b}; the other variables are as defined herein.

In some embodiments, R² is selected from the group consisting of amino, methoxy, ethoxy, aminomethyl, 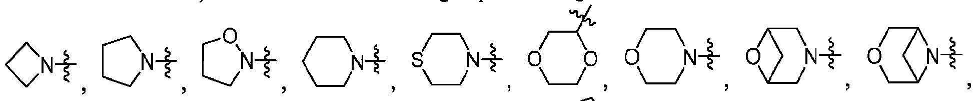 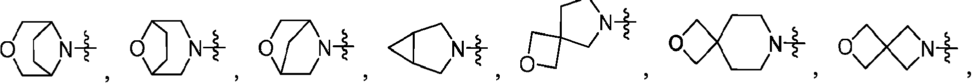 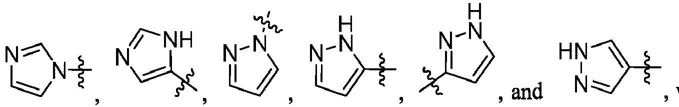 wherein the amino, methoxy, ethoxy, aminomethyl, 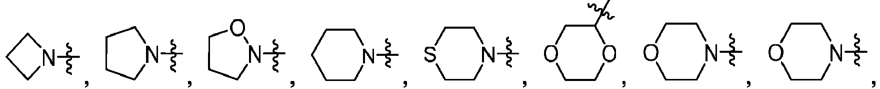 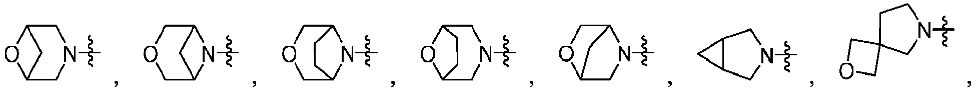 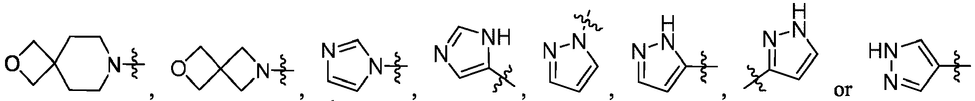 is optionally substituted with 1, 2 or 3 R^{b}; the other variables are as defined herein.

In some embodiments, R² is selected from the group consisting of 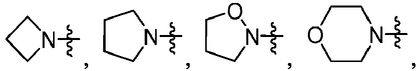 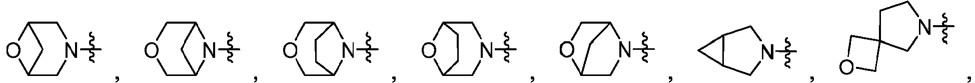 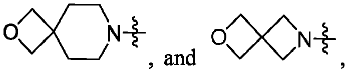 wherein the 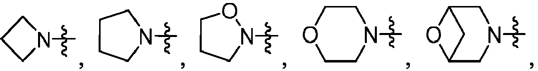 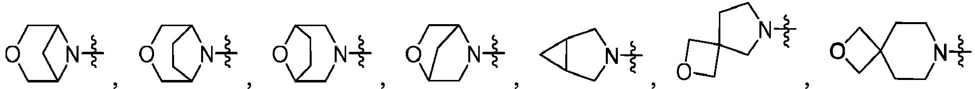 or 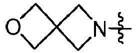 is optionally substituted with 1, 2 or 3 R^{b}; the other variables are as defined herein.

In some embodiments, R^{b} is selected from the group consisting of hydroxy, cyano, halogen, 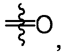 C₁₋₄ alkyl, C₁₋₄ alkoxy, C₁₋₄ alkoxy-C₁₋₄ alkyl-, C₁₋₄ alkyl-C(O)-NH-, and C₁₋₄ alkyl substituted with one or more hydroxy or halogen; the other variables are as defined herein.

In some embodiments, R^{b} is selected from the group consisting of hydroxy, cyano, fluoro, chloro, 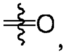 methyl, methoxy, hydroxymethyl, methoxyethyl, and acetylamino; the other variables are as defined herein.

In some embodiments, R^{b} is selected from the group consisting of hydroxy, amino, cyano, halogen, 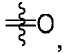 C₁₋₆ alkyl, C₁₋₆ alkoxy, and C₁₋₆ alkyl substituted with one or more hydroxy or halogen; the other variables are as defined herein.

In some embodiments, R^{b} is selected from the group consisting of hydroxy, cyano, halogen, 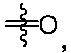 C₁₋₆ alkyl, C₁₋₆ alkoxy, and C₁₋₆ alkyl substituted with one or more hydroxy or halogen; the other variables are as defined herein.

In some embodiments, R^{b} is selected from the group consisting of hydroxy, cyano, halogen, 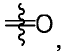 C₁₋₄ alkyl, C₁₋₄ alkoxy, and C₁₋₃ alkyl substituted with 1, 2, or 3 hydroxy; the other variables are as defined herein.

In some embodiments, R^{b} is selected from the group consisting of hydroxy, cyano, halogen, 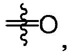 C₁₋₃ alkyl, C₁₋₃ alkoxy, and C₁₋₃ alkyl substituted with one hydroxy; the other variables are as defined herein.

In some embodiments, R^{b} is selected from the group consisting of hydroxy, cyano, fluoro, chloro, 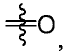 methyl, methoxy, and hydroxymethyl; the other variables are as defined herein.

In some embodiments, R² is selected from the group consisting of pyrrolidinyl, isoxazolidinyl, morpholinyl, azetidinyl, 6-oxa-3-azabicyclo[3.1.1]heptanyl, 3-oxa-6-azabicyclo[3.1.1]heptanyl, 3-oxa-8-azabicyclo[3.2.1]octanyl, 8-oxa-3-azabicyclo[3.2.1]octanyl, 2-oxa-5-azabicyclo[2.2.1]heptanyl, 3-azabicyclo[3.1.0]hexanyl, 2-oxa-6-azaspiro[3.4]octanyl, 2-oxa-7-azaspiro[3.5]nonanyl, and 2-oxa-6-azaspiro[3.3]heptanyl, wherein the pyrrolidinyl is optionally substituted with 1 or 2 hydroxy, cyano, fluoro, chloro or methoxy, wherein the azetidinyl is optionally substituted with 1 or 2 hydroxy, cyano, fluoro, methyl or hydroxymethyl, and wherein the 2-oxa-6-azaspiro[3.4]octanyl is optionally substituted with one 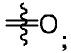 the other variables are as defined herein.

In some embodiments, R² is selected from the group consisting of amino, methoxy, ethoxy, aminomethyl, pyrrolidinyl, isoxazolidinyl, piperidinyl, morpholinyl, thiomorpholinyl, 1,4-dioxanyl, azetidinyl, 6-oxa-3-azabicyclo[3.1.1]heptanyl, 3-oxa-6-azabicyclo[3.1.1]heptanyl, 3-oxa-8-azabicyclo[3.2.1]octanyl, 8-oxa-3-azabicyclo[3.2.1]octanyl, 2-oxa-5-azabicyclo[2.2.1]heptanyl, 3-azabicyclo[3.1.0]hexanyl, 2-oxa-6-azaspiro[3.4]octanyl, 2-oxa-7-azaspiro[3.5]nonanyl, 2-oxa-6-azaspiro[3.3]heptanyl, pyrazolyl, and imidazolyl, wherein the amino is optionally substituted with 1 or 2 methyl or methoxyethyl, wherein the ethoxy is optionally substituted with 1 methoxy, wherein the aminomethyl is optionally substituted with 1 or 2 methyl or methoxy, wherein the pyrrolidinyl is optionally substituted with 1 or 2 hydroxy, cyano, fluoro, chloro, methoxy, hydroxymethyl or acetylamino, wherein the azetidinyl is optionally substituted with 1 or 2 hydroxy, cyano, fluoro, methyl or hydroxymethyl, and wherein the 2-oxa-6-azaspiro[3.4]octyl is optionally substituted with one 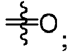 the other variables are as defined herein.

In some embodiments, R² is selected from the group consisting of methoxy, methoxyethoxy, methylamino, dimethylamino, 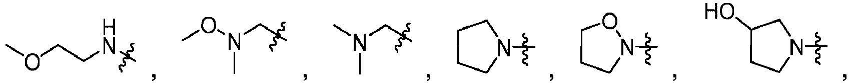 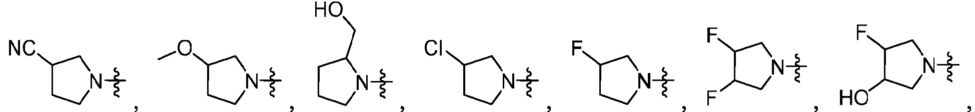 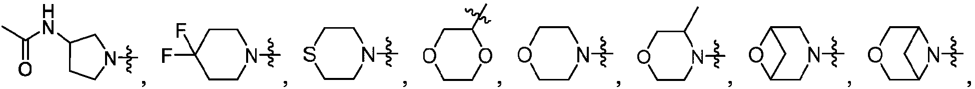 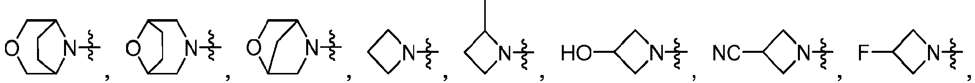 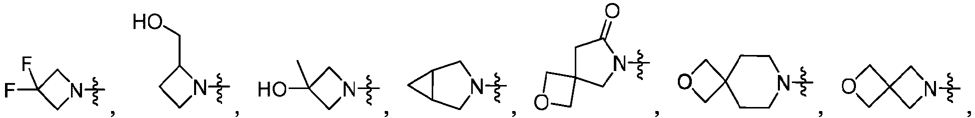 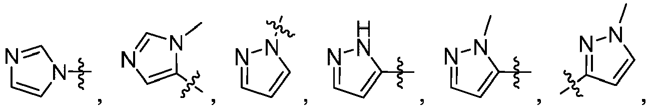 and 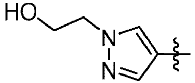 ; the other variables are as defined herein.

In some embodiments, R² is selected from the group consisting of 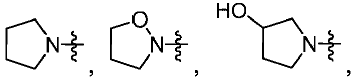 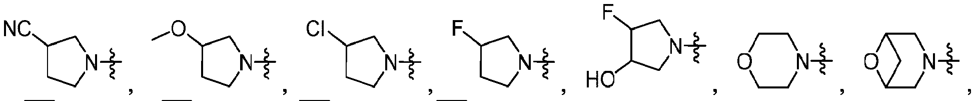 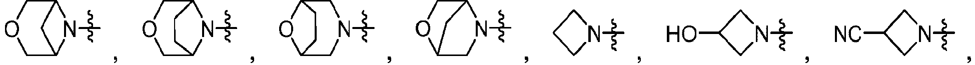 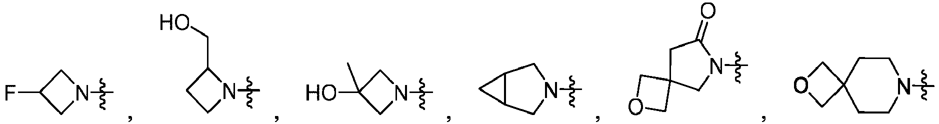 , and 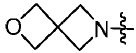 ; the other variables are as defined herein.

In some embodiments, R² is selected from the group consisting of methoxy, methoxyethoxy, methylamino, dimethylamino, 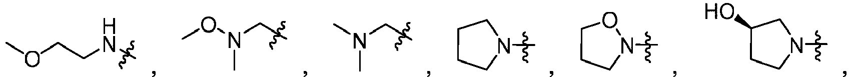 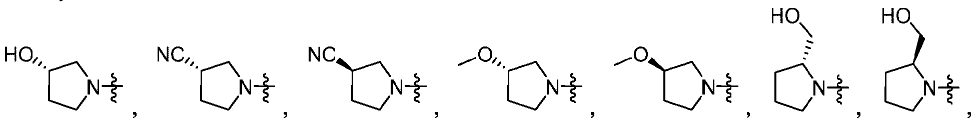 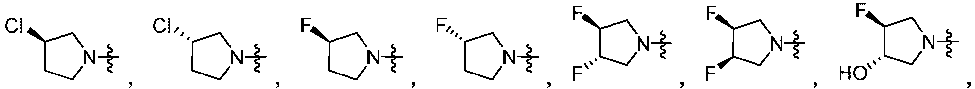 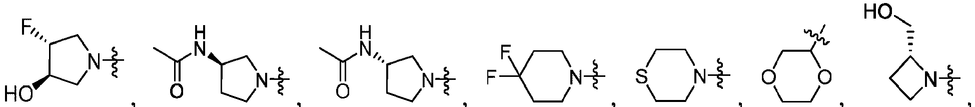 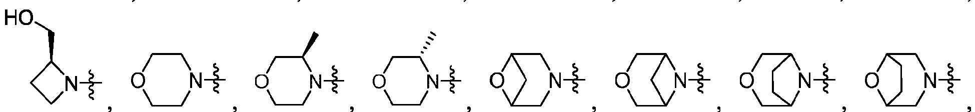 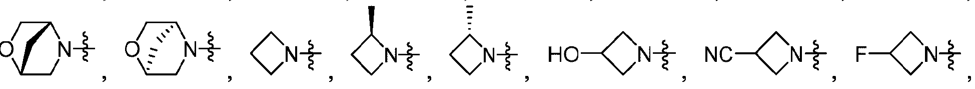 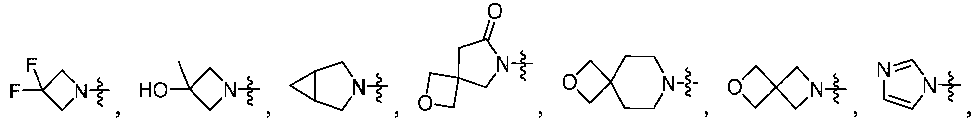 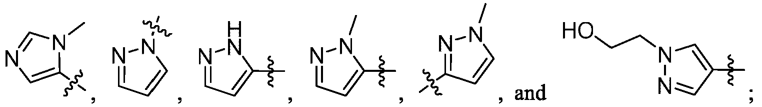 the other variables are as defined herein.

In some embodiments, R² is selected from the group consisting of 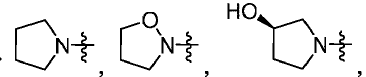 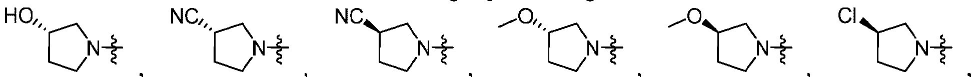 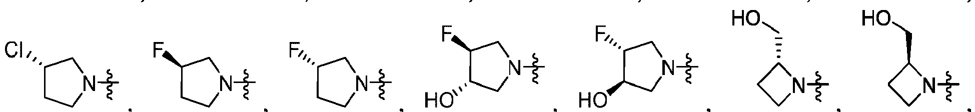 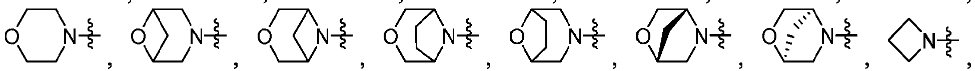 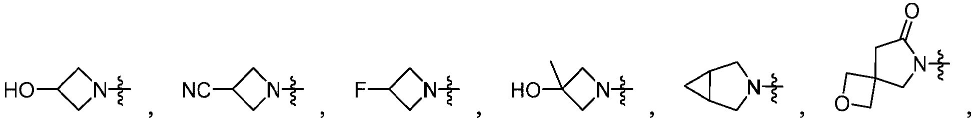 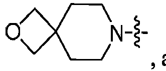 and 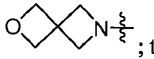 the other variables are as defined herein.

In some embodiments, R³ is selected from -OCF₂H, wherein the -OCF₂H is optionally substituted with halogen; the other variables are as defined herein.

In some embodiments, R³ is selected from -OCF₂H, wherein the -OCF₂H is optionally substituted with fluorine, chlorine, bromine, or iodine; the other variables are as defined herein. For example, R³ is selected from the group consisting of -OCF₂Cl, -OCF₂Br, -OCF₂I, and -OCF₃.

In some embodiments, R³ is selected from -OCF₂Cl; the other variables are as defined herein.

In some embodiments,
R¹ is selected from 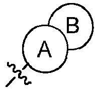 , wherein the 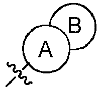 is optionally substituted with one or more R^{a'};
the ring A is selected from 5-membered heteroaryl containing 1 or 2 heteroatoms selected from the group consisting of N and O atoms;
the ring B is selected from the group consisting of 5- or 6-membered heterocycloalkyl containing 1 or 2 heteroatoms selected from the group consisting of N and O atoms, and 5-membered heteroaryl containing 1 or 2 N atoms;
R² is selected from 3- to 10-membered heterocycloalkyl, wherein the 3- to 10-membered heterocycloalkyl is optionally substituted with one or more R^{b}, and the heterocycloalkyl contains 1, 2, 3 or 4 heteroatoms or heteroatom groups independently selected from the group consisting of -O-, -NH-, and N;
R³ is selected from -OCF₂Cl;
R^{a'} is selected from the group consisting of 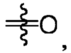 C₁₋₆ alkyl, 3- to 8-membered cycloalkyl, and C₁₋₆ alkyl substituted with one or more hydroxy or halogen;
R^{b} is selected from the group consisting of hydroxy, amino, cyano, halogen, 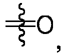 C₁₋₆ alkyl, C₁₋₆ alkoxy, and C₁₋₆ alkyl substituted with one or more hydroxy or halogen.

In some embodiments,
R¹ is selected from the group consisting of 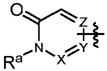 and 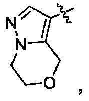 wherein the 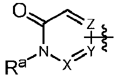 is optionally substituted with one or more R^{a'};
X, Y and Z are each independently selected from the group consisting of CH and N, and at least one of X, Y and Z is selected from CH;
R² is selected from 3- to 10-membered heterocycloalkyl, wherein the 3- to 10-membered heterocycloalkyl is optionally substituted with one or more R^{b};
R³ is selected from -OCF₂Cl;
R^{a} and R^{a'} are each independently selected from the group consisting of halogen, C₁₋₆ alkyl, 3- to 6-membered cycloalkyl, and C₁₋₆ alkyl substituted with 1, 2 or 3 hydroxy;
R^{b} is selected from the group consisting of hydroxy, cyano, halogen, 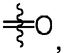 C₁₋₆ alkyl, C₁₋₆ alkoxy, and C₁₋₆ alkyl substituted with one or more hydroxy or halogen;
the heterocycloalkyl contains 1, 2, 3 or 4 heteroatoms or heteroatom groups independently selected from the group consisting of -O-, -NH-, and N.

In some embodiments, the "one or more" is selected from the group consisting of one, two, three, four, five and six. In some embodiments, the "one or more" is selected from the group consisting of one, two and three. In some embodiments, the "one or more" is selected from the group consisting of one and two.

Some other embodiments of the present application are derived from any combination of the variables described above.

In another aspect, the present application provides a compound of formula (III), a compound of formula (IV), or a pharmaceutically acceptable salt thereof, 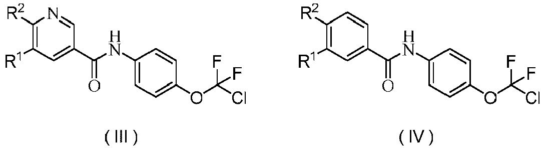 wherein,
R¹ and R² are as defined above.

In another aspect, the present application provides a compound of formula (III-A), a compound of formula (III-B), a compound of formula (IV-A), or a pharmaceutically acceptable salt thereof, 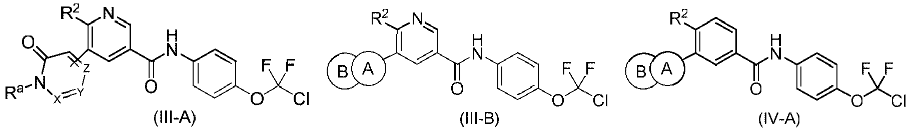 wherein 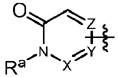 is optionally substituted with one R^{a'}, and 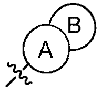 is optionally substituted with one or two R^{a'};
R², R^{a}, R^{a'}, X, Y, Z, ring A and ring B are as defined above.

In some embodiments, 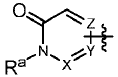 or 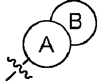 is defined as R¹.

In another aspect, the present application provides a compound of formula (V), a compound of formula (VI), a compound of formula (VII), a compound of formula (VIII), a compound of formula (IX), a compound of formula (X), or a pharmaceutically acceptable salt thereof, 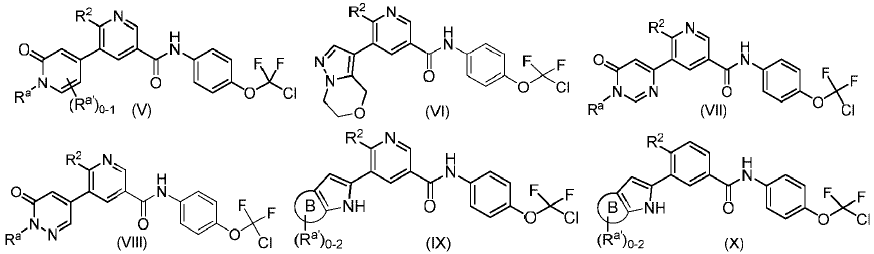 wherein,
R², R^{a}, R^{a'}, and ring B are as defined above.

In another aspect, the present application provides a compound of the following formulas or a pharmaceutically acceptable salt thereof,

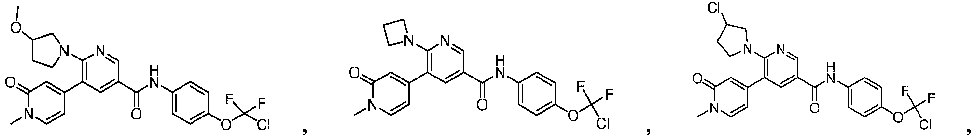

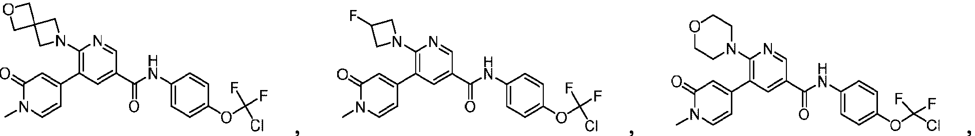

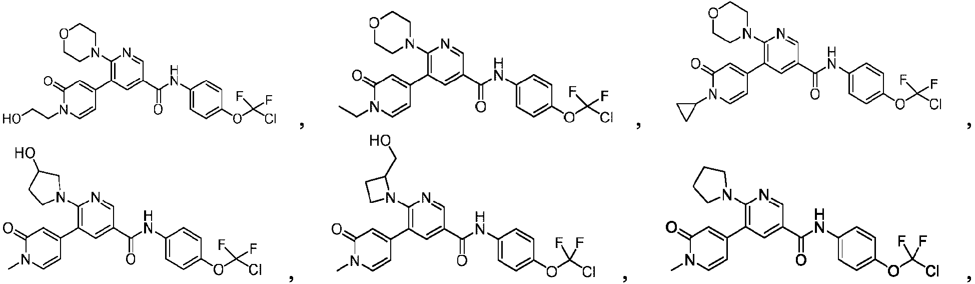

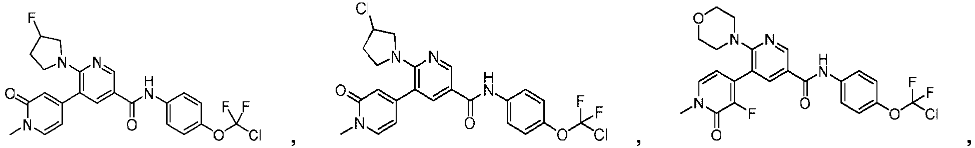

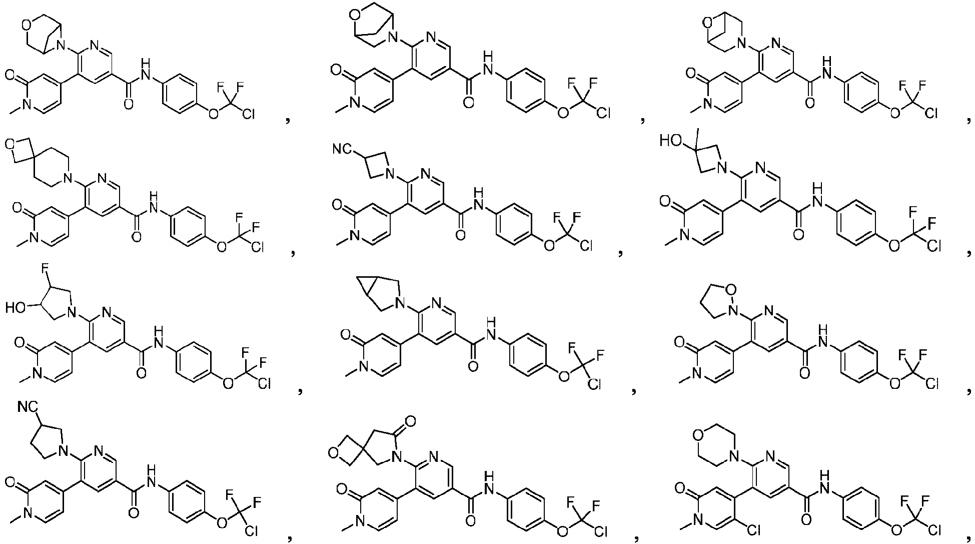

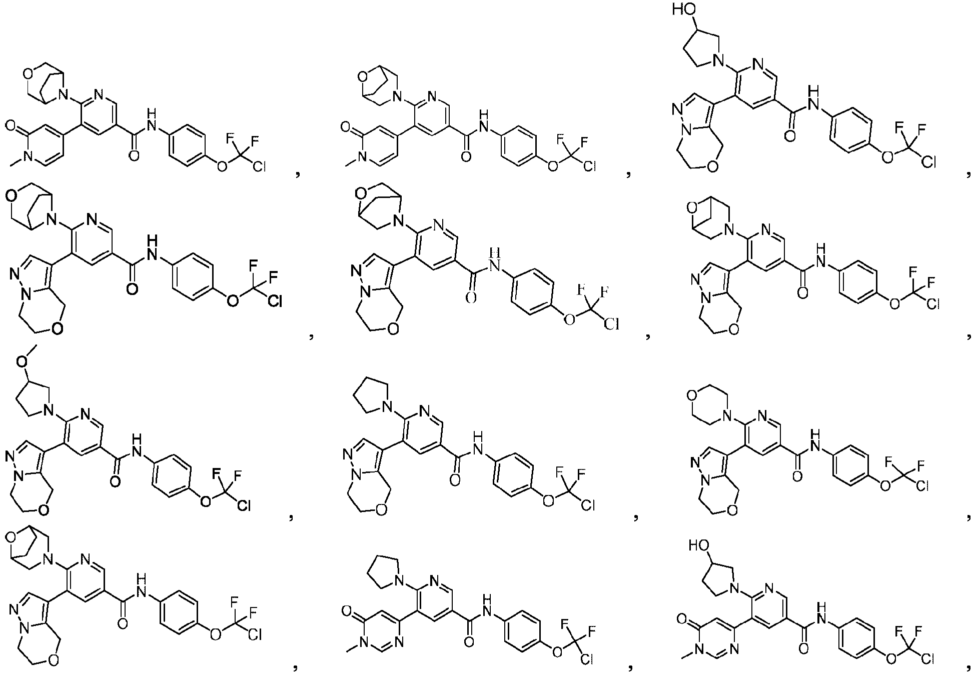

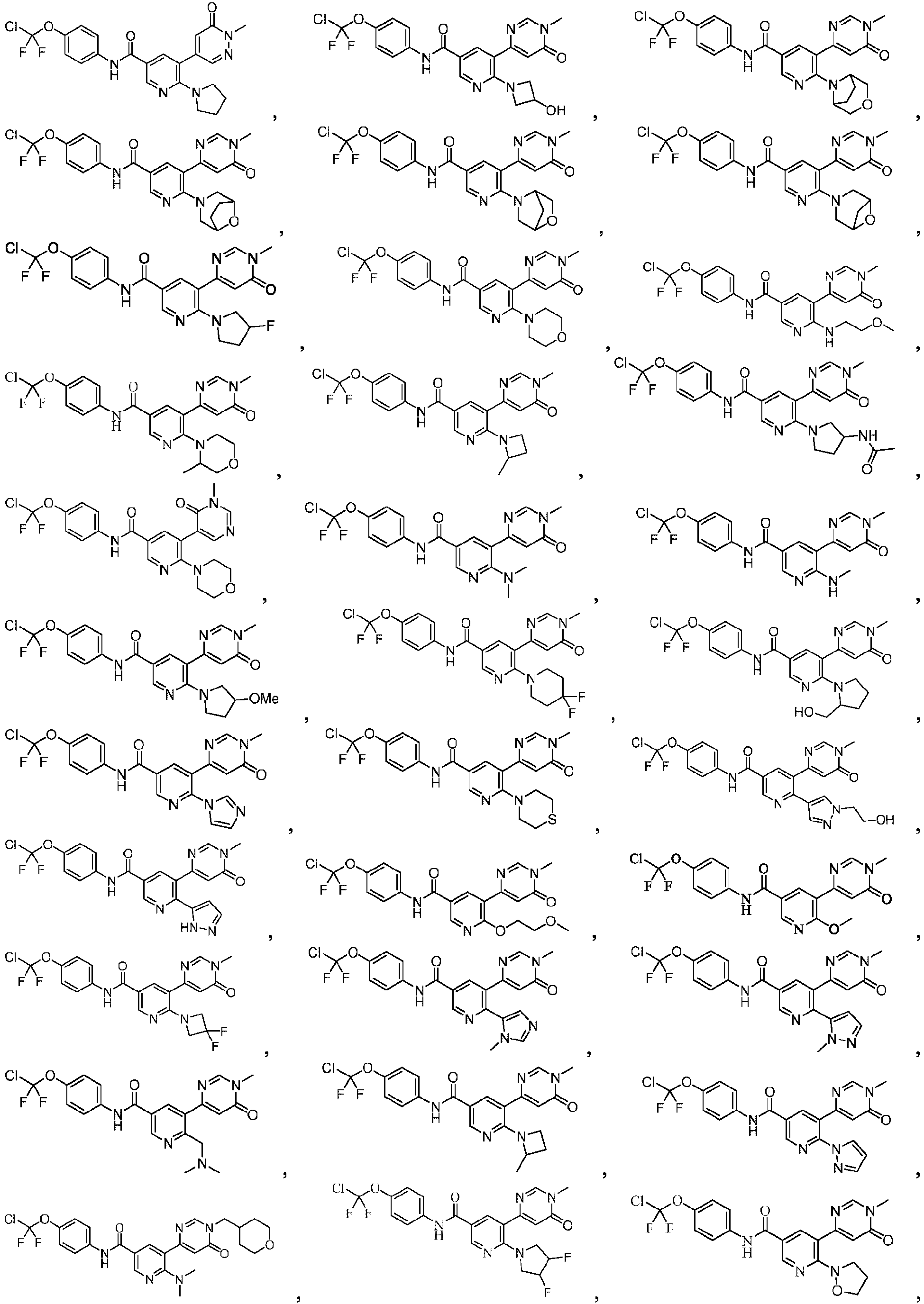

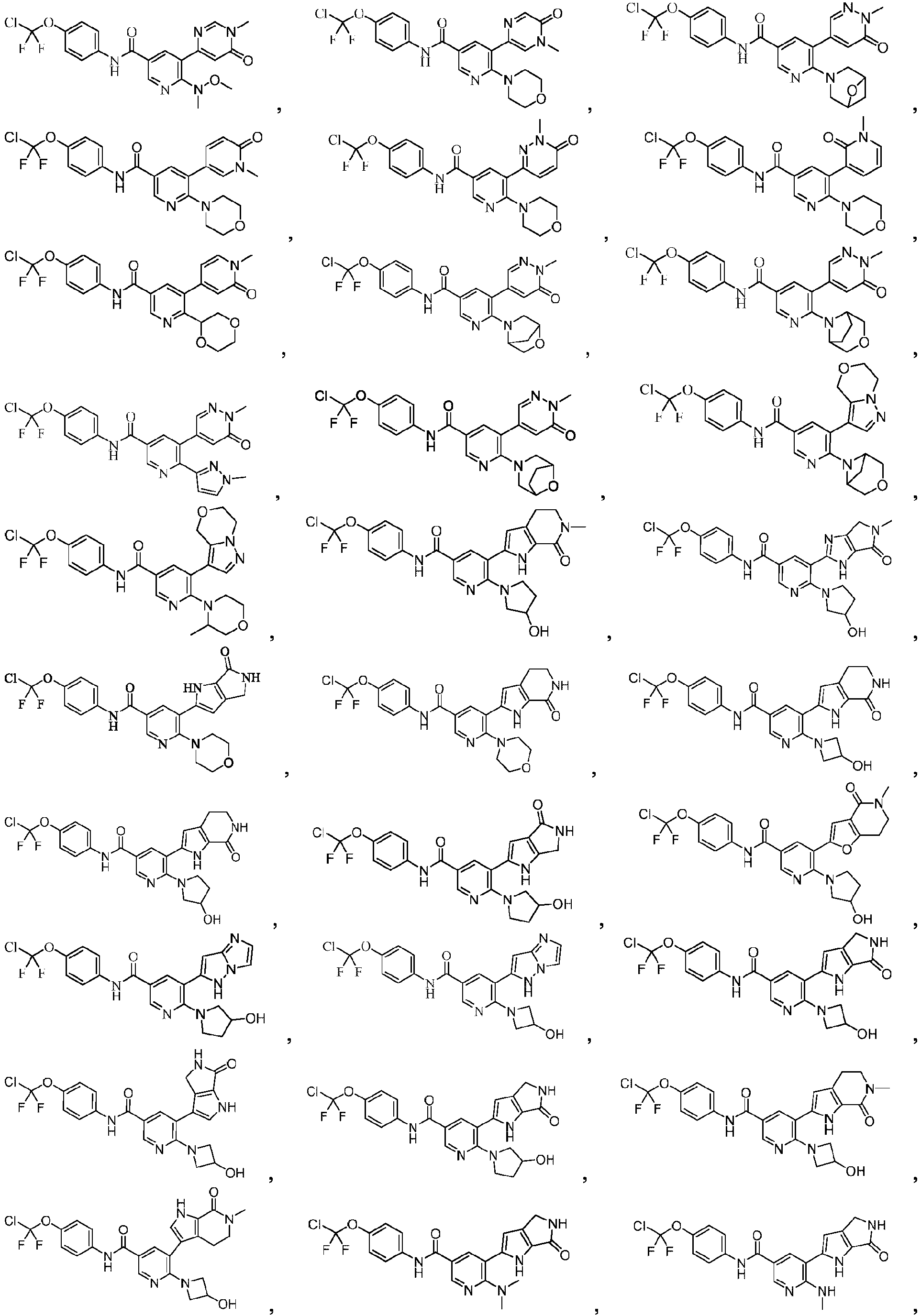

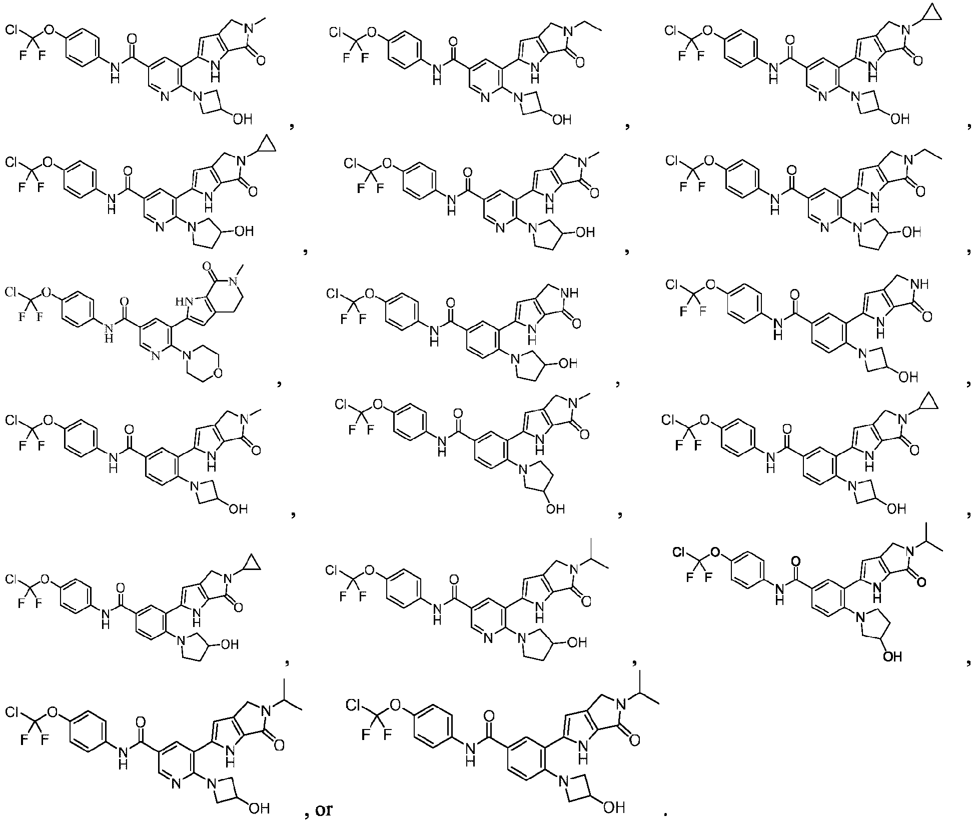

In another aspect, the present application provides a compound of the following formulas or a pharmaceutically acceptable salt thereof,

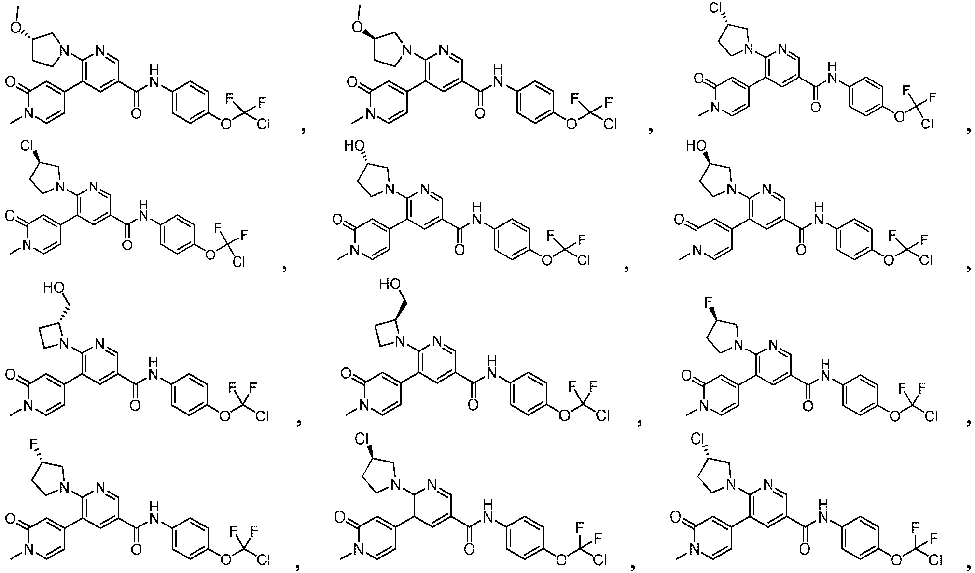

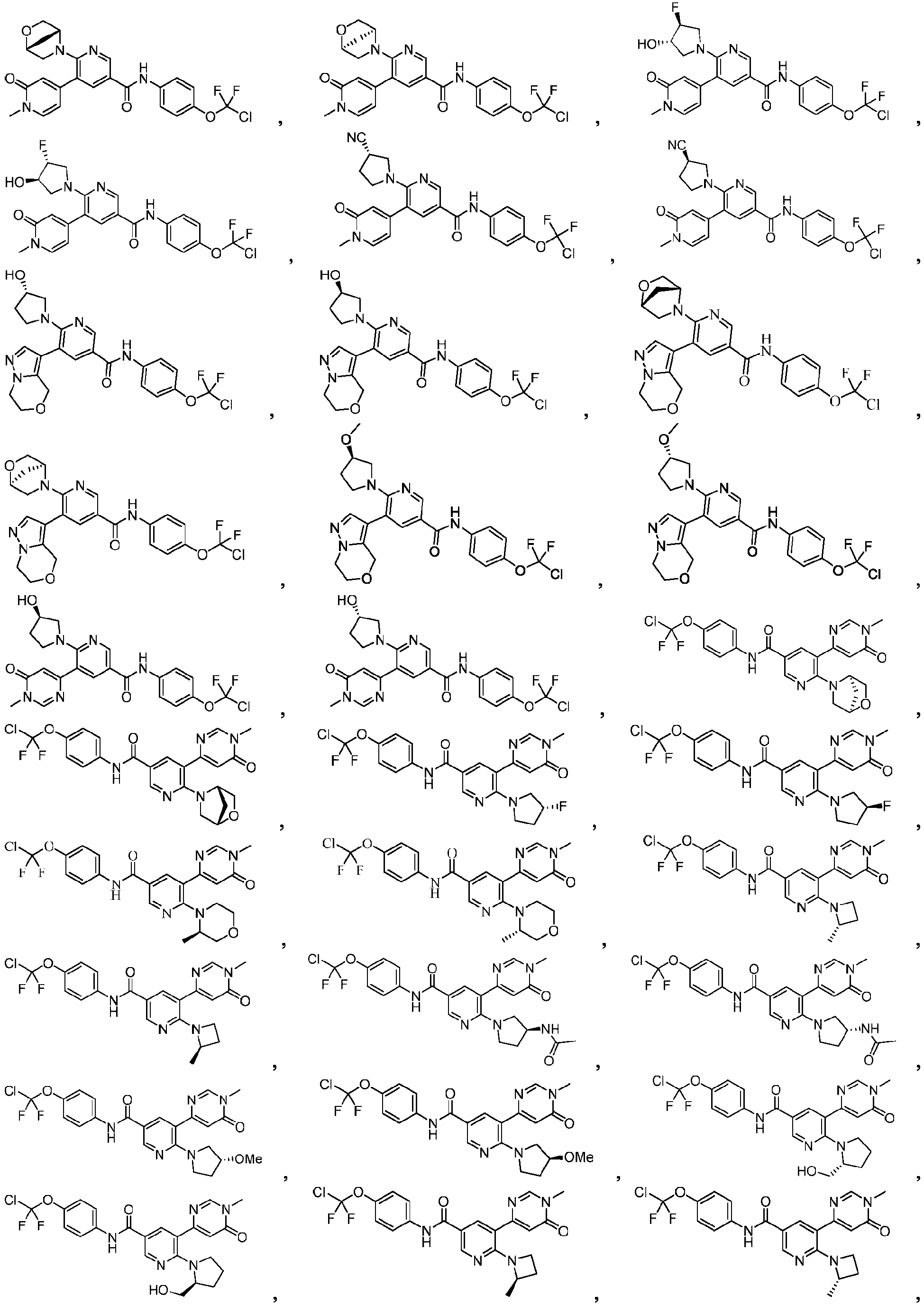

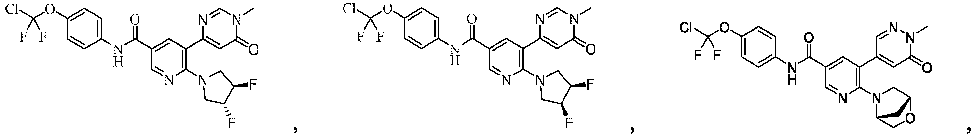

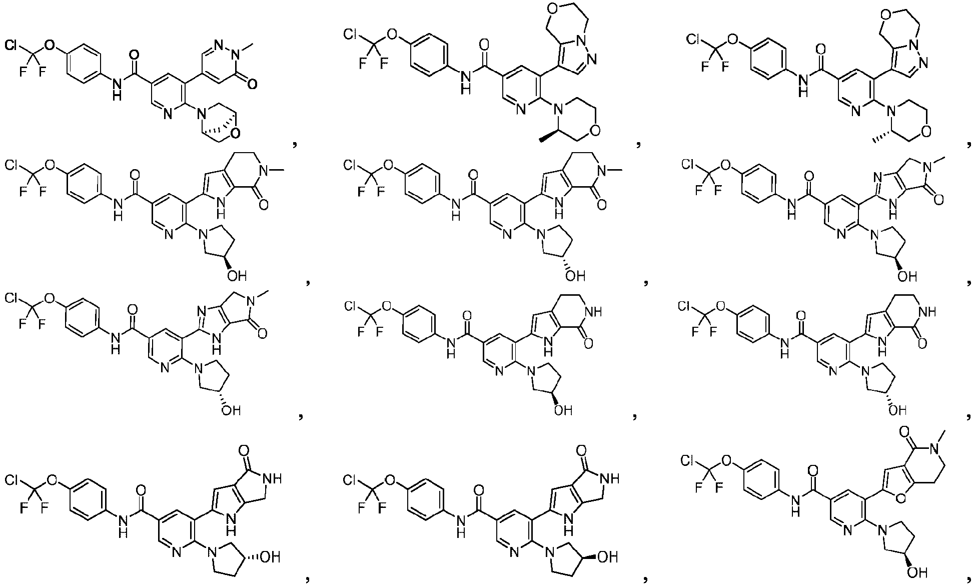

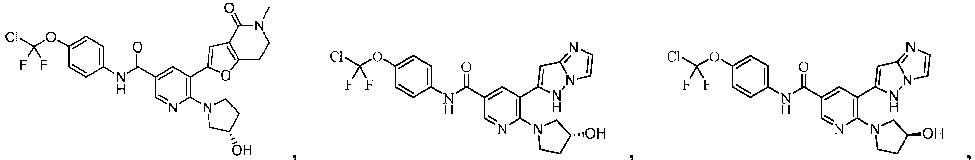

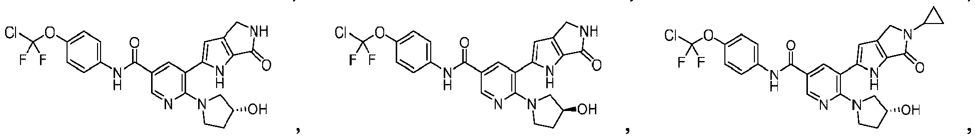

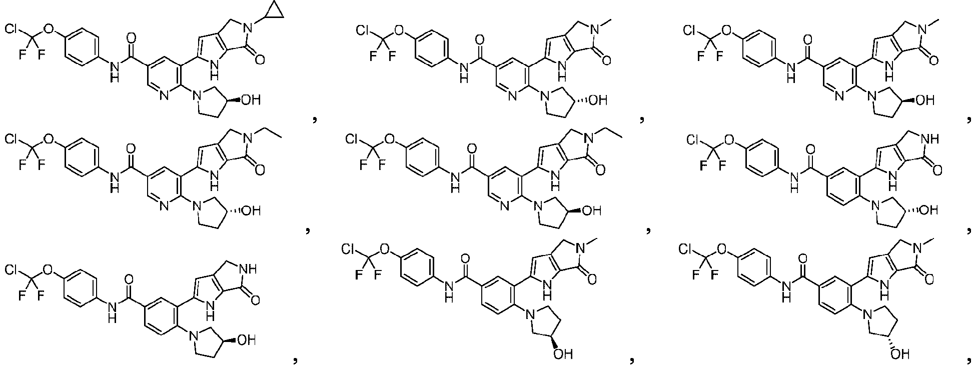

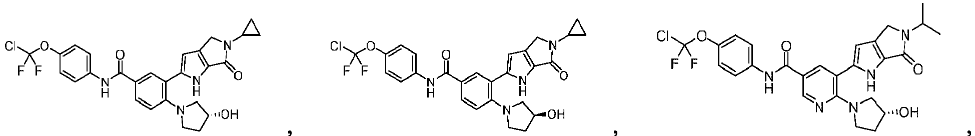

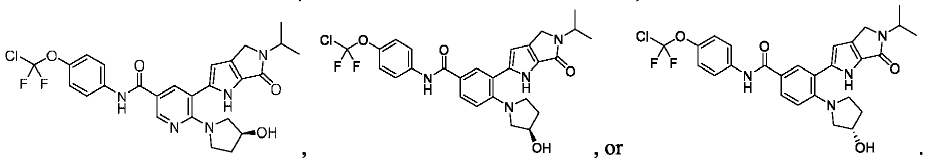

In another aspect, the present application further provides a pharmaceutical composition comprising the compound or the pharmaceutically acceptable salt thereof of the present application described above. In some embodiments, the pharmaceutical composition of the present application further comprises a pharmaceutically acceptable excipient.

In another aspect, the present application further provides a method of treating and/or preventing BCR-ABL related diseases, which comprises administering to a mammal (preferably a human) in need of the treatment a therapeutically effective amount of the compound or the pharmaceutically acceptable salt thereof, or the pharmaceutical composition thereof of the present application described above.

In another aspect, the present application further provides use of the compound or the pharmaceutically acceptable salt thereof or the pharmaceutical composition thereof of the present application described above in the preparation of a medicament for treating and/or preventing BCR-ABL related diseases.

In another aspect, the present application further provides use of the compound or the pharmaceutically acceptable salt thereof or the pharmaceutical composition thereof of the present application described above in treating and/or preventing BCR-ABL related diseases.

In another aspect, the present application further provides the compound or the pharmaceutically acceptable salt thereof or the pharmaceutical composition thereof of the present application described above for use in treating and/or preventing BCR-ABL related diseases.

In some embodiments, the BCR-ABL related diseases are selected from cancer, for example, chronic myeloid leukemia.

The compound of the present application has good cell proliferation inhibitory activity (including K562 cells and T315I mutant cells), good *in-vivo* pharmacokinetic property, low toxicity, weak inhibitory effect on hERG potassium channel, and good safety.

### Definitions

Unless otherwise stated, the following terms used in the present application shall have the following meanings. A certain term, unless otherwise specifically defined, should not be considered uncertain or unclear, but construed according to its common meaning in the field. When referring to a trade name, it is intended to refer to its corresponding commercial product or its active ingredient.

When certain structural units or groups in the present application have a covalent bond that is not connected to a specific atom, it means that the covalent bond can be connected to any atom within that structural unit or group, as long as it adheres to the rules of valence bonding.

The term "substituted" means that any one or more hydrogen atoms on a specific atom are substituted by substituents, as long as the valence of the specific atom is normal and the resulting compound is stable. When the substituent is oxo (namely =O), it means that two hydrogen atoms are substituted and oxo is not available on an aromatic group.

The terms "optional" or "optionally" means that the subsequently described event or circumstance may, but does not necessarily, occur. The description includes instances where the event or circumstance occurs and instances where it does not. "Optionally substituted" refers to substituted or unsubstituted, for example, ethyl being "optionally" substituted with halogen means that the ethyl may be unsubstituted (CH₂CH₃), monosubstituted (for example, CH₂CH₂F), polysubstituted (for example, CHFCH₂F and CH₂CHF₂), or fully substituted (CF₂CF₃). It will be appreciated by those skilled in the art that for any groups comprising one or more substituents, any substitutions or substituting patterns which may not exist or cannot be synthesized spatially are not introduced.

Cₘ₋ₙ as used herein means that the portion has an integer number of carbon atoms in the given range m-n. For example, "C₁₋₆" means that the group may have 1 carbon atom, 2 carbon atoms, 3 carbon atoms, 4 carbon atoms, 5 carbon atoms, or 6 carbon atoms. For example, C₁₋₃ means that the group may have 1 carbon atom, 2 carbon atoms, or 3 carbon atoms.

When any variable (e.g., R) occurs once or more in the constitution or structure of a compound, the definition of the variable in each case is independent. Therefore, for example, if a group is substituted with 2 R, the definition of each R is independent.

When a bond of a substituent is cross-linked to a connecting bond between two atoms on a ring, the substituent can be bonded to any atom on the ring. For example, structural unit 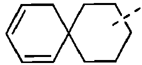 or 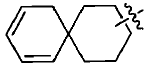 represents that substitution may occur at any one position of cyclohexyl or cyclohexadienyl.

The term "halo-" or "halogen" refers to fluorine, chlorine, bromine, and iodine.

The term "hydroxy" refers to the -OH group.

The term "amino" refers to the -NH₂ group.

The term "cyano" refers to -CN group.

The term "alkyl" refers to hydrocarbyl with a general formula of CₙH₂ₙ₊₁. The alkyl can be linear or branched. For example, the term "C₁₋₆ alkyl" refers to alkyl containing 1 to 6 carbon atoms (for example, methyl, ethyl, n-propyl, isopropyl, n-butyl, isobutyl, sec-butyl, tert-butyl, n-pentyl, 1-methylbutyl, 2-methylbutyl, 3-methylbutyl, neopentyl, hexyl, 2-methylpentyl, and the like). The alkyl moieties (namely alkyl) of alkoxy, alkylamino, dialkylamino, alkylsulfonyl, and alkylthio are similarly defined as above. As another example, the term "C₁₋₃ alkyl" refers to alkyl containing 1 to 3 carbon atoms (e.g., methyl, ethyl, propyl, and isopropyl).

The term "alkoxyl" refers to -O-alkyl.

The term "cycloalkyl" refers to a carbon ring that is fully saturated and may exist as a monocyclic, bridged cyclic or spiro structure. Unless otherwise specified, the carbocycle is generally a 3- to 10-membered ring. Non-limiting examples of cycloalkyl include, but are not limited to, cyclopropyl, cyclobutyl, cyclopentyl, cyclohexyl, norbomyl (bicyclo[2.2.1]heptyl), bicyclo[2.2.2]octyl, adamantyl, dicyclo[1.1.1]pentan-1-yl, and the like. For example, C₃₋₄ cycloalkyl includes cyclopropyl and cyclobutyl.

The term "heterocyclyl" refers to a fully saturated or partially unsaturated (but not fully unsaturated heteroaromatic group) nonaromatic ring which may exist in the form of a monocyclic, bridged cyclic, or spiro cyclic structure. Unless otherwise specified, the heterocyclyl is usually a 3- to 10-membered or 4- to 6-membered ring containing 1 to 3 heteroatoms (preferably 1 or 2 heteroatoms) independently selected from the group consisting of sulfur, oxygen, and/or nitrogen. Non-limiting examples of heterocyclyl include, but are not limited to, oxiranyl, tetrahydrofuryl, dihydrofuranyl, 3,4-dihydropyranyl, 3,6-dihydropyranyl, pyrrolidinyl, *N*-methylpyrrolidinyl, dihydropyrrolyl, piperidinyl, piperazinyl, pyrazolidinyl, 4*H*-pyranyl, morpholinyl, thiomorpholinyl, tetrahydrothienyl, 2-oxa-7-azaspiro[3.5]nonanyl, 2-oxa-6-azaspiro[3.3]heptanyl, 3-azabicyclo[3.1.0]hexanyl, and the like.

The term "heterocycloalkyl" refers to a fully saturated cyclic group which may exist in the form of a monocyclic, bridged cyclic or spiro cyclic structure. Unless otherwise specified, the heterocyclyl is usually a 3- to 10-membered or 4- to 6-membered ring containing 1 to 3 heteroatoms (preferably 1 or 2 heteroatoms) independently selected from the group consisting of sulfur, oxygen, and/or nitrogen. Examples of 3-membered heterocycloalkyl include, but are not limited to, oxiranyl, thiiranyl, and aziranyl; non-limiting examples of 4-membered heterocycloalkyl include, but are not limited to, azetidinyl, oxetanyl, and thietanyl; examples of 5-membered heterocycloalkyl include, but are not limited to, tetrahydrofuranyl, tetrahydrothienyl, pyrrolidinyl, isoxazolidinyl, oxazolidinyl, isothiazolidinyl, thiazolidinyl, imidazolidinyl, and tetrahydropyrazolyl; examples of 6-membered heterocycloalkyl include, but are not limited to, piperidinyl, tetrahydropyranyl, tetrahydrothiopyranyl, morpholinyl, piperazinyl, 1,4-oxathianyl, 1,4-dioxanyl, thiomorpholinyl, 1,3-dithianyl, and 1,4-dithianyl; examples of 7-membered heterocycloalkyl include, but are not limited to, azacycloheptanyl, oxacycloheptanyl, and thiocycloheptanyl. Monoheterocycloalkyl having 4, 5 or 6 ring atoms is preferred.

The term "monoheterocyclyl" refers to a fully saturated or partially unsaturated (but not fully unsaturated heteroaromatic group) nonaromatic ring which may exist with only one ring. Unless otherwise specified, the heterocyclyl is usually a 3- to 10-membered or 4- to 6-membered ring containing 1 to 3 heteroatoms (preferably 1 or 2 heteroatoms) independently selected from the group consisting of sulfur, oxygen, and/or nitrogen. Non-limiting examples of monoheterocyclyl include, but are not limited to, oxiranyl, tetrahydrofuranyl, dihydrofuranyl, 3,4-dihydropyranyl, 3,6-dihydropyranyl, pyrrolidinyl, *N*-methylpyrrolidinyl, dihydropyrrolyl, piperidinyl, piperazinyl, pyrazolidinyl, 4*H*-pyranyl, morpholinyl, thiomorpholinyl, tetrahydrothienyl, and the like. The term "monoheterocycloalkyl" refers to fully saturated monoheterocyclyl.

The term "bridged heterocyclyl" refers to a 5- to 14-membered polycyclic ring that is fully saturated or partially unsaturated, and shares two or more atoms with two rings, and which may contain one or more double bonds, but none of the rings have a completely conjugated π-electron system, wherein one or more of the ring atoms are heteroatoms selected from the group consisting of N, O, S(O)ₙ (where n is 0, 1 or 2), and the remaining ring atoms are carbon. It is preferably 6- to 14-membered, and is more preferably 6- to 10-membered. According to the number of the formed rings, the bridged heterocyclic ring may be bicyclic, tricyclic, tetracyclic or polycyclic bridged heterocyclic ring, preferably bicyclic or tricyclic bridged heterocyclic ring, and more preferably bicyclic bridged heterocyclic ring. Non-limiting examples of bridged heterocycles include: 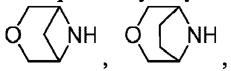 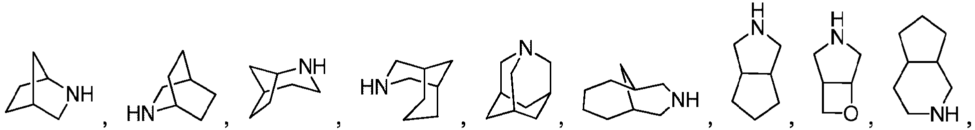 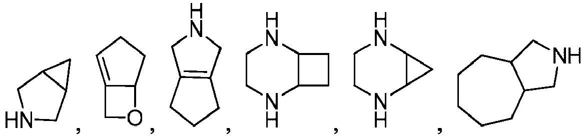 , and the like.

The term "bridged heterocycloalkyl" refers to a fully saturated bridged heterocyclyl.

The term "spiro-heterocyclyl" refers to a fully saturated or partially unsaturated (but not fully unsaturated) spiro in which one or more ring atoms are heteroatoms (preferably 1 or 2 heteroatoms) selected from the group consisting of sulfur, oxygen, and/or nitrogen, and the remaining ring atoms are carbon. It is preferably 6- to 14-membered, and is more preferably 6- to 10-membered. According to the number of spiro atoms shared among the rings, a spiro heterocyclic ring may be a monospiro heterocyclic ring, a bispiro heterocyclic ring or a polyspiro heterocyclic ring, preferably a monospiro heterocyclic ring or a bispiro heterocyclic ring, and more preferably a 4-membered/4-membered, 4-membered/5-membered, 4-membered/6-membered, 5-membered/5-membered or 5-membered/6-membered monospiro heterocyclic ring. Non-limiting examples of spiro heterocyclic ring include 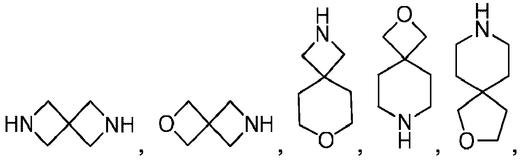 and the like.

The term "spiro-heterocycloalkyl" refers to fully saturated spiro-heterocyclyl.

The term "heteroaryl" refers to a monocyclic or fused polycyclic system which comprises at least one ring atom selected from the group consisting of N, O and S, with the remaining ring atoms being C, and which has at least one aromatic ring. Preferably, heteroaryl has a single 5- to 8-membered ring, or a plurality of fused rings comprising 6 to 14 ring atoms, particularly 6 to 10 ring atoms. Non-limiting examples of heteroaryl include, but are not limited to, pyrrolyl, furanyl, thienyl, imidazolyl, oxazolyl, pyrazolyl, pyridinyl, pyrimidinyl, pyrazinyl, quinolyl, isoquinolyl, tetrazolyl, triazolyl, triazinyl, benzofuranyl, benzothienyl, indolyl, isoindolyl, and the like.

In the present application, the group 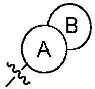 represents that ring A and ring B form a double ring structure via a common bond, wherein the common bond may be a single bond or a double bond.

The term "treat" or "treatment" means administering a compound or formulation of the present application to ameliorate or eliminate a disease or one or more symptoms associated with the disease, including:
(i) inhibiting a disease or disease state, i.e., arresting its development; and
(ii) alleviating a disease or disease state, i.e., causing its regression.

The term "prevent" or "prevention" means administering a compound or formulation of the present application to prevent a disease or one or more symptoms associated with the disease, including: preventing the occurrence of the disease or disease state in a mammal, particularly when such a mammal is predisposed to the disease state but has not yet been diagnosed with it.

The term "therapeutically effective amount" refers to an amount of the compound of the present application for (i) treating or preventing a specific disease, condition or disorder; (ii) alleviating, ameliorating or eliminating one or more symptoms of a specific disease, condition or disorder, or (iii) preventing or delaying onset of one or more symptoms of a specific disease, condition or disorder described herein. The amount of the compound of the present application composing the "therapeutically effective amount" varies depending on the compound, the disease state and its severity, the administration regimen, and the age of the mammal to be treated, but can be determined routinely by those skilled in the art in accordance with their knowledge and the present disclosure.

The term "pharmaceutically acceptable" is used herein for those compounds, materials, compositions, and/or dosage forms which are, within the scope of sound medical judgment, suitable for use in contact with the tissues of human beings and animals without excessive toxicity, irritation, allergic response, or other problems or complications, and commensurate with a reasonable benefit/risk ratio.

A pharmaceutically acceptable salt, for example, may be a metal salt, an ammonium salt, a salt formed with an organic base, a salt formed with an inorganic acid, a salt formed with an organic acid, a salt formed with a basic or acidic amino acid, and the like.

The term "pharmaceutical composition" refers to a mixture consisting of one or more of the compounds or the salts thereof of the present application and a pharmaceutically acceptable excipient. The pharmaceutical composition is intended to facilitate the administration of the compound of the present application to an organism. The term "pharmaceutically acceptable excipient" refers to those that do not have a significant irritating effect on an organism and do not impair the biological activity and properties of the active compound. Suitable excipients are well known to those skilled in the art, for example, carbohydrate, wax, water-soluble and/or water-swellable polymers, hydrophilic or hydrophobic material, gelatin, oil, solvent, water, and the like.

The word "comprise" and variations thereof such as "comprises" or "comprising" will be understood in an open, non-exclusive sense, i.e., "including but not limited to".

The compounds of the present invention may exist in the form of a specific geometric isomer or stereoisomer. All such compounds are contemplated herein, including cis and trans isomers, (-)- and (+)-enantiomers, (*R*)- and (*S*)-enantiomers, diastereoisomers, (*D*)-isomers, (*L*)-isomers, and racemic mixtures and other mixtures thereof, such as an enantiomer or diastereomer enriched mixture, all of which are encompassed within the scope of the present invention. The substituents such as alkyl may have an additional asymmetric carbon atom. All these isomers and mixtures thereof are encompassed within the scope of the present invention.

Unless otherwise stated, "(*D*)" or "(+)" stands for dextrorotation, "(*L*)" or "(-)" stands for levorotation, and "(*DL*)" or "(±)" stands for racemization.

Unless otherwise stated, the absolute configuration of a stereogenic center is represented by a wedged solid bond ( 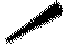 ) and a wedged dashed bond ( 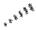 ), and the relative configuration of a stereogenic center is represented by a straight solid bond ( 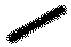 ) and a straight dashed bond ( 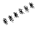 ). A wavy line ( 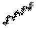 ) represents a wedged solid bond ( 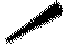 ) or a wedged dashed bond ( 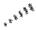 ), or a wavy line ( 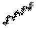 ) represents a straight solid bond ( 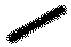 ) and a straight dashed bond ( 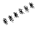 ).

Optically active (*R*)- and (*S*)-isomers and *D* and *L* isomers can be prepared by chiral synthesis or chiral reagents or other conventional techniques. If one enantiomer of a certain compound of the present invention is to be obtained, the desired pure enantiomer can be prepared by asymmetric synthesis or derivatization using a chiral additive, wherein the resulting diastereoisomeric mixture is separated and the auxiliary group is cleaved. Alternatively, when the molecule contains a basic functional group (such as amino) or an acidic functional group (such as carboxyl), the compound reacts with an appropriate optically active acid or base to form a salt of the diastereoisomer, which is then subjected to diastereomeric resolution through conventional methods in the art to give the pure enantiomer. Furthermore, the enantiomer and the diastereoisomer are generally isolated through chromatography using a chiral stationary phase, optionally in combination with chemical derivatization (e.g., carbamate generated from amines).

The compound disclosed herein may contain an unnatural proportion of atomic isotope at one or more of the atoms that constitute the compound. For example, the compound may be labeled with a radioisotope, such as tritium (³H), iodine-125 (¹²⁵I), or C-14 (¹⁴C). For another example, hydrogen may be substituted with deuterium to form a deuterated drug, for example, *d*₃-methyl indicates that all three hydrogen atoms on a methyl group are substituted with deuterium atoms, and the bond formed by deuterium and carbon is firmer than the bond formed by common hydrogen and carbon. Compared with an un-deuterated drug, the deuterated drug has the advantages of reduced toxic side effects, increased stability, enhanced efficacy, prolonged biological half-life and the like. All isotopic variations of the compound disclosed herein, whether radioactive or not, are encompassed within the scope of the present invention.

The present application also comprises isotopically labeled compounds of the present application which are identical to those recited herein but have one or more atoms replaced by an atom having an atomic mass or mass number different from the atomic mass or mass number usually found in nature. Examples of isotopes that can be incorporated into the compounds of the present application include isotopes of hydrogen, carbon, nitrogen, oxygen, phosphorus, sulfur, fluorine, iodine and chlorine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ¹²³I, ¹²⁵I and ³⁶Cl.

Certain isotopically labeled compounds of the present application (e.g., those labeled with ³H and ¹⁴C) can be used to analyze compounds and/or substrate tissue distribution. Tritiated (i.e., ³H) and carbon-14 (i.e., ¹⁴C) isotopes are particularly preferred for their ease of preparation and detectability. Positron emitting isotopes, such as ¹⁵O, ¹³N, ¹¹C, and ¹⁸F can be used in positron emission tomography (PET) studies to determine substrate occupancy. Isotopically labeled compounds of the present application can generally be prepared by following procedures analogous to those disclosed in the schemes and/or examples below while substituting a non-isotopically labeled reagent with an isotopically-labeled reagent.

Furthermore, substitution with heavier isotopes such as deuterium (i.e., ²H) may provide certain therapeutic advantages (e.g., increased *in vivo* half-life or reduced dosage) resulting from greater metabolic stability and hence may be preferred in some circumstances in which deuterium substitution may be partial or complete, wherein partial deuterium substitution refers to substitution of at least one hydrogen with at least one deuterium, and all such forms of the compounds are included within the scope of the present application.

Unless otherwise specified clearly herein, singular terms encompass plural terms, and vice versa. Similarly, unless otherwise specified clearly herein, the word "or" is intended to include "and".

Unless otherwise indicated, the parameter values should be understood as being modified by the term "about" in this context. When the term "about" is used to describe the parameters of the present application, the term "about" indicates that there is an error value; for example, it means varying within a range of ±5%, such as ±1% or ±0.1%, of a particular value.

All patents, patent applications and other identified publications are explicitly incorporated herein by reference for the purpose of description and disclosure. These publications are provided solely because they were disclosed prior to the filing date of the present application. All statements as to the dates of these documents or description as to the contents of these documents are based on the information available to the applicant and do not constitute any admission as to the correctness of the dates or the content of these documents. Moreover, in any country or region, any reference to these publications herein is not to be construed as an admission that the publications form part of the commonly recognized knowledge in the art.

The compound of the present application can be asymmetrical, for example, has one or more stereisomers. Unless otherwise stated, all stereoisomers include, for example, enantiomers and diastereoisomers. The compound with asymetrical carbon atoms of the present application can be separated in an optically pure form or in a racemic form. The optically pure form can be separated from a racemic mixture or can be synthesized using a chiral raw material or a chiral reagent.

The pharmaceutical composition of the present application can be prepared by combining the compound of the present application with a suitable pharmaceutically acceptable excipient, and can be formulated, for example, into a solid, semisolid, liquid, or gaseous formulation such as tablet, pill, capsule, powder, granule, ointment, emulsion, suspension, suppository, injection, inhalant, gel, microsphere and aerosol.

Typical routes of administration of the compound or the pharmaceutically acceptable salt thereof or the pharmaceutical composition thereof of the present application include, but are not limited to, oral, rectal, local, inhalation, parenteral, sublingual, intravaginal, intranasal, intraocular, intraperitoneal, intramuscular, subcutaneous and intravenous administration.

The pharmaceutical composition of the present application can be manufactured using methods well known in the art, such as conventional mixing, dissolving, granulating, dragee-making, levigating, emulsifying, lyophilizing, and the like.

Therapeutic dosages of the compound of the present application may be determined by, for example, the specific use of a treatment, the route of administration of the compound, the health and condition of a patient, and the judgment of a prescribing physician. The proportion or concentration of the compound of the present application in a pharmaceutical composition may not be constant and depends on a variety of factors including dosages, chemical properties (e.g., hydrophobicity), and routes of administration. For example, the compound of the present application may be provided for parenteral administration by a physiological buffered aqueous solution containing about 0.1% w/v to 10% w/v of the compound. Certain typical dosages range from about 1 µg/kg body weight/day to about 1 g/kg body weight/day. In certain embodiments, the dosage ranges from about 0.01 mg/kg body weight/day to about 100 mg/kg body weight/day. The dosage is likely to depend on such variables as the type and degree of progression of the disease or disorder, the general health condition of a particular patient, the relative biological potency of the compound selected, the excipient formulation, and its route of administration. Effective doses can be extrapolated from dose-response curves derived from *in vitro* or animal model test systems. The compounds of the present application can be prepared by a variety of synthetic methods well known to those skilled in the art, including the specific embodiments listed below, embodiments formed by combinations thereof with other chemical synthetic methods, and equivalents thereof known to those skilled in the art. The preferred embodiments include, but are not limited to, the examples of the present application.

The chemical reactions of the specific embodiments of the present application are conducted in a proper solvent that must be suitable for the chemical changes in the present application and the reagents and materials required. In order to obtain the compounds of the present application, it is sometimes necessary for those skilled in the art to modify or select a synthesis procedure or a reaction process based on the existing embodiments.

An important consideration in synthetic route planning in the art is the selection of suitable protecting groups for reactive functional groups (e.g., amino in the present application). For example, reference may be made to Greene's Protective Groups in Organic Synthesis (4th Ed.) Hoboken, New Jersey: John Wiley & Sons, Inc.

The compound of formula (I) according to the present application can be prepared by one skilled in the art of organic synthesis through the following routes: 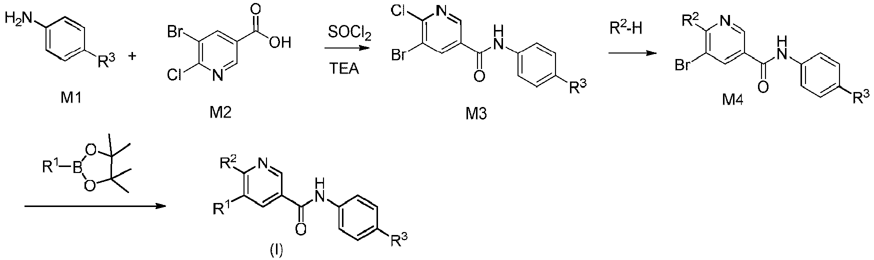 wherein,
R¹, R² and R³ are as defined above, and R² is not hydrogen.

The present application employs the following abbreviations: SOCl₂ for thionyl chloride; TEA for triethylamine; DMSO for dimethyl sulfoxide; THF for tetrahydrofuran.

### DETAILED DESCRIPTION

For clarity, the present application is further described with the following examples, which are, however, not intended to limit the scope of the present application. It will be apparent to those skilled in the art that various changes and modifications can be made to the specific examples of the present invention without departing from the spirit and scope of the present invention. All reagents used in the present application are commercially available and can be used without further purification.

### Example 1: Preparation of Compound 1

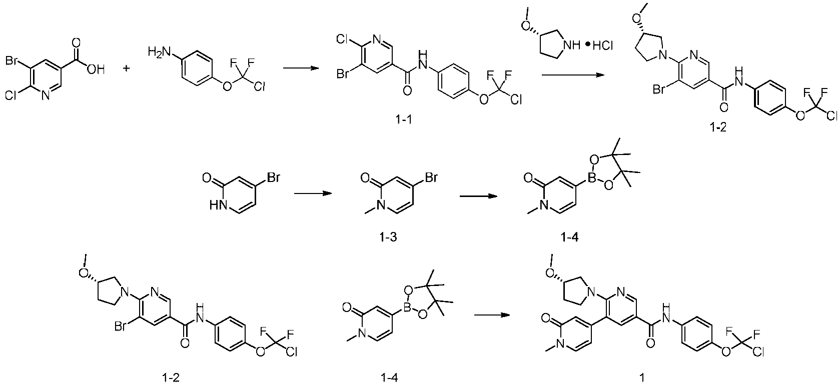

### Step A: Preparation of compound 1-1

To a 500-mL three-necked flask were added toluene (300 mL) and 5-bromo-6-chloronicotinic acid (15.0 g) in sequence, then thionyl chloride (14.79 g) was added dropwise into the system at room temperature, after the addition was completed, the system was heated to 70-80 °C and reacted for 4 h, then the reaction was stopped, and the system was concentrated under reduced pressure to obtain a brown oil; dichloromethane (300 mL) was added into the system and stirred, and 4-(chlorodifluoromethoxy)aniline (12.28 g) was added dropwise into the system; after the dropwise addition was completed, triethylamine (12.58 g) was added dropwise; after the dropwise addition was completed, the system was reacted at room temperature for 4 h. After the reaction was completed, a saturated sodium bicarbonate aqueous solution (100 mL) was added into the reaction solution described above, stirred for 10 min, and filtered, and a filter cake was collected; the mother liquor was subjected to liquid separation to obtain an organic phase, and a saturated sodium chloride aqueous solution (100 mL) was added into the organic phase, followed by stirring and liquid separation to obtain an organic phase; the filter cake was added into the organic phase, and the mixture was concentrated under reduced pressure to obtain a residue which was purified by silica gel column chromatography to obtain 20.44 g of compound 1-1.

### Step B: Preparation of compound 1-2

To a 50-mL tube were added isopropanol (10 mL), compound 1-1 (1.0 g) obtained in the step A above, (*S*)-3-methoxy pyrrolidine hydrochloride (0.334 g), *N,N*-diisopropylethylamine (1.004 g) and magnetic stir bar in sequence, and after the tube was sealed, the sealed tube was placed into a microwave reactor to react at 140 °C for 1.5 h. After the reaction solution was cooled to room temperature, ethyl acetate (15 mL) and a saturated sodium chloride aqueous solution (15 mL) were added into the reaction solution, and stirred and washed, followed by liquid separation, the organic phase was collected, dried over anhydrous sodium sulfate, filtered under vaccuum, and concentrated under reduced pressure, and the resulting residue was purified by silica gel column chromatography to obtain 0.8 g of compound 1-2. MS (ESI, [M-H]⁻) *m*/*z:* 474.0.

### Step C: Preparation of compound 1-3

To a 100-mL three-necked flask were added 4-bromopyridin-2(1*H*)-one (2.0 g) and anhydrous THF (20 mL) in sequence, after the system was cooled to 0 °C in an ice bath, 60% sodium hydride (0.919 g) was added in portions, and after the system was purged with nitrogen for 3 times, the system was heated to room temperature and stirred for 1 h. Iodomethane (3.26 g) was slowly added into the reaction solution in an ice bath, and the system was reacted at room temperature overnight. Water (10 mL) was added into the reaction solution to quench the reaction, and ethyl acetate (40 mL) was added for extraction; the organic layer was separated, and the aqueous layer was extracted with ethyl acetate (20 mL × 2). The organic layers were combined, washed with saturated sodium chloride, and dried over anhydrous sodium sulfate. The solvent was distilled off under reduced pressure to obtain compound 1-3 (1.82 g). MS (ESI, [M+H]⁺) *m*/*z*: 188.0.

### Step D: Preparation of compound 1-4

To a 10-mL microwave tube were added 1,4-dioxane (5 mL), compound 1-3 (142 mg) obtained in the step C above, bis(pinacolato)diboron (160 mg), potassium acetate (124 mg) and [1,1'-bis(diphenylphosphino)ferrocene]palladium dichloride (31 mg) in sequence, after the addition was completed, the system was purged with nitrogen, and heated to 90 °C and reacted for 4 h, the reaction was stopped, the system was cooled to room temperature, and the obtained reaction solution was directly used in the next step without separation and purification.

### Step E: Preparation of compound 1

To the reaction solution obtained in the step D were added compound 1-2 (200 mg), potassium carbonate (174 mg), deionized water (1 mL) and tetrakis(triphenylphosphine)palladium (48 mg) in sequence; after the addition was completed, the system was purged with nitrogen, and then the microwave tube was sealed and placed in a microwave reactor, heated to 140 °C, and reacted for 1.5 h. The reaction solution was cooled to room temperature and then filtered, and the mother liquor was collected and purified by silica gel column chromatography to obtain 123 mg of compound 1.

¹H NMR (500 MHz, DMSO-*d*₆) *δ* (ppm) 10.20 (s, 1H), 8.75 (d, *J* = 2.3 Hz, 1H), 8.06 (d, *J* = 2.3 Hz, 1H), 7.87 (d, *J* = 8.9 Hz, 2H), 7.75 (d, *J* = 6.9 Hz, 1H), 7.34 (d, *J* = 8.6 Hz, 2H), 6.39 (s, 1H), 6.28 (dd, *J* = 7.0, 2.0 Hz, 1H), 3.96 (dd, *J* = 5.6, 3.0 Hz, 1H), 3.54 - 3.41 (m, 5H), 3.37 - 3.34 (m, 1H), 3.26 (d, *J* = 12.0 Hz, 1H), 3.20 (s, 3H), 2.03 - 1.94 (m, 1H), 1.94 - 1.85 (m, 1H). MS (ESI, [M+H]⁺) *m*/*z:* 505.0.

### Example 2: Preparation of Compound 2

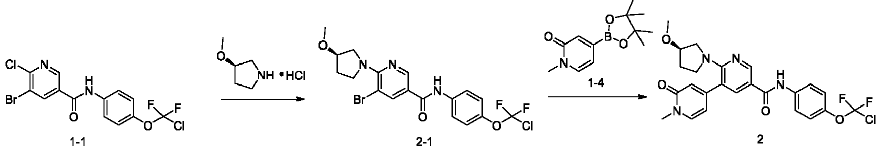

### Step A: Preparation of compound 2-1

Referring to the preparation method of the step B in Example 1, compound 1-1 was reacted with (*R*)-3-methoxypyrrolidine hydrochloride to obtain intermediate compound 2-1. MS (ESI, [M+H]⁺) *m*/*z*: 476.1.

### Step B: Preparation of compound 2

Referring to the preparation method of the step E in Example 1, compound 2-1 prepared in the step A above was added into the reaction solution of compound 1-4 for reaction to obtain compound 2.

¹H NMR (500 MHz, DMSO-*d*₆) δ (ppm) 10.20 (s, 1H), 8.75 (d, *J* = 2.3 Hz, 1H), 8.06 (d, *J* = 2.3 Hz, 1H), 7.91 - 7.82 (m, 2H), 7.75 (d, *J* = 6.9 Hz, 1H), 7.35 (d, *J* = 8.6 Hz, 2H), 6.40 (d, *J* = 2.0 Hz, 1H), 6.28 (dd, *J* = 6.9, 2.0 Hz, 1H), 4.01 - 3.93 (m, 1H), 3.54 - 3.40 (m, 5H), 3.26 (d, *J* = 12.0 Hz, 1H), 3.37 - 3.34 (m, 1H), 3.20 (s, 3H), 2.03 - 1.95 (m, 1H), 1.95 - 1.85 (m, 1H). MS (ESI, [M+H]⁺) *m*/*z:* 505.1.

### Example 3: Preparation of Compound 3

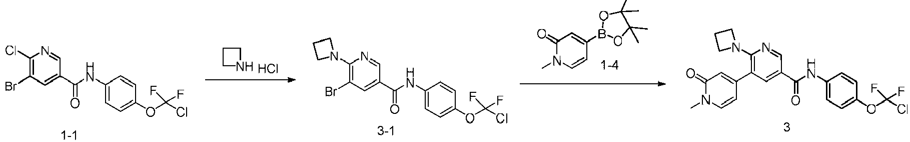

### Step A: Preparation of compound 3-1

Referring to the preparation method of the step B in Example 1, compound 1-1 was reacted with azetidine hydrochloride to obtain intermediate compound 3-1. MS (ESI, [M+H]⁺) *m*/*z*: 431.9.

### Step B: Preparation of compound 3

Referring to the preparation method of the step E in Example 1, compound 3-1 prepared in the step A above was added into the reaction solution of compound 1-4 for reaction to obtain compound 3.

¹H NMR (500 MHz, DMSO-*d*₆): δ10.22(s, 1H), 8.75(d, *J* = 2.0Hz, 1H), 8.02(d, *J* = 2.0Hz, 1H), 7.86(d, *J* = 9.0Hz, 2H), 7.75(d, *J* = 9.0Hz, 1H), 7.34(d, *J* = 9.0Hz, 2H), 6.42(d, *J* = 1.0Hz, 1H), 6.32(m, 1H), 3.89(t, *J* = 7.5Hz, 4H), 3.47(s, 3H), 2.22(m, 2H). HRMS (ESI, [M+H]⁺) *m*/*z*: 461.12323.

### Example 4: Preparation of Compound 4

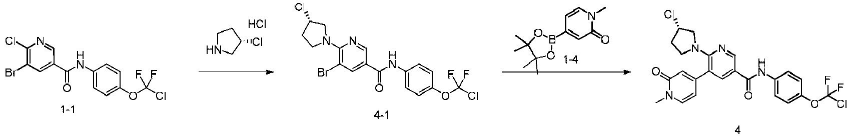

### Step A: Preparation of compound 4-1

Referring to the preparation method of the step B in Example 1, compound 1-1 was reacted with (S)-3-chloropyrrolidine hydrochloride to obtain intermediate compound 4-1. MS (ESI, [M+H]⁺) *m*/*z*: 480.00.

### Step B: Preparation of compound 4

Referring to the preparation method of the step E in Example 1, compound 4-1 prepared in the step A above was added into the reaction solution of compound 1-4 for reaction to obtain compound 4.

¹H NMR (500 MHz, DMSO-*d*₆): δ10.24(s, 1H), 8.77(d, *J* = 2.5Hz, 1H), 8.09(d, *J* = 2.5Hz, 1H), 7.87(m, 2H), 7.77(d, *J* = 7.0Hz, 1H), 7.34(d, *J* = 9.0Hz, 2H), 6.43(s, 1H), 6.30(dd, *J* = 2.0Hz, 7.0Hz, 1H), 4.80(t, *J* = 2.0Hz, 1H), 3.84(dd, *J* = 4.5Hz, 13.0Hz, 1H), 3.68(m, 1H), 3.50(s, 3H), 3.41(m, 2H), 2.34(m, 1H), 2.11(m, 1H). HRMS (ESI, [M+H]⁺) *m*/*z*: 509.10003.

### Example 5: Preparation of Compound 5

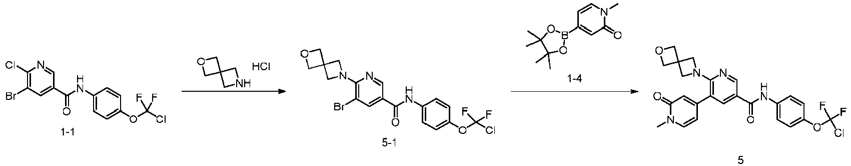

### Step A: Preparation of compound 5-1

Referring to the preparation method of the step B in Example 1, compound 1-1 was reacted with 2-oxa-6-azaspiro[3.3]heptane hydrochloride to obtain intermediate compound 5-1. MS (ESI, [M+H]⁺) *m*/*z*: 474.1.

### Step B: Preparation of compound 5

Referring to the preparation method of the step E in Example 1, compound 5-1 prepared in the step A above was added into the reaction solution of compound 1-4 for reaction to obtain compound 5.

¹H NMR (500 MHz, DMSO-*d*₆): δ10.22(s, 1H), 8.76(d, *J* = 2.5Hz, 1H), 8.03(d, *J* = 2.0Hz, 1H), 7.86(d, *J* = 9.5Hz, 2H), 7.76(d, *J* = 7.0Hz, 1H), 7.35(d, *J* = 9.0Hz, 2H), 6.42(d, *J* = 1.5Hz, 1H), 6.30(m, 1H), 4.64(s, 4H), 4.07(s, 4H), 3.49(s, 3H). HRMS (ESI, [M+H]⁺) *m*/*z*: 503.13412.

### Example 6: Preparation of Compound 6

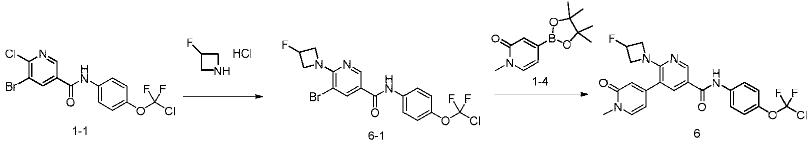

### Step A: Preparation of compound 6-1

Referring to the preparation method of the step B in Example 1, compound 1-1 was reacted with 3-fluoroazetidine hydrochloride to obtain intermediate compound 6-1. MS (ESI, [M+H]⁺) *m*/*z*: 450.0.

### Step B: Preparation of compound 6

Referring to the preparation method of the step E in Example 1, compound 6-1 prepared in the step A above was added into the reaction solution of compound 1-4 for reaction to obtain compound 6.

¹H NMR(500 MHz, DMSO-*d*₆) δ 10.27 (s, 1H), 8.78 (s, 1H), 8.09 (s, 1H), 7.86 (d, J = 9.1 Hz, 2H), 7.79 (d, J = 6.9 Hz, 1H), 7.35 (d, J = 9.0 Hz, 2H), 6.46 (s, 1H), 6.34 (d, J = 8.9 Hz, 1H), 5.38 (d, J = 55.1 Hz, 1H), 4.18-4.25 (m, 2H), 3.93-3.98 (m, 2H), 3.48 (s, 3H). HRMS (ESI, [M+H]⁺) *m*/*z*: 479.11256.

### Example 7: Preparation of Compound 7

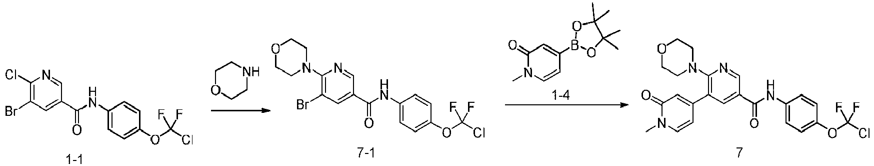

### Step A: Preparation of compound 7-1

Referring to the preparation method of the step B in Example 1, compound 1-1 was reacted with morpholine to obtain intermediate compound 7-1. MS (ESI, [M+H]⁺) *m*/*z:* 462.0.

### Step B: Preparation of compound 7

Referring to the preparation method of the step E in Example 1, compound 7-1 was added into the reaction solution of 1-4 for reaction to obtain 7.

¹H NMR (500 MHz, DMSO-*d*₆) δ (ppm) 10.37 (s, 1H), 8.80 (d, *J* = 2.3 Hz, 1H), 8.17 (d, *J* = 2.3 Hz, 1H), 7.91 - 7.85 (m, 2H), 7.78 (d, *J* = 7.0 Hz, 1H), 7.36 (d, *J* = 8.6 Hz, 2H), 6.68 (d, *J* = 1.9 Hz, 1H), 6.52 (dd, *J* = 7.0, 2.0 Hz, 1H), 3.67 - 3.58 (m, 4H), 3.47 (s, 3H), 3.28 (t, *J* = 4.6 Hz, 4H). MS (ESI, [M+H]⁺) *m*/*z*: 491.1.

### Example 8: Preparation of Compound 8

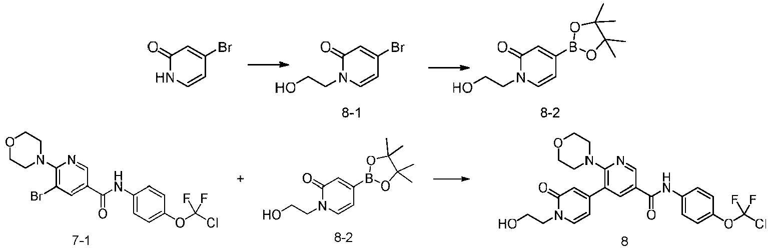

### Step A: Preparation of compound 8-1

Referring to the preparation method of the step C in Example 1, 4-bromopyridin-2(1*H*)-one was reacted with 2-iodoethan-1-ol to obtain intermediate compound 8-1.

¹H NMR (500 MHz, DMSO-*d*₆) δ 7.57 (d, J = 7.2 Hz, 1H), 6.70 (d, J = 2.1 Hz, 1H), 6.43 (d, J = 7.2 Hz, 1H), 4.88 (t, J = 5.4 Hz, 1H), 3.91 (t, J = 5.4 Hz, 2H), 3.59 (q, J = 5.4 Hz, 2H). MS (ESI, [M+H]⁺) *m*/*z*: 218.03.

### Step B: Preparation of compound 8-2

Referring to the preparation method of the step D in Example 1, compound 8-1 was reacted with bis(pinacolato)diboron to obtain a reaction solution of intermediate compound 8-2, which was directly used in the next step without separation and purification.

### Step C: Preparation of compound 8

Referring to the preparation method of the step E in Example 1, compound 7-1 was added into the reaction solution of compound 8-2 for reaction to obtain compound 8.

¹H NMR (500 MHz, DMSO-*d*₆) δ 10.33 (s, 1H), 8.79 (d, *J* = 2.2 Hz, 1H), 8.17 (d, *J* = 2.2 Hz, 1H), 7.87 (d, *J* = 9.0 Hz, 2H), 7.68 (d, *J* = 7.0 Hz, 1H), 7.36 (d, *J* = 8.8 Hz, 2H), 6.70 - 6.61 (m, 1H), 6.55 - 6.45 (m, 1H), 4.89 (t, *J* = 5.3 Hz, 1H), 3.98 (t, *J* = 5.4 Hz, 2H), 3.70 - 3.65 (m, 2H), 3.64 - 3.60 (m, 4H), 3.28 (t, *J* = 4.5 Hz, 4H). HRMS (ESI, [M+H]⁺) *m*/*z:* 521.1423.

### Example 9: Preparation of Compound 9

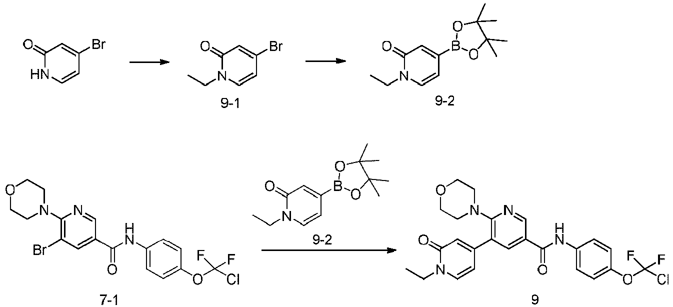

### Step A: Preparation of compound 9-1

To a 100-mL three-necked flask were added 4-bromopyridin-2(1*H*)-one (500 mg) and anhydrous tetrahydrofuran (10 mL) in sequence, the system was cooled to 0 °C in an ice bath, 60% sodium hydride (230 mg) was added in portions, and after the system was purged with nitrogen for 3 times, the system was heated to room temperature and stirred for 1 h. Iodoethane (896 mg) was slowly added into the reaction solution in an ice bath, and the reaction solution was heated to 70 °C in an oil bath and reacted for 6 h. Water (10 mL) was added into the reaction solution to quench the reaction, and ethyl acetate (15 mL) was added for extraction; the organic layer was separated, and the aqueous layer was extracted with ethyl acetate (15 mL × 2). The organic layers were combined, washed with saturated sodium chloride, and dried over anhydrous sodium sulfate. The solvent was distilled off under reduced pressure to obtain compound 9-1 (200 mg). MS (ESI, [M+H]⁺) *m*/*z:* 202.0.

### Step B: Preparation of compound 9-2

Referring to the preparation method of the step D in Example 1, compound 9-1 was reacted with bis(pinacolato)diboron to obtain intermediate compound 9-2, which was directly used in the next step without separation and purification.

### Step C: Preparation of compound 9

Referring to the preparation method of the step E in Example 1, compound 7-1 was added into the reaction solution of compound 9-2 for reaction to obtain compound 9.

¹H NMR (500 MHz, DMSO-*d*₆) δ 10.32 (s, 1H), 8.79 (d, *J* = 2.3 Hz, 1H), 8.17 (d, *J* = 2.4 Hz, 1H), 7.91 - 7.84 (m, 2H), 7.78 (d, *J* = 7.0 Hz, 1H), 7.36 (d, *J* = 8.7 Hz, 2H), 6.66 (d, *J* = 2.1 Hz, 1H), 6.53 (dd, *J* = 7.0, 2.0 Hz, 1H), 3.99 - 3.91 (m, 2H), 3.68 - 3.58 (m, 4H), 3.28 (t, *J* = 4.6 Hz, 4H), 1.24 (t, *J* = 7.1 Hz, 3H). MS (ESI, [M+H]⁺) *m*/*z:* 505.3.

### Example 10: Preparation of Compound 10

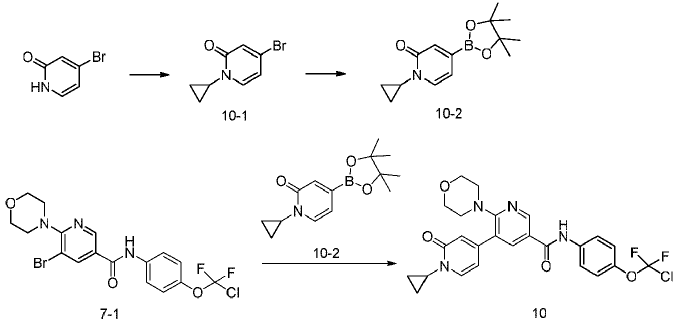

### Step A: Preparation of compound 10-1

To a 50-mL eggplant-shaped flask were added 4-bromopyridin-2(1*H*)-one 4-bromo-2-hydroxypyridine (500 mg), cyclopropylboronic acid (494 mg), pyridine (1137 mg), copper acetate (522 mg), cesium carbonate (936 mg) and toluene (10 mL) in sequence, and the system was heated to 95 °C in an oil bath in air and reacted overnight. The next day, the reaction solution was diluted with ethyl acetate (10 mL), and filtered through diatomite under vacuum, and the filtrate was washed twice with water and then washed with saturated sodium chloride, and dried over anhydrous sodium sulfate. The solvent was distilled off under reduced pressure, and the residue was separated and purified by silica gel column chromatography to obtain compound 10-1 (61 mg). MS (ESI, [M+H]⁺) *m*/*z:* 214.0.

### Step B: Preparation of compound 10-2

Referring to the preparation method of the step D in Example 1, compound 1-3 was replaced with compound 10-1 prepared in the above step, and the reaction solution of compound 10-2 was obtained after reaction and directly used in the next step.

### Step C: Preparation of compound 10

Referring to the preparation method of the step E in Example 1, compound 7-1 was added into the reaction solution of compound 10-2 for reaction to obtain compound 10.

¹H NMR (500 MHz, DMSO-*d*₆) δ 10.33 (s, 1H), 8.78 (s, 1H), 8.16 (s, 1H), 7.86 (d, *J* = 8.6 Hz, 2H), 7.61 (d, *J* = 7.2 Hz, 1H), 7.36 (d, *J* = 8.6 Hz, 2H), 6.65 (s, 1H), 6.48 (d, *J* = 7.3 Hz, 1H), 3.70 - 3.57 (m, 4H), 3.38 (s, 1H), 3.27 (s, 4H), 1.11 - 0.95 (m, 2H), 0.93 - 0.79 (m, 2H). HRMS (ESI, [M+H]⁺) *m*/*z*: 517.1451.

### Example 11: Preparation of Compound 11

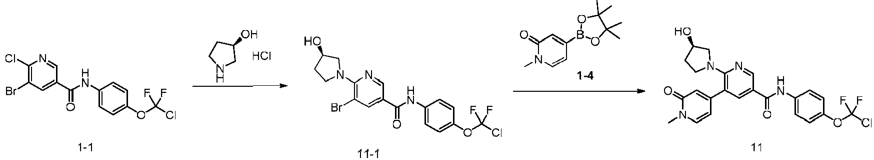

### Step A: Preparation of compound 11-1

Referring to the preparation method of the step B in Example 1, compound 1-1 was reacted with (*R*)-pyrrolidin-3-ol hydrochloride to obtain intermediate compound 11-1.

### Step B: Preparation of compound 11

Referring to the preparation method of the step E in Example 1, compound 11-1 prepared in the step A above was added into the reaction solution of compound 1-4 for reaction to obtain compound 11.

1H NMR (500 MHz, DMSO-*d*₆) δ 10.20 (s, 1H), 8.75 (s, 1H), 8.05 (d, *J* = 5.0 Hz, 1H), 8.86 (d, *J* = 10.0 Hz, 2H), 7.75 (d, *J* = 5.0 Hz, 1H), 7.34 (d, *J* = 10.0 Hz, 2H), 6.38 (s, 1H), 6.28-6.27 (m, 1H), 4.91 (s, 1H), 4.26 (s, 1H), 3.57-3.56 (m, 1H), 3.47 (s, 3H), 3.44-3.41 (m, 1H), 3.36-3.35 (m, 1H), 3.06 (d, *J* = 10.0 Hz, 1H) 1.92-1.85 (m, 1H), 1.80-1.77 (m, 1H). HRMS (ESI, [M+H]⁺) *m*/*z*: 491.1302.

### Example 12: Preparation of Compound 12

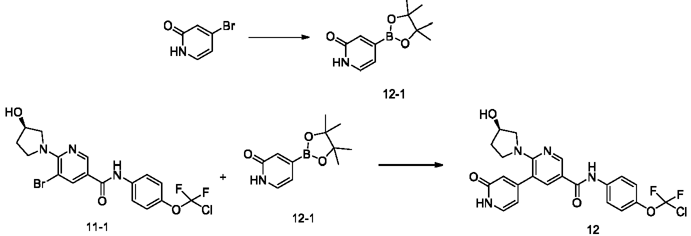

### Step A: Preparation of compound 12-1

Referring to the preparation method of the step D in Example 1, 4-bromopyridin-2(1*H*)-one was reacted with bis(pinacolato)diboron to obtain a reaction solution of intermediate compound 12-1, which was directly used in the next step.

### Step B: Preparation of compound 12

Referring to the preparation method of the step E in Example 1, compound 11-1 was added into the reaction solution of compound 12-1 for reaction to obtain compound 12.

¹H NMR (500 MHz, DMSO-*d*₆) δ 11.21 (br, 1H), 10.20 (s, 1H), 8.75 (s, 1H), 8.05 (s, 1H), 7.87-7.86 (m, 2H), 7.42 (d, *J* = 10.0 Hz, 1H), 7.34 (d, *J* = 10.0 Hz, 2H), 6.31 (s, 1H), 6.22 (d, *J* = 6.6 Hz, 1H), 4.92 (s, 1H), 4.26 (s, 1H), 3.60-3.54 (m, 1H), 3.50-3.43 (m, 1H), 3.42-3.40 (m, 1H), 3.06 (d, *J* = 10.0 Hz, 1H), 1.91-1.87 (m, 1H), 1.80-1.78 (m, 1H). HRMS (ESI, [M+H]⁺) *m*/*z*: 477.1139.

### Examples 13 and 14: Preparation of Compounds 13 and 14

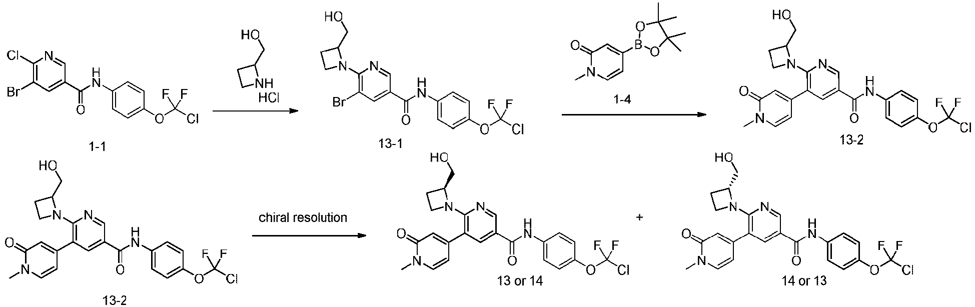

### Step A: Preparation of compound 13-1

Referring to the preparation method of the step B in Example 1, compound 1-1 was reacted with azetidin-2-ylmethanol hydrochloride to obtain compound 13-1. MS (ESI, [M-H]⁻) *m*/*z:* 460.0.

### Step B: Preparation of compound 13-2

Referring to the preparation method of the step E in Example 1, compound 13-1 prepared in the step A above was added into the reaction solution of compound 1-4 for reaction to obtain compound 13-2.

### Step C: Preparation of compounds 13 and 14

Compound 13-2 was resolved by a supercritical fluid chromatograph (model: waters SFC150G), where the separation column was CHIRALART Cellulose-SC column, the mobile phase was 25 vol% ethanol and 75 vol% carbon dioxide, and the flow rate was 60 mL/min, and compound 13 with t_{R} = 5.484 min was prepared;

¹H NMR (500 MHz, DMSO-*d*₆) δ 10.23 (s, 1H), 8.74 (d, 1H), 8.04 (d, *J* = 2.2 Hz, 1H), 7.86 (d, *J* = 9.1 Hz, 2H), 7.76 (d, *J* = 6.9 Hz, 1H), 7.35 (d, *J* = 8.9 Hz, 2H), 6.46 (s, 1H), 6.39 (dd, *J* = 6.9, 1.7 Hz, 1H), 4.88 (t, *J* = 5.8 Hz, 1H), 4.60-4.43 (m, 1H), 3.86-3.75 (m, 1H), 3.73-3.65 (m, 1H), 3.63-3.54 (m, 1H), 3.48 (s, 3H), 3.44-3.37 (m, 1H), 2.28-2.10 (m, 2H). HRMS (ESI, [M+H]⁺) *m*/*z*: 491.12806.

Also, compound 14 with t_{R} = 9.467 min was prepared.

¹H NMR (500 MHz, DMSO-*d*₆) δ 10.23 (s, 1H), 8.74 (d, 1H), 8.04 (d, *J* = 2.2 Hz, 1H), 7.86 (d, *J* = 9.1 Hz, 2H), 7.76 (d, *J* = 6.9 Hz, 1H), 7.35 (d, *J* = 8.9 Hz, 2H), 6.46 (s, 1H), 6.39 (dd, *J* = 6.9, 1.7 Hz, 1H), 4.88 (t, *J* = 5.8 Hz, 1H), 4.60 - 4.43 (m, 1H), 3.86 - 3.75 (m, 1H), 3.73 - 3.65 (m, 1H), 3.63 - 3.54 (m, 1H), 3.48 (s, 3H), 3.44 - 3.37 (m, 1H), 2.28 - 2.10 (m, 2H). HRMS (ESI, [M+H]⁺) *m*/*z*: 491.12806.

### Example 15: Preparation of Compound 15

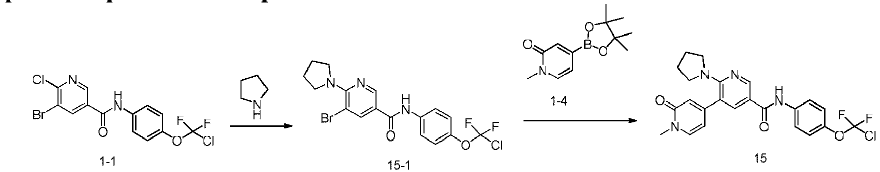

### Step A: Preparation of compound 15-1

Referring to the preparation method of the step B in Example 1, compound 1-1 was reacted with tetrahydropyrrole to obtain intermediate compound 15-1.

### Step B: Preparation of compound 15

Referring to the preparation method of the step E in Example 1, compound 15-1 prepared in the step A above was added into the reaction solution of compound 1-4 for reaction to obtain compound 15.

¹H NMR (500 MHz, DMSO-*d*₆) δ 10.19 (s, 1H), 8.75 (d, *J* = 2.1 Hz, 1H), 8.04 (d, *J* = 2.1 Hz, 1H), 7.86 (d, *J* = 9.0 Hz, 2H), 7.73 (d, *J* = 6.9 Hz, 1H), 7.34 (d, *J* = 8.8 Hz, 2H), 6.39 (d, *J* = 1.3 Hz, 1H), 6.32 - 6.21 (m, 1H), 3.47 (s, 3H), 3.31 (s, 4H), 1.82 (s, 4H). HRMS (ESI, [M+H]⁺) *m*/*z:* 475.13331.

### Example 16: Preparation of Compound 16

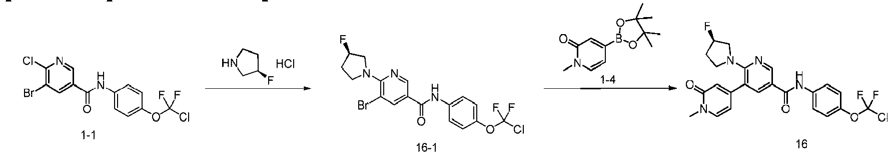

### Step A: Preparation of compound 16-1

Referring to the preparation method of the step B in Example 1, compound 1-1 was reacted with (R)-3-fluoropyrrolidine hydrochloride to obtain compound 16-1. MS (ESI, [M+H]⁺) *m*/*z*: 464.02.

### Step B: Preparation of compound 16

Referring to the preparation method of the step E in Example 1, compound 16-1 prepared in the step A above was added into the reaction solution of compound 1-4 for reaction to obtain compound 16.

¹H NMR (500 MHz, DMSO-*d*₆) δ 10.23 (s, 1H), 8.77 (d, *J* = 2.3 Hz, 1H), 8.08 (d, *J* = 2.3 Hz, 1H), 7.87 (d, *J* = 9.1 Hz, 2H), 7.76 (d, *J* = 6.9 Hz, 1H), 7.35 (d, *J* = 8.9 Hz, 2H), 6.43 (s, 1H), 6.29 (dt, *J* = 10.9, 5.4 Hz, 1H), 5.35 (d, *J* = 53.4 Hz, 1H), 3.76 - 3.62 (m, 1H), 3.54 (tt, *J* = 13.9, 6.9 Hz, 1H), 3.48 (s, 3H), 3.43 (dt, *J* = 19.2, 6.0 Hz, 2H), 2.22 - 1.95 (m, 2H). MS (ESI, [M+H]⁺) *m*/*z*: 493.29

### Example 17: Preparation of Compound 17

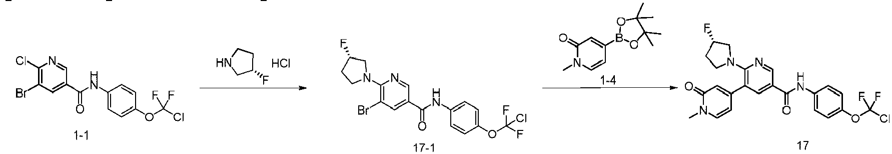

### Step A: Preparation of compound 17-1

Referring to the preparation method of the step B in Example 1, compound 1-1 was reacted with (*S*)-3-fluoropyrrolidine hydrochloride to obtain compound 17-1. MS (ESI, [M+H]⁺) *m*/*z:* 464.02.

### Step B: Preparation of compound 17

Referring to the preparation method of the step E in Example 1, compound 17-1 prepared in the step A above was added into the reaction solution of compound 1-4 for reaction to obtain compound 17.

¹H NMR (500 MHz, DMSO-*d*₆) δ 10.23 (s, 1H), 8.77 (d, *J* = 2.3 Hz, 1H), 8.08 (d, *J* = 2.3 Hz, 1H), 7.87 (d, *J* = 9.1 Hz, 2H), 7.76 (d, *J* = 6.9 Hz, 1H), 7.35 (d, *J* = 8.9 Hz, 2H), 6.43 (s, 1H), 6.29 (dt, *J* = 10.9, 5.4 Hz, 1H), 5.35 (d, *J* = 53.4 Hz, 1H), 3.76 - 3.62 (m, 1H), 3.54 (tt, *J* = 13.9, 6.9 Hz, 1H), 3.48 (s, 3H), 3.43 (dt, *J* = 19.2, 6.0 Hz, 2H), 2.22 - 1.95 (m, 2H). MS (ESI, [M+H]⁺) *m*/*z:* 493.20.

### Example 18: Preparation of Compound 18

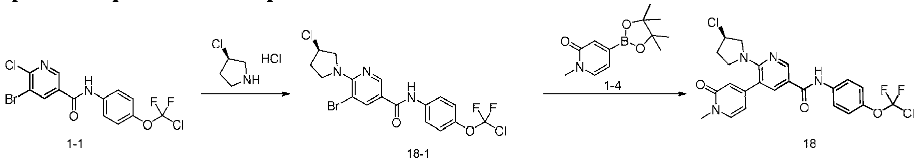

### Step A: Preparation of compound 18-1

Referring to the preparation method of the step B in Example 1, compound 1-1 was reacted with (*R*)-3-chloropyrrolidine hydrochloride to obtain compound 18-1. MS (ESI, [M+H]⁺) *m*/*z:* 479.98.

### Step B: Preparation of compound 18

Referring to the preparation method of the step E in Example 1, compound 18-1 prepared in the step A above was added into the reaction solution of compound 1-4 for reaction to obtain compound 18.

¹H NMR (500 MHz, DMSO-*d*₆) δ 10.24 (s, 1H), 8.81 (d, *J* = 2.0 Hz, 1H), 8.13 (d, *J* = 2.0 Hz, 1H), 7.92 (d, *J* = 9.0 Hz, 2H), 7.78 (d, *J* = 6.9 Hz, 1H), 7.34 (d, *J* = 8.7 Hz, 2H), 6.45 (s, 1H), 6.31 (d, *J* = 6.7 Hz, 1H), 4.80 (s, 1H), 3.90 (dd, *J* = 12.7, 4.3 Hz, 1H), 3.71 (dt, *J* = 38.1, 19.1 Hz, 1H), 3.51 (s, 3H), 3.46 (dd, *J* = 22.1, 11.0 Hz, 2H), 2.36 (dt, *J* = 13.2, 11.4 Hz, 1H), 2.15 - 1.97 (m, 1H). HRMS (ESI, [M+H]⁺) *m*/*z*: 509.09537.

### Example 19: Preparation of Compound 19

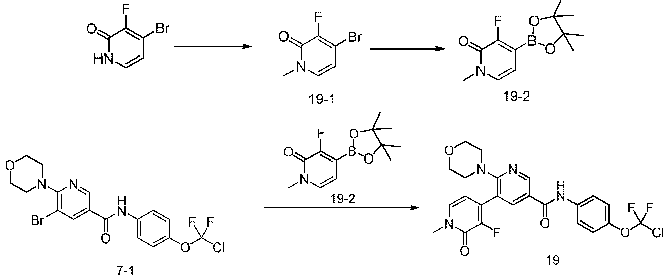

### Step A: Preparation of compound 19-1

Referring to the method of the step C in Example 1, 4-bromo-3-fluoropyridin-2(1*H*)-one was reacted with iodomethane to obtain compound 19-1. MS (ESI, [M+H]⁺) *m*/*z:* 208.02.

### Step B: Preparation of compound 19-2

Referring to the preparation method of the step D in Example 1, compound 19-1 was reacted with bis(pinacolato)diboron to obtain a reaction solution of compound 19-2, which was directly used in the next step without purification.

### Step C: Preparation of compound 19

Referring to the preparation method of the step E in Example 1, compound 7-1 was added into the reaction solution of compound 19-2 prepared in the step B above for reaction to obtain compound 19.

¹H NMR (500 MHz, DMSO-*d*₆) δ 10.31 (s, 1H), 8.83 (d, *J* = 2.3 Hz, 1H), 8.10 (dd, *J* = 24.6, 4.1 Hz, 2H), 7.86 (d, *J* = 9.1 Hz, 2H), 7.36 (d, *J* = 8.9 Hz, 2H), 6.63 (d, *J* = 7.1 Hz, 1H), 3.66 - 3.54 (m, 4H), 3.44 (d, *J* = 8.9 Hz, 3H), 3.39 - 3.34 (m, 4H). HRMS (ESI, [M+H]⁺) *m*/*z:* 509.12021.

### Example 20: Preparation of Compound 20

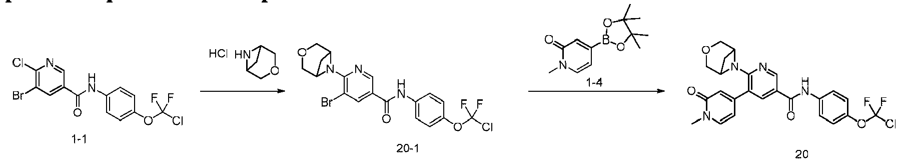

### Step A: Preparation of compound 20-1

Referring to the preparation method of the step B in Example 1, compound 1-1 was reacted with 3-oxa-6-azabicyclo[3.1.1]heptane hydrochloride to obtain compound 20-1. MS (ESI, [M+H]⁺) *m*/*z:* 474.02.

### Step B: Preparation of compound 20

Referring to the preparation method of the step E in Example 1, compound 20-1 prepared in the step A above was added into the reaction solution of compound 1-4 for reaction to obtain compound 20.

¹H NMR (500 MHz, DMSO-*d*₆) δ 10.27 (d, *J* = 25.5 Hz, 1H), 8.79 (d, *J* = 2.3 Hz, 1H), 8.05 (t, *J* = 5.5 Hz, 1H), 7.90 - 7.80 (m, 2H), 7.75 (d, *J* = 7.0 Hz, 1H), 7.35 (d, *J* = 9.0 Hz, 2H), 6.51 (d, *J* = 1.7 Hz, 1H), 6.36 (dd, *J* = 6.9, 1.9 Hz, 1H), 4.19 (s, 2H), 3.97 (s, 2H), 3.64 (d, *J* = 10.1 Hz, 2H), 3.45 (s, 3H), 2.66 (dd, *J* = 13.8, 6.6 Hz, 1H), 1.73 (d, *J* = 8.1 Hz, 1H). HRMS (ESI, [M+H]⁺) *m*/*z*: 503.12935.

### Example 21: Preparation of Compound 21

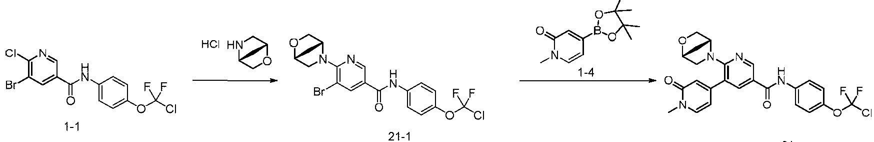

### Step A: Preparation of compound 21-1

Referring to the preparation method of the step B in Example 1, compound 1-1 was reacted with (1*R*,4*R*)-2-oxa-5-azabicyclo[2.2.1]heptane hydrochloride to obtain compound 21-1. MS (ESI, [M+H]⁺) *m*/*z:* 474.03.

### Step B: Preparation of compound 21

Referring to the preparation method of the step E in Example 1, compound 21-1 prepared in the step A above was added into the reaction solution of compound 1-4 for reaction to obtain compound 21.

¹H NMR (500 MHz, DMSO-*d*₆) δ 10.25 (s, 1H), 8.75 (s, 1H), 8.07 (s, 1H), 7.86 (d, *J* = 8.6 Hz, 2H), 7.73 (d, *J* = 6.7 Hz, 1H), 7.35 (d, *J* = 8.3 Hz, 2H), 6.51 - 6.22 (m, 2H), 4.92 (s, 1H), 4.53 (s, 1H), 3.77 (s, 2H), 3.46 (s, 3H), 3.30 (s, 1H), 2.80 (d, *J* = 10.1 Hz, 1H), 1.83 (q*, J* = 9.3 Hz, 2H). HRMS (ESI, [M+H]⁺) *m*/*z:* 503.12954.

### Example 22: Preparation of Compound 22

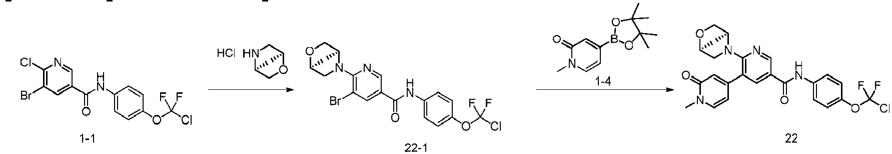

### Step A: Preparation of compound 22-1

Referring to the preparation method of the step B in Example 1, compound 1-1 was reacted with (1*S*,4*S*)-2-oxa-5-azabicyclo[2.2.1]heptane hydrochloride to obtain compound 22-1. MS (ESI, [M+H]⁺) *m*/*z:* 474.03.

### Step B: Preparation of compound 22

Referring to the preparation method of the step E in Example 1, compound 22-1 prepared in the step A above was added into the reaction solution of compound 1-4 for reaction to obtain compound 22.

¹H NMR (500 MHz, DMSO-*d*₆) δ 10.25 (s, 1H), 8.75 (d, *J* = 2.3 Hz, 1H), 8.07 (d, *J* = 2.3 Hz, 1H), 7.87 (t, *J* = 9.1 Hz, 2H), 7.73 (d, *J* = 6.9 Hz, 1H), 7.35 (d, *J* = 8.9 Hz, 2H), 6.45 (s, 1H), 6.31 (dd, *J* = 6.9, 1.5 Hz, 1H), 4.92 (s, 1H), 4.53 (s, 1H), 3.77 (s, 2H), 3.47 (d, *J* = 9.4 Hz, 3H), 3.30 (d, *J* = 3.4 Hz, 1H), 2.80 (d, *J* = 10.2 Hz, 1H), 1.91 - 1.70 (m, 2H). HRMS (ESI, [M+H]⁺) *m*/*z:* 503.12949.

### Example 23: Preparation of Compound 23

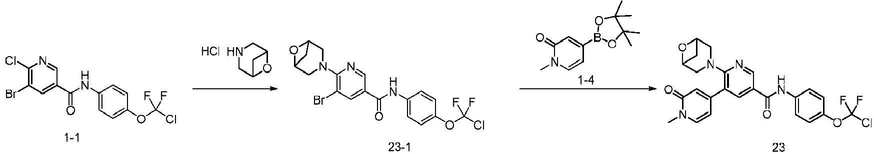

### Step A: Preparation of compound 23-1

Referring to the preparation method of the step B in Example 1, compound 1-1 was reacted with 6-oxa-3-aza-bicyclo[3,1,1]heptadecane hydrochloride to obtain compound 23-1.

MS (ESI, [M+H]⁺) *m*/*z:* 474.06.

### Step B: Preparation of compound 23

Referring to the preparation method of the step E in Example 1, compound 23-1 prepared in the step A above was added into the reaction solution of compound 1-4 for reaction to obtain compound 23.

¹H NMR (500 MHz, DMSO-*d*₆) δ 10.26 (s, 1H), 8.79 (d, *J* = 2.3 Hz, 1H), 8.08 (d, *J* = 2.3 Hz, 1H), 7.96 - 7.79 (m, 2H), 7.74 (d, *J* = 7.0 Hz, 1H), 7.35 (d, *J* = 9.0 Hz, 2H), 6.52 (d, *J* = 1.8 Hz, 1H), 6.34 (dd, *J* = 6.9, 2.0 Hz, 1H), 4.57 (d, *J* = 6.3 Hz, 2H), 3.80 (d, *J* = 12.8 Hz, 2H), 3.60 (d, *J* = 12.7 Hz, 2H), 3.46 (s, 3H), 3.07 - 2.93 (m, 1H), 1.79 (d, *J* = 8.6 Hz, 1H). HRMS (ESI, [M+H]⁺) *m*/*z*: 503.12984.

### Example 24: Preparation of Compound 24

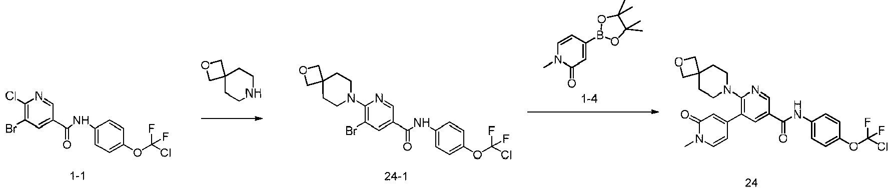

### Step A: Preparation of compound 24-1

Referring to the preparation method of the step B in Example 1, compound 1-1 and 2-oxa-7-azaspiro[3.5]nonane were taken as starting materials to prepare compound 24-1. MS (ESI, [M+H]⁺) *m*/*z*: 502.10.

### Step B: Preparation of compound 24

Referring to the preparation method of the step E in Example 1, compound 24-1 prepared in the step A above was added into the reaction solution of compound 1-4 for reaction to obtain compound 24.

¹H NMR (500 MHz, DMSO-*d*₆) δ 10.29 (s, 1H), 8.76 (d, *J* = 2.3 Hz, 1H), 8.12 (d, *J* = 2.3 Hz, 1H), 7.86 (d, *J* = 9.1 Hz, 2H), 7.76 (d, *J* = 7.0 Hz, 1H), 7.35 (d, *J* = 8.8 Hz, 2H), 6.65 (d, *J* = 1.6 Hz, 1H), 6.45 (dd, *J* = 7.0, 1.8 Hz, 1H), 4.31 (s, 4H), 3.47 (s, 3H), 3.26 - 3.17 (m, 4H), 1.84 - 1.76 (m, 4H). HRMS (ESI, [M+H]⁺) *m*/*z:* 531.1332.

### Example 25: Preparation of Compound 25

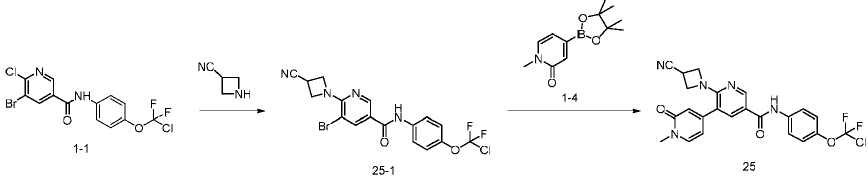

### Step A: Preparation of compound 25-1

Referring to the preparation method of the step B in Example 1, compound 1-1 and 3-acetonitrile cyclobutylamine were taken as starting materials to prepare compound 25-1. MS (ESI, [M+H]⁺) *m*/*z*: 457.02.

### Step B: Preparation of compound 25

Referring to the preparation method of the step E in Example 1, compound 25-1 prepared in the step A above was added into the reaction solution of compound 1-4 for reaction to obtain compound 25.

¹H NMR (500 MHz, DMSO-*d*₆) δ 10.29 (s, 1H), 8.79 (d, J = 2.2 Hz, 1H), 8.09 (d, J = 2.3 Hz, 1H), 7.86 (d, J = 9.1 Hz, 2H), 7.80 (d, J = 6.9 Hz, 1H), 7.35 (d, J = 9.0 Hz, 2H), 6.46 (d, J = 1.8 Hz, 1H), 6.34 (dd, J = 6.9, 1.9 Hz, 1H), 4.17 (t, J = 8.9 Hz, 2H), 4.03 (dd, J = 8.8, 5.9 Hz, 2H), 3.77 (tt, J = 9.0, 5.9 Hz, 1H), 3.48 (s, 3H). HRMS (ESI, [M+H]⁺) *m*/*z*: 486.1140.

### Example 26: Preparation of Compound 26

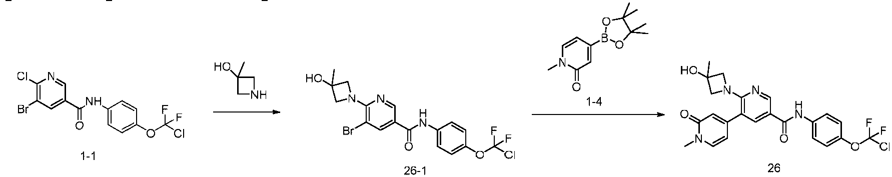

### Step A: Preparation of compound 26-1

Referring to the preparation method of the step B in Example 1, compound 1-1 and 3-methyl-3-acridinol were taken as starting materials to prepare compound 26-1. MS (ESI, [M+H]⁺) *m*/*z:* 462.02.

### Step B: Preparation of compound 26

Referring to the preparation method of the step E in Example 1, compound 26-1 prepared in the step A above was added into the reaction solution of compound 1-4 for reaction to obtain compound 26.

¹H NMR (500 MHz, DMSO-*d*₆) δ 10.22 (s, 1H), 8.76 (d, J = 2.3 Hz, 1H), 8.04 (d, J = 2.3 Hz, 1H), 7.90 - 7.83 (m, 2H), 7.77 (d, J = 6.9 Hz, 1H), 7.35 (d, J = 9.0 Hz, 2H), 6.43 (d, J = 1.9 Hz, 1H), 6.32 (dd, J = 6.9, 2.0 Hz, 1H), 5.51 (s, 1H), 3.75 (s, 4H), 3.48 (s, 3H), 1.34 (s, 3H). HRMS (ESI, [M+H]⁺) *m*/*z:* 491.1298.

### Example 27: Preparation of Compound 27

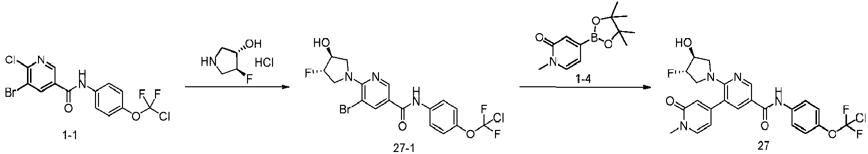

### Step A: Preparation of compound 27-1

Referring to the preparation method of the step B in Example 1, compound 1-1 was reacted with (3*S*,4*S*)-4-fluoropyrrolidin-3-ol hydrochloride to obtain compound 27-1. MS (ESI, [M+H]⁺) *m*/*z*: 480.02.

### Step B: Preparation of compound 27

Referring to the preparation method of the step E in Example 1, compound 27-1 prepared in the step A above was added into the reaction solution of compound 1-4 for reaction to obtain compound 27.

¹H NMR (500 MHz, DMSO-*d*₆) δ 10.23 (s, 1H), 8.83 - 8.70 (m, 1H), 8.14 - 8.04 (m, 1H), 7.86 (d, J = 8.9 Hz, 2H), 7.77 (d, J = 6.9 Hz, 1H), 7.34 (d, J = 8.6 Hz, 2H), 6.43 (s, 1H), 6.28 (d, J = 6.4 Hz, 1H), 5.49 (d, J = 3.1 Hz, 1H), 4.98 (d, J = 51.4 Hz, 1H), 4.23 (s, 1H), 3.83 (dd, J = 42.0, 13.4 Hz, 1H), 3.62 (d, J = 11.7 Hz, 1H), 3.54 - 3.39 (m, 4H), 3.18 (d, J = 12.0 Hz, 1H). HRMS (ESI, [M+H]⁺) *m*/*z:* 509.1248.

### Example 28: Preparation of Compound 28

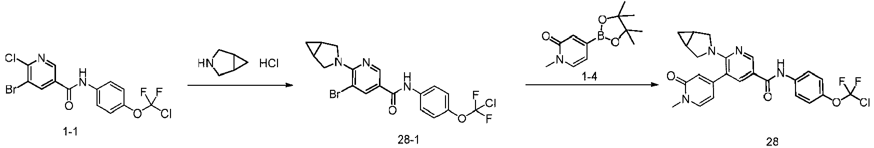

### Step A: Preparation of compound 28-1

Referring to the preparation method of the step B in Example 1, compound 1-1 was reacted with 3-azabicyclo[3.1.0]hexane hydrochloride to obtain compound 28-1. MS (ESI, [M+H]⁺) *m*/*z:* 458.06.

### Step B: Preparation of compound 28

Referring to the preparation method of the step E in Example 1, compound 28-1 prepared in the step A above was added into the reaction solution of compound 1-4 for reaction to obtain compound 28.

¹H NMR (500 MHz, DMSO-*d*₆) δ 10.19 (s, 1H), 8.71 (d, *J* = 2.3 Hz, 1H), 8.01 (d, *J* = 2.3 Hz, 1H), 7.85 (d, *J* = 9.1 Hz, 2H), 7.73 (d, *J* = 6.9 Hz, 1H), 7.34 (d, *J* = 8.9 Hz, 2H), 6.43 (d, *J* = 1.8 Hz, 1H), 6.25 (dd, *J* = 6.9, 1.9 Hz, 1H), 3.67 (d, *J* = 10.9 Hz, 2H), 3.47 (s, 3H), 3.34 (d, *J* = 11.0 Hz, 2H), 1.60 - 1.50 (m, 2H), 0.58 (td, *J* = 7.6, 4.6 Hz, 1H), 0.05 (q, *J* = 4.0 Hz, 1H). HRMS (ESI, [M+H]⁺) *m*/*z*: 487.1385.

### Example 29: Preparation of Compound 29

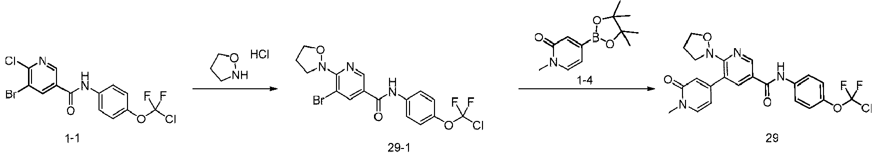

### Step A: Preparation of compound 29-1

Referring to the preparation method of the step B in Example 1, compound 1-1 was reacted with isoxazolidine hydrochloride to obtain compound 29-1. MS (ESI, [M+H]⁺) *m*/*z*: 448.03.

### Step B: Preparation of compound 29

To the reaction solution of compound 1-4 obtained in the step D of Example 1 were added compound 29-1 (500 mg) obtained in the step A above, potassium phosphate (710 mg), deionized water (3 mL), and dichloro[1,1'-bis(di-*tert*-butylphosphine)ferrocene palladium(II) (73 mg) in sequence; after the addition was completed, the system was purged with nitrogen, and reacted at room temperature for 6 h. The system was filtered, the mother liquor was collected, ethyl acetate (30 mL) was added, and the mixture was stirred and washed, followed by liquid separation, to obtain an organic phase; a saturated sodium chloride aqueous solution (30 mL) was added, and the mixture was stirred and washed, followed by liquid separation and purification by silica gel column chromatography, to obtain 280 mg of compound 29.

¹H NMR (500 MHz, DMSO-*d*₆) δ 10.42 (s, 1H), 8.81 (d, *J* = 2.2 Hz, 1H), 8.18 (d, *J* = 2.2 Hz, 1H), 7.86 (d, *J* = 9.1 Hz, 2H), 7.67 (d, *J* = 7.0 Hz, 1H), 7.36 (d, *J* = 9.0 Hz, 2H), 6.54 (d, *J* = 1.8 Hz, 1H), 6.42 (dd, *J* = 7.0, 1.9 Hz, 1H), 3.78 - 3.73 (m, 2H), 3.68 (t, *J* = 7.3 Hz, 2H), 3.45 (s, 3H), 2.13 (p, *J* = 7.3 Hz, 2H). HRMS (ESI, [M+H]⁺) *m*/*z:* 477.1177.

### Example 30: Preparation of Compound 30

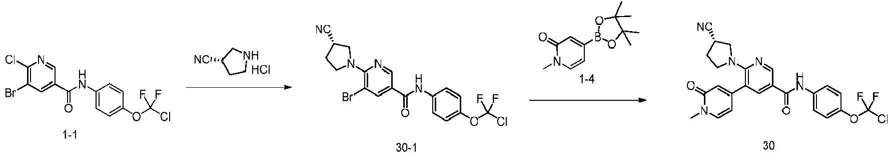

### Step A: Preparation of compound 30-1

Referring to the preparation method of the step B in Example 1, compound 1-1 was reacted with (*S*)-pyrrolidine-3-carbonitrile hydrochloride to obtain compound 30-1. MS (ESI, [M+H]⁺) *m*/*z:* 471.1.

### Step B: Preparation of compound 30

Referring to the preparation method of the step E in Example 1, compound 30-1 prepared in the step A above was added into the reaction solution of compound 1-4 for reaction to obtain compound 30.

¹H NMR (500 MHz, DMSO-*d*₆) δ 10.25 (s, 1H), 8.78 (d, *J* = 1.9 Hz, 1H), 8.16 - 8.03 (m, 1H), 7.87 (d, *J* = 8.9 Hz, 2H), 7.77 (d, *J* = 6.9 Hz, 1H), 7.35 (d, *J* = 8.7 Hz, 2H), 6.44 (s, 1H), 6.30 (dd, *J* = 6.9, 2.1 Hz, 1H), 3.73 - 3.64 (m, 1H), 3.59 - 3.52 (m, 1H), 3.48 (s, 3H), 3.47 - 3.42 (m, 2H), 3.42 - 3.36 (m, 1H), 2.27 - 2.19 (m, 1H), 2.19 - 2.09 (m, 1H). HRMS (ESI, [M+H]⁺) *m*/*z:* 500.1312.

### Example 31: Preparation of Compound 31

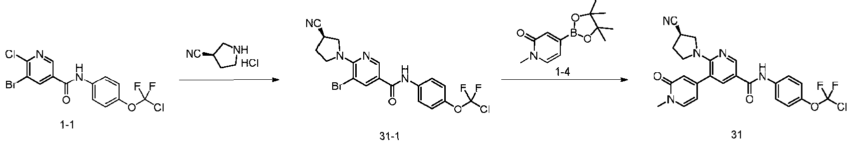

### Step A: Preparation of compound 31-1

Referring to the preparation method of the step B in Example 1, compound 1-1 was reacted with (*R*)-3-fluoropyrrolidine hydrochloride to obtain compound 31-1. MS (ESI, [M+H]⁺) *m*/*z:* 471.1.

### Step B: Preparation of compound 31

Referring to the preparation method of the step E in Example 1, compound 31-1 prepared in the step A above was added into the reaction solution of compound 1-4 for reaction to obtain 31.

¹H NMR (500 MHz, DMSO-*d*₆) δ 10.25 (s, 1H), 8.78 (d, J = 2.2 Hz, 1H), 8.10 (d, J = 2.2 Hz, 1H), 7.87 (d, J = 9.0 Hz, 2H), 7.77 (d, J = 6.9 Hz, 1H), 7.35 (d, J = 8.8 Hz, 2H), 6.44 (d, J = 1.5 Hz, 1H), 6.30 (dd, J = 6.9, 1.8 Hz, 1H), 3.71 - 3.64 (m, 1H), 3.59 - 3.52 (m, 1H),3.48 (s, 3H), 3.47 - 3.43 (m, 2H), 3.42 - 3.36 (m, 1H), 2.29 - 2.20 (m, 1H), 2.19-2.10 (m, 1H). HRMS (ESI, [M+H]⁺) *m*/*z:* 500.1321.

### Example 32: Preparation of Compound 32

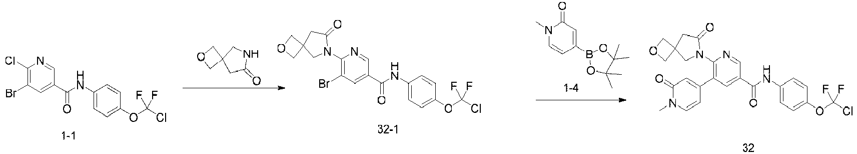

### Step A: Preparation of compound 32-1

To a 35-mL microwave tube were added dioxane (15 mL), compound 1-1 (1 g), 2-oxa-6-azaspiro[3.4]octan-7-one (0.309 g), 4,5-bis diphenylphosphino-9,9-dimethylxanthene (0.140 g), tris(dibenzylideneacetone)dipalladium(0) (0.111 g) and tripotassium phosphate (1.030 g) in sequence, and the system was placed into a microwave reactor under nitrogen atmosphere, and heated to 140 °C and reacted for 4 h. The reaction solution was cooled to room temperature and then filtered, and the mother liquor was collected and purified by silica gel column chromatography to obtain 220 mg of compound 32-1. MS (ESI, [M+H]⁺) *m*/*z*: 501.99.

### Step B: Preparation of compound 32

Referring to the preparation method of the step E in Example 1, compound 32-1 prepared in the step A above was added into the reaction solution of compound 1-4 for reaction to obtain compound 32.

¹H NMR (500 MHz, DMSO-*d*₆) δ 10.62 (s, 1H), 8.99 (d, J = 2.3 Hz, 1H), 8.38 (d, J = 2.3 Hz, 1H), 7.88 (d, J = 9.0 Hz, 2H), 7.69 (d, J = 7.0 Hz, 1H), 7.39 (d, J = 8.5 Hz, 2H), 6.54 (d, J = 2.1 Hz, 1H), 6.23 (dd, J = 7.1, 2.1 Hz, 1H), 4.71 (d, J = 6.1 Hz, 2H), 4.57 (d, J = 6.1 Hz, 2H), 4.35 (s, 2H), 3.44 (s, 3H), 2.74 (s, 2H). MS (ESI, [M+H]⁺) *m*/*z*: 531.12415.

### Example 33: Preparation of Compound 33

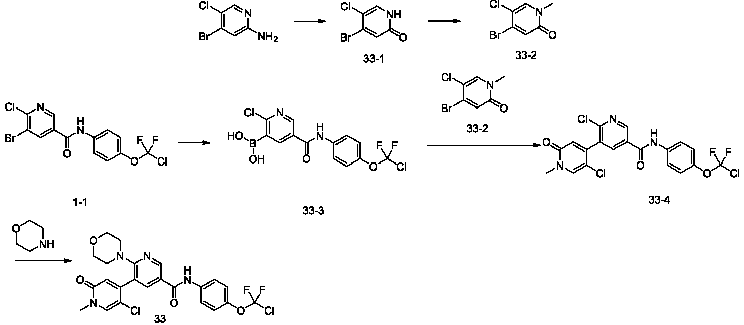

### Step A: Preparation of compound 33-1

To a 25-mL single-necked flask were added purified water (8 mL) and 4-bromo-5-chloropyridine-2-amino (500 mg) in sequence, the system was stirred and cooled to 0-5 °C, and a 98% w/w concentrated sulfuric acid aqueous solution (1 mL) was added slowly and dropwise into the system; after the addition was completed, the system was stirred for 5 min, and then a sodium nitrite aqueous solution (1 mL, 319 mg/mL) was added dropwise into the system; the temperature was maintained for reaction for 30 min, followed by filtering; the filter cake was rinsed with purified water (10 mL × 2), collected, and placed into a vacuum drying oven to be dried at 50 °C to constant weight to obtain 420 mg of compound 33-1. MS (ESI, [M-H]⁻) *m*/*z*: 205.9.

### Step B: Preparation of compound 33-2

To a 25-mL single-necked flask were added *N,N*-dimethylformamide (5 mL), compound 33-1 (200 mg) obtained in the step A above, and anhydrous potassium carbonate (199 mg) in sequence, the system was stirred and cooled to 0-5 °C, and an iodomethane (1 mL, 136 mg) solution of anhydrous *N,N*-dimethylformamide was added slowly and dropwise into the system; after the addition was completed, the temperature was maintained for reaction for 2 h; dichloromethane (20 mL) and purified water (10 mL) were added into the above system, followed by stirring and liquid separation to obtain an organic phase, which was dried over anhydrous sodium sulfate, filtered, and concentrated under reduced pressure to obtain 160 mg of compound 33-2. MS (ESI, [M+H]⁺) *m*/*z:* 222.0.

### Step C: Preparation of compound 33-3

To a 35-mL microwave tube were added 1,4-dioxane (10 mL), compound 1-1 (500 mg), bis(pinacolato)diboron (367 mg), potassium acetate (236 mg) and [1,1'-bis(diphenylphosphino)ferrocene]palladium dichloride (88 mg) in sequence, after the addition was completed, the system was purged with nitrogen, and heated to 140 °C and reacted for 2 h, the reaction was stopped, the system was cooled to room temperature, and the obtained reaction solution was directly used in the next step without separation and purification.

### Step D: Preparation of compound 33-4

To the reaction solution obtained in the step C above were added compound 33-2 (282 mg), tripotassium phosphate (639 mg), dichloro[1,1'-bis(di-*tert*-butylphosphine)ferrocene palladium(II) (78 mg) and purified water (2 mL) in sequence, and after the addition was completed, the system was purged with nitrogen and reacted at room temperature for 4 h; ethyl acetate (20 mL) was added into the system, followed by stirring, washing and liquid separation, an organic phase was collected, dried over anhydrous sodium sulfate, filtered under vacuum, and concentrated under reduced pressure to obtain a residue, and the residue was purified by silica gel column chromatography to obtain 300 mg of compound 33-4. MS (ESI, [M-H]⁻) *m*/*z:* 472.0.

### Step E: Preparation of compound 33

To a 35-mL microwave tube were added compound 33-4 (200 mg) obtained in step D above, morpholine (44.1 mg), *N,N*-diisopropylethylamine (82 mg) and *N*-methylpyrrolidone (5 mL) in sequence, the liquid level of the system was purged with nitrogen for a moment, and then the tube was sealed; the tube was heated to 160 °C by using a microwave reactor and reacted for 2 h; after the reaction solution was cooled, ethyl acetate (10 mL) was added into the system for dilution, water (10 mL) was added, followed by stirring, washing and liquid separation, an organic layer was collected, dried over anhydrous sodium sulfate, filtered under vacuum, and concentrated under reduced pressure to obtain a residue, and the residue was purified by silica gel column chromatography to obtain 20 mg of compound 33.

¹H NMR (500 MHz, DMSO-*d*₆) δ 10.29 (s, 1H), 8.82 (d, *J* = 2.4 Hz, 1H), 8.16 (s, 1H), 8.02 (d, *J* = 2.3 Hz, 1H), 7.90 - 7.81 (m, 2H), 7.35 (d, *J* = 8.7 Hz, 2H), 6.64 (s, 1H), 3.66 - 3.53 (m, 4H),3.46 - 3.36 (m, 4H), 3.48 (s, 3H). MS (ESI, [M-H]⁻) *m*/*z:* 523.1.

### Example 34: Preparation of Compound 34

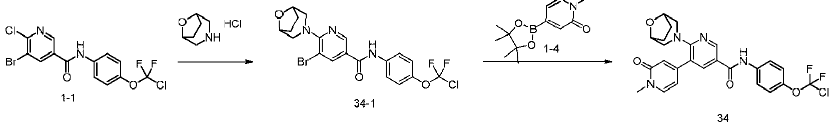

### Step A: Preparation of compound 4-1

Referring to the preparation method of the step B in Example 1, compound 1-1 was reacted with 8-oxa-3-azabicyclo[3.2.1]octane hydrochloride to obtain intermediate compound 34-1. MS (ESI, [M+H]⁺) *m*/*z*: 488.04.

### Step B: Preparation of compound 34

Referring to the preparation method of the step E in Example 1, compound 34-1 prepared in the step A above was added into the reaction solution of compound 1-4 for reaction to obtain compound 34.

¹H NMR (500 MHz, DMSO-*d*₆) δ 10.31 (s, 1H), 8.77 (d, *J* = 2.3 Hz, 1H), 8.10 (d, *J* = 2.3 Hz, 1H), 7.86 (d, *J* = 9.1 Hz, 2H), 7.80 (d, *J* = 7.0 Hz, 1H), 7.35 (d, *J* = 8.9 Hz, 2H), 6.57 (d, *J* = 1.6 Hz, 1H), 6.37 (m, *J* = 6.9, 1.8 Hz, 1H), 4.29 (s, 2H), 3.48 (d, *J* = 10.9 Hz, 5H), 3.04 (d, *J* = 11.4 Hz, 2H), 1.76 (m, *J* = 11.0, 8.0 Hz, 4H). HRMS (ESI, [M-H]⁻) *m*/*z*: 517.1452.

### Example 35: Preparation of Compound 35

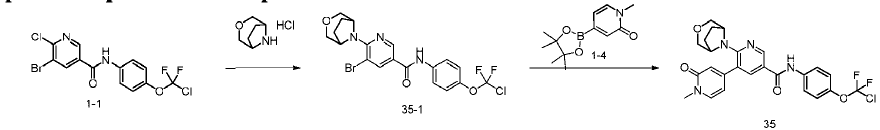

### Step A: Preparation of compound 35-1

Referring to the preparation method of the step B in Example 1, compound 1-1 was reacted with 3-oxa-8-azabicyclo[3.2.1]octane hydrochloride to obtain intermediate compound 35-1. MS (ESI, [M+H]⁺) *m*/*z*: 488.1.

### Step B: Preparation of compound 35

Referring to the preparation method of the step E in Example 1, compound 35-1 prepared in the step A above was added into the reaction solution of compound 1-4 for reaction to obtain compound 35.

¹H NMR (500 MHz, DMSO-*d*₆) δ 10.31 (s, 1H), 8.76 (d, *J* = 2.2 Hz, 1H), 8.11 (d, *J* = 2.2 Hz, 1H), 7.86 (d, *J* = 9.0 Hz, 2H), 7.77 (d, *J* = 7.0 Hz, 1H), 7.35 (d, *J* = 8.8 Hz, 2H), 6.64 (d, *J* = 1.5 Hz, 1H), 6.55 - 6.41 (m, 1H), 4.19 (s, 2H), 3.66 (d, *J* = 10.6 Hz, 2H), 3.55 - 3.41 (m, 5H), 1.98 - 1.64 (m, 4H). HRMS (ESI, [M+H]⁺) *m*/*z:* 517.1452.

### Example 36: Preparation of Compound 36

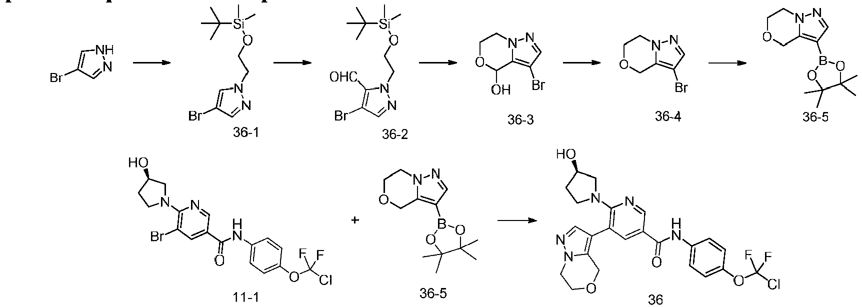

### Step A: Preparation of compound 36-1

To a 250-mL three-necked flask were added acetonitrile (80 mL), 4-bromo-1*H*-pyrazole (10 g) and cesium carbonate (44.3 g) in sequence, the reaction system was transferred to an ice salt bath and cooled to 0 °C under nitrogen atmosphere, and (2-bromoethoxy)(*tert*-butyl)dimethylsilane (19.53 g) was dissolved in acetonitrile (40 mL); the resulting solution was added slowly and dropwise into the reaction system, and after the addition was completed, the reaction system was naturally heated, and stirred at room temperature overnight. Ethyl acetate (50 mL) and water (20 mL) were added into the reaction system, followed by liquid separation, and an organic phase was collected, dried over anhydrous sodium sulfate, filtered under vacuum, and concentrated under reduced pressure; the residue was purified by silica gel column chromatography to obtain 13 g of compound 36-1.

¹H NMR (500 MHz, DMSO-*d*₆): δ7.98(s, 1H), 7.62(s, 1H), 4.27(t, *J* = 5.0Hz, 2H), 3.96(t, *J* = 5.0Hz, 2H), 0.89(s, 9H), 0.00(s, 6H). MS (ESI, [M-(CH₃)₃C]⁺) *m*/*z*: 247.

### Step B: Preparation of compound 36-2

To a 250-mL three-necked flask were added diisopropylamine (3.98 g) and anhydrous tetrahydrofuran (20 mL) in sequence under nitrogen atmosphere. The reaction system was transferred to a low-temperature tank at -80 °C, and 1.6 M *n*-hexane solution of *n*-butyllithium (23 mL) was added dropwise into the reaction system; after the addition was completed, the system was stirred at -80 °C for 30 min. Compound 36-1 (7.5 g) was dissolved in anhydrous tetrahydrofuran (15 mL), and the resulting solution was added slowly and dropwise into the above reaction system; after the addition was completed, the system was stirred at -80 °C for 30 min. *N,N*-dimethylformamide (3.23 g) was dissolved in anhydrous tetrahydrofuran (8 mL), and the resulting solution was added slowly and dropwise into the above reaction system; after the addition was completed, the reaction system was stirred at -80 °C for 3 h. Isopropanol (5 mL) was added into the reaction system to quench the reaction, and the reaction system was transferred to reach room temperature with stirring. A saturated ammonium chloride solution (40 mL) and ethyl acetate (100 mL) were added into the reaction solution, followed by liquid separation, and an organic phase was collected, dried over anhydrous sodium sulfate, filtered under vacuum, and concentrated under reduced pressure; the residue was purified by silica gel column chromatography to obtain 6 g of compound 36-2. MS (ESI, [M+H]⁺) *m*/*z*: 333.1.

### Step C: Preparation of compound 36-3

To a 100-mL single-necked flask were added compound 36-2 (4.8 g), 2-methyltetrahydrofuran (10 mL), water (10 mL), and trifluoroacetic acid (18 mL) in sequence, and the system was stirred at room temperature for 30 min. The pH of the system was adjusted to neutral with a saturated sodium bicarbonate aqueous solution, and a large amount of gas was generated. Dichloromethane (100 mL) was added into the reaction system, followed by liquid separation, and an organic phase was collected, dried over anhydrous sodium sulfate, filtered under vacuum, and concentrated under reduced pressure to obtain 3.8 g of compound 36-3. GCMS (EI, [M-*e*]⁺) *m*/*z:* 218.

### Step D: Preparation of compound 36-4

To a 250-mL three-necked flask were added dichloromethane (90 mL) and compound 36-3 (3.8 g) in sequence, the reaction system was cooled to 0 °C in an ice salt bath, triethylsilane (6.05 g) and trifluoroacetic acid (11.87 g) were added dropwise in sequence through a dropping funnel, and the reaction system was stirred at room temperature overnight. The pH of the system was adjusted to neutral with a saturated sodium bicarbonate aqueous solution. Dichloromethane (100 mL) was added into the reaction system, followed by liquid separation, and an organic phase was collected, dried over anhydrous sodium sulfate, filtered under vacuum, and concentrated under reduced pressure; the residue was purified by silica gel column chromatography to obtain 2.4 g of compound 36-4.

¹H NMR (500 MHz, DMSO-*d*₆): δ7.57(s, 1H), 4.71(s, 2H), 4.08(m, 4H). GCMS (El, [M-*e*]⁺) *m*/*z:* 202.

### Step E: Preparation of compound 36-5

To a 25-mL single-necked flask were added 1,4-dioxane (10 mL), compound 36-4 (165 mg), bis(pinacolato)diboron (247 mg), potassium acetate (159 mg) and a [1,1'-bis(diphenylphosphino)ferrocene]palladium dichloride dichloromethane complex (40 mg) in sequence; after the addition was completed, the system was purged with nitrogen and then heated to 90 °C and reacted for 2 h, the reaction was stopped, and the system was cooled to room temperature, which was directly used in the next step without separation and purification.

### Step F: Preparation of compound 36

The reaction solution obtained in the step E above was transferred into a 35 mL microwave tube, compound 11-1 (250 mg), potassium carbonate (224 mg), tetrakis(triphenylphosphine)palladium (62.4 mg) and water (2.5 mL) were added into the microwave tube in sequence, and after the addition was completed, the microwave tube was sealed and placed into a microwave reactor under nitrogen atmosphere, where the microwave reaction conditions were as follows: 150 °C and 1 h. The reaction solution was filtered through diatomite, the filtrate was collected, and ethyl acetate (50 mL) and saturated brine (25 mL) were added into the filtrate for washing, followed by liquid separation, and an organic phase was collected and purified by silica gel column chromatography to obtain 40 mg of compound 36.

¹H NMR (500 MHz, DMSO-*d*₆): δ10.14(s, 1H), 8.68(s, 1H), 7.90(m, 3H), 7.59(s, 1H), 7.33(s, 2H), 4.85(s, 1H), 4.62(m, 2H), 4.14(m, 5H), 3.24(s, 3H), 2.96(s, 1H), 1.80(m, 2H). HRMS (ESI, [M+H]⁺) *m*/*z:* 506.1413.

### Example 37: Preparation of Compound 37

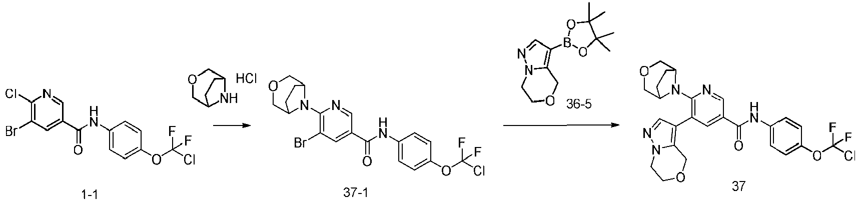

### Step A: Preparation of compound 37-1

Referring to the method of the step B in Example 1, compound 1-1 and 3-oxa-8-azabicyclo[3.2.1]octane hydrochloride were used to prepare compound 37-1.

### Step B: Preparation of compound 37

Referring to the method of the step H in Example 1, compound 37-1 and compound 36-5 were used to prepare compound 37.

¹H NMR (500 MHz, DMSO-*d*₆) δ 10.29 (s, 1H), 8.70 (d, *J* = 2.2 Hz, 1H), 7.94 (d, *J* = 2.2 Hz, 1H), 7.86 (d*, J* = 9.0 Hz, 2H), 7.71 (s, 1H), 7.35 (d, *J* = 8.8 Hz, 2H), 4.81 (s, 2H), 4.19 (t, *J* = 4.9 Hz, 2H), 4.10 (dd, *J* = 15.8, 9.5 Hz, 4H), 3.62 (d, *J* = 10.6 Hz, 2H), 3.43 (d, *J* = 10.4 Hz, 2H), 1.88 - 1.81 (m, 2H), 1.80 - 1.71 (m, 2H). HRMS (ESI, [M+H]⁺) *m*/*z:* 532.15650.

### Example 38: Preparation of Compound 38

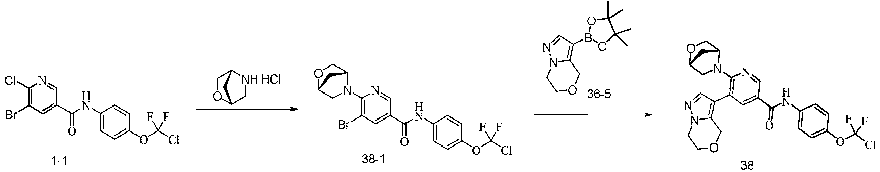

### Step A: Preparation of compound 38-1

Referring to the method of the step B in Example 1, compound 1-1 and (1*R*,4*R*)-2-oxa-5-azabicyclo[2.2.1]heptane hydrochloride were used to prepare compound 38-1.

### Step B: Preparation of compound 38

Referring to the method of the step H in Example 1, compound 38-1 and compound 36-5 were used to prepare compound 38.

¹H NMR (500 MHz, DMSO-*d*₆) δ 10.21 (s, 1H), 8.68 (d, *J* = 2.4 Hz, 1H), 7.96 (d, *J* = 2.4 Hz, 1H), 7.89 - 7.84 (m, 2H), 7.61 (s, 1H), 7.37 - 7.31 (m, 2H), 4.85 (s, 1H), 4.68 - 4.62 (m, 1H), 4.60 - 4.53 (m, 1H), 4.47 (d, *J* = 2.3 Hz, 1H), 4.20 - 4.14 (m, 2H), 4.12 - 4.05 (m, 2H), 3.83 - 3.78 (m, 1H), 3.78 - 3.72 (m, 1H), 2.93 - 2.86 (m, 1H), 2.64 - 2.59 (m, 1H), 1.88 - 1.82 (m, 1H), 1.81 - 1.74 (m, 1H). MS (ESI, [M-H]⁻) *m*/*z:* 516.1/518.1.

### Example 39: Preparation of Compound 39

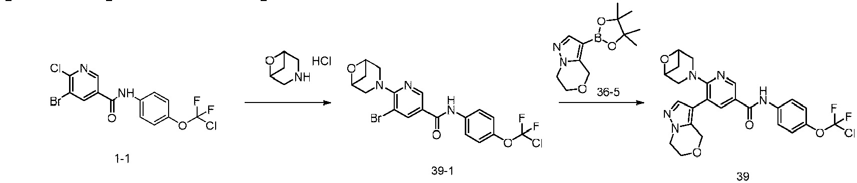

### Step A: Preparation of compound 39-1

Referring to the preparation method of the step B in Example 1, compound 1-1 and 6-oxa-3-aza-bicyclo[3,1,1]heptane hydrochloride were taken as starting materials to prepare compound 39-1. MS (ESI, [M+H]⁺) *m*/*z*: 474.04/476.06.

### Step B: Preparation of compound 39

Referring to the method of the step H in Example 1, compound 39-1 and compound 36-5 were used to prepare compound 39.

¹H NMR (500 MHz, DMSO-*d*₆) δ 10.25 (s, 1H), 8.74 (d, *J* = 2.2 Hz, 1H), 7.97 (d, *J* = 2.2 Hz, 1H), 7.87 (d, *J* = 9.0 Hz, 2H), 7.63 (s, 1H), 7.34 (d, *J* = 8.8 Hz, 2H), 4.69 (s, 2H), 4.53 (d, *J* = 6.2 Hz, 2H), 4.18 (t, *J* = 4.8 Hz, 2H), 4.09 (t, *J* = 5.0 Hz, 2H), 3.66 (d, *J* = 12.8 Hz, 2H), 3.46 (d, *J* = 12.8 Hz, 2H), 3.07 - 2.96 (m, 1H), 1.80 (d, *J* = 8.6 Hz, 1H). HRMS (ESI, [M+H]⁺) *m*/*z:* 518.1400.

### Example 40: Preparation of Compound 40

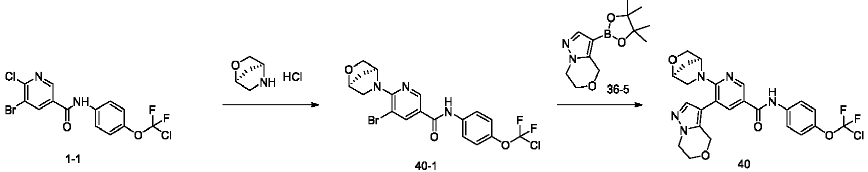

### Step A: Preparation of compound 40-1

Referring to the preparation method of the step B in Example 1, compound 1-1 and (1*S*,4*S*)-2-oxa-5-azabicyclo[2.2.1]heptane hydrochloride were taken as starting materials to prepare compound 40-1. MS (ESI, [M+H]⁺) *m*/*z*: 474.06/476.09.

### Step B: Preparation of compound 40

Referring to the method of the step H in Example 1, compound 40-1 and compound 36-5 were used to prepare compound 40.

¹H NMR (500 MHz, DMSO-*d*₆) δ 10.21 (s, 1H), 8.69 (d, *J* = 2.3 Hz, 1H), 7.96 (d, *J* = 2.3 Hz, 1H), 7.86 (d, *J* = 9.1 Hz, 2H), 7.62 (s, 1H), 7.34 (d, *J* = 8.9 Hz, 2H), 4.86 (s, 1H), 4.70 - 4.61 (m, 1H), 4.61 - 4.53 (m, 1H), 4.47 (s, 1H), 4.17 (t, *J* = 4.9 Hz, 2H), 4.09 (t, *J* = 5.5 Hz, 2H), 3.84 - 3.74 (m, 2H), 2.90 (d, *J* = 9.3 Hz, 1H), 2.61 (d, *J* = 10.2 Hz, 1H), 1.85 (d, *J* = 9.7 Hz, 1H), 1.79 (d, *J=* 9.8 Hz, 1H). HRMS (ESI, [M+H]⁺) *m*/*z*: 518.1405.

### Example 41: Preparation of Compound 41

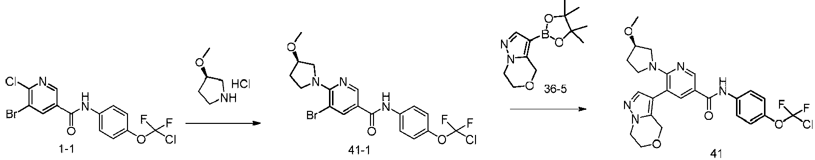

### Step A: Preparation of compound 41-1

Referring to the preparation method of the step B in Example 1, compound 1-1 and (*R*)-3-methoxypyrrolidine hydrochloride were used to prepare compound 41-1.

### Step B: Preparation of compound 41

Referring to the method of the step H in Example 1, compound 41-1 and compound 36-5 were used to prepare compound 41.

¹H NMR (500 MHz, DMSO-*d*₆) δ (ppm) 10.16 (s, 1H), 8.68 (d, *J* = 2.4 Hz, 1H), 7.94 (d, *J* = 2.4 Hz, 1H), 7.90 - 7.82 (m, 2H), 7.60 (s, 1H), 7.33 (d, *J* = 8.6 Hz, 2H), 4.69 - 4.53 (m, 2H), 4.21 - 4.15 (m, 2H), 4.11- 4.05 (m, 2H), 3.95 - 3.91 (m, 1H), 3.31 - 3.24 (m, 2H), 3.22 - 3.13 (m, 5H), 2.01 - 1.83 (m, 2H). MS (ESI, [M+H]⁺) *m*/*z*: 520.2.

### Example 42: Preparation of Compound 42

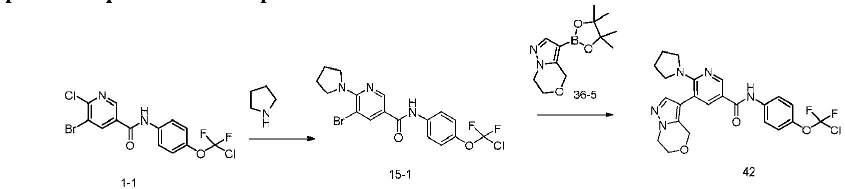

### Step A: Preparation of compound 42

Referring to the method of the step H in Example 1, compound 15-1 and compound 36-5 were used to prepare compound 42.

¹H NMR (500 MHz, DMSO-*d*₆) δ (ppm) 10.14 (s, 1H), 8.68 (d, *J* = 2.4 Hz, 1H), 7.92 (d, *J* = 2.4 Hz, 1H), 7.89 - 7.82 (m, 2H), 7.58 (s, 1H), 7.33 (d, *J* = 8.6 Hz, 2H), 4.62 (s, 2H), 4.17 (t, *J=* 5.1 Hz, 2H), 4.09 (t*, J* = 5.3 Hz, 2H), 3.23 - 3.11 (m, 4H), 1.85 - 1.71 (m, 4H). MS (ESI, [M+H]⁺) *m*/*z:* 490.1.

### Example 43: Preparation of Compound 43

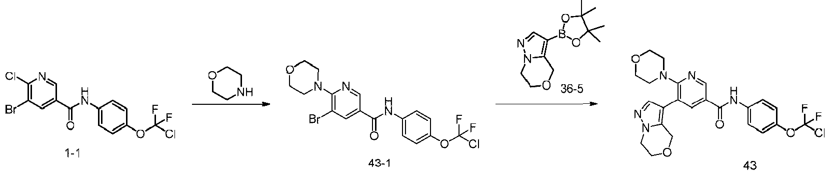

### Step A: Preparation of compound 43-1

Referring to the preparation method of the step B in Example 1, compound 1-1 was reacted with morpholine to obtain compound 43-1.

### Step B: Preparation of compound 43

Referring to the method of the step H in Example 1, compound 43-1 and compound 36-5 were used to prepare compound 43.

¹H NMR (500 MHz, DMSO-*d*₆) δ 10.31 (s, 1H), 8.73 (s, 1H), 7.97 (s, 1H), 7.87 (d, *J* = 10.0 Hz, 2H), 7.80 (s, 1H), 7.36 (d, *J* = 10.0 Hz, 2H), 4.83 (s, 2H), 4.20-4.12 (m, 4H), 3.63 (t, *J* = 5.0 Hz, 4H), 3.14 (t, *J* = 5.0, 4H). HRMS (ESI, [M+H]⁺) *m*/*z*: 506.1605.

### Example 44: Preparation of Compound 44

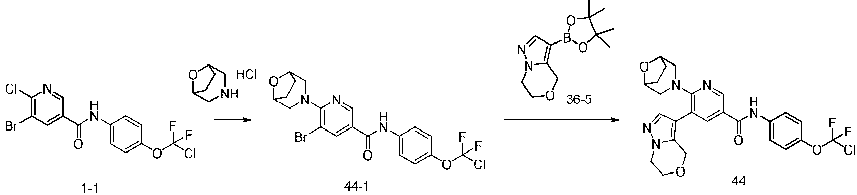

### Step A: Preparation of compound 44-1

Referring to the method of the step B in Example 1, compound 1-1 and 8-oxa-3-azabicyclo[3.2.1]octane hydrochloride were used to prepare compound 44-1.

### Step B: Preparation of compound 44

Referring to the method of the step H in Example 1, compound 44-1 and compound 36-5 were used to prepare compound 44.

¹H NMR (500 MHz, DMSO-*d*₆) δ 10.28 (s, 1H), 8.71 (d, *J* = 2.4 Hz, 1H), 7.92 (d, *J* = 2.4 Hz, 1H), 7.88 - 7.83 (m, 2H), 7.66 (s, 1H), 7.35 (d, *J* = 9.0 Hz, 2H), 4.74 (s, 2H), 4.25 (s, 2H), 4.19 (t, *J* = 5.1 Hz, 2H), 4.11 (t, *J* = 5.1 Hz, 2H), 3.37 (d, *J* = 12.3 Hz, 2H), 2.95 (d, *J* = 11.0 Hz, 2H), 1.73 (s, 4H). HRMS (ESI, [M+H]⁺) *m*/*z:* 532.15643.

### Example 45: Preparation of Compound 45

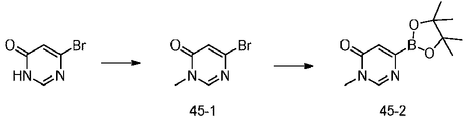

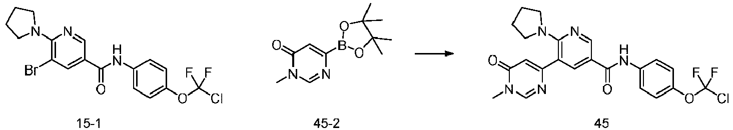

### Step A: Preparation of compound 45-1

To a 100-mL three-necked flask were added 6-bromopyrimidin-4(3*H*)-one (2.0 g), *N,N*-dimethylformamide (20 mL), iodomethane (3.24 g) and potassium carbonate (4.74 g) in sequence, and the reaction system was heated to 50 °C and reacted for 3 h under nitrogen atmosphere. Water (10 mL) was added into the reaction solution to quench the reaction, and ethyl acetate (40 mL) was added for extraction; the organic layer was separated, and the aqueous layer was extracted with ethyl acetate (20 mL × 2). The organic layers were combined, washed with saturated sodium chloride, dried over anhydrous sodium sulfate, filtered under vacuum, and concentrated under reduced pressure, and the residue was purified by silica gel column chromatography to obtain 1.4 g of compound 45-1. MS (ESI, [M+H]⁺) *m*/*z*: 189.01.

### Step B: Preparation of compound 45-2

To a 25-mL single-necked flask were added 1,4-dioxane (10 mL), compound 45-2 (127 mg), bis(pinacolato)diboron (205 mg), potassium acetate (132 mg) and [1,1'-bis(diphenylphosphino)ferrocene]palladium dichloride complex (33 mg) in sequence; after the addition was completed, the system was purged with nitrogen and then heated to 90 °C and reacted for 2 h, the reaction was stopped, the system was cooled to room temperature, and the resulting reaction solution was directly used in the next step without separation and purification.

### Step C: Preparation of compound 45

The reaction solution obtained in the step B above was transferred into a 35 mL microwave tube, compound 15-1 (200 mg), cesium carbonate (146 mg), [2'-(amino)[1,1'-biphenyl]-2-yl][[2',6'-bis(1-methylethoxy)[1,1'-biphenyl]-2-yl]dicyclohexylphosphine]palladium chloride (35 mg), 2-dicyclohexylphosphine-2',6'-diisopropoxy-1,1'-biphenyl (21 mg) and water (2.5 mL) were added into the microwave tube in sequence, and after the addition was completed, the microwave tube was sealed and placed into a microwave reactor under nitrogen atmosphere, where the microwave reaction conditions were as follows: 140 °C and 2 h. The reaction solution was filtered through diatomite, the filtrate was collected, and ethyl acetate (50 mL) and saturated brine (25 mL) were added into the filtrate for washing, followed by liquid separation, and an organic phase was collected and purified by silica gel column chromatography to obtain 15 mg of compound 45.

¹H NMR (500 MHz, DMSO-*d*₆): δ 10.21(s, 1H), 8.77(d, *J* = 2.0Hz, 1H), 8.53(s, 1H), 8.17(d, *J* = 2.0Hz, 1H), 7.86(d, *J* = 9.5Hz, 2H), 7.34(d, *J* = 9.0Hz, 2H), 6.47(s, 1H), 3.46(s, 3H), 3.32(s, 4H), 1.83(m, 4H). HRMS (ESI, [M+H]⁺) *m*/*z*: 476.13469.

### Example 46: Preparation of Compound 46

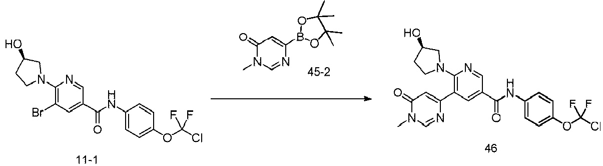

### Step A: Preparation of compound 46

Referring to the preparation method of the step C in Example 45, compound 11-1 was reacted with compound 45-2 to obtain compound 46.

¹H NMR (500 MHz, DMSO-*d*₆) δ 10.21 (s, 1H), 8.77 (d, *J* = 2.3 Hz, 1H), 8.54 (s, 1H), 8.17 (d, *J* = 2.3 Hz, 1H), 7.92 - 7.78 (m, 2H), 7.34 (d, *J* = 8.9 Hz, 2H), 6.47 (s, 1H), 4.90 (d, *J* = 3.2 Hz, 1H), 4.28 (d, *J* = 1.9 Hz, 1H), 3.66 - 3.53 (m, 1H), 3.47 (s, 3H), 3.44 (d, *J* = 4.3 Hz, 1H), 3.41 - 3.34 (m, 1H), 3.08 (d, *J=* 11.7 Hz, 1H), 1.98 - 1.73 (m, 2H). HRMS (ESI, [M+H]⁺) *m*/*z*: 492.1247.

### Example 47: Preparation of Compound 47

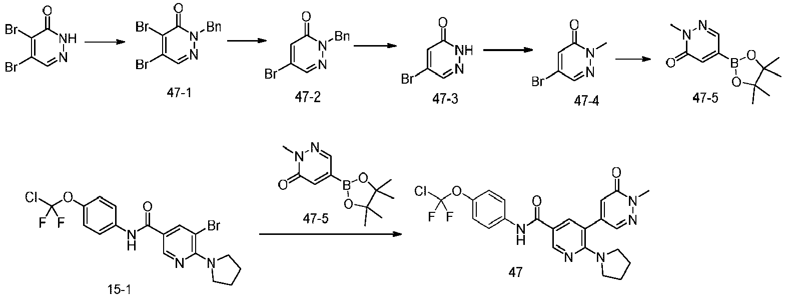

### Step A: Preparation of compound 47-1

To a 50-mL single-necked flask were added *N*-methylpyrrolidone (30 mL), 4,5-dibromopyridazine-3-one (4.0 g) and cesium carbonate (7.62 g) in sequence, followed by stirring, and bromobenzyl (2.72 g) was added slowly and dropwise into the system at room temperature; after the addition was completed, the system was reacted for 4 h and then filtered, the filtrate was collected, ethyl acetate (30 mL) and purified water (20 mL) were added into the filtrate, followed by stirring, washing and liquid separation, and an organic phase was collected and concentrated under reduced pressure to obtain a residue, which was purified by silica gel column chromatography to obtain 4.7 g of compound 47-1. MS (ESI, [M+H]⁺) *m*/*z:* 342.9.

### Step B: Preparation of compound 47-2

To a 50-mL single-necked flask were added anhydrous tetrahydrofuran (30 mL) and compound 47-1 (4.7 g) obtained in the step A above in sequence, the system was stirred and cooled to -20 °C, and then a 2 M tetrahydrofuran solution (6.9 mL) of n-butyl magnesium chloride was added slowly and dropwise into the system; after the addition was completed, the temperature was controlled and the reaction was carried out for 5 min; the reaction solution was poured into a saturated ammonium chloride aqueous solution (20 mL) to quench the reaction, and ethyl acetate was added for extraction (20 mL × 2); the organic phases were combined, stirred and washed with saturated brine, followed by liquid separation to obtain an organic phase, and the organic phase was concentrated under reduced pressure to obtain a residue, which was purified by silica gel column chromatography to obtain 1.05 g of compound 47-2.

¹H NMR (500 MHz, DMSO-*d*₆) δ 8.14 (d, *J* = 2.3 Hz, 1H), 7.48 (d, *J* = 2.3 Hz, 1H), 7.37 - 7.30 (m, 2H), 7.31 - 7.25 (m, 3H), 5.22 (s, 2H). MS (ESI, [M+H]⁺) *m*/*z*: 264.97.

### Step C: Preparation of compound 47-3

To a 50-mL single-necked flask were added toluene (20 mL), compound 47-2 (800 mg) obtained in the step B above and aluminum trichloride (2028 mg) in sequence, and the system was stirred, purged with nitrogen, and heated to 70 °C in an oil bath and reacted for 2 h; a saturated sodium bicarbonate aqueous solution (30 mL) was added into the system to quench the reaction, and ethyl acetate was added for extraction (30 mL × 2), followed by liquid separation to obtain an organic phase; a saturated sodium chloride aqueous solution (20 mL) was added into the organic phase, followed by stirring, washing and liquid separation to obtain an organic phase, and the organic phase was concentrated under reduced pressure to obtain a residue, which was purified by silica gel column chromatography to obtain 300 mg of compound 47-3. MS (ESI, [M-H]⁻) *m*/*z:* 173.0.

### Step D: Preparation of compound 47-4

To a 50-mL single-necked flask were added *N,N*-dimethylformamide (10 mL), compound 47-3 (300 mg) obtained in the step C above and cesium carbonate (838 mg), the system was stirred, and 2 mL of iodomethane (257 mg) diluted by anhydrous *N,N-*dimethylformamide was added slowly and dropwise into the system at room temperature; after the reaction was completed, the system was reacted at room temperature for 4 h; purified water (10 mL) and ethyl acetate (30 mL) were added into the system in sequence, followed by stirring, washing and liquid separation, and an organic phase was collected and concentrated under reduced pressure to obtain a residue, which was purified by silica gel column chromatography to obtain 250 mg of compound 47-4. MS (ESI, [M+H]⁺) *m*/*z:* 189.0.

### Step E: Preparation of compound 47-5

To a 35-mL microwave tube were added 1,4-dioxane (15 mL), compound 47-4 (93 mg) obtained in the step D above, bis(pinacolato)diboron (136 mg), potassium acetate (110 mg) and [1,1'-bis(diphenylphosphino)ferrocene]palladium dichloride (25 mg) in sequence, after the addition was completed, the system was purged with nitrogen, and heated to 90 °C and reacted for 2 h, the reaction was stopped, the system was cooled to room temperature, and the obtained reaction solution was directly used in the next step without separation and purification.

### Step F: Preparation of compound 47

To the reaction solution obtained in the step E above were added compound 15-1 (200 mg), potassium carbonate (154 mg), deionized water (1 mL) and tetrakis(triphenylphosphine)palladium (30 mg) in sequence, after the addition was completed, the system was purged with nitrogen, then the microwave tube was sealed and placed into a microwave reactor, and the system was heated to 140 °C and reacted for 1.5 h. The reaction solution was cooled to room temperature and then filtered, and the mother liquor was collected and purified by silica gel column chromatography to obtain 170 mg of compound 47.

¹H NMR (500 MHz, DMSO-*d*₆) δ 10.17 (s, 1H), 8.78 (d, *J* = 2.3 Hz, 1H), 8.13 (d, *J* = 2.3 Hz, 1H), 8.02 (d, *J* = 2.2 Hz, 1H), 7.89 - 7.83 (m, 2H), 7.38 - 7.31 (m, 2H), 6.90 (d, *J* = 2.2 Hz, 1H), 3.70 (s, 3H),3.30 - 3.25 (m, 4H), 1.88 - 1.79 (m, 4H). MS (ESI, [M-H]⁻) *m*/*z*: 474.09/475.99.

### Example 48: Preparation of Compound 48

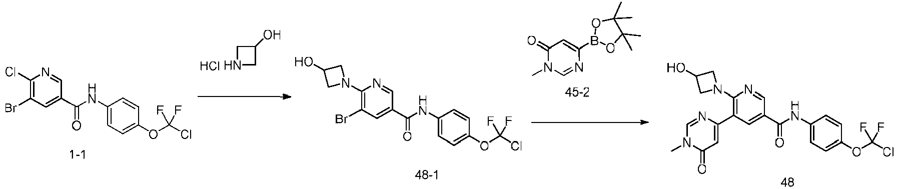

### Step A: Preparation of compound 48-1

To a 35-mL tube were added isopropanol (10 mL), compound 1-1 (1.0 g), 3-hydroxyazetidine hydrochloride (0.32 g), *N,N*-diisopropylethylamine (1.004 g) and magnetic stir bar in sequence, and after the tube was sealed, the sealed tube was placed into a microwave reactor to react at 140 °C for 1.5 h. After the reaction solution was cooled to room temperature, ethyl acetate (15 mL) and a saturated sodium chloride aqueous solution (15 mL) were added into the reaction solution, and stirred and washed, followed by liquid separation, the organic phase was collected, dried over anhydrous sodium sulfate, filtered under vaccuum, and concentrated under reduced pressure, and the resulting residue was purified by silica gel column chromatography to obtain 0.8 g of compound 48-1. MS (ESI, [M+H]⁺) *m*/*z*: 447.98.

### Step B: Preparation of compound 48

To a 35-mL microwave tube were added compound 45-2, compound 48-1 (500 mg), potassium carbonate (424 mg),
[2'-(amino)[1,1'-biphenyl]-2-yl][[2',6'-bis(1-methylethoxy)[1,1'-biphenyl]-2-yl]dicyclohexylphosphine]palladium chloride (80 mg), 2-dicyclohexylphosphine-2',6'-diisopropoxy-1,1'-biphenyl (48 mg) and water (2 mL) in sequence, and after the addition was completed, the microwave tube was placed into a microwave reactor under nitrogen atmosphere, where the microwave reaction conditions were as follows: 140 °C and 2 h. The reaction solution was filtered through diatomite, the filtrate was collected, and ethyl acetate (50 mL) and saturated brine (25 mL) were added into the filtrate for washing, followed by liquid separation, and an organic phase was collected and purified by silica gel column chromatography to obtain 100 mg of compound 48.

¹H NMR (500 MHz, DMSO-*d*₆) δ 10.26 (s, 1H), 8.78 (d, *J* = 2.2 Hz, 1H), 8.56 (s, 1H), 8.20 (d, *J* = 2.2 Hz, 1H), 7.86 (d, *J* = 9.1 Hz, 2H), 7.34 (d, *J* = 8.8 Hz, 2H), 6.48 (s, 1H), 5.63 (d, *J* = 6.4 Hz, 1H), 4.46 (h, *J* = 6.5 Hz, 1H), 4.16 - 4.05 (m, 2H), 3.69 (dd, *J* = 9.7, 4.2 Hz, 2H), 3.47 (s, 3H). HRMS (ESI, [M+H]⁺) *m*/*z*: 478.10934.

### Example 49: Preparation of Compound 49

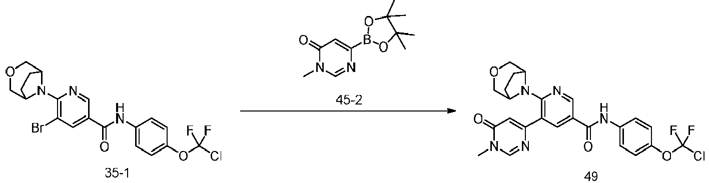

Referring to the preparation method of the step B in Example 48, compound 45-2 was reacted with compound 35-1 to obtain compound 49.

¹H NMR (500 MHz, DMSO-*d*₆) δ 10.34 (s, 1H), 8.78 (d, *J* = 2.1 Hz, 1H), 8.58 (s, 1H), 8.28 (d, *J* = 2.1 Hz, 1H), 7.86 (d, *J* = 9.0 Hz, 2H), 7.35 (d, *J* = 8.7 Hz, 2H), 6.79 (s, 1H), 4.22 (s, 2H), 3.65 (d, *J* = 10.7 Hz, 2H), 3.50 (d, *J* = 10.5 Hz, 2H), 3.45 (s, 3H), 1.83 (dd, *J* = 45.3, 5.8 Hz, 4H). HRMS (ESI, [M+H]⁺) *m*/*z*: 518.14078.

### Example 50: Preparation of Compound 50

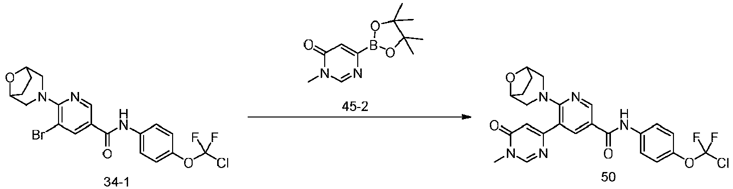

Referring to the preparation method of the step B in Example 48, compound 45-2 was reacted with compound 34-1 to obtain compound 50.

¹H NMR (500 MHz, DMSO-*d*₆) δ 10.32 (s, 1H), 8.77 (d, *J* = 2.3 Hz, 1H), 8.59 (s, 1H), 8.25 (d, *J* = 2.3 Hz, 1H), 7.86 (d, *J* = 9.1 Hz, 2H), 7.35 (d, *J* = 8.8 Hz, 2H), 6.63 (s, 1H), 4.29 (s, 2H), 3.58 (d, *J* = 12.6 Hz, 2H), 3.46 (s, 3H), 3.08 (d, *J* = 11.6 Hz, 2H), 1.73 (s, 4H). HRMS (ESI, [M+H]⁺) *m*/*z*: 518.14054.

### Example 51: Preparation of Compound 51

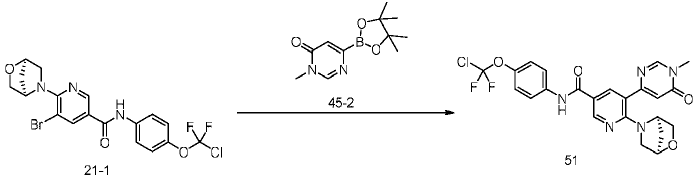

Referring to the preparation method of the step B in Example 48, compound 45-2 was reacted with compound 21-1 to obtain compound 51.

¹H NMR (500 MHz, DMSO-*d*₆) δ 10.27 (s, 1H), 8.78 (s, 1H), 8.53 (s, 1H), 8.19 (s, 1H), 7.86-7.87 (m, 2H), 7.34-7.35 (m, 2H), 6.52 (s, 1H), 4.94 (s, 1H), 4.55 (s, 1H), 3.74-3.79 (m, 2H), 3.45 (s, 3H), 3.36 (s, 1H), 2.80-2.82 (m, 1H), 1.81-1.87 (m, 2H). HRMS (ESI, [M+H]⁺) *m*/*z*: 504.12678.

### Example 52: Preparation of Compound 52

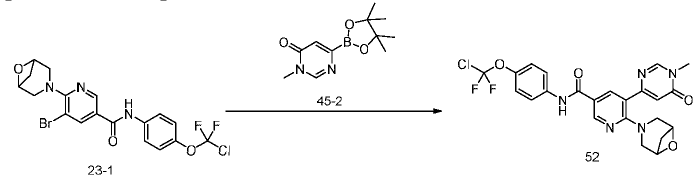

Referring to the preparation method of the step B in Example 48, compound 45-2 was reacted with compound 23-1 to obtain compound 52.

¹H NMR (500 MHz, DMSO-*d*₆) δ 10.26 (s, 1H), 8.80 (d, *J* = 2.3 Hz, 1H), 8.52 (s, 1H), 8.17 (d, *J* = 2.4 Hz, 1H), 7.92 - 7.79 (m, 2H), 7.34 (d, *J* = 8.7 Hz, 2H), 6.63 (d, *J* = 0.8 Hz, 1H), 4.57 (d, *J* = 6.4 Hz, 2H), 3.81 (d, *J* = 12.8 Hz, 2H), 3.64 (d, *J* = 12.8 Hz, 2H), 3.45 (s, 3H), 3.08 - 2.98 (m, 1H), 1.74 (d, *J* = 8.6 Hz, 1H). HRMS (ESI, [M+H]⁺) *m*/*z*: 504.12424.

### Example 53: Preparation of Compound 53

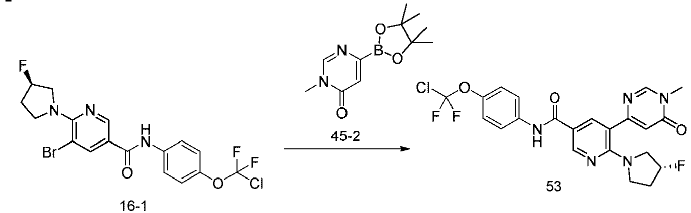

Referring to the preparation method of the step B in Example 48, compound 45-2 was reacted with compound 16-1 to obtain compound 53.

¹H NMR (500 MHz, DMSO-*d*₆) δ 10.24 (s, 1H), 8.79 (d, *J* = 2.2 Hz, 1H), 8.55 (s, 1H), 8.19 (d, *J* = 2.2 Hz, 1H), 7.87 (d, *J* = 9.1 Hz, 2H), 7.34 (d, *J* = 8.8 Hz, 2H), 6.54 (s, 1H), 5.54 - 5.17 (m, 1H), 3.81 - 3.63 (m, 1H), 3.63 - 3.48 (m, 2H), 3.47 (s, 3H), 3.45 - 3.40 (m, 1H), 2.21 - 1.97 (m, 2H). HRMS (ESI, [M+H]⁺) *m*/*z*: 494.1202.

### Example 54: Preparation of Compound 54

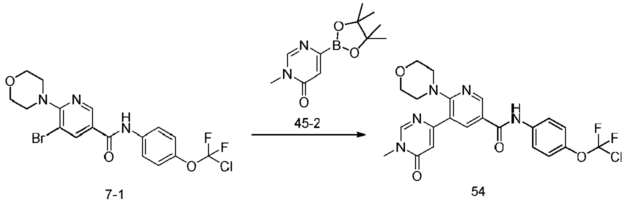

Referring to the preparation method of the step B in Example 48, compound 45-2 was reacted with compound 7-1 to obtain compound 54.

¹H NMR (500 MHz, DMSO-*d*₆) δ 10.36 (s, 1H), 8.80 (d, *J* = 2.3 Hz, 1H), 8.60 (s, 1H), 8.35 (d, *J* = 2.3 Hz, 1H), 7.87 (d, *J* = 9.0 Hz, 2H), 7.35 (d, *J* = 8.8 Hz, 2H), 6.83 (s, 1H), 3.67 - 3.59 (m, 4H), 3.46 (s, 3H), 3.40 - 3.33 (m, 4H). HRMS (ESI, [M+H]⁺) *m*/*z*: 492.1249.

### Example 55: Preparation of Compound 55

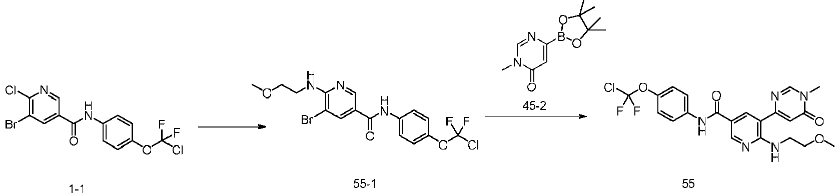

### Step A: Preparation of compound 55-1

Referring to the preparation method of the step A in Example 48, compound 1-1 was reacted with 2-methoxyethan-1-amine to obtain compound 55-1. MS (ESI, [M-H]⁻) *m*/*z:* 448.01.

### Step B: Preparation of compound 55

Referring to the preparation method of the step B in Example 48, compound 45-2 was reacted with compound 55-1 to obtain compound 55.

¹H NMR (500 MHz, DMSO-*d*₆) δ 10.19 (s, 1H), 8.89 (s, 1H), 8.75 (s, 1H), 8.64 (s, 1H), 8.44 (s, 1H), 7.94 - 7.79 (m, 2H), 7.43 - 7.29 (m, 2H), 7.01 (s, 1H), 3.76 - 3.60 (m, 2H), 3.60 - 3.51 (m, 2H), 3.48 (s, 3H), 3.30 (s, 3H). HRMS (ESI, [M+H]⁺) *m*/*z*: 480.12468.

### Example 56: Preparation of Compound 56

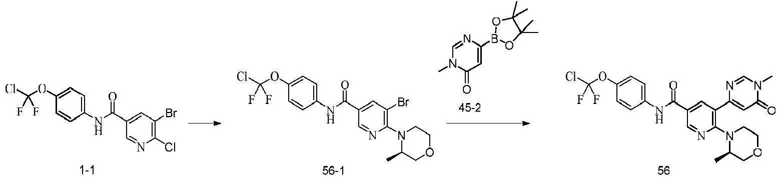

### Step A: Preparation of compound 56-1

Referring to the preparation method of the step A in Example 48, compound 1-1 was reacted with (*R*)-3-methylmorpholine hydrochloride to obtain compound 56-1. MS (ESI, [M+H]⁺) *m*/*z*: 476.04.

### Step B: Preparation of compound 56

Referring to the preparation method of the step B in Example 48, compound 45-2 was reacted with compound 56-1 to obtain compound 56.

¹H NMR (500 MHz, DMSO-*d*₆) δ 10.36 (s, 1H), 8.80 (d, *J* = 2.4 Hz, 1H), 8.59 (s, 1H), 8.32 (d, *J* = 2.4 Hz, 1H), 7.90 - 7.83 (m, 2H), 7.35 (d, *J* = 8.6 Hz, 2H), 6.81 (s, 1H), 4.09 (d, *J* = 6.4 Hz, 1H), 3.78 (d, *J* = 11.2 Hz, 1H), 3.62 - 3.55 (m, 2H), 3.50 - 3.42 (m, 4H), 3.39 - 3.33 (m, 1H), 3.31 - 3.24 (m, 1H), 1.14 (d, *J* = 6.7 Hz, 3H). HRMS (ESI, [M+H]⁺) *m*/*z:* 506.1398.

### Example 57: Preparation of Compound 57

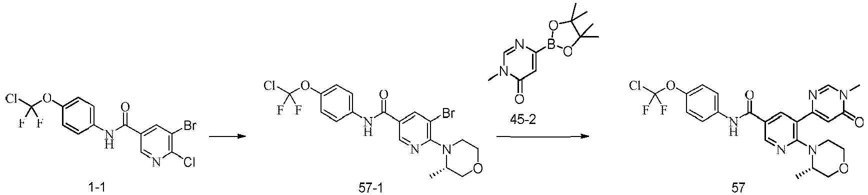

### Step A: Preparation of compound 57-1

Referring to the preparation method of the step A in Example 48, compound 1-1 was reacted with (*S*)-3-methylmorpholine hydrochloride to obtain compound 57-1. MS (ESI, [M+H]⁺) *m*/*z*: 476.04.

### Step B: Preparation of compound 57

Referring to the preparation method of the step B in Example 48, compound 45-2 was reacted with compound 57-1 to obtain compound 57.

¹H NMR (500 MHz, DMSO-*d*₆) δ 10.36 (s, 1H), 8.80 (d, *J* = 2.4 Hz, 1H), 8.59 (s, 1H), 8.32 (d, *J* = 2.4 Hz, 1H), 7.87 (d, *J* = 9.0 Hz, 2H), 7.35 (d, *J* = 8.6 Hz, 2H), 6.81 (s, 1H), 4.09 (d, *J* = 6.6 Hz, 1H), 3.78 (d, *J* = 11.1 Hz, 1H), 3.63 - 3.53 (m, 2H), 3.52 - 3.40 (m, 4H), 3.31 - 3.24 (m, 2H), 1.14 (d, *J* = 6.6 Hz, 3H). HRMS (ESI, [M+H]⁺) *m*/*z:* 506.1404.

### Example 58: Preparation of Compound 58

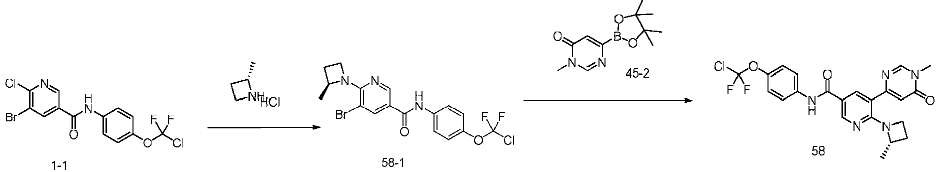

### Step A: Preparation of compound 58-1

Referring to the method of the step A in Example 48, compound 1-1 and (*S*)-2-methylazetidine hydrochloride were used to prepare compound 58-1. MS (ESI, [M+H]⁺) *m*/*z:* 446.02.

### Step B: Preparation of compound 58

Referring to the preparation method of the step B in Example 48, compound 45-2 was reacted with compound 58-1 to obtain compound 58.

¹H NMR (500 MHz, CDCl₃) δ 8.78 (d, *J* = 2.2 Hz, 1H), 8.25 - 8.09 (m, 2H), 7.97 (s, 1H), 7.69 (d, *J* = 8.9 Hz, 2H), 7.23 (d, *J* = 8.7 Hz, 2H), 6.53 (s, 1H), 4.66 - 4.56 (m, 1H), 4.16 - 4.02 (m, 1H), 3.57 (s, 3H), 3.45 (dd, *J* = 15.3, 8.8 Hz, 1H), 2.51 - 2.39 (m, 1H), 2.01 - 1.87 (m, 1H), 1.38 (d, *J* = 6.2 Hz, 3H). HRMS (ESI, [M+H]⁺) *m*/*z*: 476.1314.

### Example 59: Preparation of Compound 59

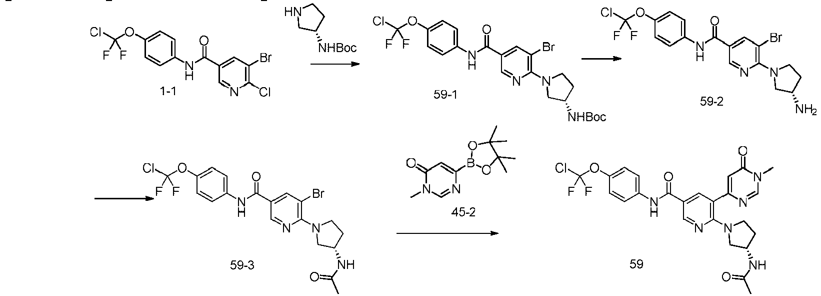

### Step A: Preparation of compound 59-1

Referring to the preparation method of the step A in Example 48, compound 1-1 was reacted with tert-butyl (*S*)-pyrrolidin-3-ylcarbamate to obtain compound 59-1. MS (ESI, [M+H]⁺) *m*/*z*: 561.09.

### Step B: Preparation of compound 59-2

To a 50-mL eggplant-shaped flask were added compound 59-1 (1 g), dichloromethane (6 mL) and trifluoroacetic acid (4.00 mL) in sequence, and the resulting mixture was reacted at room temperature. After the reaction was completed, the reaction mixture was concentrated under reduced pressure to remove the solvent. Dichloromethane (10 mL) was added into the residue, the pH thereof was adjusted to 8 by using a 1 M sodium hydroxide aqueous solution, followed by liquid separation, and an organic phase was collected. The aqueous phase was extracted with ethyl acetate (20 mL × 2), and an organic phase was collected, dried over anhydrous sodium sulfate, filtered, and concentrated to obtain 720 mg of compound 59-2. MS (ESI, [M+H]⁺) *m*/*z:* 461.04.

### Step C: Preparation of compound 59-3

To a 50-mL eggplant-shaped flask were added compound 59-2 (0.7 g), *N,N*-diisopropylethylamine (0.588 g) and dichloromethane (6 mL) in sequence, acetyl chloride (0.238 g) was added under nitrogen atmosphere, and the resulting mixture was reacted at room temperature overnight, followed by purification by silica gel column chromatography to obtain 610 mg of compound 59-3. MS (ESI, [M+H]⁺) *m*/*z*: 503.05.

### Step D: Preparation of compound 59

Referring to the preparation method of the step B in Example 48, compound 45-2 was reacted with compound 59-3 to obtain compound 59.

¹H NMR (500 MHz, DMSO-*d*₆) δ 10.22 (s, 1H), 8.79 (s, 1H), 8.54 (s, 1H), 8.19 (s, 1H), 8.07 (d, *J* = 6.0 Hz, 1H), 7.87 (d, *J* = 8.8 Hz, 2H), 7.34 (d, *J* = 8.5 Hz, 2H), 6.47 (s, 1H), 4.20 (s, 1H), 3.43-3.53 (m, 6H), 3.11-3.13 (m, 1H), 2.05 (s, 1H), 1.78 (s, 4H). HRMS (ESI, [M+H]⁺) *m*/*z:* 533.15133.

### Example 60: Preparation of Compound 60

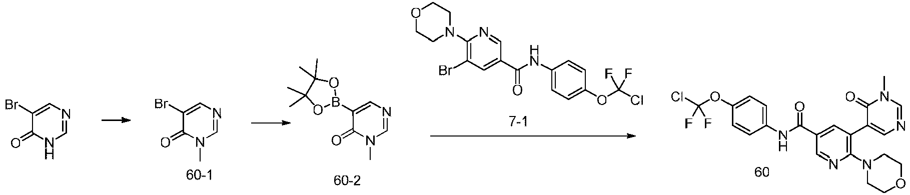

### Step A: Preparation of compound 60-1

5-Bromopyrimidin-4-one (1 g) was dissolved in tetrahydrofuran (10 mL) in a 50-mL reaction flask, the system was cooled to 0 °C, and sodium hydride (0.457 g) was added in portions. After the addition was completed, the system was purged with nitrogen for three times, and reacted for 1 h. Iodomethane (1.622 g) was added dropwise into the reaction solution by using a syringe in an ice bath, and after the dropwise addition was completed, the reaction was continued overnight. Water (10 mL) was added dropwise into the system in an ice bath to quench the reaction, ethyl acetate (15 mL) was added for liquid separation, and ethyl acetate (10 mL) was added for extraction twice; the organic phases were combined, dried over anhydrous sodium sulfate, filtered and spin-dried to obtain 0.8 g of compound 60-1. MS (ESI, [M+H]⁺) *m*/*z*: 188.96.

### Step B: Preparation of compound 60-2

Referring to the preparation method of the step B in Example 45, compound 60-1 was reacted with bis(pinacolato)diboron to obtain compound 60-2, which was directly used in the next step without separation and purification.

### Step D: Preparation of compound 60

Referring to the preparation method of the step B in Example 48, compound 7-1 was reacted with compound 60-2 to obtain compound 60.

¹H NMR (500 MHz, DMSO-*d*₆) δ 10.28 (s, 1H), 8.78 (s, 1H), 8.54 (s, 1H), 8.15 (s, 1H), 8.06 (s, 1H), 7.85-7.87 (m, 2H), 7.34-7.35 (m, 2H), 3.55-3.56 (m, 4H), 3.51 (s, 3H), 3.28-3.29 (m, 4H). HRMS (ESI, [M+H]⁺) *m*/*z:* 492.12730.

### Example 61: Preparation of Compound 61

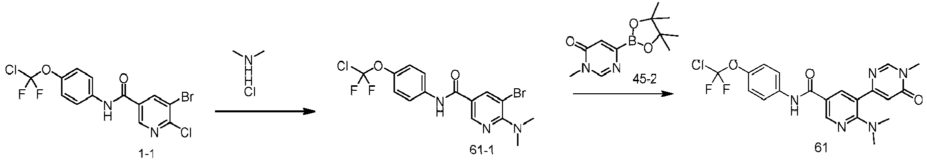

### Step A: Preparation of compound 61-1

Referring to the method of the step A in Example 48, compound 1-1 and dimethylamine hydrochloride were used to prepare compound 61-1. MS (ESI, [M+H]⁺) *m*/*z*: 420.00.

### Step B: Preparation of compound 61

Referring to the preparation method of the step B in Example 48, compound 45-2 was reacted with compound 61-1 to obtain compound 61.

¹H NMR (500 MHz, DMSO-*d*₆) δ 10.25 (s, 1H), 8.76 (d, *J* = 2.3 Hz, 1H), 8.56 (s, 1H), 8.27 (d, *J* = 2.3 Hz, 1H), 7.87 (d, *J* = 9.0 Hz, 2H), 7.34 (d, *J* = 8.8 Hz, 2H), 6.56 (s, 1H), 3.46 (s, 3H), 2.94 (s, 6H). HRMS (ESI, [M+H]⁺) *m*/*z:* 450.1141.

### Example 62: Preparation of Compound 62

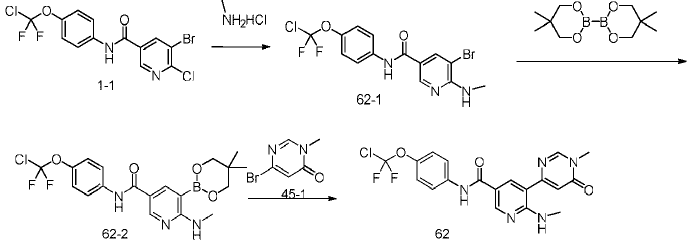

### Step A: Preparation of compound 62-1

Referring to the method of the step A in Example 48, compound 1-1 and methylamine hydrochloride were used to prepare compound 62-1. MS (ESI, [M+H]⁺) *m*/*z*: 406.04.

### Step B: Preparation of compound 62-2

To a 15-mL microwave tube were added 1,4-dioxane (4 mL), compound 62-1 (240 mg), bis(neopentyl glycolato)diboron (200 mg), potassium acetate (174 mg) and bis(triphenylphosphine)palladium(II) dichloride (41.5 mg) in sequence, and after the addition was completed, the system was reacted at 120 °C for 1 h in a microwave reactor under nitrogen atmosphere; the reaction was stopped, and the system was cooled to room temperature, which was directly used in the next step without separation and purification.

### Step C: Preparation of compound 62

To the reaction solution obtained in the step B were added compound 45-1 (145 mg), anhydrous potassium carbonate (245 mg), tetrakis(triphenylphosphine)palladium (68 mg) and water (1 mL) in sequence, and after the addition was completed, the tube was sealed and placed into a microwave reactor under nitrogen atmosphere, where the microwave reaction conditions were as follows: 140 °C and 1.5 h. Ethyl acetate (50 mL) and saturated brine (25 mL) were added into the filtrate for washing, followed by liquid separation, and an organic phase was collected and purified by silica gel column chromatography to obtain 60 mg of compound 62.

¹H NMR (500 MHz, DMSO-*d*₆) δ 10.18 (s, 1H), 8.77 (d, *J* = 2.2 Hz, 1H), 8.61 (s, 1H), 8.52 - 8.48 (m, 1H), 8.39 (d, *J* = 2.2 Hz, 1H), 7.86 (d, *J* = 9.1 Hz, 2H), 7.35 (d, *J* = 8.9 Hz, 2H), 6.96 (s, 1H), 3.48 (s, 3H), 2.99 (d, *J* = 4.7 Hz, 3H). HRMS (ESI, [M+H]⁺) *m*/*z:* 436.1336.

### Example 63: Preparation of Compound 63

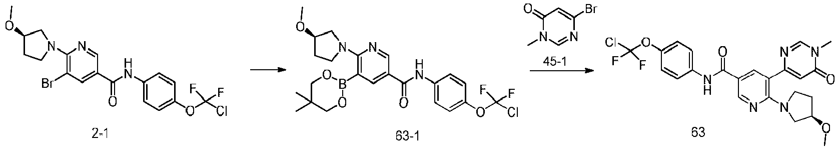

### Step A: Preparation of compound 63-1

Referring to the method of the step B in Example 62, compound 2-1 and bis(neopentyl glycolato)diboron were used to prepare compound 63-1. The reaction system was directly used in the next step without treatment.

### Step B: Preparation of compound 63

Referring to the method of the step C in Example 62, compound 63-1 and compound 45-1 were used to prepare compound 63.

¹H NMR (500 MHz, DMSO-*d*₆) δ 10.21 (s, 1H), 8.77 (d, *J* = 2.4 Hz, 1H), 8.54 (s, 1H), 8.17 (d, *J* = 2.4 Hz, 1H), 7.89 - 7.83 (m, 2H), 7.38 - 7.30 (m, 2H), 6.49 (d, *J* = 0.9 Hz, 1H), 4.00 - 3.94 (m, 1H), 3.55 - 3.44 (m, 5H), 3.37 - 3.33 (m, 1H), 3.27 (d, *J* = 12.2 Hz, 1H), 3.20 (s, 3H), 1.99 (s, 1H), 1.94 - 1.86 (m, 1H). HRMS (ESI, [M+H]⁺) *m*/*z:* 506.1471.

### Example 64: Preparation of Compound 64

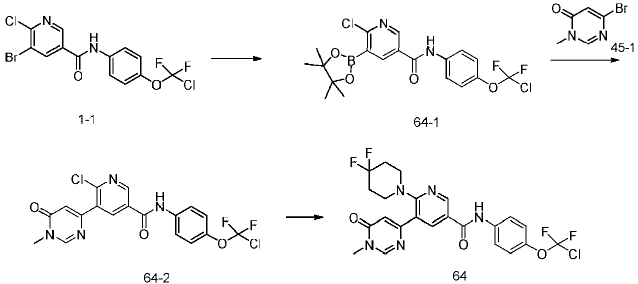

### Step A: Preparation of compound 64-1

To a 35-mL microwave tube were added 1,4-dioxane (15 mL), compound 1-1 (400 mg), bis(pinacolato)diboron (370 mg), potassium acetate (191 mg) and [1,1'-bis(diphenylphosphino)ferrocene]palladium dichloride (79 mg) in sequence, and after the addition was completed, the system was reacted at 140 °C for 1.5 h in a microwave reactor under nitrogen atmosphere; the reaction was stopped, and the system was cooled to room temperature, which was directly used in the next step without separation and purification.

### Step B: Preparation of compound 64-2

To the reaction solution obtained in the step A above were added compound 45-1 (150 mg), anhydrous potassium carbonate (90 mg), tetrakis(triphenylphosphine)palladium (57 mg) and water (2 mL) in sequence, and after the addition was completed, the reaction was carried out at 70 °C for 5 h under nitrogen atmosphere. Ethyl acetate (50 mL) and saturated brine (25 mL) were added into the reaction solution for washing, followed by liquid separation, and an organic phase was collected and purified by silica gel column chromatography to obtain 150 mg of compound 64-2. MS (ESI, [M+H]⁺) *m*/*z:* 441.02.

### Step C: Preparation of compound 64

To a 35-mL microwave tube were added compound 64-2 (100 mg), 4,4-difluoropiperidine (30 mg), *N,N*-diisopropylethylamine (64 mg) and *N*-methylpyrrolidone (10 mL) in sequence, and the reaction was carried out in a microwave reactor at 180 °C for 2 h. Ethyl acetate (50 mL) and saturated brine (25 mL) were added into the reaction solution for washing, followed by liquid separation, and an organic phase was collected and purified by silica gel column chromatography to obtain 75 mg of compound 64.

¹H NMR (500 MHz, DMSO-*d*₆) δ 10.38 (s, 1H), 8.81 (d, *J* = 2.4 Hz, 1H), 8.62 (s, 1H), 8.37 (d, *J* = 2.4 Hz, 1H), 7.91 - 7.84 (m, 2H), 7.36 (d, *J* = 8.7 Hz, 2H), 6.85 (s, 1H), 3.53 - 3.43 (m, 7H), 2.03 (td, *J* = 13.6, 6.4 Hz, 4H). HRMS (ESI, [M+H]⁺) *m*/*z*: 526.1279.

### Example 65: Preparation of Compound 65

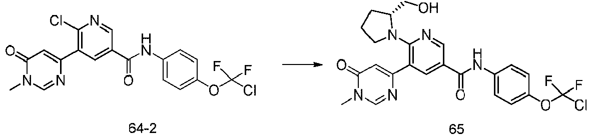

Referring to the method of the step C in Example 64, compound 64-2 and R-prolinol were used to prepare compound 65.

¹H NMR (500 MHz, DMSO-*d*₆) δ 10.26 (s, 1H), 8.77 (d, *J* = 2.4 Hz, 1H), 8.55 (s, 1H), 8.27 (d, *J* = 2.4 Hz, 1H), 7.91 - 7.83 (m, 2H), 7.34 (d, *J* = 8.8 Hz, 2H), 6.42 (d, *J* = 0.8 Hz, 1H), 4.74 (t, *J* = 5.5 Hz, 1H), 4.47 - 4.39 (m, 1H), 3.69 - 3.62 (m, 1H), 3.61 - 3.51 (m, 1H), 3.30 - 3.21 (m, 1H), 2.85 - 2.74 (m, 1H), 2.03 - 1.95 (m, 1H), 1.94 - 1.84 (m, 2H), 1.71 - 1.55 (m, 1H). HRMS (ESI, [M+H]⁺) *m*/*z:* 506.1407.

### Example 66: Preparation of Compound 66

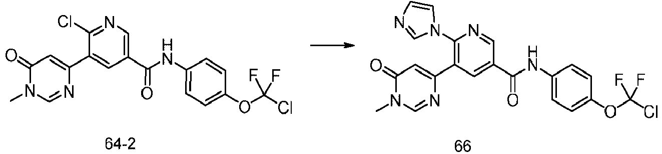

To a 15-mL microwave tube were added compound 64-2 (100 mg), imidazole (30.9 mg), potassium *tert*-butoxide (76 mg) and dimethyl sulfoxide (4 mL). The reaction was carried out in a microwave reactor at 120 °C for 15 min under nitrogen atmosphere. Ethyl acetate (50 mL) and saturated brine (25 mL) were added into the reaction system for washing, followed by liquid separation, and an organic phase was collected and purified by silica gel column chromatography to obtain 50 mg of compound 66.

¹H NMR (500 MHz, DMSO-*d*₆) δ 10.75 (s, 1H), 9.14 (s, 1H), 8.66 (s, 1H), 8.53 (s, 1H), 7.96 (s, 1H), 7.91 (d, *J* = 9.1 Hz, 2H), 7.42 (d, *J* = 21.3 Hz, 3H), 7.05 (s, 1H), 6.56 (s, 1H), 3.45 (s, 3H). HRMS (ESI, [M+H]⁺) *m*/*z:* 473.09304.

### Example 67: Preparation of Compound 67

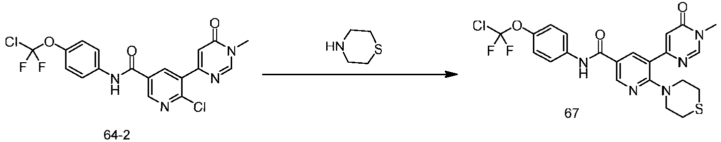

Referring to the method of the step C in Example 64, compound 64-2 and thiomorpholine were used to prepare compound 67.

¹H NMR (500 MHz, DMSO-*d*₆) δ 10.37 (s, 1H), 8.80 (d, *J* = 2.3 Hz, 1H), 8.61 (s, 1H), 8.34 (d, *J* = 2.3 Hz, 1H), 7.87 (d, *J* = 9.1 Hz, 2H), 7.35 (d, *J* = 8.9 Hz, 2H), 6.80 (s, 1H), 3.68 - 3.57 (m, 4H), 3.46 (s, 3H), 2.69 - 2.59 (m, 4H). HRMS (ESI, [M+H]⁺) *m*/*z*: 508.1188.

### Example 68: Preparation of Compound 68

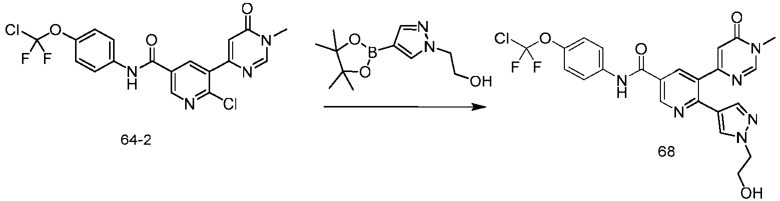

To a 35-mL microwave tube were added compound 64-2 (150 mg), dioxane (10 mL), 2-(4-(4,4,5,5-tetramethyl-1,3,2-dioxaborolan-2-yl)-1*H*-pyrazol-1-yl)ethan-1-ol (97 mg), anhydrous potassium carbonate (94 mg), tetrakis(triphenylphosphine)palladium (59 mg), and water (1 mL) in sequence. After the addition was completed, the system was placed into a microwave reactor under nitrogen atmosphere, where the microwave reaction conditions were as follows: 140 °C and 1.5 h. Ethyl acetate (50 mL) and saturated brine (25 mL) were added into the reaction solution for washing, followed by liquid separation, and an organic phase was collected and purified by silica gel column chromatography to obtain 100 mg of compound 68.

¹H NMR (500 MHz, DMSO-*d*₆) δ10.58 (s, 1H), 9.12 (d, *J* = 2.2 Hz, 1H), 8.57 (s, 1H), 8.25 (d, *J* = 2.2 Hz, 1H), 7.97 (s, 1H), 7.93 - 7.86 (m, 2H), 7.60 (s, 1H), 7.38 (d, *J* = 9.0 Hz, 2H), 6.64 (s, 1H), 4.90 (t, *J* = 5.2 Hz, 1H), 4.15 (t*, J* = 5.6 Hz, 2H), 3.72 (q*, J* = 5.5 Hz, 2H), 3.51 (d*, J* = 7.6 Hz, 3H). HRMS (ESI, [M+H]⁺) *m*/*z*: 517.1368.

### Example 69: Preparation of Compound 69

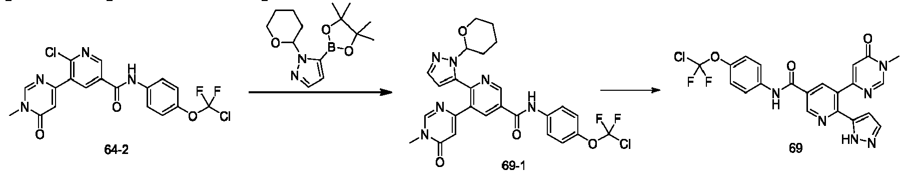

### Step A: Preparation of compound 69-1

Referring to the preparation method of Example 68, compound 64-2 was reacted with 1-(2-tetrahydropyranyl)-1*H-*pyrazole-5-boronic acid pinacol ester to obtain compound 69-1. MS (ESI, [M-H]⁻) *m*/*z:* 555.14.

### Step B: Preparation of compound 69

To a 25-mL eggplant-shaped flask were added compound 69-1 (330 mg) and dichloromethane (10 mL) in sequence, trifluoroacetic acid (3 mL) was added slowly and dropwise into the flask, and after the addition was completed, the reaction was carried out at room temperature for 2 h. The reaction solution was concentrated to remove the solvent, and dichloromethane (10 mL) was added for reconstitution, followed by purification by silica gel column chromatography to obtain 30 mg of compound 69.

¹H NMR (500 MHz, DMSO-*d*₆) δ 13.05 (s, 1H), 10.68 (s, 1H), 9.19 (d, *J=* 2.1 Hz, 1H), 8.52 (s, 1H), 8.36 (d, *J* = 2.1 Hz, 1H), 7.91 (d, *J* = 9.1 Hz, 2H), 7.70 (s, 1H), 7.39 (d, *J* = 8.9 Hz, 2H), 6.58 (d, *J* = 76.3 Hz, 2H), 3.46 (s, 3H). HRMS (ESI, [M+H]⁺) *m*/*z:* 473.09353.

### Example 70: Preparation of Compound 70

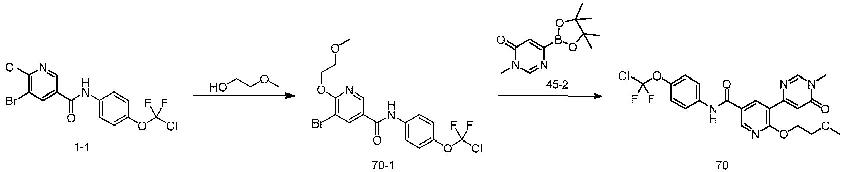

### Step A: Preparation of compound 70-1

Referring to the preparation method of the step A in Example 48, compound 1-1 was reacted with ethylene glycol methyl ether to obtain compound 70-1. MS (ESI, [M+H]⁺) *m*/*z:* 450.98.

### Step B: Preparation of compound 70

Referring to the preparation method of the step B in Example 48, compound 45-2 was reacted with compound 70-1 to obtain compound 70.

¹H NMR (500 MHz, DMSO-*d*₆) δ 10.59 (s, 1H), 8.96 (d, *J* = 2.5 Hz, 1H), 8.85 (d, *J* = 2.5 Hz, 1H), 8.61 (s, 1H), 7.89 (d, *J* = 9.1 Hz, 2H), 7.37 (d, *J* = 9.0 Hz, 2H), 7.27 (s, 1H), 4.66 - 4.61 (m, 2H), 3.80 - 3.73 (m, 2H), 3.46 (s, 3H), 3.34 (s, 3H). HRMS (ESI, [M+H]⁺) *m*/*z*: 481.10819.

### Example 71: Preparation of Compound 71

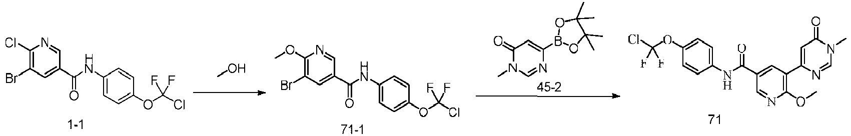

### Step A: Preparation of compound 71-1

To a 50-mL reaction flask were added compound 1-1 (0.6 g), potassium hydroxide (245 mg) and methanol (20 mL) in sequence, and the system was purged with nitrogen for 3 times and then reacted in a microwave reactor at 60 °C for 6 h. The reaction solution was concentrated and extracted with ethyl acetate (60 mL); the organic layer was separated, and the aqueous layer was extracted with ethyl acetate (40 mL × 2). The organic layers were combined, washed with a saturated sodium chloride aqueous solution, and dried over anhydrous sodium sulfate. The solvent was distilled off under reduced pressure, and the residue was purified by silica gel column chromatography to obtain 0.5 g of compound 71-1. MS (ESI, [M+H]⁺) *m*/*z*: 406.9.

### Step B: Preparation of compound 71

Referring to the preparation method of the step B in Example 48, compound 1-4 was reacted with compound 25-1 to obtain compound 71.

¹H NMR (500 MHz, DMSO-*d*₆) δ 10.60 (s, 1H), 8.93 (d, *J* = 2.3 Hz, 1H), 8.88 (d, *J* = 2.4 Hz, 1H), 8.61 (s, 1H), 7.90 (d, *J* = 9.0 Hz, 2H), 7.38 (d, *J* = 8.8 Hz, 2H), 7.17 (s, 1H), 4.09 (s, 3H), 3.46 (s, 3H). HRMS (ESI, [M+H]⁺) *m*/*z*: 437.08180.

### Example 72: Preparation of Compound 72

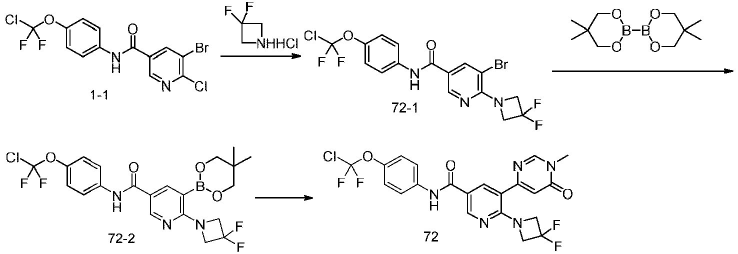

### Step A: Preparation of compound 72-1

Referring to the preparation method of the step A in Example 48, compound 1-1 was reacted with 3,3-difluoroazetidine hydrochloride to obtain compound 72-1. MS (ESI, [M+H]⁺) *m*/*z:* 467.91.

### Step B: Preparation of compound 72-2

Referring to the preparation method of the step B in Example 62, compound 72-1 was reacted with bis(neopentyl glycolato)diboron to obtain a reaction solution of compound 72-2, which was directly used in the next step without purification.

### Step C: Preparation of compound 72

Referring to the preparation method of the step C in Example 62, the reaction solution of compound 72-2 obtained in the step B and compound 45-1 were used to prepare compound 72.

¹H NMR (500 MHz, DMSO-*d*₆) δ 10.36 (s, 1H), 8.84 (s, 1H), 8.57 (s, 1H), 8.28 (s, 1H), 7.87 (d, *J* = 8.7 Hz, 2H), 7.36 (d, *J* = 8.4 Hz, 2H), 6.61 (s, 1H), 4.35 (t, *J* = 12.4 Hz, 4H), 3.47 (s, 3H). HRMS (ESI, [M+H]⁺) *m*/*z*: 498.0951.

### Example 73: Preparation of Compound 73

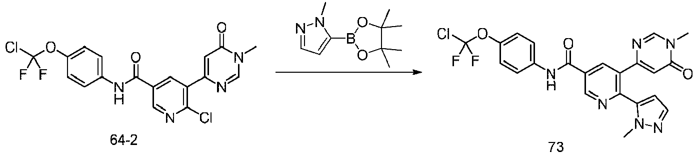

To a 50-mL eggplant-shaped flask were added compound 64-2 (330 mg), 1,4-dioxane (20 mL), 1-methyl-1*H*-pyrazole-5-boronic acid pinacol ester (171 mg), potassium carbonate (310 mg), deionized water (2 mL), and bis(triphenylphosphine)palladium(II) dichloride (53 mg) in sequence, and the system was purged with nitrogen for three times, placed into an oil bath and heated to 100 °C and reacted for 8 h. The reaction solution was cooled to room temperature and then filtered, the mother liquor was collected, the reaction solution was concentrated under reduced pressure to obtain a brown residue, and ethyl acetate (50 mL) and water (30 mL) were added, followed by stirring, washing and liquid separation, to obtain an organic phase; a saturated sodium chloride aqueous solution (30 mL) was added into the organic phase, followed by stirring, washing and liquid separation, and the residue was purified by silica gel column chromatography to obtain 43 mg of compound 73.

¹H NMR (500 MHz, DMSO-*d*₆) δ 10.78 (s, 1H), 9.27 (d, *J* = 2.1 Hz, 1H), 8.55 (d, *J* = 2.1 Hz, 1H), 8.52 (s, 1H), 7.92 (d, *J* = 9.0 Hz, 2H), 7.45 - 7.37 (m, 3H), 6.46 (s, 1H), 6.19 (d, *J* = 1.8 Hz, 1H), 3.86 (s, 3H), 3.42 (s, 3H). HRMS (ESI, [M+H]⁺) *m*/*z*: 487.10835.

### Example 74: Preparation of Compound 74

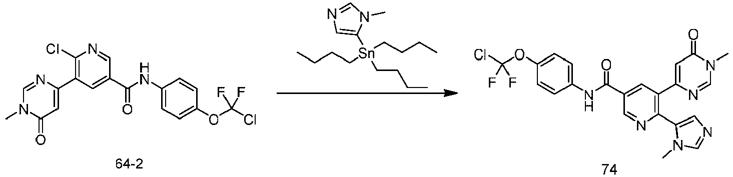

To a 30-mL tube were added compound 64-2 (250 mg), *N,N*-dimethylformamide (10 mL), 1-methyl-5-(tri-n-butyltin)imidazole (275 mg) and [1,1'-bis(diphenylphosphino)ferrocene]palladium dichloride (45 mg) in sequence, and after the tube was sealed, the sealed tube was placed into a microwave reactor at 135 °C and reacted for 2 h. The reaction solution was cooled to room temperature and then filtered, the mother liquor was collected, the reaction solution was concentrated under reduced pressure to obtain a brown residue, and ethyl acetate (50 mL) and water (30 mL) were added, followed by stirring, washing and liquid separation, to obtain an organic phase; a saturated sodium chloride aqueous solution (30 mL) was added into the organic phase, followed by stirring, washing and liquid separation, and the residue was purified by silica gel column chromatography to obtain 160 mg of compound 74.

¹H NMR (500 MHz, DMSO-*d*₆) δ 10.70 (s, 1H), 9.23 (d, *J* = 2.2 Hz, 1H), 8.54 (s, 1H), 8.45 (d, *J* = 2.2 Hz, 1H), 7.91 (d, *J* = 9.1 Hz, 2H), 7.79 (s, 1H), 7.39 (d, *J* = 8.9 Hz, 2H), 6.84 (s, 1H), 6.54 (s, 1H), 3.79 (s, 3H), 3.45 (s, 3H). HRMS (ESI, [M+H]⁺) *m*/*z*: 487.1080.

### Example 75: Preparation of Compound 75

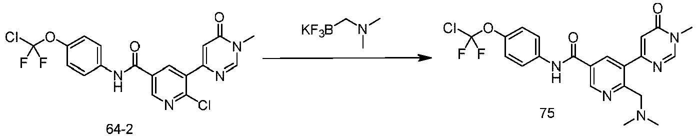

To a 35-mL microwave tube were added compound 64-2 (400 mg), cesium carbonate (591 mg), *N*,*N*-dimethyl-1-(trifluoro-λ⁴-boryl)methylamine potassium salt (194 mg), methanesulfonic acid(2-dicyclohexylphosphino-2',6'-diisopropoxy-1,1'-biphenyl)(2-amino-1,1'-biphenyl-2-yl)palladium(II) (76 mg), 2-dicyclohexylphosphino-2',6'-diisopropoxy-1,1'-biphenyl (85 mg), dioxane (10 mL), and water (1 mL) in sequence. After the addition was completed, the reaction system was purged with nitrogen for 30 s, and placed into a microwave reactor, where the microwave conditions were as follows: 120 °C, and 90 min. After the reaction was completed, the system was filtered through diatomite under vacuum and purified by silica gel column chromatography to obtain 50 mg of compound 75.

¹H NMR (500 MHz, DMSO-*d*₆) δ 10.68 (s, 1H), 9.09 (d, *J* = 2.0 Hz, 1H), 8.61 (s, 1H), 8.42 (d, *J* = 2.0 Hz, 1H), 7.90 (d, *J* = 9.0 Hz, 2H), 7.39 (d, *J* = 8.8 Hz, 2H), 6.98 (s, 1H), 3.77 (s, 2H), 3.49 (s, 3H), 2.13 (s, 6H). HRMS (ESI, [M+H]⁺) *m*/*z*: 464.1294.

### Example 76: Preparation of Compound 76

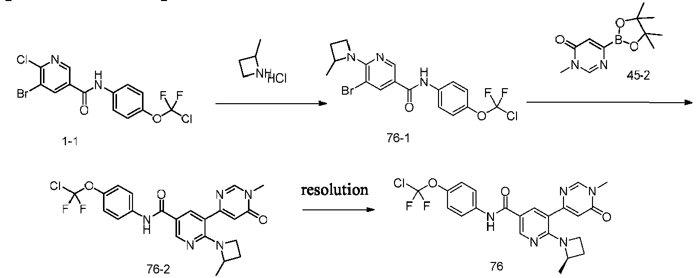

### Step A: Preparation of compound 76-1

Referring to the method of the step A in Example 48, compound 1-1 and 2-methylazetidine hydrochloride were used to prepare compound 76-1. MS (ESI, [M+H]⁺) m/z: 446.02.

### Step B: Preparation of compound 76-2

Referring to the preparation method of the step B in Example 48, compound 45-2 was reacted with compound 76-1 to obtain compound 76-2. MS (ESI, [M+H]⁺) *m*/*z:* 476.15.

### Step C: Preparation of compound 76

Compound 76-2 was subjected to chiral resolution and purified by YMC high pressure preparative chromatography (CHIRALART Cellulose-SC column, flow rate: 40 mL/min, mobile phase: ethanol/n-hexane = 2:3) to obtain compound 76.

¹H NMR (500 MHz, DMSO-*d*₆) δ 10.28 (s, 1H), 8.80 (d, *J* = 2.3 Hz, 1H), 8.57 (s, 1H), 8.23 (d, *J* = 2.3 Hz, 1H), 7.87 (d, *J* = 9.1 Hz, 2H), 7.34 (d, *J* = 8.9 Hz, 2H), 6.47 (s, 1H), 4.57 - 4.44 (m, 1H), 4.01 - 3.87 (m, 1H), 3.50 - 3.47 (m, 1H), 3.46 (s, 3H), 2.45 - 2.36 (m, 1H), 1.93 - 1.84 (m, 1H), 1.34 (d, *J* = 6.2 Hz, 3H). HRMS (ESI, [M+H]⁺) *m*/*z*: 476.13008.

### Example 77: Preparation of Compound 77

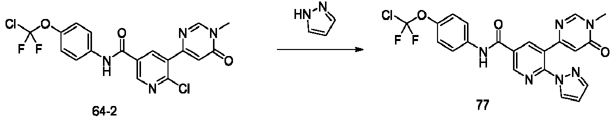

To a 10-mL microwave tube were added compound 64-2 (150 mg), dimethyl sulfoxide (5 mL), potassium *tert*-butoxide (114 mg) and pyrazole (35 mg) in sequence, and after the addition was completed, the system was placed into a microwave reactor at 120 °C and reacted for 0.5 h under nitrogen atmosphere; ethyl acetate (50 mL) and a saturated brine solution (25 mL) were added into the reaction solution for washing, following by liquid separation, and an organic phase was collected and purified by silica gel column chromatography to obtain 70 mg of compound 77.

¹H NMR (500 MHz, DMSO-*d*₆) δ 10.73 (s, 1H), 9.10 (d, *J* = 2.2 Hz, 1H), 8.61 (d, *J* = 2.2 Hz, 1H), 8.51 - 8.43 (m, 2H), 7.96 - 7.88 (m, 2H), 7.69 (d, *J* = 1.0 Hz, 1H), 7.41 (d, *J* = 8.9 Hz, 2H), 6.59 - 6.50 (m, 1H), 6.39 (s, 1H), 3.44 (s, 3H). HRMS (ESI, [M+H]⁺) *m*/*z*: 473.09397.

### Example 78: Preparation of Compound 78

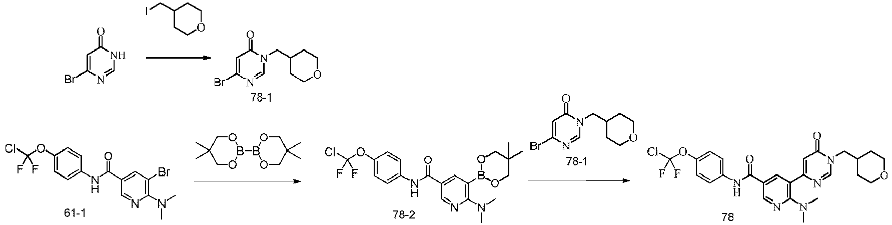

### Step A: Preparation of compound 78-1

Referring to the preparation method of the step A in Example 45, 6-bromopyrimidin-4(3*H*)-one was reacted with 4-iodomethyl tetrahydropyran to obtain compound 78-1. MS (ESI, [M+H]⁺) *m*/*z*: 273.04.

### Step B: Preparation of compound 78-2

Referring to the preparation method of the step B in Example 62, compound 61-1 was reacted with bis(neopentyl glycolato)diboron to obtain a reaction solution of compound 78-2, which was directly used in the next step without purification.

### Step C: Preparation of compound 78

Referring to the preparation method of the step C in Example 62, the reaction solution of compound 78-2 obtained in the step B above was reacted with compound 78-1 obtained in the step A to obtain compound 78.

¹H NMR (500 MHz, DMSO-*d*₆) δ 10.27 (s, 1H), 8.81 (d, *J* = 30.3 Hz, 2H), 8.31 (s, 1H), 7.87 (d, *J* = 8.0 Hz, 2H), 7.35 (d, *J* = 7.5 Hz, 2H), 7.11 (s, 1H), 4.27 (d, *J* = 5.5 Hz, 2H), 3.88 (d, *J* = 8.6 Hz, 2H), 2.87 (s, 6H), 2.06 (s, 1H), 1.67 (d, *J* = 11.7 Hz, 2H), 1.46 - 1.23 (m, 2H). HRMS (ESI, [M+H]⁺) *m*/*z*: 534.17145.

### Example 79: Preparation of Compound 79

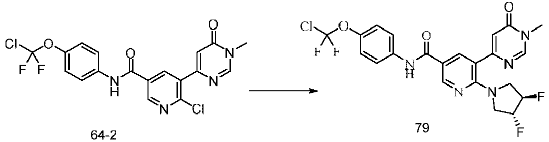

Referring to the method of the step C in Example 64, compound 64-2 and (3R,4R)-3,4-difluoropyrrolidine hydrochloride were used to prepare compound 79.

¹H NMR (500 MHz, DMSO-*d*₆) δ 10.29 (s, 1H), 8.81 (d, *J* = 2.1 Hz, 1H), 8.57 (s, 1H), 8.22 (d, *J* = 2.1 Hz, 1H), 7.87 (d, *J* = 9.0 Hz, 2H), 7.35 (d, *J* = 8.8 Hz, 2H), 6.63 (s, 1H), 5.51 - 5.39 (m, 1H), 5.38 - 5.27 (m, 1H), 3.96 - 3.84 (m, 1H), 3.83 - 3.73 (m, 1H), 3.61 (dd, *J* = 26.2, 14.1 Hz, 2H), 3.47 (s, 3H).

HRMS (ESI, [M+H]⁺) *m*/*z*: 512.11122.

### Example 80: Preparation of Compound 80

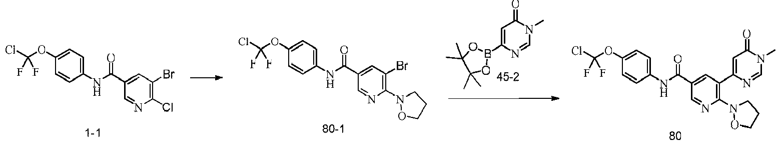

### Step A: Preparation of compound 80-1

Referring to the method of the step A in Example 48, compound 1-1 and isoxazolidine hydrochloride were used to prepare compound 80-1.

### Step B: Preparation of compound 80

Referring to the preparation method of the step B in Example 48, compound 45-2 was reacted with compound 80-1 to obtain compound 80.

¹H NMR (500 MHz, DMSO-*d*₆) δ 10.49 (s, 1H), 8.85 (s, 1H), 8.55 (s, 1H), 8.35 (s, 1H), 7.88 (d, *J* = 8.9 Hz, 2H), 7.37 (d, *J* = 8.6 Hz, 2H), 6.67 (s, 1H), 3.80-3.83 (m, 2H), 3.68-3.70 (m, 2H), 3.47 (s, 3H), 2.13-2.18 (m, 2H). HRMS (ESI, [M+H]⁺) *m*/*z*: 478.10924.

### Example 81: Preparation of Compound 81

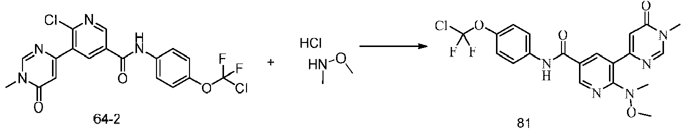

To a 10-mL microwave tube were added compound 64-2 (150 mg), 1,4-dioxane (4 mL) and dimethylhydroxylamine hydrochloride (663 mg) in sequence. The liquid level of the system was purged with nitrogen for a moment, and then the tube was sealed; the sealed tube was reacted in a microwave reactor at 140 °C for 6 h, followed by purification by silica gel column chromatography to obtain compound 81.

¹H NMR (500 MHz, DMSO-*d*₆) δ 10.45 (s, 1H), 8.85 (s, 1H), 8.55 (s, 1H), 8.16 (s, 1H), 7.87 (d, *J* = 9.0 Hz, 2H), 7.36 (d, *J* = 8.8 Hz, 2H), 6.52 (s, 1H), 3.47 (s, 3H), 3.35 (s, 3H), 3.19 (s, 3H). HRMS (ESI, [M+H]⁺) *m*/*z*: 466.1083.

### Example 82: Preparation of Compound 82

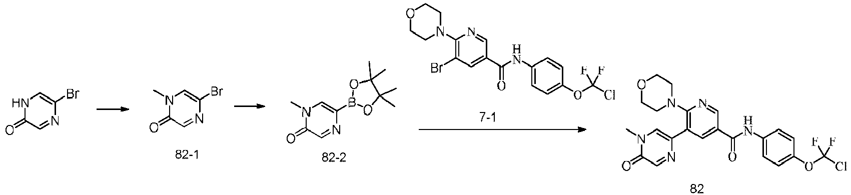

### Step A: Preparation of compound 82-1

To a 50-mL single-necked flask were added *N*,*N*-dimethylformamide (10 mL), 5-bromopyrazine-2(1*H*)-one (500 mg) and cesium carbonate (1369 mg) in sequence, and after the addition was completed, the system was stirred; iodomethane (419 mg/2 mL) diluted by *N,N*-dimethylformamide was added slowly and dropwise into the system at room temperature, and after the addition was completed, the system was reacted at room temperature for 4 h; purified water (10 mL) and ethyl acetate (30 mL) were added into the system, followed by stirring, washing and liquid separation; the aqueous phase was extracted with ethyl acetate (20 mL × 1), and the organic phases were combined, and stirred and washed with purified water (20 mL) and a saturated brine solution (20 mL) twice, followed by liquid separation, to obtain an organic phase; the organic phase was concentrated under reduced pressure, and the resulting crude product was subjected to silica gel column chromatography to obtain 120 mg of compound 82-1. MS (ESI, [M+H]⁺) *m*/*z*: 188.97.

### Step B: Preparation of compound 82-2

Referring to the method of the step D in Example 1, compound 82-1 was reacted with bis(pinacolato)diboron to obtain a reaction solution containing compound 82-2, which was cooled to room temperature and directly used in the next step without separation and purification.

### Step C: Preparation of compound 82

Referring to the method of the step B in Example 48, compound 7-1 was reacted with compound 82-2 to obtain compound 82.

¹H NMR (500 MHz, DMSO-*d*₆) δ 10.39 (s, 1H), 8.77 (d, *J* = 2.3 Hz, 1H), 8.25 (d, *J* = 2.4 Hz, 1H), 8.22 (d, *J* = 1.1 Hz, 1H), 8.14 (d, *J* = 1.1 Hz, 1H), 7.92 - 7.83 (m, 2H), 7.41 - 7.29 (m, 2H), 3.64 (t, *J* = 4.5 Hz, 4H), 3.53 (s, 3H), 3.27 (t, *J* = 4.6 Hz, 4H). HRMS (ESI, [M+H]⁺) *m*/*z:* 492.12502.

### Example 83: Preparation of Compound 83

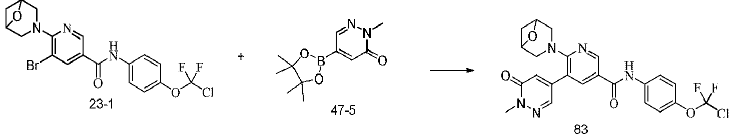

Referring to the preparation method of the step B in Example 48, compound 23-1 was reacted with compound 47-5 to obtain compound 83.

¹H NMR (500 MHz, DMSO-*d*₆) δ 10.25 (s, 1H), 8.83 (d, *J* = 2.3 Hz, 1H), 8.16 (d, *J* = 2.4 Hz, 1H), 8.09 (d, *J* = 2.1 Hz, 1H), 7.89 - 7.82 (m, 2H), 7.40 - 7.30 (m, 2H), 7.05 (d, *J* = 2.2 Hz, 1H), 4.57 (d, *J* = 6.3 Hz, 2H), 3.78 (d, *J* = 12.7 Hz, 2H), 3.70 (s, 3H), 3.64 - 3.56 (m, 2H), 3.08 - 2.99 (m, 1H), 1.78 (d, *J* = 8.7 Hz, 1H). HRMS (ESI, [M+H]⁺) *m*/*z:* 504.12506.

### Example 84: Preparation of Compound 84

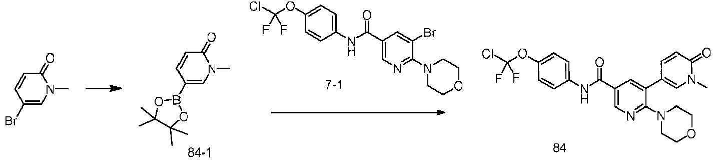

### Step A: Preparation of compound 84-1

Referring to the preparation method of the step D in Example 1, 5-bromo-1-methylpyridin-2(1*H*)-one was reacted with bis(pinacolato)diboron to obtain a reaction solution of compound 84-1, which was directly used in the next step without separation and purification.

### Step B: Preparation of compound 84

Referring to the preparation method of the step B in Example 48, the reaction solution of compound 84-1 was reacted with compound 7-1 to obtain compound 84.

¹H NMR (500 MHz, DMSO-*d*₆) δ 10.36 (s, 1H), 8.75 (s, 1H), 8.06 (d, *J* = 14.7 Hz, 2H), 7.88 (d, *J* = 8.6 Hz, 2H), 7.79 (d, *J* = 8.8 Hz, 1H), 7.36 (d, *J* = 8.4 Hz, 2H), 6.50 (d, *J* = 9.4 Hz, 1H), 3.63 (s, 4H), 3.51 (s, 3H), 3.21 (s, 4H). HRMS (ESI, [M+H]⁺) *m*/*z*: 491.1295.

### Example 85: Preparation of Compound 85

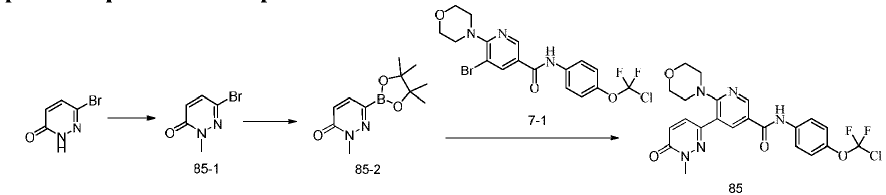

### Step A: Preparation of compound 85-1

Referring to the method of the step A in Example 82, 6-bromopyridazin-3(2*H*)-one was reacted with iodomethane to obtain compound 85-1. MS (ESI, [M+H]⁺) *m*/*z*: 189.0.

### Step B: Preparation of compound 85-2

Referring to the preparation method of the step D in Example 1, compound 85-1 was reacted with bis(pinacolato)diboron to obtain a reaction solution of compound 85-2, which was directly used in the next step without separation and purification.

### Step C: Preparation of compound 85

Referring to the preparation method of the step B in Example 48, the reaction solution of compound 85-2 was reacted with compound 7-1 to obtain compound 85.

¹H NMR (500 MHz, DMSO-*d*₆) δ 10.43 (s, 1H), 8.86 (d, *J* = 2.4 Hz, 1H), 8.25 (d, *J* = 2.4 Hz, 1H), 7.91 - 7.84 (m, 3H), 7.42 - 7.32 (m, 2H), 7.05 (d, *J* = 9.6 Hz, 1H), 3.75 (s, 3H), 3.67 - 3.60 (m, 4H), 3.26 - 3.20 (m, 4H). HRMS (ESI, [M+H]⁺) *m*/*z*: 492.12480.

### Example 86: Preparation of Compound 86

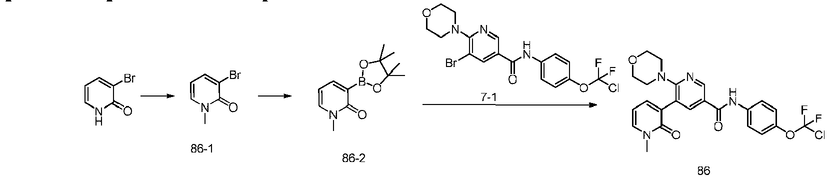

### Step A: Preparation of compound 86-1

Referring to the method of the step C in Example 1, 3-bromopyridin-2(1*H*)-one was reacted with iodomethane to obtain compound 86-1.

¹H NMR (500 MHz, DMSO-*d*₆) δ 7.90 (dd, *J* = 7.3, 1.8 Hz, 1H), 7.77 (dd, *J* = 6.7, 1.8 Hz, 1H), 6.17 (t, *J* = 7.0 Hz, 1H), 3.50 (s, 3H). MS (ESI, [M+H]⁺) *m*/*z*: 187.99.

### Step B: Preparation of compound 86-2

Referring to the preparation method of the step D in Example 1, compound 86-1 was reacted with bis(pinacolato)diboron to obtain a reaction solution containing compound 86-2, which was directly used in the next step without purification.

### Step C: Preparation of compound 86

Referring to the preparation method of the step B in Example 48, a reaction solution containing compound 86-2 was reacted with compound 7-1 to obtain compound 86.

¹H NMR (500 MHz, DMSO-*d*₆) δ 10.28 (s, 1H), 8.74 (d, *J* = 2.2 Hz, 1H), 8.05 (d, *J* = 2.2 Hz, 1H), 7.87 (d, *J* = 9.0 Hz, 2H), 7.79 (dd, *J* = 6.7, 1.7 Hz, 1H), 7.63 (dd, *J* = 6.9, 1.7 Hz, 1H), 7.34 (d, *J* = 8.8 Hz, 2H), 6.35 (t, *J=* 6.8 Hz, 1H), 3.59 - 3.54 (m, 4H), 3.53 (s, 3H), 3.29 - 3.23 (m, 4H). HRMS (ESI, [M+H]⁺) *m*/*z*: 491.1307.

### Example 87: Preparation of Compound 87

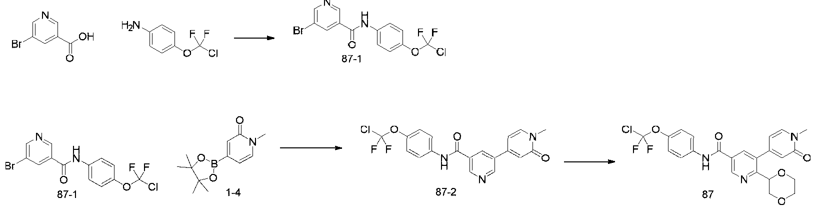

### Step A: Preparation of compound 87-1

Referring to the preparation method of the step A in Example 1, 5-bromonicotinic acid was reacted with 4-(chlorodifluoromethoxy)aniline to obtain compound 87-1. MS (ESI, [M+H]⁺) *m*/*z:* 377.0.

### Step B: Preparation of compound 87-2

Referring to the preparation method of the step E in Example 1, compound 87-1 was reacted with compound 1-4 to obtain compound 87-2. MS (ESI, [M+H]⁺) *m*/*z:* 406.17.

### Step C: Preparation of compound 87

To a microwave tube were added compound 87-2 (100 mg), dibenzoyl peroxide (103 mg), trifluoroacetic acid (36 mg) and dioxane (8 mL) in sequence, and after the addition was completed, the tube was sealed and placed into a microwave reactor under nitrogen atmosphere, where the microwave reaction conditions were as follows: 100 °C and 2 h. The reaction solution was filtered through diatomite, the filtrate was collected, and ethyl acetate (50 mL) and a saturated brine solution (25 mL) were added into the filtrate for washing, followed by liquid separation, and an organic phase was collected and purified by silica gel column chromatography to obtain 26 mg of compound 87.

¹H NMR (500 MHz, DMSO-*d*₆) δ 10.69 (s, 1H), 9.16 (s, 1H), 8.70 (s, 1H), 8.26 (s, 1H), 7.90 (d, *J* = 8.3 Hz, 2H), 7.81 (d, *J* = 6.6 Hz, 1H), 7.36 (d, *J* = 8.1 Hz, 2H), 6.20 (d, *J* = 6.6 Hz, 1H), 4.60 (d, *J* = 9.3 Hz, 1H), 3.97 - 3.82 (m, 1H), 3.76 - 3.63 (m, 1H), 3.56 - 3.53 (m, 1H), 3.50 (s, 3H), 3.47 - 3.40 (m, 1H), 3.37 - 3.20 (m, 2H). HRMS (ESI, [M+H]⁺) *m*/*z*: 492.11330.

### Example 88: Preparation of Compound 88

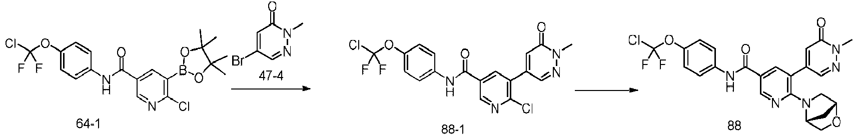

### Step A: Preparation of compound 88-1

Referring to the preparation method of the step B in Example 64, compound 64-1 was reacted with compound 47-4 to obtain compound 88-1. MS (ESI, [M+H]⁺) *m*/*z*: 441.05.

### Step B: Preparation of compound 88

Referring to the method of the step C in Example 64, compound 88-1 and (1*R*,4*R*)-2-oxa-5-azabicyclo[2.2.1]heptane hydrochloride were used to prepare compound 88.

¹H NMR (500 MHz, DMSO-*d*₆) δ 10.24 (s, 1H), 8.79 (d, *J* = 1.9 Hz, 1H), 8.17 (d, *J* = 1.9 Hz, 1H), 8.07 - 7.99 (m, 1H), 7.86 (d, *J* = 9.0 Hz, 2H), 7.35 (d, *J* = 8.7 Hz, 2H), 6.98 (s, 1H), 4.95 (s, 1H), 4.55 (s, 1H), 3.79 (s, 2H), 3.70 (s, 3H), 3.23 (d, *J* = 9.6 Hz, 1H), 2.81 (d, *J* = 9.9 Hz, 1H), 1.90 (d, *J* = 8.8 Hz, 1H), 1.82 (d, *J* = 9.8 Hz, 1H). HRMS (ESI, [M+H]⁺) *m*/*z*: 504.12449.

### Example 89: Preparation of Compound 89

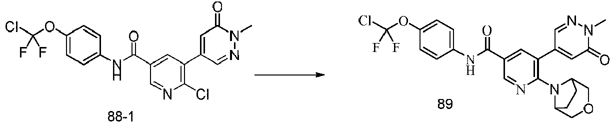

Referring to the method of the step C in Example 64, compound 88-1 and 3-oxa-8-azabicyclo[3.2.1]octane hydrochloride were used to prepare compound 89.

¹H NMR (500 MHz, DMSO-*d*₆) δ 10.32 (s, 1H), 8.81 (d, *J* = 2.2 Hz, 1H), 8.20 (dd, *J* = 10.7, 2.1 Hz, 2H), 7.86 (d, *J* = 9.0 Hz, 2H), 7.36 (d, *J* = 8.8 Hz, 2H), 7.18 (d, *J* = 2.0 Hz, 1H), 4.15 (s, 2H), 3.69 (s, 3H), 3.66 (d, *J* = 10.7 Hz, 2H), 3.50 (d, *J* = 10.4 Hz, 2H), 1.89 - 1.82 (m, 2H), 1.79 (dd, *J* = 13.8, 8.8 Hz, 2H). HRMS (ESI, [M+H]⁺) *m*/*z:* 518.14261.

### Example 90: Preparation of Compound 90

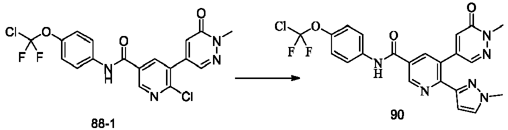

To a 35-mL microwave tube were added compound 88-1 (200 mg), 1-methyl-4-(4,4,5,5-tetramethyl-1,3,2-dioxaborolan-2-yl)-1*H*-pyrazole (113 mg), potassium carbonate (63 mg), tetrakis(triphenylphosphine)palladium (20 mL), and dioxane (15 mL) in sequence, and after the addition was completed, the tube was sealed and placed into a microwave reactor under nitrogen atmosphere, where the microwave reaction conditions were as follows: 140 °C and 2 h. The reaction solution was filtered through diatomite, the filtrate was collected, and ethyl acetate (50 mL) and a saturated brine solution (25 mL) were added into the filtrate for washing, followed by liquid separation, and an organic phase was collected and purified by silica gel column chromatography to obtain 100 mg of compound 90.

¹H NMR (500 MHz, CDCl₃) δ 9.06 (s, 1H), 8.41 (s, 1H), 8.19 (s, 1H), 8.12 (s, 1H), 7.80 (s, 1H), 7.71 (d, *J* = 8.7 Hz, 2H), 7.40 (s, 1H), 7.26 (d, *J* = 6.6 Hz, 2H), 6.58 (s, 1H), 3.88 (s, 3H), 3.57 (s, 3H). HRMS (ESI, [M+H]⁺) *m*/*z*: 487.10909.

### Example 91: Preparation of Compound 91

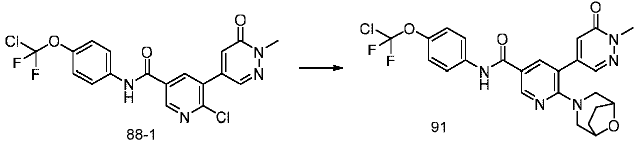

Referring to the method of the step C in Example 64, compound 88-1 and 8-oxo-3-azabicyclo[3,2,1]octane hydrochloride were used to prepare compound 91.

¹H NMR (500 MHz, DMSO-*d*₆) δ 10.31 (s, 1H), 8.82 (d, *J* = 2.2 Hz, 1H), 8.20 (d, *J* = 2.2 Hz, 1H), 8.09 (d, *J* = 2.0 Hz, 1H), 7.86 (d, *J* = 9.0 Hz, 2H), 7.36 (d, *J* = 8.8 Hz, 2H), 7.11 (d, *J* = 2.0 Hz, 1H), 4.30 (s, 2H), 3.71 (s, 3H), 3.45 (d, *J* = 12.4 Hz, 2H), 3.08 (d, *J* = 11.6 Hz, 2H), 1.74 (s, 4H). HRMS (ESI, [M+H]⁺) *m*/*z*: 518.1404.

### Example 92: Preparation of Compound 92

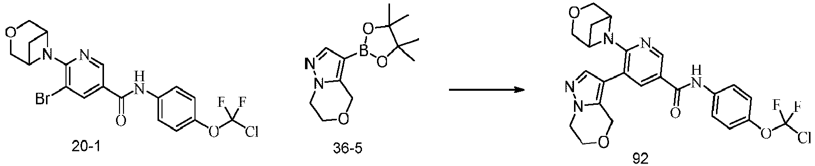

Referring to the preparation method of the step B in Example 48, compound 20-1 was reacted with compound 36-5 to obtain compound 92.

¹H NMR (500 MHz, DMSO-*d*₆) δ 10.26 (s, 1H), 8.72 (d, *J* = 2.3 Hz, 1H), 7.95 (d, *J* = 2.3 Hz, 1H), 7.90 - 7.82 (m, 2H), 7.66 (s, 1H), 7.39 - 7.26 (m, 2H), 4.69 (s, 2H), 4.21 - 4.14 (m, 2H), 4.13 - 4.06 (m, 2H), 4.03 - 3.85 (m, 4H), 3.61 - 3.51 (m, 2H), 2.70 - 2.60 (m, 1H), 1.70 (d, *J* = 8.1 Hz, 1H). HRMS (ESI, [M+H]⁺) *m*/*z*: 518.14062.

### Example 93: Preparation of Compound 93

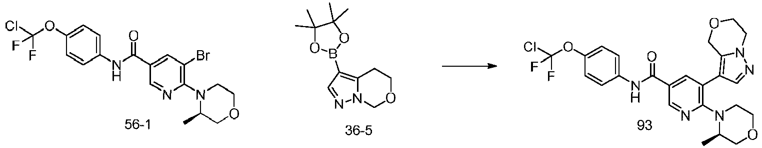

### Step A: Preparation of compound 93

Referring to the preparation method of the step B in Example 48, compound 56-1 was reacted with compound 36-5 to obtain compound 93.

¹H NMR (500 MHz, DMSO-*d*₆) δ 10.35 (s, 1H), 8.74 (d, *J* = 2.3 Hz, 1H), 7.97 (d, *J* = 2.3 Hz, 1H), 7.87 (d, *J* = 9.1 Hz, 2H), 7.79 (s, 1H), 7.35 (d, *J* = 8.9 Hz, 2H), 5.01 (d, *J* = 15.1 Hz, 1H), 4.68 (d, *J* = 15.1 Hz, 1H), 4.23 - 4.06 (m, 4H), 3.77 - 3.64 (m, 2H), 3.62 (dd, *J* = 11.2, 2.8 Hz, 1H), 3.58 - 3.50 (m, 1H), 3.46 (dd, *J* = 11.3, 3.4 Hz, 1H), 3.21 (ddd, *J* = 12.7, 9.6, 3.0 Hz, 1H), 3.10 (d, *J* = 13.4 Hz, 1H), 0.99 (d, *J* = 6.5 Hz, 3H). HRMS (ESI, [M+H]⁺) *m*/*z*: 520.15643.

### Example 94: Preparation of Compound 94

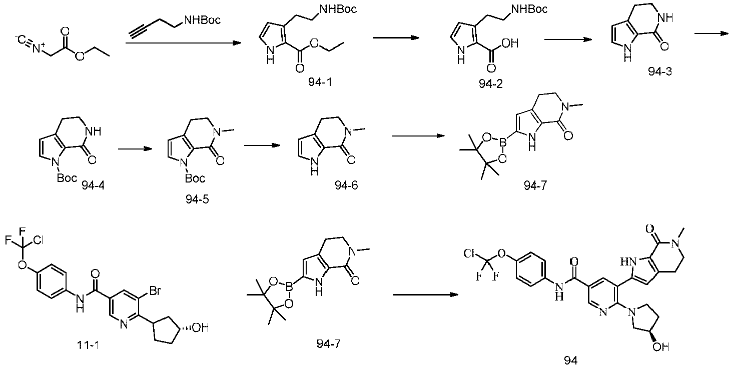

### Step A: Preparation of compound 94-1

To a 100-mL single-necked flask were added ethyl 2-isocyanatoacetate (1 g), anhydrous dioxane (10 mL), N BOC-3-butyn-1-amine (2.24 g) and silver carbonate (0.25 g) in sequence, and the resulting mixture was heated to 80 °C in an oil bath under nitrogen atmosphere and reacted overnight. After the reaction was completed, ethyl acetate (50 mL) and a saturated brine solution (25 mL) were added into the reaction solution for washing, followed by liquid separation, and an organic phase was collected and purified by silica gel column chromatography to obtain 1 g of compound 94-1. MS (ESI, [M+Na]⁺) *m*/*z:* 305.14.

### Step B: Preparation of compound 94-2

To a 50-mL single-necked flask were added compound 94-1 (1.2 g), methanol (10 mL) and a 2 M lithium hydroxide aqueous solution (5 mL) in sequence, and the system was reacted in an oil bath at 50 °C overnight. After the reaction was completed, the pH was adjusted to 3-4 by using 2 M diluted hydrochloric acid, and ethyl acetate (50 mL) and a saturated brine solution (25 mL) were added into the reaction solution for washing, followed by liquid separation; an organic phase was collected, dried over anhydrous sodium sulfate, filtered, and concentrated to obtain 0.6 g of compound 94-2. MS (ESI, [M-H]⁻) m/z: 253.13.

### Step C: Preparation of compound 94-3

To a 50-mL single-necked flask were added compound 94-2 (0.47 g) and dichloromethane (10 mL) in sequence, thionyl chloride (0.27 g) was added slowly and dropwise, and after the addition was completed, the system was stirred at room temperature for 2 h. After the reaction was completed, the pH was adjusted to alkalinity by using a saturated sodium carbonate aqueous solution, and the system was extracted with ethyl acetate (100 mL × 5); the organic phases were combined, dried over anhydrous sodium sulfate, filtered, and concentrated to obtain 0.25 g of compound 94-3. MS (ESI, [M+H]⁺) *m*/*z:* 137.1.

### Step D: Preparation of compound 94-4

To a 100-mL single-necked flask were added compound 94-3 (0.22 g), dichloromethane (10 mL), triethylamine (0.33 g), di-*tert*-butyl dicarbonate (0.36 g) and 4-dimethylaminopyridine (10 mg) in sequence, and the resulting mixture was stirred at room temperature for 2 h under nitrogen atmosphere. After the reaction was completed, ethyl acetate (50 mL) and a saturated brine solution (25 mL) were added into the reaction solution for washing, followed by liquid separation, and an organic phase was collected and purified by silica gel column chromatography to obtain 0.13 g of compound 94-4. MS (ESI, [M+H-C(CH₃)₃]⁺) *m*/*z*: 181.10.

### Step E: Preparation of compound 94-5

To a 50-mL single-necked flask were added compound 94-4 (0.1 g), acetonitrile (10 mL), iodomethane (0.1 g), and cesium carbonate (0.28 g) in sequence, and the resulting mixture was reacted in an oil bath at 80 °C overnight under nitrogen atmosphere. After the reaction was completed, ethyl acetate (50 mL) and a saturated brine solution (25 mL) were added into the reaction solution for washing, followed by liquid separation, and an organic phase was collected and purified by silica gel column chromatography to obtain 69 mg of compound 94-5. MS (ESI, [M+H-C(CH₃)₃]⁺) *m*/*z*: 195.10.

### Step F: Preparation of compound 94-6

To a 50-mL single-necked flask were added compound 94-5 (1 g) and a 2 M dioxane solution of hydrochloric acid (10 mL) in sequence, and the resulting mixture was stirred at room temperature for 2 h under nitrogen atmosphere. After the reaction was completed, triethylamine (5 mL) was added into the reaction system to adjust the pH to alkalinity, and ethyl acetate (50 mL) and a saturated brine solution (25 mL) were added into the reaction solution for washing, followed by liquid separation; an organic phase was collected and purified by silica gel column chromatography to obtain 0.5 g of compound 94-6. MS (ESI, [M+H]⁺) *m*/*z:* 151.06.

### Step G: Preparation of compound 94-7

To a 50-mL single-necked flask were added compound 94-6 (0.1 g), anhydrous tetrahydrofuran (10 mL), bis(pinacolato)diboron (0.25 g), 3,4,7,8-tetramethyl-1,10-phenanthroline (10 mg), and (1,5-cyclooctadiene)(methoxy)iridium(I) dimer (25 mg) in sequence, and the resulting mixture was heated in an oil bath to 70 °C under nitrogen atmosphere and refluxed for 4 h. After the reaction was completed, the reaction mixture was concentrated under reduced pressure to remove the solvent, which was directly used in the next step without separation and purification. MS (ESI, [M+H]⁺) *m*/*z*: 277.18.

### Step H: Preparation of compound 94

To a 50-mL single-necked flask were added compound 11-1 (100 mg), dioxane (10 mL), compound 94-7 (72 mg), anhydrous potassium carbonate (30 mg), tetrakis(triphenylphosphine)palladium (30 mg), and water (1 mL) in sequence. After the addition was completed, the system was heated in an oil bath to 100 °C and reacted overnight under nitrogen atmosphere. Ethyl acetate (50 mL) and a saturated brine solution (25 mL) were added into the reaction solution for washing, followed by liquid separation, and an organic phase was collected and purified by silica gel column chromatography to obtain 30 mg of compound 94.

¹H NMR (500 MHz, DMSO-*d*₆) δ 11.80 (d, *J* = 1.6 Hz, 1H), 10.14 (s, 1H), 8.71 (d, *J* = 2.4 Hz, 1H), 8.05 (d, *J* = 2.4 Hz, 1H), 7.93 - 7.84 (m, 2H), 7.33 (d, *J* = 9.0 Hz, 2H), 6.01 (d, *J* = 2.0 Hz, 1H), 4.87 (d, *J* = 3.4 Hz, 1H), 4.22 (d, *J* = 2.4 Hz, 1H), 3.58 - 3.49 (m, 2H), 3.49 - 3.41 (m, 1H), 3.30 (dd, *J* = 6.9, 4.5 Hz, 2H), 2.99 (d, *J* = 11.7 Hz, 1H), 2.93 (s, 3H), 2.78 (t, *J* = 6.9 Hz, 2H), 1.84 (ddd, *J* = 12.6, 8.6, 4.3 Hz, 1H), 1.78 - 1.70 (m, 1H). HRMS (ESI, [M+H]⁺) *m*/*z*: 532.15638.

### Example 95: Preparation of Compound 95

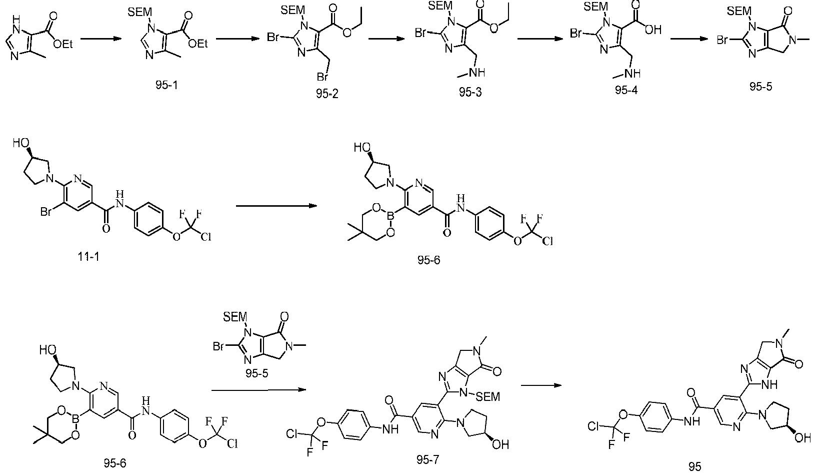

### Step A: Preparation of compound 95-1

To a 250-mL single-necked flask was added anhydrous *N*,*N*-dimethylformamide (150 mL), the system was stirred, and then 4-methyl-5-imidazole ethyl formate (10.0 g) was added into the system; the system was cooled to 0 °C, sodium hydrogen (3.11 g to the content of 60% w/w) was added into the system in portions, and after the addition was completed, the system was stirred at a constant temperature for 30 min, and then 2-(trimethylsilyl)ethoxymethyl chloride (12.98 g) was added dropwise into the system; after the addition was completed, the system was heated to room temperature and reacted for 4 h. Ethyl acetate (100 mL) and a saturated ammonium chloride aqueous solution (100 mL) were added into the system, followed by stirring, washing and liquid separation, and the aqueous phase was extracted with ethyl acetate (100 mL × 1); the organic phases were combined, and a saturated sodium chloride aqueous solution (150 mL) was added into the system, followed by stirring, washing and liquid separation; the organic phase was concentrated under reduced pressure to obtain a residue, which was purified by silica gel column chromatography to obtain 13.18 g of compound 95-1. MS (ESI, [M+H]⁺) *m*/*z:* 285.20.

### Step B: Preparation of compound 95-2

To a 250-mL single-necked flask were added 1,2-dichloroethane (100 mL), compound 95-1 (4.98 g), *N*-bromosuccinimide (6.48 g) and azobisisobutyronitrile (0.257 g) in sequence, and after the addition was completed, the system was purged with nitrogen and heated to 70-80 °C and reacted for 6 h. The reaction solution was cooled to room temperature and filtered, and the filtrate was concentrated under reduced pressure to obtain a residue, which was purified by silica gel column chromatography to obtain 2.20 g of compound 95-2. MS (ESI, [M+Na]⁺) *̅m̅*̅/̅*̅z̅*̅: 463.03.

### Step C: Preparation of compound 95-3

To a 100-mL single-necked flask were added anhydrous ethanol (50 mL), methylamine hydrochloride (1.527 g) and *N*,*N*-diisopropylethylamine (5.85 g) in sequence, and after the addition was completed, the system was stirred for 30 min, and compound 95-2 (2.0 g) obtained in the step B above was then added into the system; after the addition was completed, the reaction solution was reacted at room temperature overnight, and then concentrated under reduced pressure, and the resulting residue was subjected to silica gel column chromatography to obtain 570 mg of compound 95-3. MS (ESI, [M+H]⁺) *m*/*z:* 392.05.

### Step D: Preparation of compound 95-4

To a 100-mL single-necked flask was added methanol (30 mL), the system was stirred, then compound 95-3 (570 mg) obtained in the step C above, lithium hydroxide (0.311 g) and purified water (10 mL) were added into the system in sequence, and after the addition was completed, the system was reacted at room temperature for 3 h; the system was concentrated under reduced pressure, ethyl acetate (20 mL) and purified water (10 mL) were added into the resulting residue, and then a 1 M hydrochloric acid aqueous solution was added dropwise into the system to adjust the pH to 6-7; the system became turbid, and a solid precipitated; the solid was filtered, and the filter cake was sequentially rinsed with purified water (5 mL) and ethyl acetate (5 mL), collected, placed into a vacuum drying oven, and dried under vacuum at 50 °C to constant weight to obtain 390 mg of compound 95-4. MS (ESI, [M+H]⁺) *m*/*z*: 364.2.

### Step E: Preparation of compound 95-5

To a 25-mL single-necked flask was added *N*,*N*-dimethylformamide (10 mL), the system was stirred, and 2-(7-azobenzotriazole)-*N*,*N*,*N'*,*N'*-tetramethylurea hexafluorophosphate (488 mg), and *N,N*-diisopropylethylamine (415 mg) were added into the system in sequence, followed by stirring for 5 min; an *N*,*N*-dimethylformamide (5 mL) dissolved *N*,*N*-dimethylformamide solution of compound 95-4 (390 mg) obtained in the step D above was added dropwise into the system; after the addition was completed, the system was reacted at room temperature for 1 h; ethyl acetate (30 mL) and purified water (10 mL) were added into the system, followed by stirring, washing and liquid separation, an organic phase was collected, and the aqueous phase was extracted with ethyl acetate (10 mL × 2); the organic phases were combined, purified water (15 mL) and a saturated sodium chloride aqueous solution (15 mL) were respectively added for stirring and washing twice, followed by liquid separation, an organic phase was collected and concentrated under reduced pressure, and the resulting residue was subjected to silica gel column chromatography to obtain 200 mg of compound 95-5. MS (ESI, [M+H]⁺) *m*/*z:* 346.1.

### Step F: Preparation of compound 95-6

Referring to the method of the step B in Example 62, compound 11-1 was reacted with bis(neopentyl glycolato)diboron to obtain compound 95-6. The reaction product was directly used in the next step without treatment.

### Step G: Preparation of compound 95-7

Referring to the preparation method of the step C in Example 62, compound 95-6 was reacted with compound 95-5 to obtain compound 95-7. MS (ESI, [M-H]⁻) *m*/*z:* 647.3.

### Step H: Preparation of compound 95

To a 25-mL single-necked flask was added dichloromethane (10 mL), the system was stirred, compound 95-7 (90 mg) obtained in the step G above and trifluoroacetic acid (4467 mg) were added into the system in sequence, and after the addition was completed, the system was reacted at room temperature for 2 h; the reaction solution was concentrated under reduced pressure, ethyl acetate (15 mL) and a saturated sodium bicarbonate aqueous solution (10 mL) were added into the resulting residue, followed by stirring, washing, and liquid separation, to obtain an organic phase; a saturated sodium chloride aqueous solution (10 mL) was then added, followed by stirring, washing and liquid separation, an organic phase was collected and concentrated under reduced pressure to obtain a concentrate, which was purified by reversed-phase chromatography to obtain compound 95 (20 mg).

¹H NMR (500 MHz, DMSO-*d*₆) δ 13.09 (d, *J* = 115.1 Hz, 1H), 10.23 (d, *J* = 8.5 Hz, 1H), 8.98 - 8.68 (m, 1H), 8.19 (d, *J* = 2.5 Hz, 1H), 7.87 (d, *J* = 8.7 Hz, 2H), 7.33 (d, *J* = 8.6 Hz, 2H), 4.89 (d, *J* = 3.2 Hz, 1H), 4.33 (t, *J* = 49.6 Hz, 3H), 3.50 - 3.39 (m, 1H), 3.27 (s, 1H), 3.03 (d, *J* = 11.5 Hz, 3H), 2.92 (d, *J* = 11.8 Hz, 1H), 1.95 - 1.68 (m, 2H). HRMS (ESI, [M+H]⁺) *m*/*z*: 519.13584.

### Example 96: Preparation of Compound 96

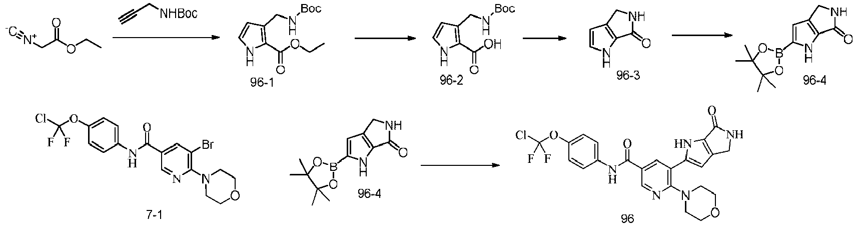

### Step A: Preparation of compound 96-1

Referring to the method of the step A in Example 94, 2-isocyanatoethyl acetate was reacted with N-Boc-aminopropyne to obtain compound 96-1. MS (ESI, [M+Na-Boc]⁺) *m*/*z:* 191.1.

### Step B: Preparation of compound 96-2

Referring to the method of the step B in Example 94, compound 96-1 was used under an alkaline condition to prepare compound 96-2. MS (ESI, [M-H]⁻) *m*/*z*: 239.214.

### Step C: Preparation of compound 96-3

Referring to the method of the step C in Example 94, compound 96-2 was reacted with thionyl chloride to obtain compound 96-3. MS (ESI, [M+H]⁺) *m*/*z*: 123.06.

### Step D: Preparation of compound 96-4

Referring to the method of the step G in Example 94, compound 96-3 was reacted with bis(pinacolato)diboron to obtain 96-4. MS (ESI, [M+H]⁺) *m*/*z*: 249.23.

### Step E: Preparation of compound 96

Referring to the method of the step H in Example 94, compound 7-1 was reacted with compound 96-4 to obtain compound 96.

¹H NMR (500 MHz, DMSO-*d*₆) δ 11.99 (s, 1H), 10.35 (s, 1H), 8.72 (d, *J* = 1.8 Hz, 1H), 8.22 (d, *J* = 1.8 Hz, 1H), 8.06 - 7.74 (m, 3H), 7.36 (d, *J* = 8.7 Hz, 2H), 6.52 (s, 1H), 4.15 (s, 2H), 3.65 (s, 4H), 3.17 (s, 4H). HRMS (ESI, [M+H]⁺) *m*/*z:* 504.12491.

### Example 97: Preparation of Compound 97

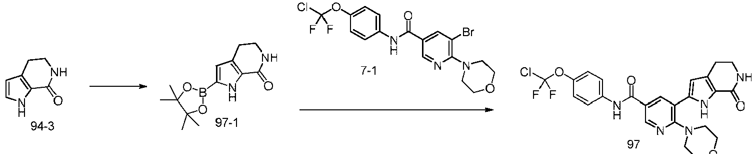

### Step A: Preparation of compound 97-1

Referring to the method of the step G in Example 94, compound 94-3 was reacted with bis(pinacolato)diboron to obtain compound 97-1. MS (ESI, [M+H]⁺) *m*/*z:* 263.23.

### Step B: Preparation of compound 97

Referring to the method of the step H in Example 94, compound 7-1 was reacted with compound 97-1 to obtain compound 97.

¹H NMR (500 MHz, DMSO-*d*₆) δ 11.82 (s, 1H), 10.32 (s, 1H), 8.69 (d, *J* = 2.2 Hz, 1H), 8.26 (d, *J* = 2.2 Hz, 1H), 7.88 (d, *J* = 9.1 Hz, 2H), 7.36 (d, *J* = 8.8 Hz, 2H), 7.21 (s, 1H), 6.44 (d, *J* = 1.8 Hz, 1H), 3.90 - 3.55 (m, 4H), 3.43 - 3.35 (m, 2H), 3.21 - 3.13 (m, 4H), 2.71 (t, *J* = 6.8 Hz, 2H). HRMS (ESI, [M+H]⁺) *m*/*z:* 518.14061.

### Example 98: Preparation of Compound 98

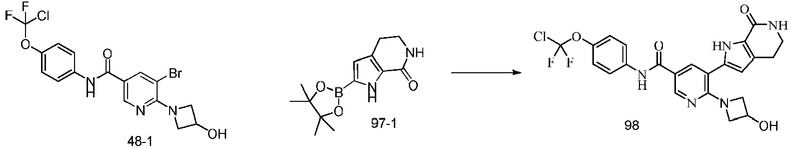

### Step A: Preparation of compound 98

Referring to the method of the step H in Example 94, compound 48-1 was reacted with compound 97-1 to obtain compound 98.

¹H NMR (500 MHz, DMSO-*d*₆) δ 11.82 (s, 1H), 10.17 (s, 1H), 8.70 (d, *J* = 2.2 Hz, 1H), 8.08 (d, *J* = 2.2 Hz, 1H), 7.87 (d, *J* = 9.1 Hz, 2H), 7.34 (d, *J* = 8.8 Hz, 2H), 7.17 (s, 1H), 6.07 (d, *J* = 1.5 Hz, 1H), 5.55 (d, *J* = 5.8 Hz, 1H), 4.42 (dd, *J* = 10.7, 5.1 Hz, 1H), 4.01 - 3.89 (m, 2H), 3.57 (dd, *J* = 9.6, 4.4 Hz, 2H), 3.39 (dd, *J* = 6.5, 4.7 Hz, 2H), 2.70 (t, *J* = 6.7 Hz, 2H). HRMS (ESI, [M+H]⁺) *m*/*z:* 504.12481.

### Example 99: Preparation of Compound 99

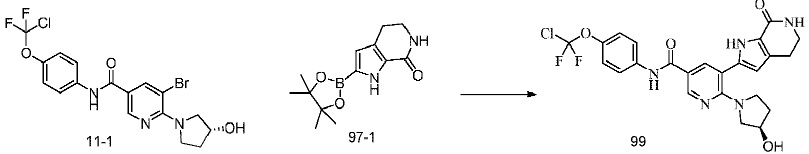

### Step A: Preparation of compound 99

Referring to the method of the step H in Example 94, compound 11-1 was reacted with compound 97-1 to obtain compound 99.

¹H NMR (500 MHz, DMSO-*d*₆) δ 11.80 (s, 1H), 10.14 (s, 1H), 8.71 (d, *J=* 2.0 Hz, 1H), 8.06 (d, *J=* 2.0 Hz, 1H), 7.87 (d, *J* = 9.0 Hz, 2H), 7.33 (d, *J* = 8.7 Hz, 2H), 7.13 (s, 1H), 6.03 (s, 1H), 4.88 (d, *J=* 3.2 Hz, 1H), 4.23 (s, 1H), 3.47 (dd, *J* = 17.5, 9.7 Hz, 1H), 3.39 (t, *J* = 5.6 Hz, 2H), 3.31 (d, *J* = 5.2 Hz, 2H), 2.99 (d, *J* = 11.6 Hz, 1H), 2.70 (t, *J* = 6.7 Hz, 2H), 1.89 - 1.79 (m, 1H), 1.76 (d, *J* = 3.5 Hz, 1H). HRMS (ESI, [M+H]⁺) *m*/*z*: 518.14020.

### Example 100: Preparation of Compound 100

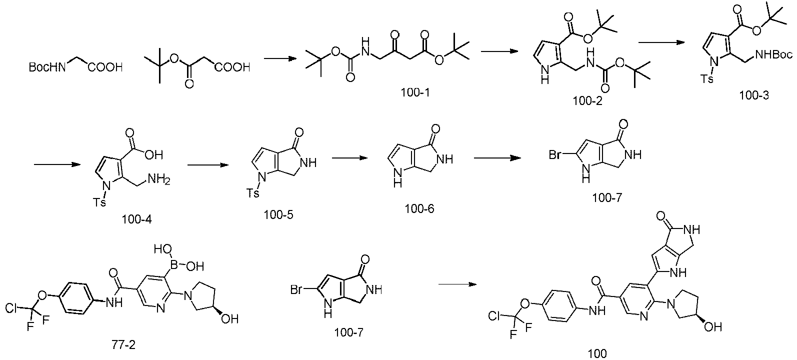

### Step A: Preparation of compound 100-1

Reaction system A: potassium *tert*-butoxide (3.50 g) was added slowly into a stirred solution of 3-(*tert*-butoxy)-3-oxopropanoic acid (5 g) and magnesium chloride (2.97 g) in tetrahydrofuran (200 mL) at -30 °C under nitrogen atmosphere, and after the dropwise addition was carried out for 30 min and completed, the resulting mixture was stirred at room temperature for 5 h. Reaction system B: *N*,*N'*-carbonyldiimidazole (4.56 g) was added into a stirred solution of (*tert*-butoxycarbonyl)glycine (4.37 g) in tetrahydrofuran (200 mL) at -30 °C under nitrogen atmosphere, and after the dropwise addition was completed, the resulting mixture was stirred at room temperature and reacted for 3 h. The reaction system B was added dropwise into the reaction system A at 0 °C, and after the dropwise addition was completed, the reaction system was transferred to reach room temperature and stirred for 2 d. Ethyl acetate (500 mL) was added into the reaction system for extraction, and the organic phase was sequentially washed with 1 M hydrochloric acid (200 mL), a saturated sodium bicarbonate aqueous solution (200 mL), and water (200 mL). After the organic phase was dried over anhydrous sodium sulfate, the solvent was distilled off under reduced pressure to obtain 8 g of compound 100-1. MS (ESI, [M+Na]⁺) m/z: 296.21.

### Step B: Preparation of compound 100-2

To a 50-mL single-necked flask were added compound 100-1 (7 g), methanol (6 mL), ammonium hydroxide (4.36 g), ammonium acetate (19.74 g) and 2-chloroacetaldehyde (20.10 g) in sequence, and the resulting mixture was heated to 50 °C for 3 h under nitrogen atmosphere. The stirring was stopped, and the reaction solution was purified by silica gel column chromatography to obtain 6.5 g of compound 100-2. MS (ESI, [M+Na]⁺) *m*/*z*: 319.25.

### Step C: Preparation of compound 100-3

Sodium *tert*-butoxide (4.22 g) was added slowly into a stirred solution of compound 100-2 (6.5 g) in tetrahydrofuran (10 mL) at 0 °C under nitrogen atmosphere, and after the addition was completed, the resulting mixture was stirred at room temperature for 1 h. A solution of 4-*p*-toluenesulfonyl chloride (5.02 g) in tetrahydrofuran (10 mL) was added slowly and dropwise at 0 °C, and after the dropwise addition was carried out for 10 min and completed, the mixture was heated to room temperature and stirred and reacted overnight. The stirring was stopped, then a saturated ammonium chloride solution (50 mL) was added into the reaction solution to quench the reaction, and ethyl acetate (200 mL) and water (200 mL) were added for extraction; the organic phase was separated, and the aqueous phase was extracted with ethyl acetate (50 mL × 2); the organic phases were combined, washed with a saturated brine solution (300 mL), dried over anhydrous sodium sulfate, and filtered, and the filtrate was purified by silica gel column chromatography to obtain 8.5 g of compound 100-3. MS (ESI, [M+Na]⁺) *m*/*z*: 473.3.

### Step D: Preparation of compound 100-4

To a 50-mL eggplant-shaped flask were added compound 100-3 (8.5 g) and dichloromethane (10 mL) in sequence, then trifluoroacetic acid (10 mL) was added under nitrogen atmosphere, and the resulting mixture was reacted at room temperature overnight. The stirring was stopped, and the solvent was distilled off from the reaction system under reduced pressure to obtain 5.6 g of compound 100-4. MS (ESI, [M+H]⁺) m/z: 295.11.

### Step E: Preparation of compound 100-5

To a 50-mL single-necked flask were added tetrahydrofuran (5 mL), *N*,*N*-diisopropylethylamine (4.61 g), and compound 100-4 (3.5 g) in sequence, then 1-propylphosphoric anhydride (4.54 g) was added dropwise at 0 °C, and the resulting mixture was reacted at room temperature for 3 h under nitrogen atmosphere, which was purified by silica gel column chromatography to obtain 3 g of compound 100-5. MS (ESI, [M+H]⁺) *m*/*z*: 277.13.

### Step F: Preparation of compound 100-6

To a 50-mL single-necked flask were added methanol (5 mL), compound 100-5 (1 g) and potassium carbonate (1.501 g) in sequence, and the resulting mixture was reacted at room temperature overnight under nitrogen atmosphere, and concentrated to dryness under reduced pressure to obtain a crude product as a white solid, which was diluted with ethyl acetate (50 mL), and then washed with water (30 mL); the organic phase was washed with saturated sodium chloride to obtain 300 mg of compound 100-6. MS (ESI, [M+H]⁺) *m*/*z*: 123.04.

### Step G: Preparation of compound 100-7

To a 25-mL single-necked flask were added tetrahydrofuran (100 mL) and compound 100-6 (200 mg) in sequence, and after the addition was completed, the system was stirred, purged with nitrogen, and then cooled to -80 °C; A-bromosuccinimide (357 mg) was added, and the system was stirred for 0.5 h. After the reaction was completed, 1 drop of water was added dropwise into the reaction system, ethanol (5 mL) was added, and the reaction system was purified by silica gel column chromatography to obtain 200 mg of compound 100-7. MS (ESI, [M+H]⁺) *m*/*z:* 201.06.

### Step H: Preparation of compound 100

Referring to the preparation method of the step C in Example 62, compound 95-6 was reacted with compound 100-7 to obtain compound 100.

¹H NMR (500 MHz, DMSO-*d*₆) δ11.73 (s, 1H), 10.18 (s, 1H), 8.74 (s, 1H), 8.08 (s, 1H), 7.88 (d, J = 8.8 Hz, 2H), 7.54 (s, 1H), 7.33 (d, J = 8.6 Hz, 2H), 6.17 (s, 1H), 4.87 (s, 1H), 4.23 (s, 3H), 3.46-3.51(m, 1H), 3.30-3.31 (m, 2H), 3.01-3.04 (m, 1H), 1.76-1.86 (m, 2H). HRMS (ESI, [M+H]⁺) m/z: 504.12477.

### Example 101: Preparation of Compound 101

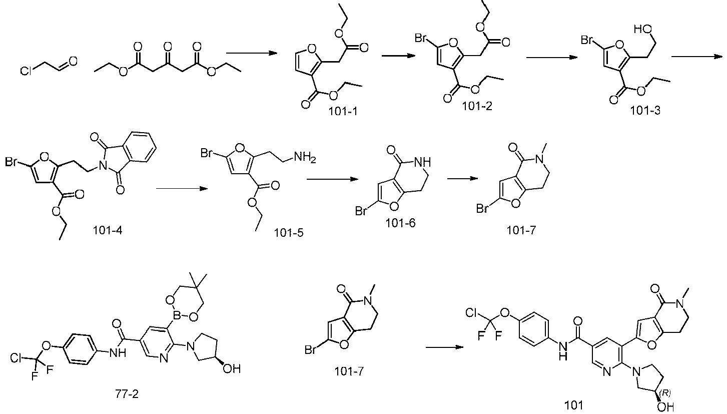

### Step A: Preparation of compound 101-1

2-Chloroacetaldehyde (24.53 g) was added slowly into a stirred solution of diethyl 1,3-acetonedicarboxylate (31.6 g) in acetonitrile (80 mL) at 0 °C under nitrogen atmosphere, and after the addition was completed, the resulting mixture was stirred at 50 °C and reacted for 3 h. The stirring was stopped, then a saturated ammonium chloride solution (50 mL) was added into the reaction solution to quench the reaction, and ethyl acetate (200 mL) and water (200 mL) were added for extraction; the organic phase was separated, and the aqueous phase was extracted with ethyl acetate (50 mL × 2); the organic phases were combined, washed with a saturated brine solution (300 mL), dried over anhydrous sodium sulfate, and filtered, and the filtrate was purified by silica gel column chromatography to obtain 22.6 g of compound 101-1. MS (ESI, [M+H]⁺) *m*/*z*: 227.2.

### Step B: Preparation of compound 101-2

To a 50-mL single-necked flask were added acetonitrile (400 mL) and compound 101-1 (22 g) in sequence. After the addition was completed, the system was cooled to 0 °C, then *N*-bromosuccinimide (21.19 g) was added, and the system was purged with nitrogen and then heated to room temperature and reacted, followed by filtration; the mother liquor was collected and purified by silica gel column chromatography to obtain 25 g of compound 101-2. MS (ESI, [M+H]⁺) *m*/*z:* 305.1.

### Step C: Preparation of compound 101-3

To a 50-mL single-necked flask were added compound 101-2 (24 g) and ethanol (20 mL) in sequence, the system was then cooled in an ice bath, sodium borohydride (8.93 g) was added, and the resulting mixture was stirred at room temperature for 3 h. A saturated ammonium chloride aqueous solution (100 mL) was added into the reaction system to quench the reaction, and ethyl acetate (50 mL) was then added for extraction; the organic phase was extracted with ethyl acetate (50 mL × 2), and the organic phases were combined and purified by silica gel column chromatography to obtain 7 g of compound 101-3. MS (ESI, [M+H]⁺) *m*/*z*: 263.02.

### Step D: Preparation of compound 101-4

To a 50-mL three-necked flask were added compound 101-3 (6.5 g), phthalimide (4.36 g), triphenylphosphine (9.72 g) and tetrahydrofuran (10 mL) in sequence, diisopropyl azodicarboxylate (7.49 g) was added dropwise into the system at 0 °C under nitrogen atmosphere, and after the addition was completed, the resulting mixture was transferred to reach room temperature and stirred and reacted overnight. Water and ethyl acetate were added into the reaction solution for extraction and liquid separation. The organic phase was respectively washed with water and saturated brine, dried over anhydrous sodium sulfate, and purified by silica gel column chromatography to obtain 8.5 g of compound 101-4. MS (ESI, [M+H]⁺) *m*/*z:* 392.14.

### Step E: Preparation of compound 101-5

To a 200-mL three-necked flask were added compound 101-4 (8 g) and ethanol (600 mL) in sequence, hydrazine hydrate (2.403 g) was added at 0 °C, and after the addition was completed, the resulting mixture was heated to 50 °C and reacted overnight, and purified by silica gel column chromatography to obtain 5 g of compound 101-5. MS (ESI, [M+H]⁺) *m*/*z:* 262.09.

### Step F: Preparation of compound 101-6

To a 50-mL reaction flask were added compound 101-5 (1 g), 1,5,7-triazabicyclo(4.4.0)dec-5-ene (0.159 g) and tetrahydrofuran (5 mL) in sequence, and the resulting mixture was stirred at 75 °C and reacted for 30 min, and purified by silica gel column chromatography to obtain 600 mg of compound 101-6. MS (ESI, [M+H]⁺) *m*/*z:* 216.03.

### Step G: Preparation of compound 101-7

Referring to the preparation method of the step A in Example 60, compound 101-6 was reacted with iodomethane to obtain compound 101-7. MS (ESI, [M+H]⁺) *m*/*z:* 230.0.

### Step H: Preparation of compound 101

Referring to the preparation method of the step C in Example 62, compound 95-6 was reacted with compound 101-7 to obtain compound 101.

¹H NMR (500 MHz, DMSO-*d*₆) δ 11.73 (s, 1H), 10.18 (s, 1H), 8.74 (s, 1H), 8.08 (s, 1H), 7.88 (d, *J* = 8.8 Hz, 2H), 7.54 (s, 1H), 7.33 (d, *J=* 8.6 Hz, 2H), 6.17 (s, 1H), 4.87 (s, 1H), 4.23 (s, 3H), 3.46-3.51(m, 1H), 3.30-3.31 (m, 2H), 3.01-3.04 (m, 1H), 1.76-1.86 (m, 2H). HRMS (ESI, [M+H]⁺) *m*/*z:* 533.14074.

### Example 102: Preparation of Compound 102

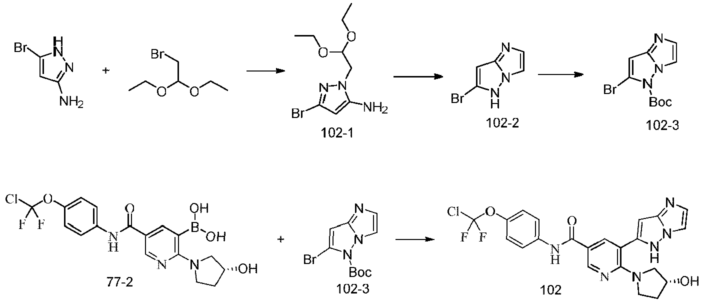

### Step A: Preparation of compound 102-1

To a 100-mL single-necked flask were added N,N-dimethylformamide (50 mL), 3-bromo-1*H*-pyrazol-5-amine (3.0 g), cesium carbonate (9.05 g), and bromoacetaldehyde diethyl acetal (4.01 g); after the addition was completed, the system was heated to 100 °C under nitrogen atmosphere and reacted overnight. After the system was cooled to room temperature, ethyl acetate (50 mL) and purified water (50 mL) were added into the system, followed by stirring, washing and liquid separation; an organic phase was collected, and the aqueous phase was extracted with ethyl acetate (50 mL × 2); the organic phases were combined, and purified water (50 mL) and a saturated sodium chloride aqueous solution (50 mL) were added for stirring and washing twice respectively, followed by liquid separation; an organic phase was collected, and concentrated under reduced pressure to obtain a residue, which was purified by silica gel column chromatography to obtain 3.15 g of compound 102-1. MS (ESI, [M+H]⁺) *m*/*z*: 278.1.

### Step B: Preparation of compound 102-2

To a 100-mL single-necked flask were added anhydrous ethanol (50 mL), compound 102-1 (3.0 g), and a 1 M sulfuric acid aqueous solution (7.5 mL); after the addition was completed, the system was heated to 80 °C under nitrogen atmosphere and reacted for 4 h. The system was cooled to room temperature and then concentrated under reduced pressure, dichloromethane (20 mL) and a saturated sodium bicarbonate solution (10 mL) were added into the resulting residue, followed by stirring, washing and liquid separation to obtain an organic phase; a saturated sodium chloride solution (10 mL) was added into the organic phase, followed by stirring, washing and liquid separation, and an organic phase was collected and concentrated under reduced pressure to obtain a residue, which was purified by silica gel column chromatography to obtain 500 mg of compound 102-2. MS (ESI, [M+H]⁺) m/z: 186.0.

### Step C: Preparation of compound 102-3

To a 50-mL single-necked flask were added dichloromethane (10 mL), compound 102-3 (200 mg), di-*tert*-butyl dicarbonate (258 mg), *N*,*N*-diisopropylethylamine (208 mg), and 4-dimethylaminopyridine (15 mg) in sequence, and after the addition was completed, the system was reacted at room temperature for 2 h; 1 moL/L hydrochloric acid aqueous solution (10 mL) was added into the system for stirring and washing twice, and a saturated sodium chloride aqueous solution was added into the system for stirring and washing once, followed by liquid separation; an organic phase was collected and concentrated under reduced pressure to obtain a residue, which was purified by silica gel column chromatography to obtain 200 mg of compound 102-3. MS (ESI, [M+H]⁺) *m*/*z:* 285.9.

### Step D: Preparation of compound 102

Referring to the preparation method of the step C in Example 62, compound 95-6 was reacted with compound 102-3 to obtain compound 102.

¹H NMR (500 MHz, DMSO-*d*₆) δ 11.09 (s, 1H), 10.16 (s, 1H), 8.72 (d, *J* = 2.4 Hz, 1H), 8.08 (d, *J* = 2.5 Hz, 1H), 7.97 - 7.81 (m, 2H), 7.53 (d, *J* = 2.2 Hz, 1H), 7.32 (d, *J* = 8.7 Hz, 2H), 7.18 (d, *J* = 2.2 Hz, 1H), 5.79 (s, 1H), 4.81 (d, *J* = 3.3 Hz, 1H), 4.25 - 4.11 (m, 1H), 3.56 - 3.45 (m, 1H), 3.08 - 2.97 (m, 1H), 1.88 - 1.77 (m, 1H), 1.77 - 1.64 (m, 1H). HRMS (ESI, [M+H]⁺) *m*/*z*: 489.12628.

### Example 103: Preparation of Compound 103

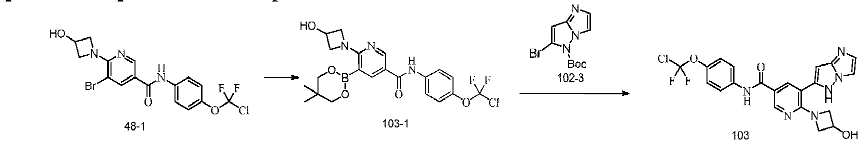

### Step A: Preparation of compound 103-1

Referring to the preparation method of the step B in Example 62, compound 48-1 was reacted with bis(neopentyl glycolato)diboron to obtain compound 103-1.

### Step B: Preparation of compound 103

Referring to the preparation method of the step C in Example 62, compound 103-1 was reacted with compound 102-3 to obtain compound 103.

¹H NMR (500 MHz, DMSO-*d*₆) δ 11.10 (s, 1H), 10.22 (s, 1H), 8.71 (d, *J=* 2.3 Hz, 1H), 8.11 (d, *J* = 2.4 Hz, 1H), 7.92 - 7.84 (m, 2H), 7.56 (d, *J* = 1.8 Hz, 1H), 7.36 - 7.29 (m, 2H), 7.20 (t, *J* = 2.4 Hz, 1H), 5.82 (s, 1H), 5.48 (d, *J* = 5.9 Hz, 1H), 4.41 - 4.34 (m, 1H), 4.04 - 3.97 (m, 2H), 3.63 - 3.55 (m, 2H). HRMS (ESI, [M+H]⁺)m/z: 475.10915.

### Example 104: Preparation of Compound 104

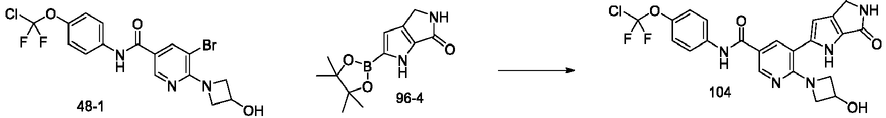

Referring to the preparation method of the step H in Example 94, compound 48-1 was reacted with compound 96-4 to obtain compound 104.

¹H NMR (500 MHz, DMSO-*d*₆) δ 11.94 (s, 1H), 10.21 (s, 1H), 8.73 (d, *J* = 1.9 Hz, 1H), 8.08 (d, *J* = 1.8 Hz, 1H), 7.87 (d, *J* = 9.0 Hz, 2H), 7.82 (s, 1H), 7.34 (d, *J* = 8.7 Hz, 2H), 6.19 (s, 1H), 5.55 (d, *J* = 5.7 Hz, 1H), 4.48 - 4.34 (m, 1H), 4.15 (s, 2H), 4.02 - 3.88 (m, 2H), 3.57 (dd, *J* = 9.4, 4.3 Hz, 2H). HRMS (ESI, [M-H]⁻) *m*/*z*: 488.09439.

### Example 105: Preparation of Compound 105

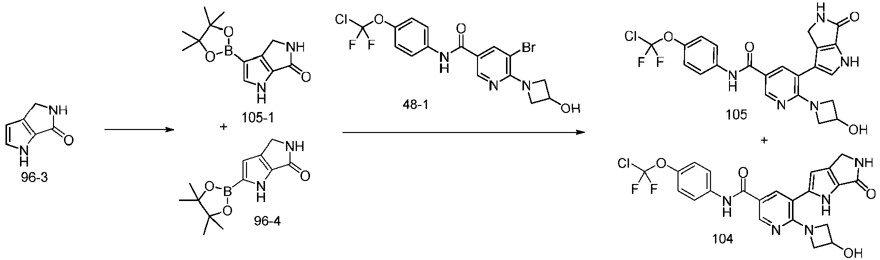

### Step A: Preparation of compound 105-1

To a 35-mL microwave tube were added compound 96-3 (0.1 g), anhydrous tetrahydrofuran (10 mL), bis(pinacolato)diboron (0.25 g), 3,4,7,8-tetramethyl-1,10-phenanthroline (10 mg), and (1,5-cyclooctadiene)(methoxy)iridium(I) dimer (25 mg) in sequence, and the resulting mixture was placed into a microwave reactor under nitrogen atmosphere, where the microwave conditions were as follows: 100 °C and 2 h. After the reaction was completed, the reaction mixture was concentrated under reduced pressure to remove the solvent. Compound 96-4 and compound 105-1 were simultaneously produced in the reaction. The resulting mixture was directly used in the next step without separation and purification.

### Step B: Preparation of compound 105

Referring to the preparation method of the step H in Example 94, compound 48-1 was reacted with the mixture containing compound 105-1 obtained in the step A above to obtain a mixture of compound 104 and compound 105, which was separated by using YMC high pressure preparative chromatography (Ultimate XB-Phenyl column, flow rate: 40 mL/min, mobile phase: pure water/acetonitrile = 13:7) to finally obtain compound 105.

¹H NMR (500 MHz, DMSO-*d*₆) δ 11.89 (s, 1H), 10.20 (s, 1H), 8.66 (s, 1H), 8.01 (s, 1H), 7.94 - 7.82 (m, 3H), 7.34 (d, *J* = 8.1 Hz, 2H), 7.25 (s, 1H), 5.50 (d, *J* = 5.4 Hz, 1H), 4.39 (s, 1H), 4.09 (s, 2H), 3.97 - 3.74 (m, 2H), 3.50 (d, *J* = 4.7 Hz, 2H). HRMS (ESI, [M-H]⁻) *m*/*z*: 488.09448.

### Example 106: Preparation of Compound 106

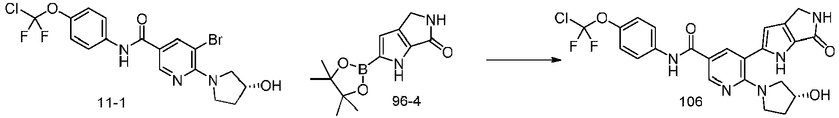

Referring to the preparation method of the step H in Example 94, compound 11-1 was reacted with compound 96-4 to obtain compound 106.

¹H NMR (500 MHz, DMSO-*d*₆) δ 11.90 (s, 1H), 10.17 (s, 1H), 8.74 (d, *J* = 2.4 Hz, 1H), 8.08 (d, *J* = 2.4 Hz, 1H), 7.90 - 7.85 (m, 2H), 7.79 (s, 1H), 7.33 (d, *J* = 9.0 Hz, 2H), 6.16 (d, *J* = 1.2 Hz, 1H), 4.88 (d, *J* = 3.1 Hz, 1H), 4.22 (s, 1H), 4.14 (s, 2H), 3.47 (dt, *J* = 9.4, 7.3 Hz, 1H), 3.32 - 3.26 (m, 2H), 3.05 - 2.94 (m, 1H), 1.85 (ddd, *J* = 12.7, 11.9, 7.7 Hz, 1H), 1.80 - 1.71 (m, 1H). HRMS (ESI, [M+H]⁺) *m*/*z*: 504.12506

### Example 107: Preparation of Compound 107

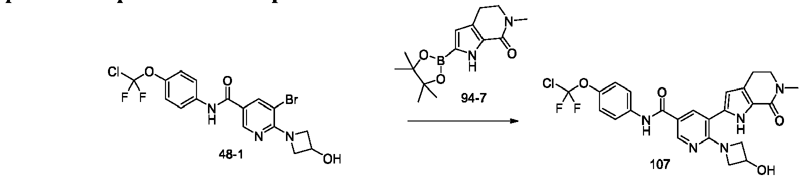

Referring to the preparation method of the step H in Example 94, compound 48-1 was reacted with compound 94-7 to obtain compound 107.

¹H NMR (500 MHz, DMSO-*d*₆) δ 11.82 (s, 1H), 10.18 (s, 1H), 8.69 (d, J = 2.0 Hz, 1H), 8.07 (d, J = 1.9 Hz, 1H), 7.87 (d, J = 9.0 Hz, 2H), 7.34 (d, J = 8.7 Hz, 2H), 6.05 (s, 1H), 5.54 (d, J = 5.5 Hz, 1H), 4.47 - 4.35 (m, 1H), 4.01 - 3.90 (m, 2H), 3.62 - 3.49 (m, 4H), 2.94 (s, 3H), 2.79 (t, J = 6.8 Hz, 2H). HRMS (ESI, [M+H]⁺) *m*/*z*:518.1399.

### Example 108: Preparation of Compound 108

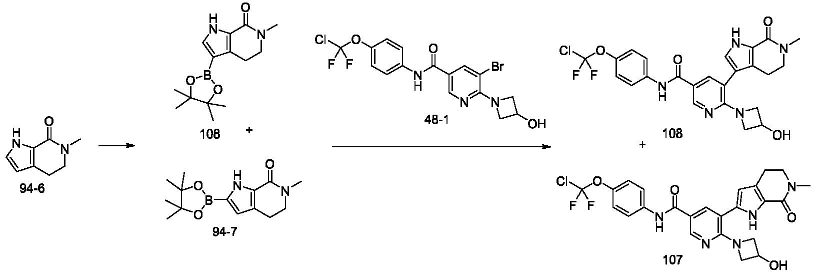

### Step A: Preparation of compound 108-1

Referring to the preparation method of the step A in Example 105, compound 94-6 was reacted with bis(pinacolato)diboron to obtain a crude product containing compound 108-1, which was used immediately in the next step without separation and purification.

### Step B: Preparation of compound 108

Referring to the preparation method of the step H in Example 94, compound 48-1 was reacted with the crude product containing compound 108-1 obtained in the step A above to obtain a mixture of compound 107 and compound 108, which was separated by using YMC high pressure preparative chromatography (Ultimate XB-Phenyl column, flow rate: 40 mL/min, mobile phase: pure water/acetonitrile = 13:7) to finally obtain compound 108.

¹H NMR (500 MHz, DMSO-*d*₆) δ 11.80 (s, 1H), 10.17 (s, 1H), 8.73 - 8.59 (m, 1H), 7.95 - 7.89 (m, 1H), 7.87 (d, J = 8.9 Hz, 2H), 7.33 (d, J = 8.6 Hz, 2H), 7.00 (d, J = 2.4 Hz, 1H), 5.49 (d, J = 5.9 Hz, 1H), 4.37 (h, J = 6.4 Hz, 1H), 3.97 - 3.86 (m, 2H), 3.58 - 3.46 (m, 4H), 2.94 (s, 3H), 2.63 (t, J = 6.7 Hz, 2H). HRMS (ESI, [M+H]⁺) *m*/*z*:518.1408.

### Example 109: Preparation of Compound 109

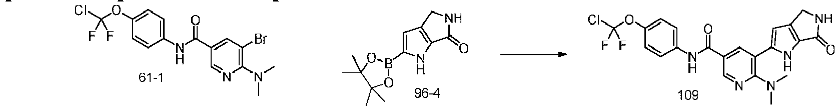

Referring to the preparation method of the step H in Example 94, compound 61-1 was reacted with compound 96-4 to obtain compound 109.

¹H NMR (500 MHz, DMSO-*d*₆) δ 11.90 (s, 1H), 10.27 (s, 1H), 8.71 (d, *J* = 1.9 Hz, 1H), 8.15 (d, *J* = 1.9 Hz, 1H), 7.88 (d, *J* = 9.0 Hz, 2H), 7.81 (s, 1H), 7.34 (d, *J* = 8.7 Hz, 2H), 6.30 (s, 1H), 4.14 (s, 2H), 2.82 (s, 6H). HRMS (ESI, [M-H]⁻) *m*/*z:* 460.10014.

### Example 110: Preparation of Compound 110

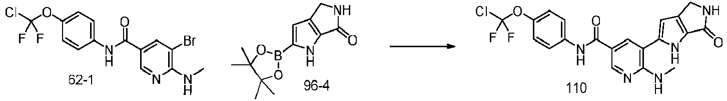

Referring to the preparation method of the step H in Example 94, compound 62-1 was reacted with compound 96-4 to obtain compound 110.

¹H NMR (500 MHz, DMSO-*d*₆) δ 11.96 (s, 1H), 10.14 (s, 1H), 8.68 (s, 1H), 8.05 (s, 1H), 7.94 - 7.65 (m, 3H), 7.34 (d, *J* = 8.5 Hz, 2H), 6.65 (d, *J* = 4.3 Hz, 1H), 6.43 (s, 1H), 4.15 (s, 2H), 2.92 (d, *J* = 4.3 Hz, 3H). HRMS (ESI, [M-H]⁻) *m*/*z*: 446.08408.

### Example 111: Preparation of Compound 111

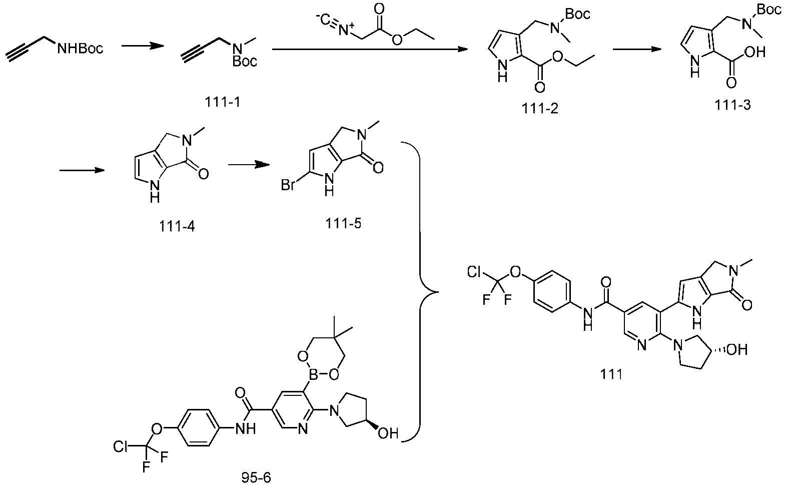

### Step A: Preparation of compound 111-1

To a 250-mL single-necked flask were added *N*-Boc-aminopropyne (10 g) and anhydrous *N,N*-dimethylformamide (150 mL) in sequence, sodium hydride (1.5 g) was added in portions in an ice bath, and then iodomethane (8 g) was added; after the reaction was completed, water was slowly added to quench the reaction, ethyl acetate was added for extraction (200 mL × 3), and organic phases were combined, dried over anhydrous sodium sulfate, and purified by silica gel column chromatography to obtain 9 g of compound 111-1. GC-MS (EI, [M-*e*]⁺) *m*/*z:* 169.0.

### Step B: Preparation of compound 111-2

Referring to the method of the step A in Example 94, compound 111-1 was used with the addition of silver carbonate to prepare compound 111-2. MS (ESI, [M+H]⁺) m/z: 283.10.

### Step C: Preparation of compound 111-3

Referring to the method of the step B in Example 94, compound 111-2 was subjected to hydrolysis with the addition of lithium hydroxide to prepare compound 111-3. MS (ESI, [M-H]⁻) *m*/*z:* 253.17.

### Step D: Preparation of compound 111-4

Referring to the method of the step C in Example 94, compound 111-3 was used with the addition of thionyl chloride to prepare compound 111-4. MS (ESI, [M+H]⁺) *m*/*z:* 137.0.

### Step E: Preparation of compound 111-5

To a 50-mL single-necked flask were added compound 111-4 (0.5 g) and anhydrous tetrahydrofuran (30 mL) in sequence, *N*-bromosuccinimide (0.6 g) was added in an ice bath in portions, and after the reaction was completed, the system was purified by silica gel column chromatography to obtain 0.2 g of compound 111-5. MS (ESI, [M+H]⁺) *m*/*z:* 215.01

### Step F: Preparation of compound 111

Referring to the method of the step G in Example 95, compound 111-5 and compound 95-6 were reacted with the addition of tetrakis(triphenylphosphine)palladium and potassium carbonate to prepare compound 111.

¹H NMR (500 MHz, DMSO-*d*₆) δ 11.95 (s, 1H), 10.17 (s, 1H), 8.74 (s, 1H), 8.06 (s, 1H), 7.87 (d, *J* = 8.5 Hz, 2H), 7.33 (d, *J* = 8.5 Hz, 2H), 6.17 (s, 1H), 4.87 (s, 1H), 4.22 (s, 3H), 3.46 (q, *J* = 9.0 Hz, 1H), 3.00 (d, *J* = 12.0 Hz, 4H), 1.95 - 1.66 (m, 2H), 1.45 - 1.16 (m, 1H), 0.84 (s, 1H). HRMS (ESI, [M-H]⁻) *m*/*z:* 516.1256.

### Example 112: Preparation of Compound 112

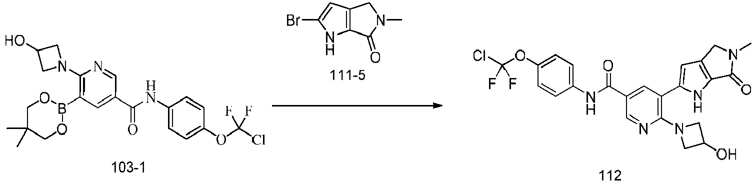

Referring to the method of the step G in Example 95, compound 103-1 and compound 111-5 were reacted with the addition of tetrakis(triphenylphosphine)palladium and potassium carbonate to prepare compound 112.

¹H NMR (500 MHz, DMSO-*d*₆) δ 11.99 (d, *J* = 1.8 Hz, 1H), 10.21 (s, 1H), 8.73 (d, *J* = 2.3 Hz, 1H), 8.07 (d, *J* = 2.4 Hz, 1H), 7.92 - 7.84 (m, 2H), 7.34 (d, *J* = 8.6 Hz, 2H), 6.20 (d, *J* = 1.6 Hz, 1H), 5.54 (d, *J* = 5.5 Hz, 1H), 4.41 (q, *J* = 5.5 Hz, 1H), 4.23 (s, 2H), 3.95 (dd, *J* = 9.6, 6.6 Hz, 2H), 3.56 (dd, *J* = 9.8, 4.5 Hz, 2H), 3.01 (s, 3H). HRMS (ESI, [M-H]⁻) *m*/*z*: 502.1112.

### Example 113: Preparation of Compound 113

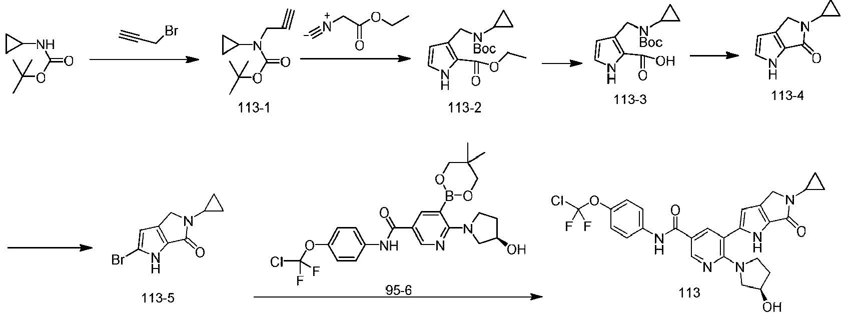

### Step A: Preparation of compound 113-1

*N*-Boc-cyclopropylamine (5 g) was dissolved in tetrahydrofuran (50 mL) in a 100-mL three-necked flask, the system was purged with nitrogen for three times, and sodium hydride (1.908 g) was added in an ice bath in portions; after the addition was completed, the system was purged with nitrogen once, and stirred for 30 min, and 3-bromopropyne (4.54 g) was further added dropwise. After the dropwise addition was completed, the reaction was continued for 3 h, and then the reaction was completed. Water (50 mL) was added dropwise to quench the reaction, the system was extracted with ethyl acetate (40 mL × 3), and the organic phase was washed with saturated sodium chloride (50 mL), dried over anhydrous sodium sulfate, filtered, and spin-dried to obtain compound 20-1 (6 g) which was directly used in the next step without further purification.

### Step B: Preparation of compound 113-2

To a 250-mL single-necked flask were added compound 113-1 (6 g), dioxane (60 mL), ethyl isocyanate (3.13 g), and silver carbonate (1.695 g) in sequence, and after the addition was completed, the system was purged with nitrogen for three times, and then heated to 100 °C and reacted overnight; after the reaction was completed, the solid was filtered off with the addition of diatomite, and the filtrate was directly spin-dried and purified by silica gel column chromatography to obtain compound 113-2 (6 g). MS (ESI, [M+Na]⁺) *m*/*z:* 331.2.

### Step C: Preparation of compound 113-3

To a 50-mL single-necked flask were added compound 113-2 (1 g), methanol (8 mL), water (4 mL) and sodium hydroxide (0.259 g) in sequence, and after the addition was completed, the system was heated to 55 °C and reacted overnight; after the reaction was completed, the system was cooled to room temperature, 2 M diluted hydrochloric acid was added dropwise in an ice bath to adjust the pH to 6, and the system was directly spin-dried to obtain a crude product (1.2 g), which was directly used in the next step without further purification. MS (ESI, [M-H]⁻) *m*/*z:* 279.1.

### Step D: Preparation of compound 113-4

To a 50-mL single-necked flask were added compound 113-3 (630 mg), dioxane (8 mL) and thionyl chloride (2673 mg) in sequence, and the system was heated to 60 °C and reacted for 3 h; after the reaction was completed, the system was cooled to room temperature, the solvent was removed by rotary evaporation, and the resulting residue was redissolved with dichloromethane and N,N-dimethylformamide; diisopropylethylamine (1452 mg) was added dropwise into the system in an ice bath, and after the dropwise addition was completed, the reaction was carried out at room temperature for 30 min. After the reaction was completed, the solvent was directly removed by rotary evaporation, and the resulting residue was subjected to silica gel column chromatography to obtain compound 113-4 (250 mg). MS (ESI, [M+H]⁺) *m*/*z*: 163.0.

### Step E: Preparation of compound 113-5

To a 100-mL single-necked flask were added compound 113-4 (310 mg) and tetrahydrofuran (40 mL) in sequence, the system was cooled to -60 °C, and a solution of *N*-bromosuccinimide (306 mg) in tetrahydrofuran (10 mL) was added dropwise; after the dropwise addition was completed, the temperature was kept for 1 h, and the temperature was naturally raised to room temperature and the reaction was carried out overnight; after the reaction was completed, the reaction system was directly spin-dried at room temperature, dissolved in dichloromethane (2 mL), and subjected to silica gel column chromatography to obtain compound 113-5 (160 mg). MS (ESI, [M+H]⁺) *m*/*z:* 241.1.

### Step F: Preparation of compound 113

Referring to the method of the step G in Example 95, compound 113-5 and compound 95-6 were reacted with the addition of tetrakis(triphenylphosphine)palladium and potassium carbonate to prepare compound 113.

¹H NMR (500 MHz, DMSO-*d*₆) δ 11.94 (s, 1H), 10.18 (s, 1H), 8.74 (d, *J* = 2.2 Hz, 1H), 8.04 (d, *J* = 2.2 Hz, 1H), 7.87 (d, *J* = 9.0 Hz, 2H), 7.33 (d, *J* = 8.8 Hz, 2H), 6.14 (s, 1H), 4.91 (s, 1H), 4.19 (d, *J* = 28.5 Hz, 3H), 3.46 (dd, *J* = 17.3, 9.8 Hz, 1H), 3.30 - 3.25 (m, 1H), 2.99 (d, J = 11.5 Hz, 1H), 2.86 - 2.72 (m, 1H), 1.82 (ddd, *J* = 39.1, 19.5, 3.8 Hz, 2H), 0.81-0.68 (m, 4H). HRMS (ESI, [M+H]⁺) *m*/*z:* 544.1556.

### Example 114: Preparation of Compound 114

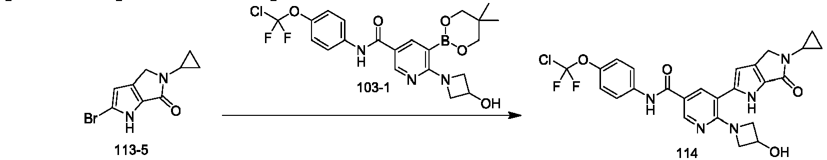

Referring to the method of the step G in Example 95, compound 113-5 and compound 103-1 were reacted with the addition of tetrakis(triphenylphosphine)palladium and cesium carbonate to prepare compound 114.

¹H NMR (500 MHz, DMSO-*d*₆) δ 11.99 (s, 1H), 10.22 (s, 1H), 8.72 (d, *J* = 1.8 Hz, 1H), 8.05 (d, *J* = 1.8 Hz, 1H), 7.87 (d, *J* = 8.9 Hz, 2H), 7.34 (d, *J* = 8.7 Hz, 2H), 6.17 (s, 1H), 5.55 (s, 1H), 4.40 (d, *J* = 4.6 Hz, 1H), 4.16 (s, 2H), 4.04 - 3.88 (m, 2H), 3.56 (dd, *J* = 9.4, 4.3 Hz, 2H), 2.80 (dd, *J* = 8.6, 4.6 Hz, 1H), 0.82 - 0.68 (m, 4H). HRMS (ESI, [M+H]⁺) *m*/*z:* 530.1397.

### Example 115: Preparation of Compound 115

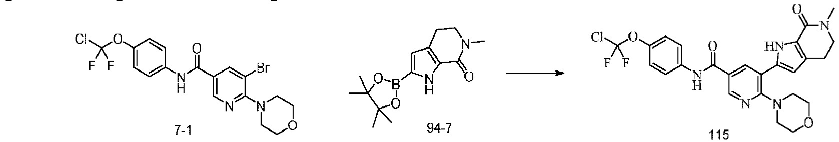

To a 50-mL single-necked flask were added compound 7-1 (100 mg), dioxane (10 mL), compound 94-7 (72 mg), anhydrous potassium carbonate (30 mg), tetrakis(triphenylphosphine)palladium (30 mg), and water (1 mL) in sequence. After the addition was completed, the system was heated in an oil bath to 100 °C and reacted overnight under nitrogen atmosphere. Ethyl acetate (50 mL) and saturated brine (25 mL) were added into the reaction solution for washing, followed by liquid separation, and an organic phase was collected and purified by silica gel column chromatography to obtain 30 mg of compound 115.

¹H NMR (500 MHz, Chloroform-*d*) δ 10.32 (s, 1H), 8.80 (s, 1H), 8.67 (s, 1H), 8.32 - 7.97 (m, 1H), 7.92 - 7.53 (m, 2H), 7.29 - 7.20 (m, 2H), 6.36 (s, 1H), 4.02 - 3.67 (m, 4H), 3.66 - 3.43 (m, 2H), 3.23 - 2.94 (m, 7H), 2.94 - 2.69 (m, 2H). HRMS (ESI, [M+H]⁺) *m*/*z:* 532.15640.

### Example 116: Preparation of Compound 116

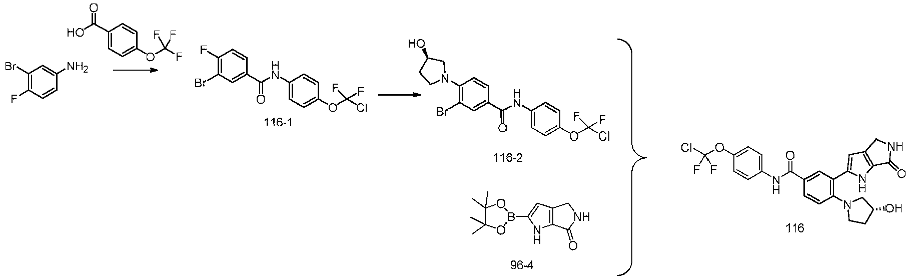

### Step A: Preparation of compound 116-1

To a 100-mL single-necked flask were added 4-trifluoromethoxybenzoic acid (0.29 g), anhydrous toluene (20 mL) and thionyl chloride (0.35 g) in sequence at room temperature, and the resulting mixture was heated in an oil bath to 80 °C and reacted overnight under nitrogen atmosphere. After the reaction was completed, the solvent was removed by concentration, then dichloromethane (20 mL) was added, and 3-bromo-4-fluoroaniline (0.3 g) and diisopropylethylamine (0.3 g) were added dropwise in sequence into the system while stirring; after the addition was completed and the reaction was completed, ethyl acetate (50 mL) and saturated brine (25 mL) were added into the reaction solution for washing, followed by liquid separation, and an organic phase was collected, and purified by silica gel column chromatography to obtain 0.5 g of compound 116-1. MS (ESI, [M-H]⁻) *m*/*z:* 392.053/394.06.

### Step B: Preparation of compound 116-2

To a 30-mL microwave tube were added compound 116-1 (0.8 g), potassium carbonate (560 mg), and (*R*)-3-pyrrolidinol (2 g) in sequence, and the system was purged with nitrogen for 3 times and reacted in an microwave reactor at 160 °C for 2 h. Water (50 mL) was added into the reaction solution to quench the reaction, and ethyl acetate (60 mL) was added for extraction; the organic layer was separated, and the aqueous layer was extracted with ethyl acetate (40 mL × 2). The organic layers were combined, washed with saturated sodium chloride, and dried over anhydrous sodium sulfate. The solvent was distilled off under reduced pressure, and the residue was subjected to silica gel column chromatography to obtain compound 116-2 (0.7 g). MS (ESI, [M+H]⁺) *m*/*z*: 461.1/463.0.

### Step C: Preparation of compound 116

To a 50-mL single-necked flask were added compound 116-2 (100 mg), ethylene glycol dimethyl ether (10 mL), compound 96-4 (60 mg), anhydrous potassium carbonate (30 mg), tetrakis(triphenylphosphine)palladium (30 mg), and water (0.2 mL) in sequence. After the addition was completed, the system was heated in an oil bath to 100 °C and reacted overnight under nitrogen atmosphere. Ethyl acetate (50 mL) and saturated brine (25 mL) were added into the reaction solution, followed by washing and liquid separation, and an organic phase was collected and purified by silica gel column chromatography to obtain 30 mg of compound 116.

¹H NMR (500 MHz, DMSO-*d₆*) δ 11.77 (s, 1H), 10.09 (s, 1H), 7.97 - 7.83 (m, 4H), 7.73 (s, 1H), 7.31 (d, *J* = 8.7 Hz, 2H), 6.84 (d, *J* = 8.7 Hz, 1H), 6.12 (s, 1H), 4.91 - 4.82 (m, 1H), 4.27 - 4.18 (m, 1H), 4.13 (s, 2H), 3.30 - 3.25 (m, 1H), 3.19 - 3.07 (m, 2H), 2.84 - 2.74 (m, 1H), 1.94 - 1.69 (m, 2H). HRMS (ESI, [M+H]⁺) *m*/*z:* 503.1295.

### Example 117: Preparation of Compound 117

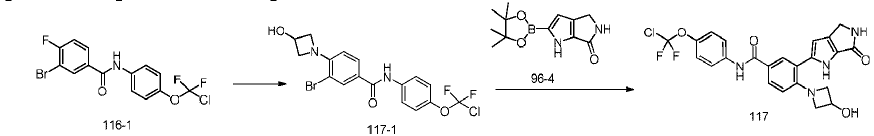

### Step A: Preparation of compound 117-1

To a 30-mL microwave tube were added compound 116-1 (0.8 g), potassium carbonate (560 mg), and azetidin-3-ol (2 g) in sequence, and the system was purged with nitrogen for 3 times and then reacted in a microwave reactor at 160 °C for 2 h. Water (50 mL) was added into the reaction solution to quench the reaction, and ethyl acetate (60 mL) was added for extraction; the organic layer was separated, and the aqueous layer was extracted with ethyl acetate (40 mL × 2). The organic layers were combined, washed with saturated sodium chloride, and dried over anhydrous sodium sulfate. The solvent was distilled off under reduced pressure, and the residue was subjected to silica gel column chromatography to obtain compound 117-1 (0.7 g). MS (ESI, [M+H]⁺) *m*/*z*: 447.1/449.0.

### Step B: Preparation of compound 117

To a 50-mL single-necked flask were added compound 117-1 (100 mg), ethylene glycol dimethyl ether (10 mL), compound 96-4 (60 mg), anhydrous potassium carbonate (30 mg), tetrakis(triphenylphosphine)palladium (30 mg), and water (0.2 mL) in sequence. After the addition was completed, the system was heated in an oil bath to 100 °C and reacted overnight under nitrogen atmosphere. Ethyl acetate (50 mL) and saturated brine (25 mL) were added into the reaction solution, followed by washing and liquid separation, and an organic phase was collected and purified by silica gel column chromatography to obtain 30 mg of compound 117.

¹H NMR (500 MHz, DMSO-*d₆*) δ 11.81 (s, 1H), 10.11 (s, 1H), 7.88 (d, *J* = 9.3 Hz, 4H), 7.75 (s, 1H), 7.32 (d, *J* = 8.8 Hz, 2H), 6.60 (d, *J* = 9.0 Hz, 1H), 6.12 (s, 1H), 5.51 (s, 1H), 4.40 (q, *J* = 6.0, 5.6 Hz, 1H), 4.14 (s, 2H), 3.81 (t, *J* = 7.5 Hz, 2H), 3.39 (dd, *J* = 8.7, 4.8 Hz, 2H). HRMS (ESI, [M+H]⁺) m/z: 489.1143.

### Example 118: Preparation of Compound 118

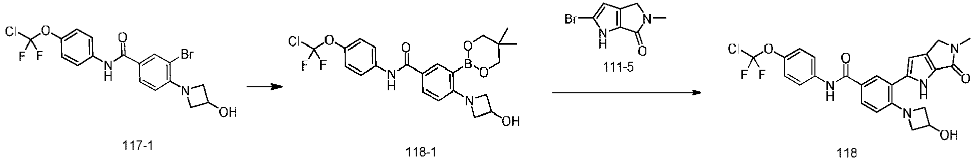

### Step A: Preparation of compound 118-1

Referring to the method of the step F in Example 95, compound 117-1 was reacted with bis(neopentyl glycolato)diboron to obtain compound 118-1.

### Step B: Preparation of compound 118

Referring to the method of the step G in Example 95, compound 118-1 and compound 111-5 were reacted with the addition of tetrakis(triphenylphosphine)palladium and potassium carbonate to prepare compound 118.

¹H NMR (500 MHz, DMSO-*d*₆) δ 11.85 (s, 1H), 10.11 (s, 1H), 7.97 - 7.79 (m, 4H), 7.32 (d, *J* = 8.6 Hz, 2H), 6.60 (d, *J* = 8.5 Hz, 1H), 6.13 (s, 1H), 5.49 (d, *J* = 5.8 Hz, 1H), 4.38 (p, *J* = 5.6 Hz, 1H), 4.22 (s, 2H), 3.80 (t, *J* = 7.5 Hz, 2H), 3.38 (dd, *J* = 8.7, 4.8 Hz, 2H), 3.01 (s, 3H). HRMS (ESI, [M-H]⁻) *m*/*z:* 501.1147.

### Example 119: Preparation of Compound 119

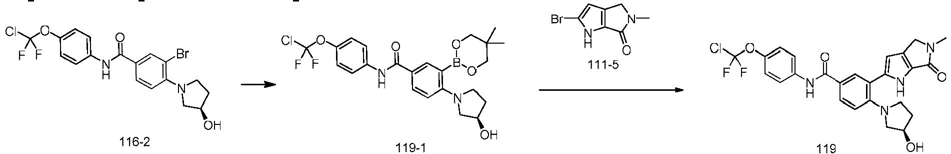

### Step A: Preparation of compound 119-1

Referring to the method of the step F in Example 95, compound 116-2 was reacted with bis(neopentyl glycolato)diboron to obtain compound 119-1.

### Step B: Preparation of compound 119

Referring to the method of the step G in Example 95, compound 119-1 and compound 111-5 were reacted with the addition of tetrakis(triphenylphosphine)palladium and potassium carbonate to prepare compound 119.

¹H NMR (500 MHz, DMSO-*d*₆) δ 11.81 (s, 1H), 10.09 (s, 1H), 7.88 (q, *J* = 4.8, 4.4 Hz, 4H), 7.32 (d, *J* = 8.6 Hz, 2H), 6.84 (d, *J* = 8.9 Hz, 1H), 6.13 (s, 1H), 4.86 (d, *J* = 3.6 Hz, 1H), 4.21 (s, 2H), 3.30 - 3.24 (m, 1H), 3.12 (ddt, *J* = 21.1, 8.6, 4.0 Hz, 2H), 3.00 (s, 3H), 2.79 (d, *J* = 10.7 Hz, 1H), 1.93 - 1.71 (m, 2H). HRMS (ESI, [M-H]⁻) 515.1302.

### Example 120: Preparation of Compound 120

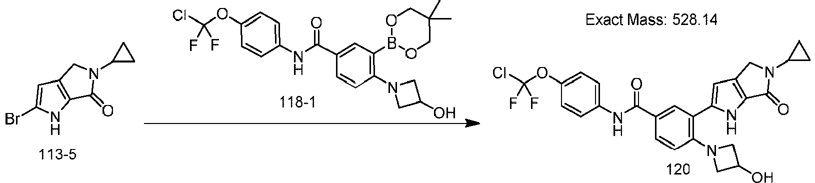

Referring to the preparation method of the step G in Example 95, compound 113-5 and compound 118-1 were reacted with the addition of tetrakis(triphenylphosphine)palladium and potassium carbonate to prepare compound 120.

¹H NMR (500 MHz, DMSO-*d*₆) δ 13.20 - 12.76 (m, 1H), 10.22 (s, 1H), 7.93 - 7.85 (m, 2H), 7.83 - 7.51 (m, 2H), 7.49 - 7.28 (m, 3H), 6.54 (d, *J* = 18.6 Hz, 1H), 4.68 - 4.41 (m, 1H), 4.18 (s, 2H), 3.44 - 3.32 (m, 4H), 2.91 - 2.69 (m, 2H). HRMS (ESI, [M+H]⁺) *m*/*z*: 529.1446.

### Example 121: Preparation of Compound 121

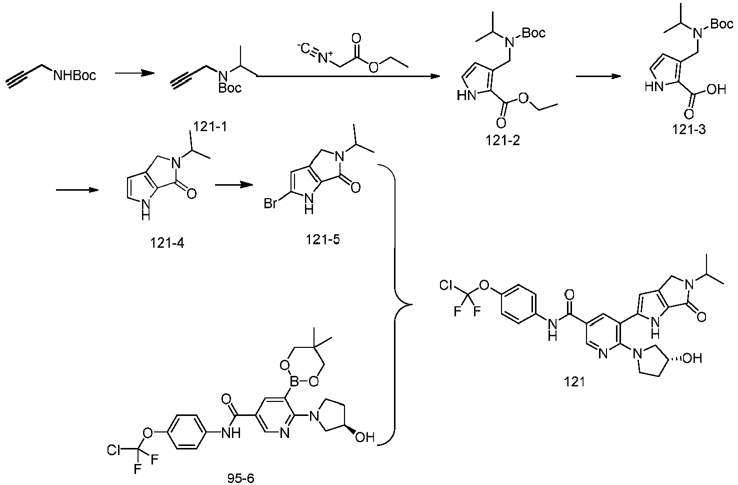

### Step A: Preparation of compound 121-1

Referring to the method of the step A in Example 111, N-Boc-aminopropyne was reacted with iodoisopropane to obtain compound 121-1.

### Step B: Preparation of compound 121-2

Referring to the method of the step A in Example 94, compound 121-1 was used with the addition of silver carbonate to prepare compound 121-2. MS (ESI, [M+H-Boc]⁺) *m*/*z:* 211.0.

### Step C: Preparation of compound 121-3

Referring to the method of the step B in Example 94, compound 121-2 was subjected to hydrolysis with the addition of lithium hydroxide to prepare compound 121-3. MS (ESI, [M-H]⁻) *m*/*z*: 281.13.

### Step D: Preparation of compound 121-4

Referring to the method of the step C in Example 94, compound 121-3 was used with the addition of thionyl chloride to prepare compound 121-4. MS (ESI, [M+H]⁺) *m*/*z*: 165.11.

### Step E: Preparation of compound 121-5

Referring to the method of the step E in Example 111, compound 121-4 was reacted with *N*-bromosuccinimide to obtain compound 121-5. MS (ESI, [M+H+2]⁺) *m*/*z:* 245.05.

### Step F: Preparation of compound 121

Referring to the method of the step G in Example 95, compound 121-5 and compound 77-2 were reacted with the addition of tetrakis(triphenylphosphine)palladium and potassium carbonate to prepare compound 121.

¹H NMR (500 MHz, DMSO-*d*₆) δ 11.94 (s, 1H), 10.18 (s, 1H), 8.73 (s, 1H), 8.07 (s, 1H), 7.87 (d, J = 8.8 Hz, 2H), 7.33 (d, J = 8.5 Hz, 2H), 6.19 (s, 1H), 4.29-4.32 (m, 1H), 4.23 (s, 1H), 4.18 (s, 2H), 3.50 (s, 1H), 3.22-3.34 (m, 3H), 3.00-3.02 (m, 1H), 1.77-1.78 (m, 2H), 1.20 (d, J = 6.6 Hz, 6H). HRMS (ESI, [M+H]⁺) *m*/*z:* 546.1715.

### Example 122: Preparation of Compound 122

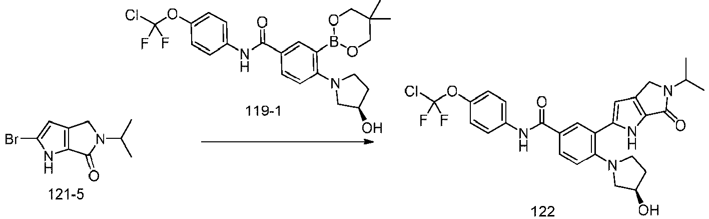

### Step A: Preparation of compound 122

Referring to the method of the step G in Example 95, compound 119-1 and compound 121-5 were reacted with the addition of tetrakis(triphenylphosphine)palladium and potassium carbonate to prepare compound 122.

¹H NMR (500 MHz, DMSO-*d*₆) δ 11.80 (s, 1H), 10.07 (s, 1H), 7.87-7.89 (m, 4H), 7.31-7.33 (m, 2H), 6.84-6.85 (m, 1H), 6.14 (s, 1H), 4.87 (s, 1H), 4.33 (s, 1H), 4.28-4.33 (m, 1H), 4.17 (s, 2H), 3.28-3.30 (m, 1H), 3.12-3.19 (m, 2H), 2.79-2.81 (m, 1H), 1.78-1.91 (m, 2H), 1.20 (d, *J* = 6.7 Hz, 6H). HRMS (ESI, [M-H]⁻) *m*/*z*: 545.1762.

### Example 123: Preparation of Compound 123

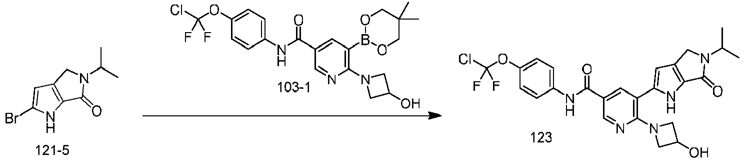

Referring to the method of the step G in Example 95, compound 121-5 and compound 103-1 were reacted with the addition of tetrakis(triphenylphosphine)palladium and potassium carbonate to prepare compound 123.

¹H NMR (500 MHz, DMSO-*d*₆) δ 11.97 (s, 1H), 10.20 (s, 1H), 8.73 (s, 1H), 8.06 (s, 1H), 7.87 (d, *J* = 9.1 Hz, 2H), 7.34 (d, *J* = 8.8 Hz, 2H), 6.21 (s, 1H), 5.54 (s, 1H), 4.40-4.43 (m, 1H), 4.30-4.32 (m, 1H), 4.18 (s, 2H), 3.94-3.98 (m, 2H), 3.55-3.58 (m, 2H), 1.20 (d, *J* = 6.8 Hz, 6H). HRMS (ESI, [M-H]⁻) *m*/*z*: 532.1564.

### Example 124: Preparation of Compound 124

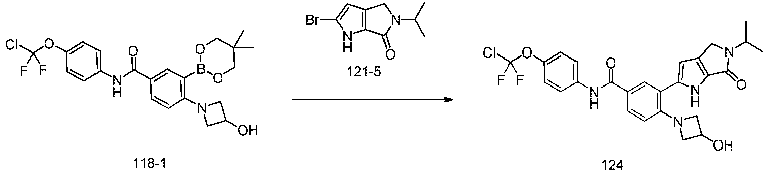

### Step A: Preparation of compound 124

Referring to the method of the step G in Example 95, compound 118-1 and compound 121-5 were reacted with the addition of tetrakis(triphenylphosphine)palladium and potassium carbonate to prepare compound 124.

¹H NMR (500 MHz, DMSO-*d*₆) δ 11.84 (s, 1H), 10.10 (s, 1H), 7.86-7.89 (m, 4H), 7.32 (d, *J* = 8.9 Hz, 2H), 6.61 (d, *J* = 8.6 Hz, 1H), 6.14 (s, 1H), 5.50 (s, 1H), 4.38-4.41 (m, 1H), 4.29-4.32 (m, 1H), 4.17 (s, 2H), 3.80-3.83 (m, 2H), 3.38-3.40 (m, 2H), 1.20 (d, *J* = 6.8 Hz, 6H). HRMS (ESI, [M-H]⁻) *m*/*z*: 531.1602.

### Experimental Example 1: Inhibitory Effect of Compounds on Proliferation of K562 Cells

K562 cells in logarithmic growth phase and good cell condition were added to a centrifuge tube and centrifuged at 1000 rpm for 5 min in a low speed centrifuge. The supernatant was discarded, and 5 mL of complete medium (RPMI 1640 basic medium + 10% FBS) was added using a pipette for cell resuspension. The cells were counted using a cell counter, diluted with complete medium to a cell density of 6 × 10⁴ cells/mL, and an equivalent amount of RPMI 1640 basic medium was added to adjust the serum concentration to 5% and the cell density to 3 × 10⁴ cells/mL for plate seeding. The cells were seeded on a 96-well plate at 100 µL/well using a multi-channel pipette and cultured in a cell incubator at 37 °C, 5% CO₂ and saturated humidity. After 24 h incubation, compounds were loaded using a nanoliter pipettor, 8 concentrations (1000 nM, 250 nM, 63 nM, 16 nM, 3.9 nM, 0.98 nM, 0.24 nM, 0.061 nM) were set for each compound, and 2 replicate wells were set for each concentration; cells with no compounds added were taken as a negative control, and a plating medium with no cells added was taken as a blank control. After 72 h, CCK-8 was added at 10 µL/well for incubation for 2 h, then absorbance was measured at 450 nm with an Envision plate reader, and inhibition rate was calculated. Inhibition rate (%) = (mean value of negative control group - mean value of experimental group) / (mean value of negative control group - mean value of blank group) ×100%. A dose-response curve was fitted by four-parameter analysis, with the logarithm of compound concentration serving as abscissa and inhibition rate serving as ordinate, such that IC₅₀ was calculated. The results are shown in Table 1.

**Table 1**

| Example | IC₅₀ (nM) K562 | Example | IC₅₀ (nM) K562 |
|---|---|---|---|
| 1 | 9.3 | 55 | 26 |
| 2 | 4.8 | 56 | 18 |
| 3 | 3.8 | 57 | 59 |
| 4 | 16 | 60 | 6.3 |
| 5 | 16 | 61 | 5.6 |
| 6 | 4.1 | 62 | 18 |
| 7 | 11 | 63 | 5.2 |
| 8 | 52 | 64 | 9.1 |
| 9 | 9.3 | 66 | 31 |
| 10 | 9 | 67 | 12 |
| 11 | 12 | 69 | 47 |
| 14 | 3.8 | 70 | 95 |
| 15 | 3.1 | 71 | 43 |
| 16 | 2.1 | 72 | 9.2 |
| 17 | 13 | 73 | 14 |
| 18 | 4.9 | 74 | 23 |
| 19 | 3.7 | 76 | 2.2 |
| 20 | 31 | 77 | 13 |
| 21 | 17 | 78 | 46 |
| 22 | 57 | 79 | 17 |
| 23 | 10 | 80 | 14 |
| 24 | 6.3 | 81 | 10 |
| 25 | 9 | 82 | 57 |
| 26 | 26 | 83 | 3.2 |
| 27 | 18 | 84 | 45 |
| 28 | 6.5 | 86 | 24 |
| 29 | 6.8 | 88 | 10 |
| 30 | 10 | 89 | 17 |
| 31 | 12 | 90 | 14 |
| 33 | 4.9 | 91 | 6.6 |
| 34 | 20 | 92 | 15 |
| 35 | 16 | 93 | 12 |
| 36 | 1.7 | 94 | 0.71 |
| 37 | 7.8 | 95 | 39 |
| 38 | 7.2 | 96 | 4.7 |
| 39 | 5.9 | 97 | 5.7 |
| 40 | 14 | 98 | 4.7 |
| 41 | 2.1 | 99 | 3.2 |
| 42 | 2.3 | 101 | 10 |
| 43 | 2.2 | 104 | 1.2 |
| 44 | 4.5 | 106 | 1.1 |
| 45 | 4.2 | 109 | 0.95 |
| 46 | 26 | 110 | 3.1 |
| 47 | 2.6 | 111 | 0.5 |
| 48 | 13 | 112 | 0.54 |
| 49 | 10 | 113 | 0.62 |
| 50 | 10 | 114 | 0.56 |
| 51 | 13 | 115 | 5.5 |
| 52 | 6.1 | 116 | 0.82 |
| 53 | 3 | 117 | 1 |
| 54 | 8.5 | 120 | 0.21 |

### Experimental Example 2: Inhibitory Effect of Compounds on Proliferation of BCR-ABL T315I-transfected Ba/F3 cells

Ba/F3-BCR-ABL1-T315I-C3 cells in logarithmic growth phase and good cell condition were added to a centrifuge tube and centrifuged at 1000 rpm for 5 min in a low speed centrifuge. The supernatant was discarded, and 5 mL of complete medium (RPMI 1640 basic medium + 10% FBS) was added using a pipette for cell resuspension. The cells were counted using a cell counter, diluted with complete medium to a cell density of 6 × 10⁴ cells/mL, and an equivalent amount of RPMI 1640 basic medium was added to adjust the serum concentration to 5% and the cell density to 3 × 10⁴ cells/mL for plate seeding. The cells were seeded on a 96-well plate at 100 µL/well using a multi-channel pipette and cultured in a cell incubator at 37 °C, 5% CO₂ and saturated humidity. After 24 h incubation, compounds were loaded using a nanoliter pipettor, 8 concentrations (1000 nM, 250 nM, 63 nM, 16 nM, 3.9 nM, 0.98 nM, 0.24 nM, 0.061 nM) were set for each compound, and 2 replicate wells were set for each concentration; cells with no compounds added were taken as a negative control, and a plating medium with no cells added was taken as a blank control. After 72 h, CCK-8 was added into Ba/F3-BCR-ABL1 T315I cells at 10 µL/well, the cells were placed at 37 °C for 1 h, and then the absorbance was measured at 450 nm with an Envision plate reader, and inhibition rate was calculated. Inhibition rate (%) = (mean value of negative control group - mean value of experimental group) / (mean value of negative control group - mean value of blank group) ×100%. A dose-response curve was fitted by four-parameter analysis, with the logarithm of compound concentration serving as abscissa and inhibition rate serving as ordinate, such that IC₅₀ was calculated. The results are shown in Table 2.

**Table 2**

| Example | IC₅₀ (nM) Ba/F3-BCR-ABL1 T315I | Example | IC₅₀ (nM) Ba/F3-BCR-ABL1 T315I |
|---|---|---|---|
| 1 | 19 | 44 | 2.4 |
| 2 | 9.6 | 45 | 8.1 |
| 3 | 7.5 | 46 | 26 |
| 4 | 21 | 47 | 1.8 |
| 5 | 28 | 53 | 2.7 |
| 6 | 13 | 54 | 6.4 |
| 7 | 16 | 56 | 12 |
| 8 | 23 | 60 | 4.0 |
| 10 | 20 | 61 | 3.0 |
| 11 | 54 | 63 | 6.0 |
| 14 | 14 | 64 | 8.1 |
| 15 | 9.6 | 67 | 6.1 |
| 16 | 4.7 | 72 | 6.3 |
| 17 | 33 | 76 | 2.2 |
| 18 | 8.1 | 83 | 4.1 |
| 19 | 8.4 | 86 | 14 |
| 20 | 38 | 89 | 9.5 |
| 21 | 20 | 90 | 18 |
| 22 | 35 | 91 | 4.1 |
| 23 | 18 | 94 | 0.88 |
| 24 | 12 | 96 | 0.59 |
| 25 | 20 | 97 | 2.3 |
| 26 | 53 | 98 | 3.8 |
| 27 | 67 | 99 | 1.4 |
| 28 | 11 | 102 | 76 |
| 29 | 12 | 104 | 0.86 |
| 30 | 18 | 106 | 0.54 |
| 31 | 21 | 107 | 1.1 |
| 33 | 9.1 | 109 | 0.27 |
| 34 | 7.8 | 110 | 0.44 |
| 35 | 13 | 111 | 0.16 |
| 36 | 4.3 | 112 | 0.29 |
| 37 | 16 | 113 | 0.18 |
| 38 | 5.7 | 114 | 0.21 |
| 39 | 2.4 | 115 | 3.0 |
| 40 | 7.4 | 116 | 0.12 |
| 41 | 3.4 | 117 | 0.15 |
| 42 | 3.5 | 120 | 0.084 |
| 43 | 4.0 | | |

### Experimental Example 3: Evaluation of Pharmacokinetics in Mice

ICR mice (purchased from Shanghai Sippe-Bk Lab Animal Co., Ltd.) of 18-22 g were randomized into groups of 9 after 3-5 days of acclimatization and administered with the compounds at a dose of 10 mg/kg by intragastric administration. The animals to be tested (ICR mice) were fasted overnight before administration and food was provided 4 h after administration, and water was freely drunk before and after the experiment and during the experiment. After intragastric administration, about 0.1 mL of blood was collected from the orbit at 0.25 (15 min), 0.5 (30 min), 1, 2, 3, 4, 6, 8, 10, and 24 h (Each mouse was subjected to blood collection at 3-4 time points, with three mice per time point). The blood samples were mixed with EDTA-K2 anticoagulant, transferred to a centrifuge within 30 min, and then centrifuged at 4000 rpm for 10 min at 4 °C to separate the plasma. All the plasma samples were collected and immediately stored at -20 °C for testing.

300 µL of acetonitrile solution containing an internal standard (20 ng/mL diazepam) was added to 30 µL of the plasma sample and standard curve sample. The mixture was shaken and mixed well for 5 min, and centrifuged at 13,000 rpm for 10 min. 70 µL of supernatant was taken and diluted with 70 µL of ultrapure water. After being mixed well, 1 µL of the resulting sample was subjected to LC/MS/MS, and a chromatogram was recorded.

Oral exposure of the compounds was evaluated by *in vivo* pharmacokinetic experiments in mice. The relevant pharmacokinetic parameters were fitted by using DAS3.2.5 software, where absolute bioavailability F_{abs}(%) = (intragastric AUC × intravenous dosage)/(intravenous AUC × intragastric dosage) × 100%. The results are shown in Table 3.

**Table 3**

| Compound | | Example 7 | Example 15 | Example 16 | Example 61 |
|---|---|---|---|---|---|
| Route of administration | | ig | ig | ig | ig |
| Dose (mg/kg) | | 10 | 10 | 10 | 10 |
| AUC(0-24) | (h•ng/mL) | 2847 | 12300 | 9693 | 24056 |
| MRT(0-24) | h | 3.03 | 3.64 | 3.33 | 4.83 |
| t_{1/2} | h | 1.05 | 2.12 | 1.46 | 10.41 |
| Tmax | h | 3 | 2 | 3 | 2 |
| Cmax | ng/mL | 772 | 2333 | 1878 | 3197 |
| F_{abs}(%) | / | 74.14% | 106.36% | 93.20% | 95.12% |

### Experimental Example 4: Evaluation of Cardiac Safety of Compounds

The cell strains were derived from HEK-293 cells over-expressing hERG potassium channel. The cells were cultured in an incubator at 37 °C, 5% CO₂. When the cell density reached 80% of the culture dish, the cells were pre-washed with phosphate buffered saline (PBS), and then digested with trypsin/EDTA for 2-3 min; a cell culture medium was added and the digestion was stopped; the cells were gently blown down and transferred into a centrifuge tube, and then centrifuged at 1000 rpm for 3 min; the supernatant was discarded, a cell culture medium was added, and the cells were gently pipetted and mixed uniformly, and then transferred into the culture dish for subculture, or the cells were added dropwise onto a circular slide and placed in a culture dish for experiments after their adhesion to the wall.

The cell culture medium is composed of DMEM, 15% fetal bovine serum, and 1% 100× penicillin-streptomycin.

The stably-transformed cells were seeded onto a slide at a cell density of less than 50% and cultured overnight. The experimental cells were transferred into an approximately 1 mL bath embedded in an inverted microscope stage and perfused with an extracellular fluid at a rate of 2.7 mL/min. The experiment was carried out after 5 min of stabilization. Membrane currents were recorded by using a HEKA EPC-10 patch clamp amplifier and PATCHMASTER acquisition system (HEKA Instruments Inc., D - 67466 Lambrecht, Pfalz, Germany) and analyzed and counted by using Origin 8.5 (OriginLab Corporation, Northampton, MA) software and Microsoft Excel (results in Table 4). All experiments were performed at room temperature (22-24 °C). A P-97 micropipette puller (Sutter Instrument Company, One Digital Drive, Novato, CA 94949) was used to straighten the electrodes (BF150-110-10) during the experiments. The inner diameter of the electrodes was 1-1.5 mm, and the water inlet resistance after the electrodes were filled with an intracellular fluid was 2-4 MΩ.

The electrophysiological stimulation protocol for the hERG potassium channel was that, the membrane voltage was firstly clamped at -80 mV, the cells were given a voltage stimulation of +20 mV for 2 s, the hERG potassium channel was activated, followed by repolarization to -50 mV for 5 s, and an outward tail current was generated, where the stimulus frequency was once every 15 s. The current value was the peak value of the tail current. Channel currents were recorded in the experiments using a whole-cell recording mode. An extracellular fluid (approximately 2 mL per min) was firstly perfused, the process was continuously recorded, and the current was waited for stabilization (current decay (Run-Down) was less than 5% in 5 min); at this point, the tail current peak value was the control current value. Then, the extracellular fluid containing the test drug was perfused, and the process was continuously recorded until the inhibitory effect of the drug on the hERG current reached a stable state; at this point, the tail current peak value was the current value after dosing. The criterion for stable state is to determine whether the nearest consecutive 3 current recording lines coincide. After a stable state was reached, if the hERG currents, after the cells were flushed by perfusion with an extracellular fluid, returned to or reached the level before dosing, the perfusion test could be continuously carried out for other concentrations or drugs. 30 µM Quinidine was used in the experiment as a positive control to ensure that the cell response used was normal.

**Table 4**

| Example | hERG IC₅₀ (µM) |
|---|---|
| 104 | > 30 |
| 106 | > 30 |

Those skilled in the art will recognize that the scope of the present disclosure is not limited to the various embodiments and examples described above. Instead, various modifications, substitutions, or recombinations can be made without departing from the spirit and conception of the present disclosure, all of which fall within the protection scope of the present disclosure.

## Claims

1. A compound of formula (I) or a pharmaceutically acceptable salt thereof, 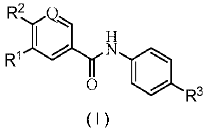 wherein,
Q is selected from the group consisting of N and CH;
R¹ is selected from the group consisting of 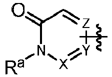 and 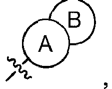 wherein the 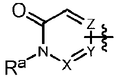 or 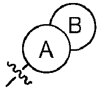 is optionally substituted with one or more R^{a'};
X, Y and Z are each independently selected from the group consisting of CH and N, and at least one of X, Y and Z is selected from CH;
ring A is selected from 5-membered heteroaryl containing 1-3 heteroatoms selected from the group consisting of N, O and S atoms;
ring B is selected from the group consisting of 5- to 10-membered heterocyclyl containing 1-3 heteroatoms selected from the group consisting of N, O and S atoms, and 5- to 8-membered heteroaryl containing 1-3 heteroatoms selected from the group consisting of N, O and S atoms;
R² is selected from the group consisting of hydrogen, amino, C₁₋₆ alkyl, C₁₋₆ alkoxy, amino-C₁₋₆ alkyl-, 3- to 10-membered heterocyclyl, and 5- to 6-membered heteroaryl, wherein the amino, C₁₋₆ alkyl, C₁₋₆ alkoxy, amino-C₁₋₆ alkyl-, 3- to 10-membered heterocyclyl, or 5- to 6-membered heteroaryl is optionally substituted with one or more R^{b};
R³ is selected from -OCF₂H, wherein the -OCF₂H is optionally substituted with halogen;
R^{a} and R^{a'} are each independently selected from the group consisting of hydroxy, amino, cyano, halogen, 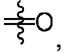 C₁₋₆ alkyl, 3- to 8-membered cycloalkyl, 3- to 8-membered heterocycloalkyl, 3- to 8-membered cycloalkyl-C₁₋₆ alkyl-, 3- to 8-membered heterocycloalkyl-C₁₋₆ alkyl-, and C₁₋₆ alkyl substituted with one or more hydroxy or halogen;
R^{b} is selected from the group consisting of hydroxy, amino, cyano, halogen, 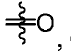 C₁₋₆ alkyl, C₁₋₆ alkoxy, C₁₋₆ alkoxy-C₁₋₆ alkyl-, C₁₋₆ alkyl-C(O)-NH-, and C₁₋₆ alkyl substituted with one or more hydroxy or halogen.

2. The compound of formula (I) or the pharmaceutically acceptable salt thereof according to claim 1, being selected from the group consisting of a compound of formula (II) and a pharmaceutically acceptable salt thereof, 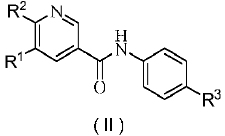 wherein,
R¹ is selected from the group consisting of 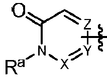 and 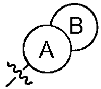 , wherein the 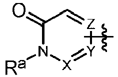 or 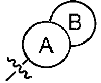 is optionally substituted with one or more R^{a'};
X, Y and Z are each independently selected from the group consisting of CH and N, and at least one of X, Y and Z is selected from CH;
the ring A is selected from 5-membered heteroaryl containing 1-3 heteroatoms selected from the group consisting of N, O and S atoms;
the ring B is selected from the group consisting of 5- to 10-membered heterocyclyl containing 1-3 heteroatoms selected from the group consisting of N, O and S atoms, and 5- to 8-membered heteroaryl containing 1-3 heteroatoms selected from the group consisting of N, O and S atoms;
R² is selected from the group consisting of hydrogen, amino, C₁₋₆ alkyl, C₁₋₆ alkoxy, amino-C₁₋₆ alkyl-, 3- to 10-membered heterocyclyl, and 5- to 6-membered heteroaryl, wherein the amino, C₁₋₆ alkyl, C₁₋₆ alkoxy, amino-C₁₋₆ alkyl-, 3- to 10-membered heterocyclyl, or 5- to 6-membered heteroaryl is optionally substituted with one or more R^{b};
R³ is selected from -OCF₂H, wherein the -OCF₂H is optionally substituted with halogen;
R^{a} and R^{a'} are each independently selected from the group consisting of hydroxy, amino, cyano, halogen, 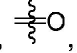 C₁₋₆ alkyl, 3- to 8-membered cycloalkyl, 3- to 8-membered heterocycloalkyl, 3- to 8-membered cycloalkyl-C₁₋₆ alkyl-, 3- to 8-membered heterocycloalkyl-C₁₋₆ alkyl-, and C₁₋₆ alkyl substituted with one or more hydroxy or halogen;
R^{b} is selected from the group consisting of hydroxy, amino, cyano, halogen, 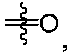 C₁₋₆ alkyl, C₁₋₆ alkoxy, C₁₋₆ alkoxy-C₁₋₆ alkyl-, C₁₋₆ alkyl-C(O)-NH-, and C₁₋₆ alkyl substituted with one or more hydroxy or halogen.

3. The compound of formula (I) or the pharmaceutically acceptable salt thereof according to claim 1, wherein Q is selected from CH.

4. The compound of formula (I) or the pharmaceutically acceptable salt thereof according to any one of claims 1-3, wherein X, Y and Z are all selected from CH; or, one of X, Y and Z is selected from N, and the others are selected from CH; or, X is selected from N, and Y and Z are selected from CH; or, Y is selected from N, and X and Z are selected from CH; or; Z is selected from N, and X and Y are selected from CH; or, one of X, Y and Z is selected from CH, and the others are selected from N.

5. The compound of formula (I) or the pharmaceutically acceptable salt thereof according to any one of claims 1-4, wherein the ring A is selected from 5-membered heteroaryl containing 1 or 2 heteroatoms selected from the group consisting of N and O atoms; or, the ring A is selected from the group consisting of pyrrolyl, pyrazolyl, imidazolyl, furanyl, thienyl, thiazolyl, oxazolyl and isoxazolyl; or, the ring A is selected from the group consisting of pyrrolyl, pyrazolyl, imidazolyl and furanyl.

6. The compound of formula (I) or the pharmaceutically acceptable salt thereof according to any one of claims 1-5, wherein the ring B is selected from the group consisting of 5- to 8-membered heterocycloalkyl containing 1-3 heteroatoms selected from the group consisting of N and O atoms, and 5- to 8-membered heteroaryl containing 1-3 heteroatoms selected from the group consisting of N and O atoms; or, the ring B is selected from the group consisting of 5- or 6-membered heterocycloalkyl containing 1 or 2 heteroatoms selected from the group consisting of N and O atoms, and 5-membered heteroaryl containing 1 or 2 N atoms; or, the ring B is selected from the group consisting of tetrahydropyrrolyl, piperidinyl, piperazinyl, morpholinyl, imidazolyl, and pyrazolyl; or, the ring B is selected from the group consisting of tetrahydropyrrolyl, piperidinyl, morpholinyl, and imidazolyl.

7. The compound of formula (I) or the pharmaceutically acceptable salt thereof according to any one of claims 1-6, wherein R¹ is selected from 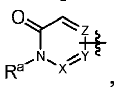 wherein the 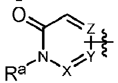 is optionally substituted with one or more R^{a'}; or, R¹ is selected from the group consisting of 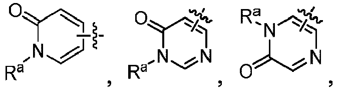 and 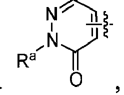 wherein the 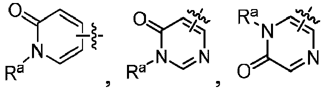 or 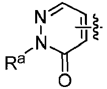 is optionally substituted with one or more R^{a'}; or, R¹ is selected from the group consisting of 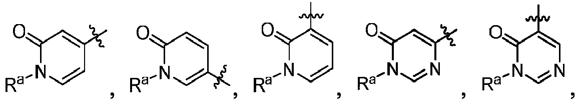 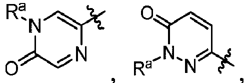 and 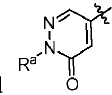 , wherein the 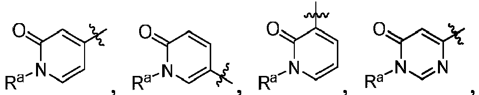 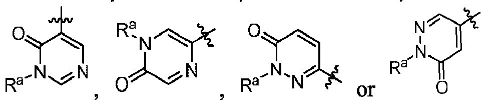 is optionally substituted with one or more R^{a'}; or, R¹ is selected from 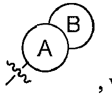 wherein the 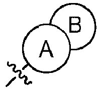 is optionally substituted with one or more R^{a'}; or, R¹ is selected from the group consisting of 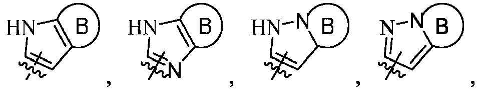 and 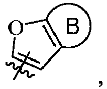 wherein the 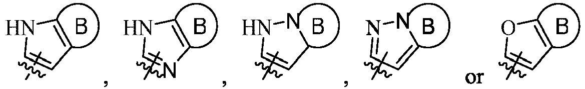 is optionally substituted with one or more R^{a'}; or, R¹ is selected from the group consisting of 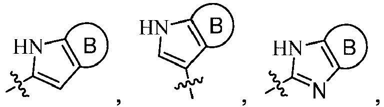 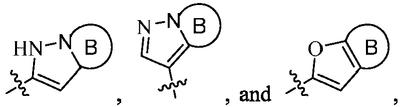 wherein the 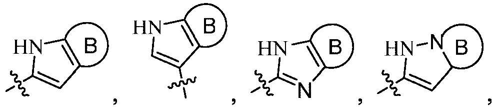 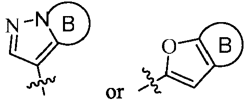 is optionally substituted with one or more R^{a'}; or, R¹ is selected from the group consisting of 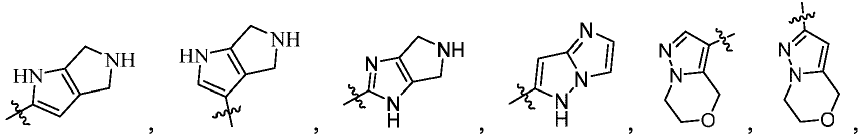 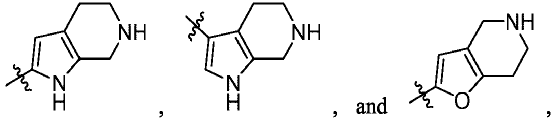 wherein the 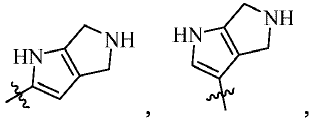 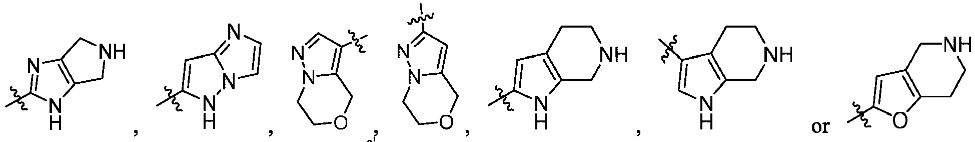 is optionally substituted with one or more R^{a'}.

8. The compound of formula (I) or the pharmaceutically acceptable salt thereof according to any one of claims 1-7, wherein R^{a} and R^{a'} are each independently selected from the group consisting of halogen, 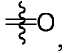 C₁₋₆ alkyl, 3- to 6-membered cycloalkyl, 3- to 8-membered heterocycloalkyl-C₁₋₆ alkyl-, and C₁₋₆ alkyl substituted with 1, 2 or 3 hydroxy; or, R^{a} and R^{a'} are each independently selected from the group consisting of halogen, 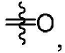 C₁₋₄ alkyl, 3- to 6-membered cycloalkyl, 3- to 6-membered heterocycloalkyl-C₁₋₄ alkyl-, and C₁₋₄ alkyl substituted with 1, 2 or 3 hydroxyl;
or, R^{a} is selected from the group consisting of C₁₋₄ alkyl, 3- to 6-membered cycloalkyl, 3- to 6-membered heterocycloalkyl-C₁₋₄ alkyl-, and C₁₋₄ alkyl substituted with 1, 2 or 3 hydroxy; or, R^{a} is selected from the group consisting of methyl, ethyl, cyclopropyl, 2-hydroxyethyl, and 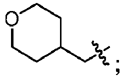
or, R^{a'} is selected from the group consisting of halogen, 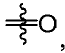 C₁₋₄ alkyl, and 3- to 6-membered cycloalkyl; or,
R^{a'} is selected from the group consisting of fluoro, chloro, 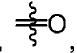 methyl, ethyl, isopropyl, and cyclopropyl; or,
R^{a'} is selected from the group consisting of fluoro, chloro, 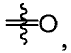 methyl, ethyl, and cyclopropyl.

9. The compound of formula (I) or the pharmaceutically acceptable salt thereof according to any one of claims 1-8, wherein R¹ is selected from the group consisting of 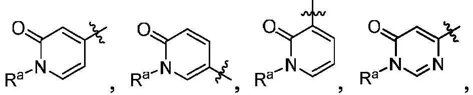 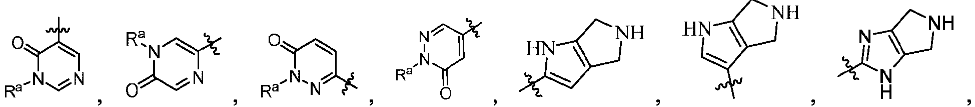 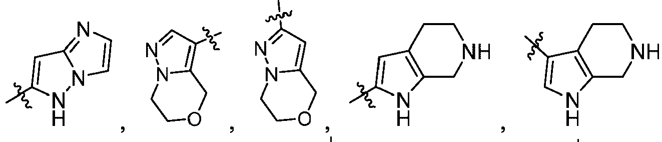 , and 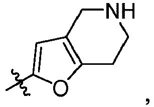 wherein the 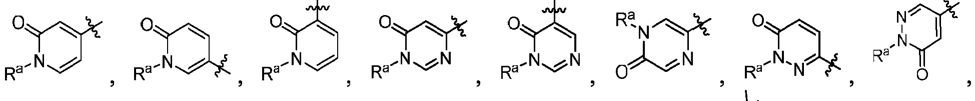 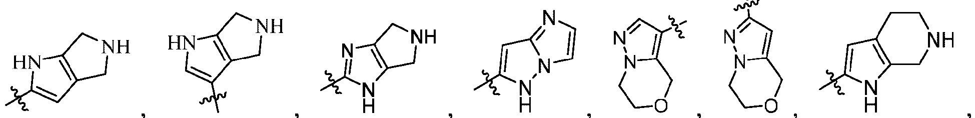 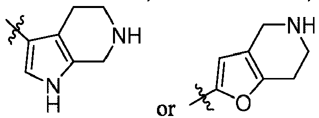 is optionally substituted with one or more R^{a'}; or, R¹ is selected from the group consisting of 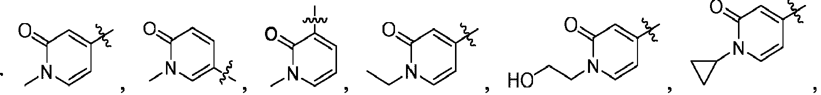 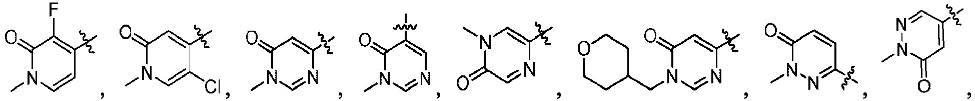 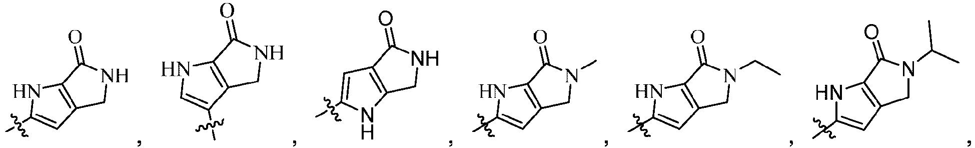 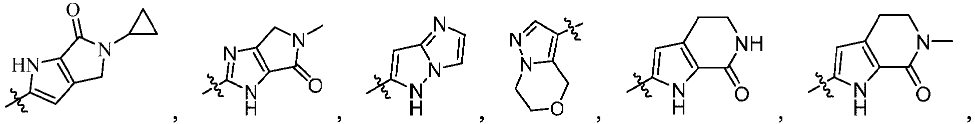 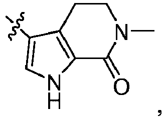 and

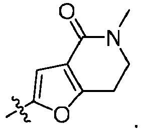

10. The compound of formula (I) or the pharmaceutically acceptable salt thereof according to any one of claims 1-9, wherein R² is selected from the group consisting of hydrogen, amino, C₁₋₄ alkoxy, amino-C₁₋₄ alkyl-, 3- to 10-membered heterocycloalkyl, and 5- to 6-membered heteroaryl, wherein the amino, C₁₋₄ alkoxy, amino-C₁₋₄ alkyl-, 3- to 10-membered heterocycloalkyl, or 5- to 6-membered heteroaryl is optionally substituted with one or more R^{b}; or, R² is selected from the group consisting of hydrogen, amino, C₁₋₄ alkoxy, amino-C₁₋₄ alkyl-, 4- to 6-membered monoheterocycloalkyl, 6- to 9-membered bridged heterocycloalkyl, 7- to 9-membered spiro-heterocycloalkyl, and 5- to 6-membered heteroaryl, wherein the amino, C₁₋₄ alkoxy, amino-C₁₋₄ alkyl-, 4- to 6-membered monoheterocycloalkyl, 6- to 9-membered bridged heterocycloalkyl, 7- to 9-membered spiro-heterocycloalkyl, or 5- to 6-membered heteroaryl is optionally substituted with one or more R^{b}; or, R² is selected from the group consisting of amino, methoxy, ethoxy, aminomethyl, 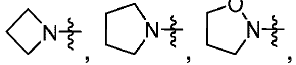 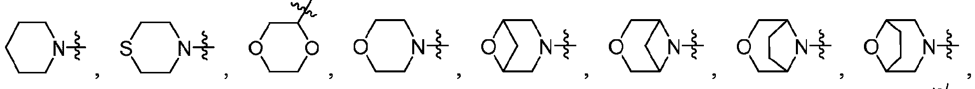 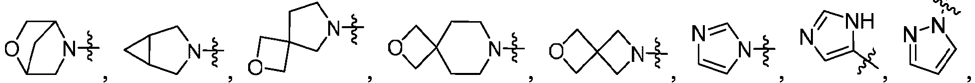 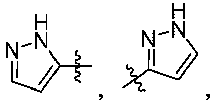 and 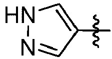 , wherein the amino, methoxy, ethoxy, aminomethyl, 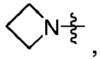 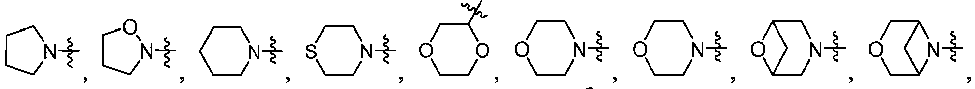 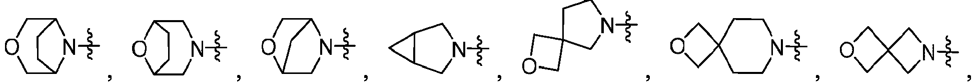 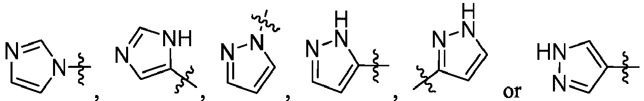 is optionally substituted with 1, 2 or 3 R^{b}; or, R² is selected from the group consisting of amino, methoxy, ethoxy, aminomethyl, pyrrolidinyl, isoxazolidinyl, piperidinyl, morpholinyl, thiomorpholinyl, 1,4-dioxanyl, azetidinyl, 6-oxa-3-azabicyclo[3.1.1]heptanyl, 3-oxa-6-azabicyclo[3.1.1]heptanyl, 3-oxa-8-azabicyclo[3.2.1]octanyl, 8-oxa-3-azabicyclo[3.2.1]octanyl, 2-oxa-5-azabicyclo[2.2.1]heptanyl, 3-azabicyclo [3.1.0]hexanyl, 2-oxa-6-azaspiro[3.4]octanyl, 2-oxa-7-azaspiro[3.5]nonanyl, 2-oxa-6-azaspiro[3.3]heptanyl, pyrazolyl, and imidazolyl, wherein the amino is optionally substituted with 1 or 2 methyl or methoxyethyl, wherein the ethoxy is optionally substituted with 1 methoxy, wherein the aminomethyl is optionally substituted with 1 or 2 methyl or methoxy, wherein the pyrrolidinyl is optionally substituted with 1 or 2 hydroxy, cyano, fluoro, chloro, methoxy, hydroxymethyl or acetylamino, wherein the azetidinyl is optionally substituted with 1 or 2 hydroxy, cyano, fluoro, methyl or hydroxymethyl, and wherein the 2-oxa-6-azaspiro[3.4]octyl is optionally substituted with one 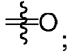 or, R² is selected from the group consisting of methoxy, methoxyethoxy, methylamino, dimethylamino, 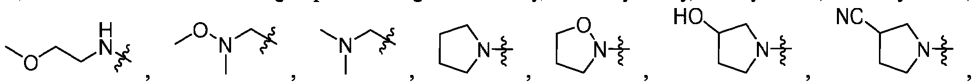 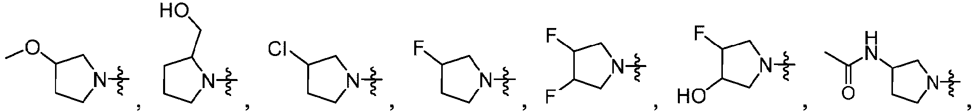 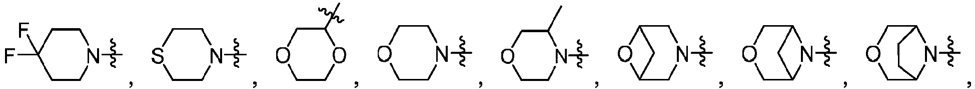 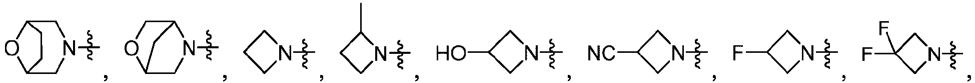 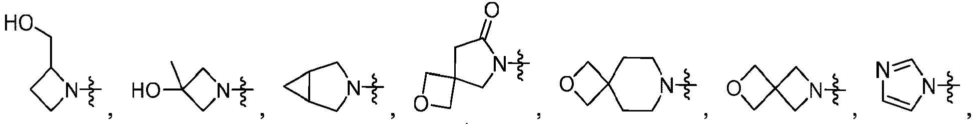 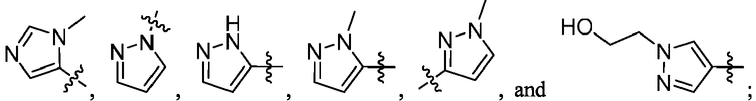 or R² is selected from the group consisting of methoxy, methoxyethoxy, methylamino, dimethylamino, 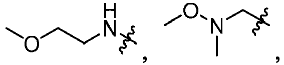 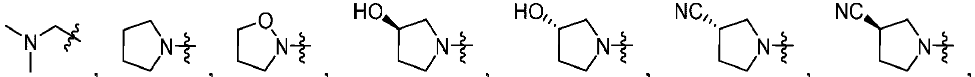 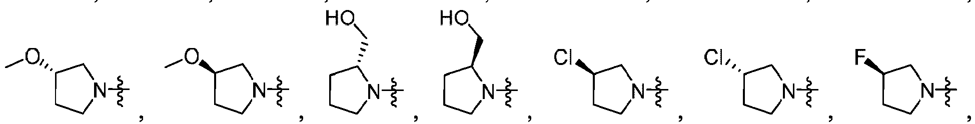 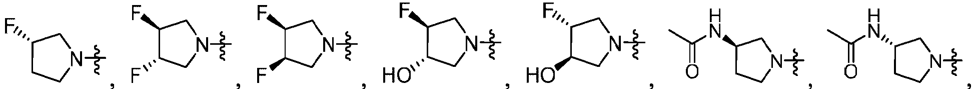 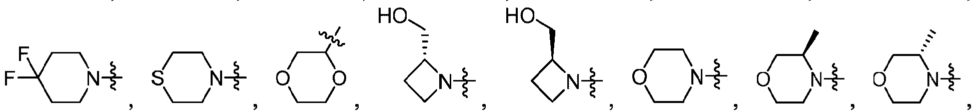 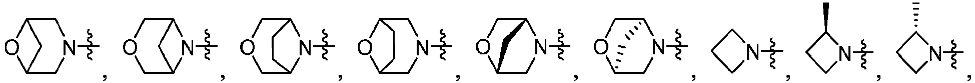 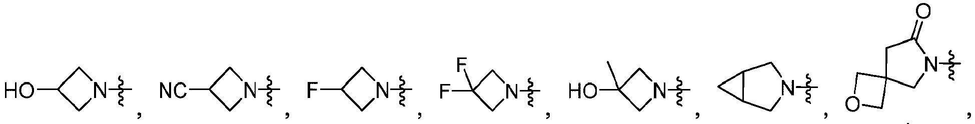 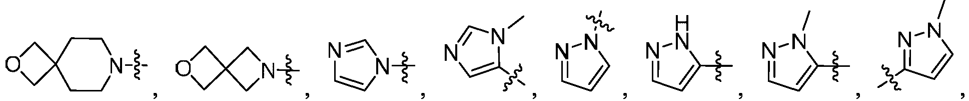 and

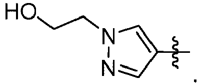

11. The compound of formula (I) or the pharmaceutically acceptable salt thereof according to any one of claims 1-10, wherein R^{b} is selected from the group consisting of hydroxy, cyano, halogen, , C₁₋₄ alkyl, C₁₋₄ alkoxy, C₁₋₄ alkoxy-C₁₋₄ alkyl-, C₁₋₄ alkyl-C(O)-NH-, and C₁₋₄ alkyl substituted with one or more hydroxy or halogen; or, R^{b} is selected from the group consisting of hydroxy, cyano, fluoro, chloro, , methyl, methoxy, hydroxymethyl, methoxyethyl, and acetylamino.

12. The compound of formula (I) or the pharmaceutically acceptable salt thereof according to any one of claims 1-11, wherein the compound of formula (I) is a compound of formula (III-A), a compound of formula (III-B), a compound of formula (IV-A), a compound of formula (V), a compound of formula (VI), a compound of formula (VII), a compound of formula (VIII), a compound of formula (IX), or a compound of formula (X): 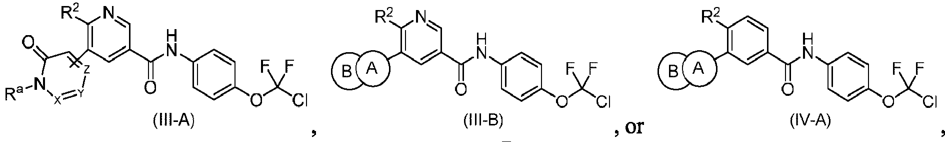 and R² are as defined in claim 1, and 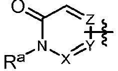 and 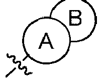 are as defined in claim 7; 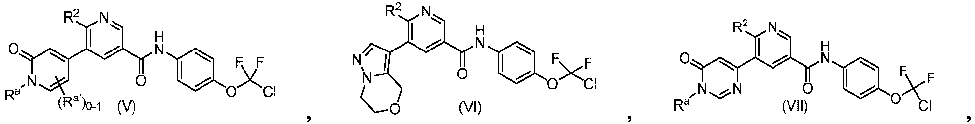 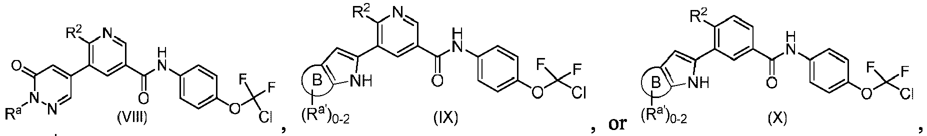 and R², R^{a}, R^{a'}, and the ring B are as defined in claim 1.

13. The compound of formula (I) or the pharmaceutically acceptable salt thereof according to any one of claims 1-12, wherein the compound of formula (I) or the pharmaceutically acceptable salt thereof is the following compound or a pharmaceutically acceptable salt thereof

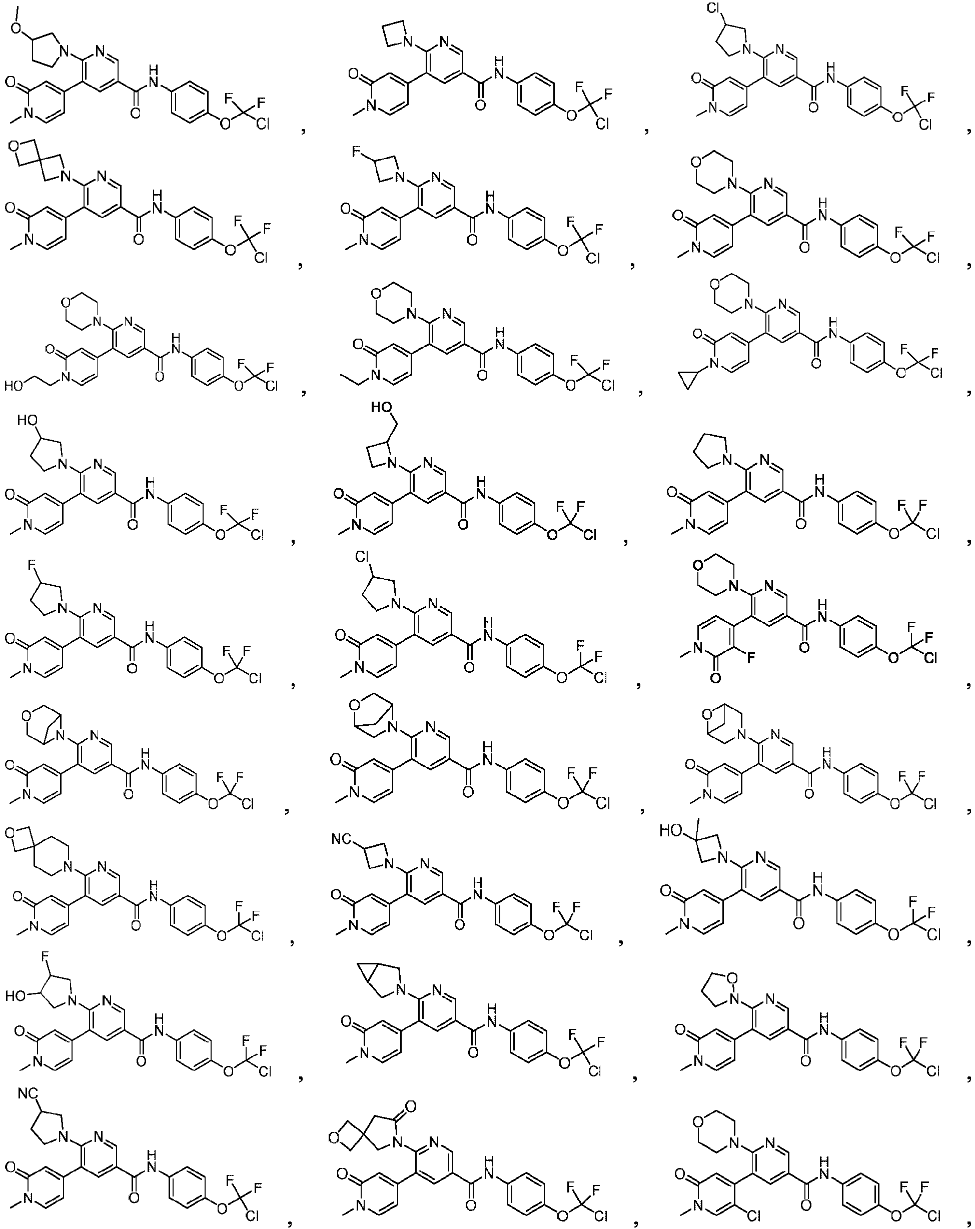

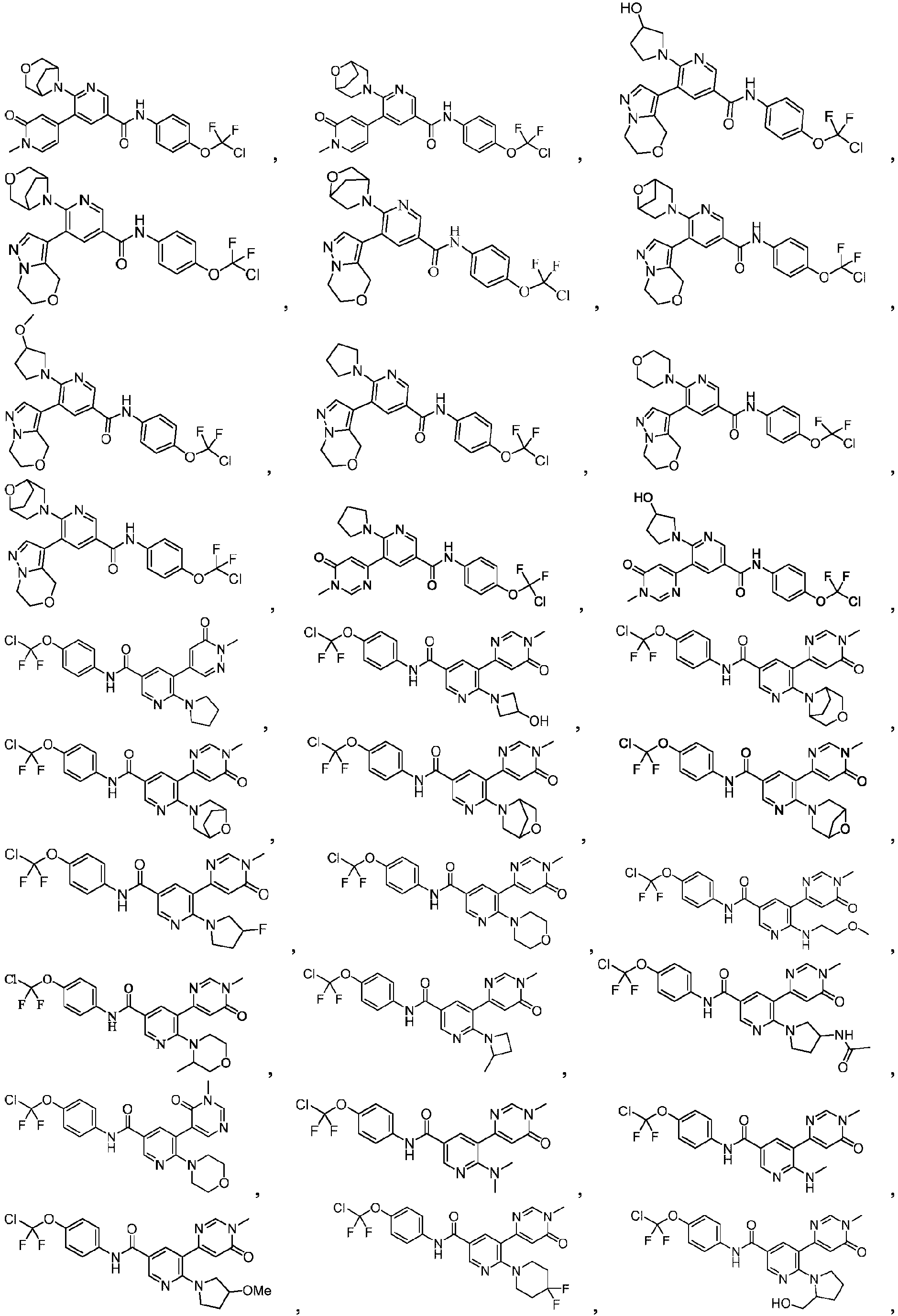

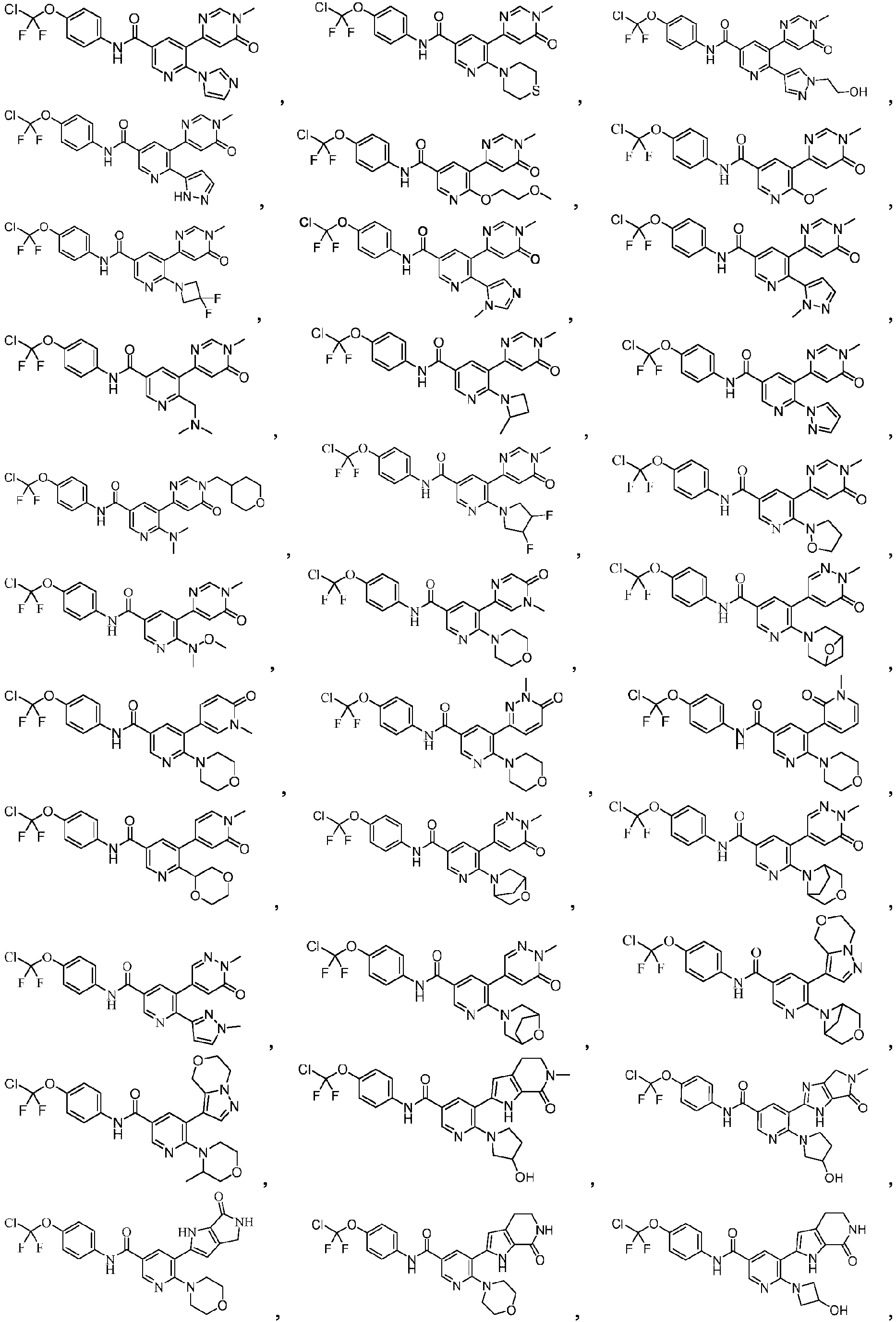

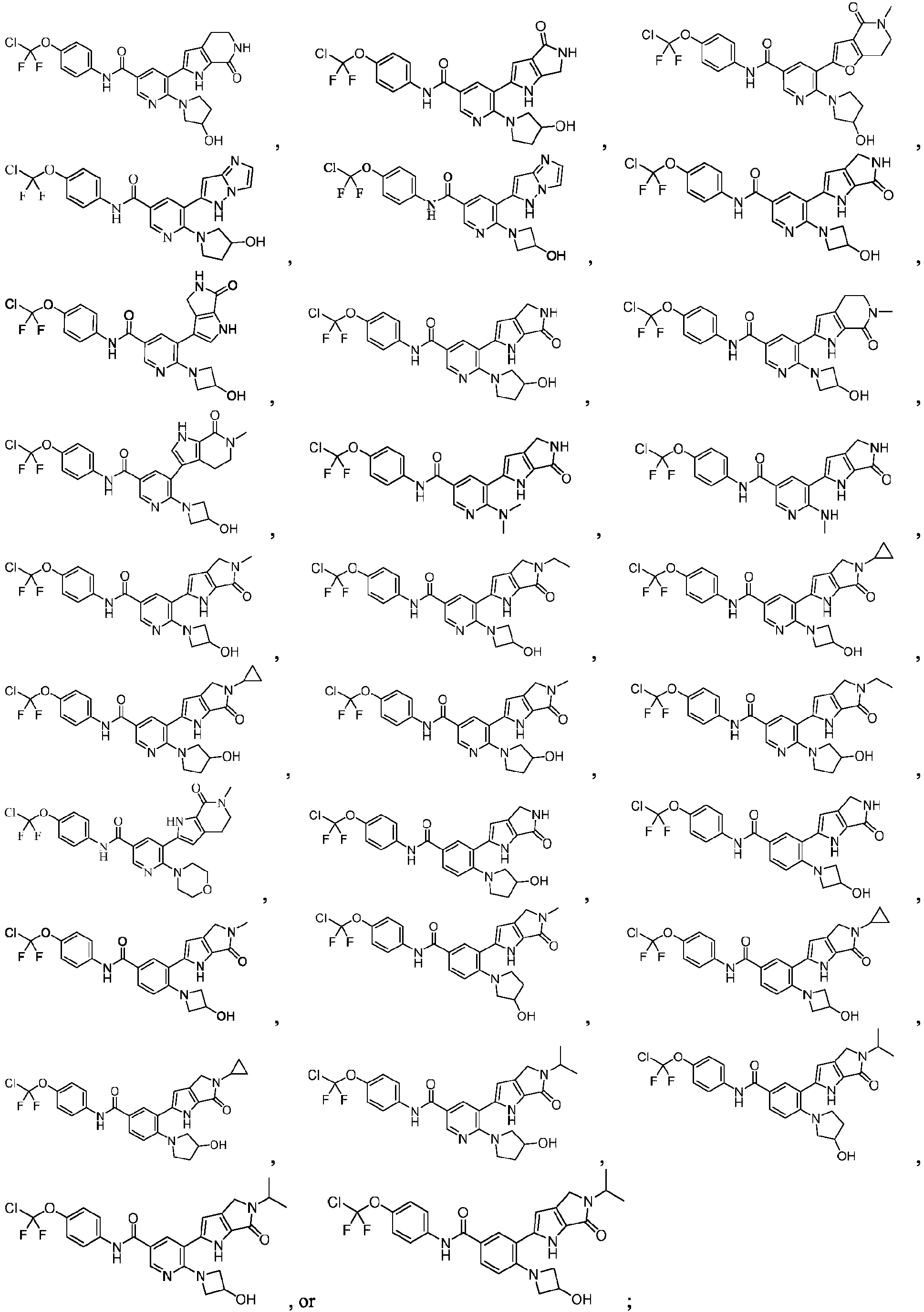

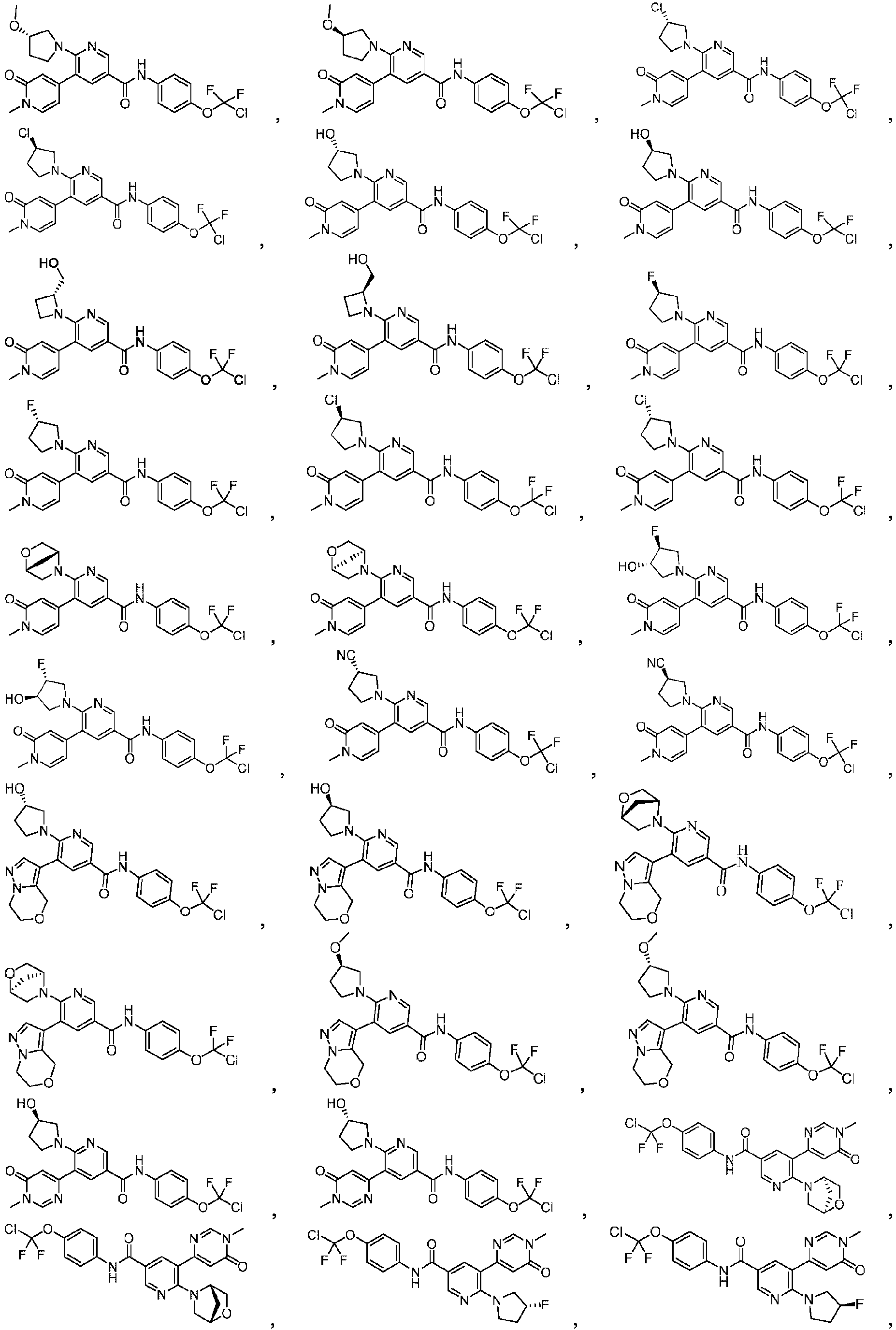

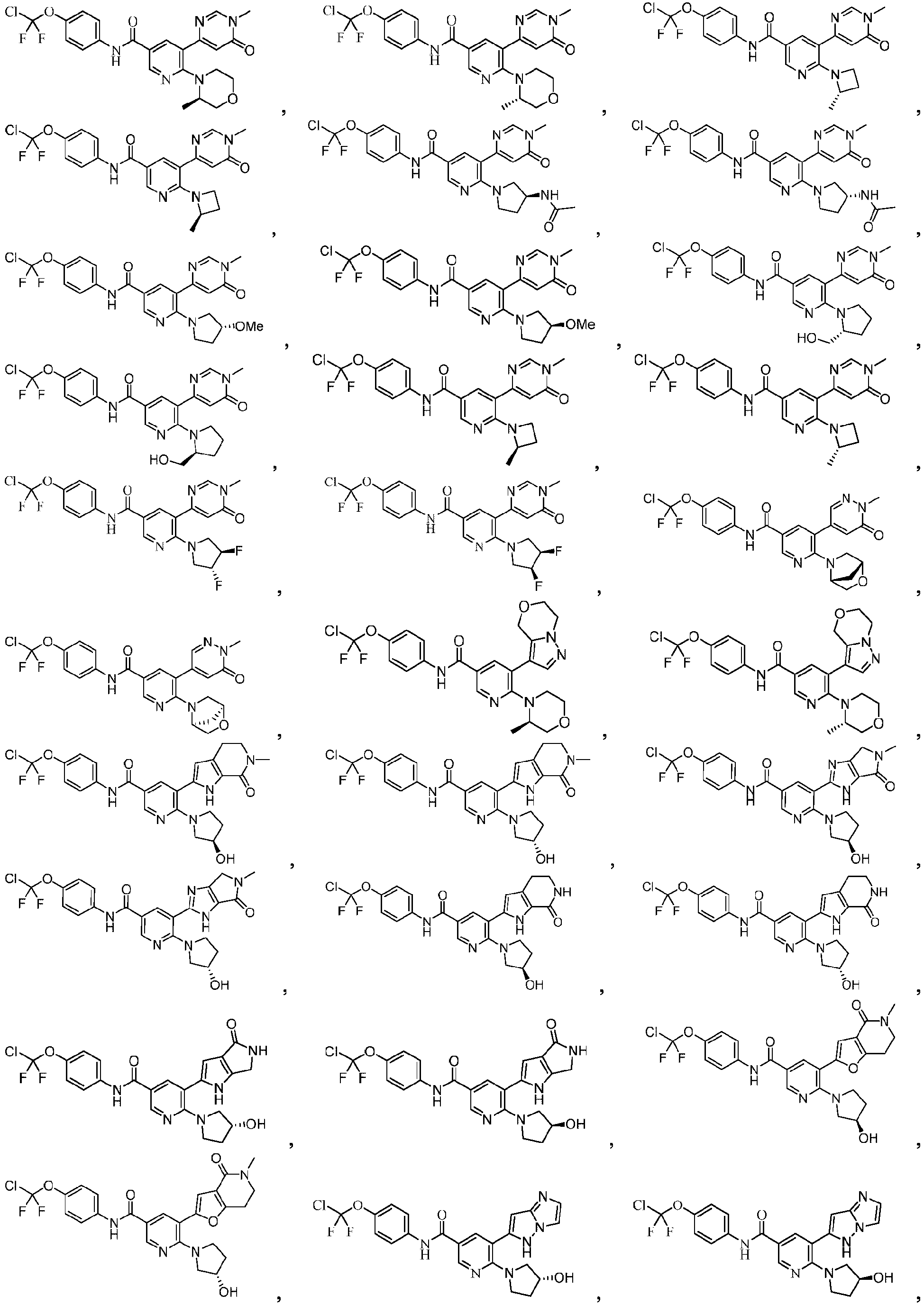

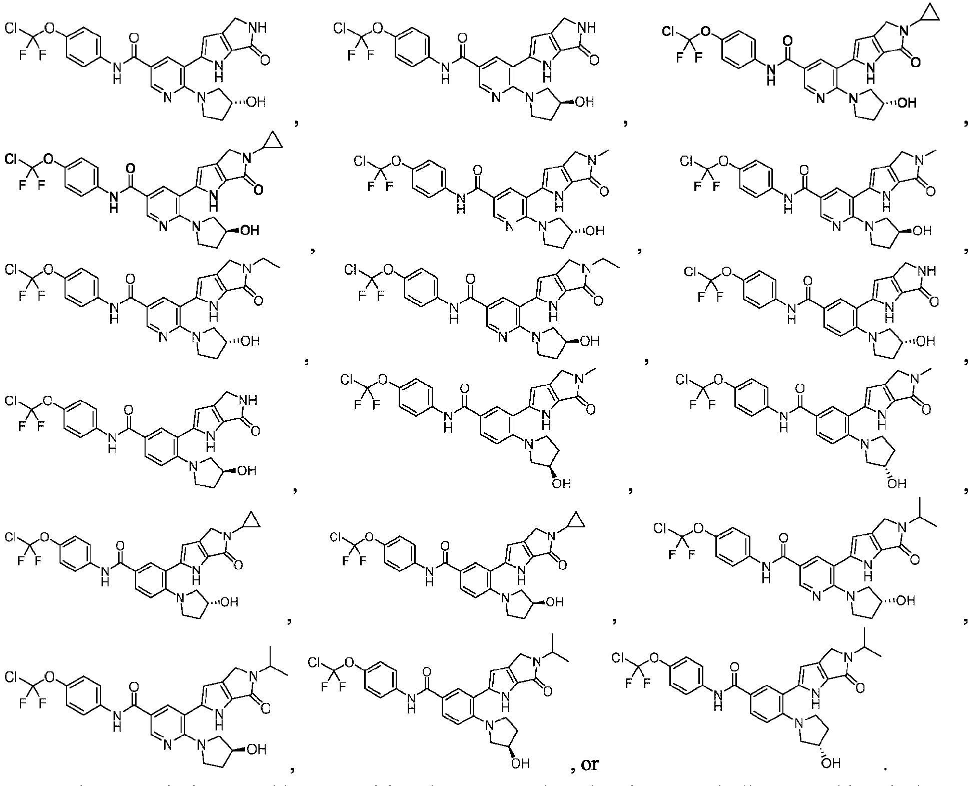

14. A pharmaceutical composition comprising the compound or the pharmaceutically acceptable salt thereof according to any one of claims 1-13.

15. A compound or a pharmaceutically acceptable salt thereof according to any one of claims 1-13, or a pharmaceutical composition according to claim 14, for use in treating and/or preventing a BCR-ABL related disease; wherein preferably, the disease is selected from cancer; or, the disease is selected from chronic myeloid leukemia.
